# EUROPEAN PATENT SPECIFICATION

(11) **EP 0 703 783 B1**
(45) Date of publication and mention of the grant of the patent: **05.05.2010**
(21) Application number: 94910837.7
(22) Date of filing: 04.03.1994
(51) Int. Cl.: C07K 7/06, C07K 14/02, C07K 14/47, A61K 38/17, A61K 38/08

(54) **METHODS OF MAKING IMMUNOGENIC HLA-A2.1 BINDING PEPTIDES**
VERFAHREN ZUR HERSTELLUNG VON IMMUNOGENEN HLA-A2.1-BINDENDEN PEPTIDEN
PROCÉDÉS POUR PRÉPARER DE PEPTIDES IMMUNOGÈNES SE LIANT A HLA-A2.1

(30) Priority: 05.03.1993 US 27146; 04.06.1993 US 73205; 29.11.1993 US 159184
(43) Date of publication of application: 03.04.1996
(73) Proprietor: Epimmune Inc., San Diego, CA 92121 (US)
(72) Inventor: GREY, Howard M., La Jolla, CA 92037 (US); SETTE, Alessandro, La Jolla, CA 92037 (US); SIDNEY, John, La Jolla, CA 92037 (US); KAST, W. Martin, NL-2331 NX Leiden (NL)
(74) Representative: Inspicos A/S
(86) International application number: PCT/US1994/002353
(87) International publication number: WO 1994/020127

(56) References cited:
- WO-A-93/22338
- WO-A-93/24525
- WO-A-94/03205
- WO-A-94/11738
- WO-A-94/26903
- WO-A-95/22561
- NAYERSINA, RAMIN ET AL: "HLA A2 restricted cytotoxic T lymphocyte responses to multiple hepatitis B surface antigen epitopes during hepatitis B virus infection" J. IMMUNOL. (1993), 150(10), 4659-71 CODEN: JOIMA3;ISSN: 0022-1767,1993, XP000609159
- NIJMAN H W: "IDENTIFICATION OF PEPTIDE SEQUENCES THAT POTENTIALLY TRIGGER HLA-A2.1-RESTRICTED CYTOTOXIC T LYMPHOCYTES" EUROPEAN JOURNAL OF IMMUNOLOGY, vol. 23, June 1993, pages 1215-1219, XP000674255
- RUPPERT J ET AL: "PROMINENT ROLE OF SECONDARY ANCHOR RESIDUES IN PEPTIDE BINDING TO HLA-A2.1 MOLECULES" CELL, vol. 74, no. 5, 10 September 1993, pages 929-937, XP000609144
- JOURNAL OF IMMUNOLOGY, Volume 147, No. 11, issued 01 December 1991, SETTE et al., "Random Association Between the Peptide repertoire of A2.1 Class I and Several Different DR Class II Molecules", pages 3893, see pages 3897-3900, Table III.
- SCIENCE, Volume 255, issued 06 March 1992, HENDERSON et al., "HLA-A2.1 Associated Peptides from a Mutant Cell Line: A Second Pathway of Antigen Presentation", pages 1264-1266, see page 1265.
- NATURE, Volume 351, issued 23 May 1991, FALK et al., "Allelespecific Motifs Revealed by Sequencing of Self-Peptides Eluted from MHC Molecules", pages 290-296, see page 293.
- A. LEHNINGER, "Principles of Biochemistry" published 1982 by Worth Publishers, Inc. (N.Y.), pages 100-101, see pages 100-101.
- EUROPEAN JOURNAL OF IMMUNOLOGY, Volume 21, issued June 1991, SAROBE et al., "Induction of Antibodies Against a Peptide Hapten Does Not Require Covalent Linkage Between the Hapten and a Class II Presentable T Helper Peptide", pages 1555-1558, see page 1556.

## Description

The present application is a continuation in part of USSN 08/159,184, which is a continuation in part of USSN 08/073,205, which is a continuation in part of USSN 08/027,146, all of which are incorporated herein by reference.

### BACKGROUND OF THE INVENTION

The present invention relates to compositions and methods for preventing, treating or diagnosing a number of pathological states such as viral diseases and cancers. In particular, it provides methods of making immunogenic peptides capable of binding selected major histocompatibility complex (MHC) molecules and inducing an immune response.

MHC molecules are classified as either Class I or Class II molecules. Class II MHC molecules are expressed primarily on cells involved in initiating and sustaining immune responses, such as T lymphocytes, B lymphocytes, macrophages, etc. Class II MHC molecules are recognized by helper T lymphocytes and induce proliferation of helper T lymphocytes and amplification of the immune response to the particular immunogenic peptide that is displayed. Class I MHC molecules are expressed on almost all nucleated cells and are recognized by cytotoxic T lymphocytes (CTLs), which then destroy the antigen-bearing cells. CTLs are particularly important in tumor rejection and in fighting viral infections.

The CTL recognizes the antigen in the form of a peptide fragment bound to the MHC class I molecules rather than the intact foreign antigen itself. The antigen must normally be endogenously synthesized by the cell, and a portion of the protein antigen is degraded into small peptide fragments in the cytoplasm. Some of these small peptides translocate into a pre-Golgi compartment and interact with class I heavy chains to facilitate proper folding and association with the subunit β2 microglobulin. The peptide-MHC class I complex is then routed to the cell surface for expression and potential recognition by specific CTLs.

Investigations of the crystal structure of the human MAC class I molecule, HLA-A2.1, indicate that a peptide binding groove is created by the folding of the α1 and α2 domains of the class I heavy chain (Bjorkman et al., Nature 329:506 ( 1987). In these investigations, however, the identity of peptides bound to the groove was not determined.

Buus et al., Science 242:1065 (1988) first described a method for acid elution of bound peptides from MHC. Subsequently, Rammensee and his coworkers (Falk et al., Nature 351:290 (1991) have developed an approach to characterize naturally processed peptides bound to class I molecules. Other investigators have successfully achieved direct amino acid sequencing of the more abundant peptides in various HPLC fractions by conventional automated sequencing of peptides eluted from class I molecules of the B type (Jardetzky, et al., Nature 353:326 (1991) and of the A2.1 type by mass spectrometry (Hunt, et al., Science 225:1261 (1992). A review of the characterization of naturally processed peptides in MHC Class I has been presented by Rötzschke and Falk (Rötzschke and Falk, Immunol. Today 12:447 (1991). Nayersina et al, The Journal of Immunology Vol 150, 4659- 4671 (1993) describes the CLT response to several HLA-A1-restricted eptitopes in patients with acute viral hepatitis.

Sette et al., Proc. Natl. Acad. Sci. USA 86:3296 (1989) showed that MHC allele specific motifs could be used to predict MHC binding capacity. Schaeffer et al., Proc. Natl. Acad. Sci. USA 86:4649 (1989) showed that MHC binding was related to immunogenicity. Several authors (De Bruijn et al., Eur. J. Immunol., 21:2963-2970 (1991);'Pamer et al., 991 Nature 353:852-955 (1991)) have provided preliminary evidence that class I binding motifs can be applied to the identification of potential immunogenic peptides in animal models. Class I motifs specific for a number of human alleles of a given class I isotype have yet to be described. It is desirable that the combined frequencies of these different alleles should be high enough to cover a large fraction or perhaps the majority of the human outbred population.

Despite the developments in the art, the prior art has yet to provide a useful human peptide-based vaccine or therapeutic agent based on this work. The present invention provides these and other advantages.

### SUMMARY OF THE INVENTION

The present invention provides methods of making immunogenic peptides having binding motifs for HLA-A2.1 molecules. The immunogenic peptides, which bind to the appropriate MHC allele, are preferably 9 to 10 residues in length and comprise conserved residues at certain positions such as positions 2 and 9. Moreover, the peptides do not comprise negative binding residues as defined herein at other positions such as positions 1, 3, 6 and/or 7 in the case of peptides 9 amino acids in length and positions 1, 3, 4, 5, 7, 8 and/or 9 in the case of peptides 10 amino acids in length. The present invention defines positions within a motif enabling the selection of peptides which will bind efficiently to HLA A2.1.

Epitopes on a number of immunogenic target proteins can be identified using the peptides of the invention. Examples of suitable antigens include prostate cancer specific antigen (PSA), hepatitis B core and surface antigens (HBVc, HBVs) hepatitis C antigens, Epstein-Barr virus antigens; human immunodeficiency type-1 virus (HIV1) and papilloma virus antigens. The peptides are thus useful in pharmaceutical compositions for both in vivo and ex vivo therapeutic and diagnostic applications.

### Definitions

The term "peptide" is used interchangeably with "oligopeptide" in the present specification to designate a series of residues, typically L-amino acids, connected one to the other typically by peptide bonds between the alpha-amino and carbonyl groups of adjacent amino acids. The oligopeptides of the invention are less than about 15 residues in length and usually consist of between about 8 and about 11 residues, preferably 9 or 10 residues.

An "immunogenic peptide" is a peptide which comprises an allele-specific motif such that the peptide will bind an MHC molecule and induce a CTL response. Immunogenic peptides of the invention are capable of binding to an appropriate HLA-A2.1 molecule and inducing a cytotoxic T cell response against the antigen from which the immunogenic peptide is derived.

Immunogenic peptides are conveniently identified using the algorithms of the invention. The algorithms are mathematical procedures that produce a score which enables the selection of immunogenic peptides. Typically one uses the algorithmic score with a "binding threshold" to enable selection of peptides that have a high probability of binding at a certain affinity and will in turn be immunogenic. The algorithm is based upon either the effects on MHC binding of a particular amino acid at a particular position of a peptide or the effects on binding of a particular substitution in a motif containing peptide.

A "conserved residue" is an amino acid which occurs in a significantly higher frequency than would be expected by random distribution at a particular position in a peptide. Typically a conserved residue is one where the MHC structure may provide a contact point with the immunogenic peptide. One to three, preferably two, conserved residues within a peptide of defined length defines a motif for an immunogenic peptide. These residues are typically in close contact with the peptide binding groove, with their side chains buried in specific pockets of the groove itself. Typically, an immunogenic peptide will comprise up to three conserved residues, more usually two conserved residues.

As used herein, "negative binding residues" are amino acids which if present at certain positions (for example, positions 1, 3 and/or 7 of a 9-mer) will result in a peptide being a nonbinder or poor binder and in turn fail to be immunogenic i.e. induce a CTL response.

The term "motif" refers to the pattern of residues in a peptide of defined length, usually about 8 to about 11 amino acids, which is recognized by a particular MHC allele. The peptide motifs are typically different for each human MHC allele and differ in the pattern of the highly conserved residues and negative residues.

The binding motif for an allele can be defined with increasing degrees of precision. In one case, all of the conserved residues are present in the correct positions in a peptide and there are no negative residues in positions 1,3 and/or 7.

The phrases "isolated" or "biologically pure" refer to material which is substantially or essentially free from components which normally accompany it as found in its native state. Thus, the peptides of this invention do not contain materials normally associated with their in situ environment, e.g., MHC I molecules on antigen presenting cells. Even where a protein has been isolated to a homogenous or dominant band, there are trace contaminants in the range of 5-10% of native protein which co-purify with the desired protein. Isolated peptides of this invention do not contain such endogenous co-purified protein.

The term "residue" refers to an amino acid or amino acid mimetic incorporated in an oligopeptide by an amide bond or amide bond mimetic.

### BRIEF DESCRIPTION OF THE DRAWINGS

Fig. 1 is a flow diagram of an HLA-A purification scheme.
Fig. 2 shows a scattergram of the log of relative binding plotted against the "Grouped Ratio" algorithm for 9 mer peptides.
Fig. 3 shows a scattergram of the log of relative binding plotted against the average "Log of Binding" algorithm score for 9 mer peptides.
Figs. 4 and 5 show scattergrams of a set of 10-mer peptides containing preferred residues in positions 2 and 10 as scored by the "Grouped Ratio" and "Log of Binding" algorithms.

### DESCRIPTION OF THE PREFERRED EMBODIMENTS

The present invention relates to the determination of allele-specific peptide motifs for human Class I MHC (sometimes referred to as HLA) allele subtypes, in particular, peptide motifs recognized by HLA-A2.1 alleles. These motifs are then used to define T cell epitopes from any desired antigen, particularly those associated with human viral diseases, cancers or autoiummune diseases, for which the amino acid sequence of the potential antigen or autoantigen targets is known.

Epitopes on a number of potential target proteins can be identified in this manner. Examples of suitable antigens include prostate specific antigen (PSA), hepatitis B core and surface antigens (HBVc, HBVs) hepatitis C antigens, Epstein-Barr virus antigens, melanoma antigens (e.g., MAGE-1), human immunodeficiency virus (HIV) antigens and human papilloma virus (HPV) antigens.

The peptides made by the methods of the invention may also be employed to relieve the symptoms of, treat or prevent the occurrence or reoccurrence of autoimuune diseases. Such diseases include, for example, multiple sclerosis (MS), rheumatoid arthritis (RA), Sjogren syndrome, scleroderma, polymyositis, dermatomyositis, systemic lupus erythematosus, juvenile rheumatoid arthritis, ankylosing spondylitis, myasthenia gravis (MG), bullous pemphigoid (antibodies to basement membrane at dermal-epidermal junction), pemphigus (antibodies to mucopolysaccharide protein complex or intracellular cement substance), glomerulonephritis (antibodies to glomerular basement membrane), Goodpasture's syndrome, autoimmune hemolytic anemia (antibodies to erythrocytes), Hashimoto's disease (antibodies to thyroid), pernicious anemia (antibodies to intrinsic factor), idiopathic thrombocytopenic purpura (antibodies to platelets), Grave's disease, and Addison's disease (antibodies to thyroglobulin), and the like.

The autoantigens associated with a number of these diseases have been identified. For example, in experimentally induced autoimmune diseases, antigens involved in pathogenesis have been characterized: in arthritis in rat and mouse, native type-II collagen is identified in collagen-induced arthritis, and mycobacterial heat shock protein in adjuvant arthritis; thyroglobulin has been identified in experimental allergic thyroiditis (EAT) in mouse; acetyl choline receptor (AChR) in experimental allergic myasthenia gravis (EAMG); and myelin basic protein (MBP) and proteolipid protein (PLP) in experimental allergic encephalomyelitis (EAE) in mouse and rat. In addition, target antigens have been identified in humans: type-II collagen in human rheumatoid arthritis; and acetyl choline receptor in myasthenia gravis.

Peptides comprising the epitopes from these antigens are synthesized and then tested for their ability to bind to the appropriate MHC molecules in assays using, for example, purified class I molecules and radioiodonated peptides and/or cells expressing empty class I molecules by, for instance, immunofluorescent staining and flow microfluorometry, peptide-dependent class I assembly assays, and inhibition of CTL recognition by peptide competition. Those peptides that bind to the class I molecule are further evaluated for their ability to serve as targets for CTLs derived from infected or immunized individuals, as well as for their capacity to induce primary in vitro or in vivo CTL responses that can give rise to CTL populations capable of reacting with virally infected target cells or tumor cells as potential therapeutic agents.

The MHC class I antigens are encoded by the HLA-A, B, and C loci. HLA-A and B antigens are expressed at the cell surface at approximately equal densities, whereas the expression of HLA-C is significantly lower (perhaps as much as 10-fold lower). Each of these loci have a number of alleles. The peptide binding motifs of the invention are relatively specific for each allelic subtype.

For peptide-based vaccines, the peptides of the present invention preferably comprise a motif recognized by an MHC I molecule having a wide distribution in the human population. Since the MHC alleles occur at different frequencies within different ethnic groups and races, the choice of target MHC allele may depend upon the target population. Table 1 shows the frequency of various alleles at the HLA-A locus products among different races. For instance, the majority of the Caucasoid population can be covered by peptides which bind to four HLA-A allele subtypes, specifically HLA-A2.1, A1, A3.2, and A24.1. Similarly, the majority of the Asian population is encompassed with the addition of peptides binding to a fifth allele HLA-A11.2.

**TABLE 1**

| A Allele/Subtype | N(69)* | A(54) | C(502) |
|---|---|---|---|
| A1 | 10.1 (7) | 1.8(1) | 27.4(138) |
| A2.1 | 11.5(8) | 37.0(20) | 39.8(199) |
| A2.2 | 10.1(7) | 0 | 3.3(17) |
| A2.3 | 1.4(1) | 5.5(3) | 0.8(4) |
| A2.4 | - | - | - |
| A2.5 | - | - | - |
| A3.1 | 1.4(1) | 0 | 0.2(0) |
| A3.2 | 5.7(4) | 5.5(3) | 21.5(108) |
| A11.1 | 0 | 5.5(3) | 0 |
| A11.2 | 5.7(4) | 31.4(17) | 8.7(44) |
| A11.3 | 0 | 3.7(2) | 0 |
| A23 | 4.3(3) | - | 3.9(20) |
| A24 | 2.9 (2) | 27.7(15) | 15.3(77) |
| A24.2 | - | - | - |
| A24.3 | - | - | - |
| A25 | 1.4(1) | - | 6.9(35) |
| A26.1 | 4.3(3) | 9.2(5) | 5.9(30) |
| A26.2 | 7.2(5) | - | 1.0(5) |
| A26V | - | 3.7(2) | - |
| A28.1 | 10.1(7) | - | 1.6(8) |
| A28.2 | 1.4(1) | - | 7.5(38) |
| A29.1 | 1.4(1) | - | 1.4(7) |
| A29.2 | 10.1(7) | 1.8(1) | 5.3(27) |
| A30.1 | 8.6(6) | - | 4.9(25) |
| A30.2 | 1.4(1) | - | 0.2(1) |
| A30.3 | 7.2(5) | - | 3.9(20) |
| A31 | 4.3(3) | 7.4(4) | 6.9(35) |
| A32 | 2.8(2) | - | 7.1(36) |
| Aw33.1 | 8.6(6) | - | 2.5(13) |
| Aw33.2 | 2.8(2) | 16.6(9) | 1.2(6) |
| Aw34.1 | 1.4(1) | - | - |
| Aw34.2 | 14.5(10) | - | 0.8(4) |
| Aw36 | 5.9(4) | - | - |

| | | | |
|---|---|---|---|
| Table compiled from B. DuPont, Immunobiology of HLA, Vol. I, Histocompatibility Testing 1987, Springer-Verlag, New York 1989. * N - negroid; A = Asian; C = caucasoid. Numbers in parenthesis represent the number of individuals included in the analysis. | | | |

The nomenclature used to describe peptide compounds follows the conventional practice wherein the amino group is presented to the left (the N-terminus) and the carboxyl group to the right (the C-terzninus) of each amino acid residue. In the formulae representing selected specific embodiments of the present invention, the amino- and carboxyl-terminal groups, although not specifically shown, are in the form they would assume at physiologic pH values, unless otherwise specified. In the amino acid structure formulae, each residue is generally represented by standard three letter or single letter designations. The L-form of an amino acid residue is represented by a capital single letter or a capital first letter of a three-letter symbol, and the D-form for those amino acids having D-forms is represented by a lower case single letter or a lower case three letter symbol. Glycine has no asymmetric carbon atom and is simply referred to as "Gly" or G.

The procedures used to identify peptides of the present invention generally follow the methods disclosed in Falk et al., Nature 351:290 (1991), which is incorporated herein by reference. Briefly, the methods involve large-scale isolation of MHC class I molecules, typically by immunoprecipitation or affinity chromatography, from the appropriate cell or cell line. Examples of other methods for isolation of the desired MHC molecule equally well known to the artisan include ion exchange chromatography, lectin chromatography, size exclusion, high performance ligand chromatography, and a combination of all of the above techniques.

In the typical case, immunoprecipitation is used to isolate the desired allele. A number of protocols can be used, depending upon the specificity of the antibodies used. For example, allele-specific mAb reagents can be used for the affinity purification of the HLA-A, HLA-B₁. and HLA-C molecules. Several mAb reagents for the isolation of HLA-A molecules are available. The monoclonal BB7.2 is suitable for isolating HLA-A2 molecules. Affinity columns prepared with these mAbs using standard techniques are successfully used to purify the respective HLA-A allele products.

In addition to allele-specific mAbs, broadly reactive anti-HLA-A, B, C mAbs, such as W6/32 and H9.12.1, and one anti-HLA-B, C mAb, B1.23.2, could be used in alternative affinity purification protocols as described in the example section below.

The peptides bound to the peptide binding groove of the isolated MHC molecules are eluted typically using acid treatment. Peptides can also be dissociated from class I molecules by a variety of standard denaturing means, such as heat, pH, detergents, salts, chaotropic agents, or a combination thereof.

Peptide fractions are further separated from the MHC molecules by reversed-phase high performance liquid chromatography (HPLC) and sequenced. Peptides can be separated by a variety of other standard means well known to the artisan, including filtration, ultrafiltration, electrophoresis, size chromatography, precipitation with specific antibodies, ion exchange chromatography, isoelectrofocusing, and the like.

Sequencing of the isolated peptides can be performed according to standard techniques such as Edman degradation (Hunkapiller, M.W., et al., Methods Enzymol. 91, 399 [1983]). Other methods suitable for sequencing include mass spectrometry sequencing of individual peptides as previously described (Hunt, et al., Science 225:1261 (1992), which is incorporated herein by reference). Amino acid sequencing of bulk heterogenous peptides (e.g., pooled HPLC fractions) from different class I molecules typically reveals a characteristic sequence motif for each class I allele.

Definition of motifs specific for different class I alleles allows the identification of potential peptide epitopes from an antigenic protein whose amino acid sequence is known. Typically, identification of potential peptide epitopes is initially carried out using a computer to scan the amino acid sequence of a desired antigen for the presence of motifs. The epitopic sequences are then synthesized. The capacity to bind MHC Class molecules is measured in a variety of different ways. One means is a Class I molecule binding assay as described in Example 4, below. Other alternatives described in the literature include inhibition of antigen presentation (Sette, et al., J. Immunol. 141:3893 (1991), in vitro assembly assays (Townsend, et al., Cell 62:285 (1990), and FACS based assays using mutated ells, such as RMA.S (Melief, et al., Eur. J. Immunol. 21:2963 (1991)).

Next, peptides that test positive in the MHC class I binding assay are assayed for the ability of the peptides to induce specific CTL responses in vitro. For instance, Antigen-presenting cells that have been incubated with a peptide can be assayed for the ability to induce CTL responses in responder cell populations. Antigen-presenting cells can be normal cells such as peripheral blood mononuclear cells or dendritic cells (Inaba, et al., J. Exp. Med. 166:182 (1987); Boog, Eur. J. Immunol. 18:219 [1988]).

Alternatively, mutant mammalian cell lines that are deficient in their ability to load class I molecules with internally processed peptides, such as the mouse cell lines RMA-S (Kärre, et al.. Nature, 319:675 (1986); Ljunggren, et al., Eur. J. Immunol. 21:2963-2970 (1991)), and the human somatic T cell hybrid, T-2 (Cerundolo, et al., Nature 345:449-452 (1990)) and which have been transfected with the appropriate human class I genes are conveniently used, when peptide is added to them, to test for the capacity of the peptide to induce in vitro primary CTL responses. Other eukaryotic cell lines which could be used include various insect cell lines such as mosquito larvae (ATCC cell lines CCL 125, 126, 1660, 1591, 6585, 6586), silkworm (ATTC CRL 8851), armyworm (ATCC CRL 1711), moth (ATCC CCL 80) and Drosophila cell lines such as a Schneider cell line (see Schneider J. Embryol. Exp. Morphol. 27:353-365 [1927]).

Peripheral blood lymphocytes are conveniently isolated following simple venipuncture or leukapheresis of normal donors or patients and used as the responder cell sources of CTL precursors. In one embodiment, the appropriate antigen-presenting cells are incubated with 10-100 µM of peptide in serum-free media for 4 hours under appropriate culture conditions. The peptide-loaded antigen-presenting cells are then incubated with the responder cell populations in vitro for 7 to 10 days under optimized culture conditions. Positive CTL activation can be determined by assaying the cultures for the presence of CTLs that kill radiolabeled target cells, both specific peptide-pulsed targets as well as target cells expressing endogenously processed form of the relevant virus or tumor antigen from which the peptide sequence was derived.

Specificity and MHC restriction of the CTL is determined by testing against different peptide target cells expressing appropriate or inappropriate human MHC class I. The peptides that test positive in the MHC binding assays and give rise to specific CTL responses are referred to herein as immunogenic peptides.

The immunogenic peptides can be prepared synthetically, or by recombinant DNA technology or from natural sources such as whole viruses or tumors. Although the peptide will preferably be substantially free of other naturally occurring host cell proteins and fragments thereof, in some embodiments the peptides can be synthetically conjugated to native fragments or particles.

The polypeptides or peptides can be a variety of lengths, either in their neutral (uncharged) forms or in forms which are salts, and either free of modifications such as glycosylation, side chain oxidation, or phosphorylation or containing these modifications, subject to the condition that the modification not destroy the biological activity of the polypeptides as herein described.

Desirably, the peptide will be as small as possible while still maintaining substantially all of the biological activity of the large peptide. When possible, it may be desirable to optimize peptides of the invention to a length of about 8 to about 10 amino acid residues, commensurate in size with endogenously processed viral peptides or tumor cell peptides that are bound to MHC class I molecules on the cell surface.

Peptides having the desired activity may be modified as necessary to provide certain desired attributes, e.g., improved pharmacological characteristics, while increasing or at least retaining substantially all of the biological activity of the unmodified peptide to bind the desired MHC molecule and activate the appropriate T cell. For instance, the peptides may be subject to various changes, such as substitutions, either conservative or non-conservative, where such changes might provide for certain advantages in their use, such as improved MHC binding. By conservative substitutions is meant replacing an amino acid residue with another which is biologically and/or chemically similar, e.g., one hydrophobic residue for another, or one polar residue for another. The substitutions include combinations such as Gly, Ala; Val, Ile, Leu, Met; Asp, Glu; Asn, Gln; Ser, Thr; Lys, Arg; and Phe, Tyr. The effect of single amino acid substitutions may also be probed using D-amino acids. Such modifications may be made using well known peptide synthesis procedures, as described in e.g., Merrifield, Science 232:341-347 (1986), Barany and Merrifield, The Peptides, Gross and Meienhofer, eds. (N.Y., Academic Press), pp. 1-284 (1979); and Stewart and Young, Solid Phase Peptide Synthesis, (Rockford, I11., Pierce), 2d Ed. (1984), incorporated by reference herein.

The peptides can also be modified by extending or decreasing the compound's amino acid sequence, e.g., by the addition or deletion of amino acids. The peptides or analogs of the invention can also be modified by altering the order or composition of certain residues, it being readily appreciated that certain amino acid residues essential for biological activity, e.g., those at critical contact sites or conserved residues, may generally not be altered without an adverse effect on biological activity. The non-critical amino acids need not be limited to those naturally occurring in proteins, such as L-α-amino acids, or their D-isomers, but may include non-natural amino acids as well, such as β-γ-δ-amino acids, as well as many derivatives of L-α-amino acids.

Typically, a series of peptides with single amino acid substitutions are employed to determine the effect of electrostatic charge, hydrophobicity, etc. on binding. For instance, a series of positively charged (e.g., Lys or Arg) or negatively charged (e.g., Glu) amino acid substitutions are made along the length of the peptide revealing different patterns of sensitivity towards various MHC molecules and T cell receptors. In addition, multiple substitutions using small, relatively neutral moieties such as Ala, Gly, Pro, or similar residues may be employed. The substitutions may be homo-oligomers or hetero-oligomers. The number and types of residues which are substituted or added depend on the spacing necessary between essential contact points and certain functional attributes which are sought (e.g., hydrophobicity versus hydrophilicity). Increased binding affinity for an MHC molecule or T cell receptor may also be achieved by such substitutions, compared to the affinity of the parent peptide. In any event, such substitutions should employ amino acid residues or other molecular fragments chosen to avoid, for example, steric and charge interference which might disrupt binding.

Amino acid substitutions are typically of single residues. Substitutions, deletions, insertions or any combination thereof may be combined to arrive at a final peptide. Substitutional variants are those in which at least one residue of a peptide has been removed and a different residue inserted in its place. Such substitutions generally are made in accordance with the following Table 2 when it is desired to finely modulate the characteristics of the peptide.

**TABLE 2**

| Original Residue | Exemplary Substitution |
|---|---|
| Ala | Ser |
| Arg | Lys, His |
| Asn | Gln |
| Asp | Glu |
| Cys | Ser |
| Gln | Asn |
| Glu | Asp |
| Gly | Pro |
| His | Lys; Arg |
| Ile | Leu; Val |
| Leu | Ile; Val |
| Lys | Arg; His |
| Met | Leu; Ile |
| Phe | Tyr; Trp |
| Ser | Thr |
| Thr | Ser |
| Trp | Tyr; Phe |
| Tyr | Trp; Phe |
| Val | Ile; Leu |

Substantial changes in function (e.g., affinity for MHC molecules or T cell receptors) are made by selecting substitutions that are less conservative than those in Table 2, i.e., selecting residues that differ more significantly in their effect on maintaining (a) the structure of the peptide backbone in the area of the substitution, for example as a sheet or helical conformation, (b) the charge or hydrophobicity of the molecule at the target site or (c) the bulk of the side chain. The substitutions which in general are expected to produce the greatest changes in peptide properties will be those in which (a) hydrophilic residue, e.g. seryl, is substituted for (or by) a hydrophobic residue, e.g. leucyl, isoleucyl, phenylalanyl, valyl or alanyl; (b) a residue having an electropositive side chain, e.g., lysl, arginyl, or histidyl, is substituted for (or by) an electronegative residue, e.g. glutamyl or aspartyl; or (c) a residue having a bulky side chain, e.g. phenylalanine, is substituted for (or by) one not having a side chain, e.g., glycine.

The peptides may also comprise isosteres of two or more residues in the immunogenic peptide. An isostere as defined here is a sequence of two or more residues that can be substituted for a second sequence because the steric conformation of the first sequence fits a binding site specific for the second sequence. The term specifically includes peptide backbone modifications well known to those skilled in the art. Such modifications include modifications of the amide nitrogen, the α-carbon, amide carbonyl, complete replacement of the amide bond, extensions, deletions or backbone crosslinks. See, generally. Spatola, Chemistry and Biochemistry of Amino Acids, peptides and Proteins, Vol. VII (Weinstein ed., 1983).

Modifications of peptides with various amino acid mimetics or unnatural amino acids are particularly useful in increasing the stability of the peptide in vivo. Stability can be assayed in a number of ways. For instance, peptidases and various biological media, such as human plasma and serum, have been used to test stability. See, e.g., Verhoef et al., Eur. J. Drug Metab. Pharmacokin. 11:291-302 (1986). Half life of the peptides of the present invention is conveniently determined using a 25% human serum (v/v) assay. The protocol is generally as follows. Pooled human serum (Type AB, non-heat inactivated) is delipidated by centrifugation before use. The serum is then diluted to 25% with RPMI tissue culture media and used to test peptide stability. At predetermined time intervals a small amount of reaction solution is removed and added to either 6% aqueous trichloracetic acid or ethanol. The cloudy reaction sample is cooled (4°C) for 15 minutes and then spun to pellet the precipitated serum proteins. The presence of the peptides is then determined by reversed-phase HPLC using stability-specific chromatography conditions.

The peptides made by the methods of the present invention or analogs thereof which have CTL stimulating activity may be modified to provide desired attributes other than improved serum half life. For instance, the ability of the peptides to induce CTL activity can be enhanced by linkage to a sequence which contains at least one epitope that is capable of inducing a T helper cell response.

In some embodiments, the T helper peptide is one that is recognized by T helper cells in the majority of the population. This can be accomplished by selecting amino acid sequences that bind to many, most, or all of the MHC class II molecules. These are known as "loosely MHC-restricted" T helper sequences. Examples of amino acid sequences that are loosely MHC-restricted include sequences from antigens such as Tetanus toxin at positions 830-843 (QYIKANSKFIGITE), *Plasmodium falciparum* CS protein at positions 378-398 (DIEKKIAKMERASSVFNWNS), and Streptococcus 18kD protein at positions 1-16 (YGAVDSILGGVATYGAA).

Alternatively, it is possible to prepare synthetic peptides capable of stimulating T helper lymphocytes, in a loosely MHC-restricted fashion, using amino acid sequences not found in nature. These synthetic compounds called Pan-DR-binding epitope (PADRE) are designed on the basis of their binding activity to most, HLA-DR (human MHC class II) molecules (see, copending application USSN 08/121,101).

Particularly preferred immunogenic peptides/T helper conjugates are linked by a spacer molecule. The spacer is typically comprised of relatively small, neutral molecules, such as amino acids or amino acid mimetics, which are substantially uncharged under physiological conditions. The spacers are typically selected from, e.g., Ala, Gly, or other neutral spacers of nonpolar amino acids or neutral polar amino acids. It will be understood that the optionally present spacer need not comprise the same residues and thus may be a hetero- or homo-oligomer. When present, the spacer will usually be at least one or two residues, more usually three to six residues. Alternatively, the CTL peptide may be linked to the T helper peptide without a spacer.

The immunogenic peptide may be linked to the T helper peptide either directly or via a spacer either at the amino or carboxy terminus of the CTL peptide. The amino terminus of either the immunogenic peptide or the T helper peptide may be acylated. Exemplary T helper peptides include tetanus toxoid 830-843, influenza 307-319, malaria circumsporozoite 382-398 and 378-389.

In some embodiments it,may be desirable to include in the pharmaceutical compositions related to the methods of making immunogenic peptide of the invention at least one component which primes CTL. Lipids have been identified as agents capable of priming CTL in vivo against viral antigens. For example, palmitic acid residues can be attached to the alpha and epsilon amino groups of a Lys residue and then linked, e.g., via one or more linking residues such as Gly, Gly-Gly-, Ser, Ser-Ser, or the like, to an immunogenic peptide. The lipidated peptide can then be injected directly in a micellar form, incorporated into a liposome or emulsified in an adjuvant, e.g., incomplete Freund's adjuvant. In a preferred embodiment a particularly effective immunogen comprises palmitic acid attached to alpha and epsilon amino groups of Lys, which is attached via linkage, e.g., Ser-Ser, to the amino terminus of the immunogenic peptide.

As another example of lipid priming of CTL responses, E. coli lipoproteins, such as tripalmitoyl-S-glycerylcysteinlyseryl-serine (P₃CSS) can be used to prime virus specific CTL when covalently attached to an appropriate peptide. See, Deres et al., Nature 342:561-564 (1989), incorporated herein by reference. Peptides of the invention can be coupled to P₃CSS, for example, and the lipopeptide administered to an individual to specifically prime a CTL response to the target antigen. Further, as the induction of neutralizing antibodies can also be primed with P₃CSS conjugated to a peptide which displays an appropriate epitope, the two compositions can be combined to more effectively elicit both humoral and cell-mediated responses to infection.

In addition, additional amino acids can be added to the termini of a peptide to provide for ease of linking peptides one to another, for coupling to a carrier support, or larger peptide, for modifying the physical or chemical properties of the peptide or oligopeptide, or the like. Amino acids such as tyrosine, cysteine, lysine, glutamic or aspartic acid, or the like, can be introduced at the C- or N-terminus of the peptide or oligopeptide. Modification at the C terminus in some cases may alter binding characteristics of the peptide. In addition, the peptide or oligopeptide sequences can differ from the natural sequence by being modified by terminal-NH₂ acylation, e.g., by alkanoyl (C₁-C₂₀) or thioglycolyl acetylation, terminal-carboxyl amidation, e.g., ammonia, methylamine, etc. In some instances these modifications may provide sites for linking to a support or other molecule.

The peptides made by the methods of the invention can be prepared in a wide variety of ways. Because of their relatively short size, the peptides can be synthesized in solution or on a solid support in accordance with conventional techniques. Various automatic synthesizers are commercially available and can be used in accordance with known protocols. See, for example, Stewart and Young, Solid Phase Peptide Synthesis, 2d. ed., Pierce Chemical Co. (1984), supra.

Alternatively, recombinant DNA technology may be employed wherein a nucleotide sequence which encodes an immunogenic peptide of interest is inserted into an expression vector, transformed or transfected into an appropriate host cell and cultivated under conditions suitable for expression. These procedures are generally known in the art, as described generally in Sambrook et al., Molecular Cloning, A Laboratory Manual, Cold Spring Harbor Press, Cold Spring Harbor, New York (1982), which is incorporated herein by reference. Thus, fusion proteins which comprise one or more peptide sequences of the invention can be used to present the appropriate T cell epitope.

As the coding sequence for peptides of the length contemplated herein can be synthesized by chemical techniques, for example, the phosphotriester method of Matteucci et al., J. Am. Chem. Soc. 103:3185 (1981), modification can be made simply by substituting the appropriate base(s) for those encoding the native peptide sequence. The coding sequence can then be provided with appropriate linkers and ligated into expression vectors commonly available in the art, and the vectors used to transform suitable hosts to produce the desired fusion protein. A number of such vectors and suitable host systems are now available. For expression of the fusion proteins, the coding sequence will be provided with operably linked start and stop codons, promoter and terminator regions and usually a replication system to provide an expression vector for expression in the desired cellular host. For example, promoter sequences compatible with bacterial hosts are provided in plasmids containing convenient restriction sites for insertion of the desired coding sequence. The resulting expression vectors are transformed into suitable bacterial hosts. Of course, yeast or mammalian cell hosts may also be used, employing suitable vectors and control sequences.

The peptides made by the methods of the present invention and pharmaceutical and vaccine compositions thereof are useful for administration to mammals, particularly humans, to treat and/or prevent viral infection and cancer. Examples of diseases which can be treated using the immunogenic peptides of the invention include prostate cancer, hepatitis B, hepatitis C, AIDS, renal carcinoma, cervical carcinoma, lymphoma, CMV and condlyloma acuminatum.

For pharmaceutical compositions, the immunogenic peptides made by the methods of the invention are administered to an individual already suffering from cancer or infected with the virus of interest. Those in the incubation phase or the acute phase of infection can be treated with the immunogenic peptides separately or in conjunction with other treatments, as appropriate. In therapeutic applications, compositions are administered to a patient in an amount sufficient to elicit an effective CTL response to the virus or tumor antigen and to cure or at least partially arrest symptoms and/or complications. An amount adequate to accomplish this is defined as "therapeutically effective dose." Amounts effective for this use will depend on, e.g., the peptide composition, the manner of administration, the stage and severity of the disease being treated, the weight and general state of health of the patient, and the judgment of the prescribing physician, but generally range for the initial immunization (that is for therapeutic or prophylactic administration) from about 1.0 µg to about 5000 µg of peptide for a 70 kg patient, followed by boosting dosages of from about 1.0 µg to about 1000 µg of peptide pursuant to a boosting regimen over weeks to months depending upon the patient's response and condition by measuring specific CTL activity in the patient's blood. It must be kept in mind that the peptides and compositions of the present invention may generally be employed in serious disease states, that is, life-threatening or potentially life threatening situations. In such cases, in view of the minimization of extraneous substances and the relative nontoxic nature of the peptides, it is possible and may be felt desirable by the treating physician to administer substantial excesses of these peptide compositions.

For therapeutic use, administration should begin at the first sign of viral infection or the detection or surgical removal of tumors or shortly after diagnosis in the case of acute infection. This is followed by boosting doses until at least symptoms are substantially abated and for a period thereafter. In chronic infection, loading doses followed by boosting doses may be required.

Treatment of an infected individual with the compositions of the invention may hasten resolution of the infection in acutely infected individuals. For those individuals susceptible (or predisposed) to developing chronic infection the compositions are particularly useful in methods for preventing the evolution from acute to chronic infection. Where the susceptible individuals are identified prior to or during infection, for instance, as described herein, the composition can be targeted to them, minimizing need for administration to a larger population.

The peptide compositions can also be used for the treatment of chronic infection and to stimulate the immune system to eliminate virus-infected cells in carriers. It is important to provide an amount of immuno-potentiating peptide in a formulation and mode of administration sufficient to effectively stimulate a cytotoxic T cell response. Thus, for treatment of chronic infection, a representative dose is in the range of about 1.0 µg to about 5000 µg, preferably about 5 µg to 1000 µg for a 70 kg patient per dose. Immunizing doses followed by boosting doses at established intervals, e.g., from one to four weeks, may be required, possibly for a prolonged period of time to effectively immunize an individual. In the case of chronic infection, administration should continue until at least clinical symptoms or laboratory tests indicate that the viral infection has been eliminated or substantially abated and for a period thereafter.

The pharmaceutical compositions for therapeutic treatment are intended for parenteral, topical, oral or local administration. Preferably, the pharmaceutical compositions are administered parenterally, e.g., intravenously, subcutaneously, intradermally, or intramuscularly. Thus, the invention provides compositions for parenteral administration which comprise a solution of the immunogenic peptides dissolved or suspended in an acceptable carrier, preferably an aqueous carrier. A variety of aqueous carriers may be used, e.g., water, buffered water, 0.8% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

The concentration of CTL stimulatory peptides of the invention in the pharmaceutical formulations can vary widely, i.e., from less than about 0.1%, usually at or at least about 2% to as much as 20% to 50% or more by weight, and will be selected primarily by fluid volumes, viscosities, etc., in accordance with the particular mode of administration selected.

The peptides made by the methods of the invention may also be administered via liposomes, which serve to target the peptides to a particular tissue, such as lymphoid tissue, or targeted selectively to infected cells, as well as increase the half-life of the peptide composition. Liposomes include emulsions, foams, micelles, insoluble monolayers, liquid crystals, phospholipid dispersions, lamellar layers and the like. In these preparations the peptide to be delivered is incorporated as part of a liposome, alone or in conjunction with a molecule which binds to, e.g., a receptor prevalent among lymphoid cells, such as monoclonal antibodies which bind to the CD45 antigen, or with other therapeutic or immunogenic compositions. Thus, liposomes either filled or decorated with a desired peptide of the invention can be directed to the site of lymphoid cells, where the liposomes then deliver the selected therapeutic/immunogenic peptide compositions. Liposomes for use in the invention are formed from standard vesicle-forming lipids, which generally include neutral and negatively charged phospholipids and a sterol, such as cholesterol. The selection of lipids is generally guided by consideration of, e.g., liposome size, acid lability and stability of the liposomes in the blood stream. A variety of methods are available for preparing liposomes, as described in, e.g., Szoka et al., Ann. Rev. Biophys. Bioeng. 9:467 (1980), U.S. Patent Nos. 4,235,871, 4,501,728, 4,837,028, and 5,019,369, incorporated herein by reference.

For targeting to the immune cells, a ligand to be incorporated into the liposome can include, e.g., antibodies or fragments thereof specific for cell surface determinants of the desired immune system cells. A liposome suspension containing a peptide may be administered intravenously, locally, topically, etc. in a dose which varies according to, inter alia, the manner of administration, the peptide being delivered, and the stage of the disease being treated.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more peptides of the invention, and more preferably at a concentration of 25%-75%.

For aerosol administration, the immunogenic peptides are preferably supplied in finely divided form along with a surfactant and propellant. Typical percentages of peptides are 0.01%-20% by weight, preferably 1%-10%. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and.oleic acids with an aliphatic polyhydric alcohol or its cyclic anhydride. Mixed esters, such as mixed or natural glycerides may be employed. The surfactant may constitute 0.1%-20% by weight of the composition, preferably 0.25-5%. The balance of the composition is ordinarily propellant. A carrier can also be included, as desired, as with, e.g., lecithin for intranasal delivery.

Another aspect is directed to vaccines which contain as an active ingredient an immunogenically effective amount of an immunogenic peptide made by the methods as described herein. The peptide(s) may be introduced into a host, including humans, linked to its own carrier or as a homopolymer or heteropolymer of active peptide units. Such a polymer has the advantage of increased immunological reaction and, where different peptides are used to make up the polymer, the additional ability to induce antibodies and/or CTLs that react with different antigenic determinants of the virus or tumor cells. Useful carriers are well known in the art, and include, e.g., thyroglobulin, albumins such as human serum albumin, tetanus toxoid, polyamino acids such as poly(lysine:glutamic acid), influenza, hepatitis B virus core protein, hepatitis B virus recombinant vaccine and the like. The vaccines can also contain a physiologically tolerable (acceptable) diluent such as water, phosphate buffered saline, or saline, and further typically include an adjuvant. Adjuvants such as incomplete Freund's adjuvant, aluminum phosphate, aluminum hydroxide, or alum are materials well known in the art. And, as mentioned above, CTL responses can be primed by conjugating peptides of the invention to lipids, such as P₃CSS. Upon immunization with a peptide composition as described herein, via injection, aerosol, oral, transdermal or other route, the immune system of the host responds to the vaccine by producing large amounts of CTLs specific for the desired antigen, and the host becomes at least partially immune to later infection, or resistant to developing chronic infection.

Vaccine compositions containing the peptides made by the methods of the invention are administered to a patient susceptible to or otherwise at risk of viral infection or cancer to elicit an immune response against the antigen and thus enhance the patient's own immune response capabilities. Such an amount is defined to be an "immunogenically effective dose." In this use, the precise amounts again depend on the patient's state of health and weight, the mode of administration, the nature of the formulation, etc., but generally range from about 1.0 µg to about 5000 µg per 70 kilogram patient, more commonly from about 10 µg to about 500 µg mg per 70 kg of body weight.

In some instances it may be desirable to combine the peptide vaccines of the invention with vaccines which induce neutralizing antibody responses to the virus of interest, particularly to viral envelope antigens.

For therapeutic or immunization purposes, the peptides made by the methods of the invention can also be expressed by attenuated viral hosts, such as vaccinia or fowlpox. This approach involves the use of vaccinia virus as a vector to express nucleotide sequences that encode the peptides of the invention. Upon introduction into an acutely or chronically infected host or into a non-infected host, the recombinant vaccinia virus expresses the immunogenic peptide, and thereby elicits a host CTL response. Vaccinia vectors and methods useful in immunization protocols are described in, e.g., U.S. Patent No. 4,722,848, incorporated herein by reference. Another vector is BCG (Bacille Calmette Guerin). BCG vectors are described in Stover et al. (Nature 351:456-460 (1991)) which is incorporated herein by reference. A wide variety of other vectors useful for therapeutic administration or immunization of the peptides of the invention, e.g., Salmonella typhi vectors and the like, will be apparent to those skilled in the art from the description herein.

Antigenic peptides may be used to elicit CTL ex vivo, as well. The resulting CTL, can be used to treat chronic infections (viral or bacterial) or tumors in patients that do not respond to other conventional forms of therapy, or will not respond to a peptide vaccine approach of therapy. Ex vivo CTL responses to a particular pathogen (infectious agent or tumor antigen) are induced by incubating in tissue culture the patient's CTL precursor cells (CTLp) together with a source of antigen-presenting cells (APC) and the appropriate immunogenic peptide. After an appropriate incubation time (typically 1-4 weeks), in which the CTLp are activated and mature and expand into effector CTL, the cells are infused back into the patient, where they will destroy their specific target cell (an infected cell or a tumor cell).

The peptides may also find use as diagnostic reagents. For example, a peptide made by the methods of the invention may be used to determine the susceptibility of a particular individual to a treatment regimen which employs the peptide or related peptides, and thus may be helpful in modifying an existing treatment protocol or in determining a prognosis for an affected individual. In addition, the peptides may also be used to predict which individuals will be at substantial risk for developing chronic infection.

The following examples are offered by way of illustration.

### Example 1

### Class I antigen isolation

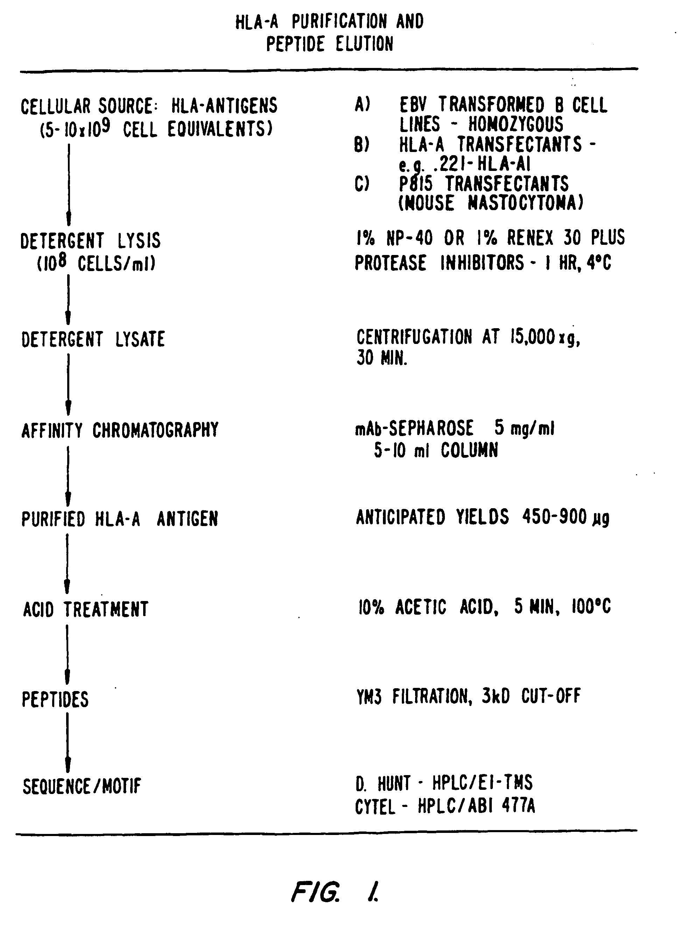

A flow diagram of an HLA-A antigen purification scheme is presented in Figure 1. Briefly, the cells bearing the appropriate allele were grown in large batches (6-8 liters yielding -5 x 10⁹ cells), harvested by centrifugation and washed. All cell lines were maintained in RPMI 1640 media (Sigma) supplemented with 10% fetal bovine serum (FBS) and antibiotics. For large-scale cultures, cells were grown in roller bottle culture in RPMI 1640 with 10% FBS or with 10% horse serum and antibiotics. Cells were harvested by centrifugation at 1500 RPM IEC-CRU5000 centrifuge with 259 rotor and washed three times with phosphate-buffered saline (PBS)(0.01 M PO₄, 0.154 M NaCl, pH 7.2).

Cells were pelleted and stored at -70°C or treated with detergent lysing solution to prepare detergent lysates. Cell lysates were prepared by the addition of stock detergent solution [1% NP-40 (Sigma) or Renex 30 (Accurate Chem. Sci. Corp., Westbury, NY 11590), 150 mM NaCl, 50 mM Tris, pH 8.0] to the cell pellets (previously counted) at a ratio of 50-100 x 10⁶ cells per ml detergent solution. A cocktail of protease inhibitors was added to the premeasured volume of stock detergent solution immediately prior to the addition to the cell pellet. Addition of the protease inhibitor cocktail produced final concentrations of the following: phenylmethylsulfonyl fluoride (PMSF), 2 mM; aprotinin, 5 µg/ml; leupeptin, 10 µg/ml; pepstatin, 10 µg/ml; iodoacetamide, 100 µM; and EDTA, 3 ng/ml. Cell lysis was allowed to proceed at 4°C for 1 hour with periodic mixing. Routinely 5-10 x 10⁹ cells were lysed in 50-100 ml of detergent solution. The lysate was clarified by centrifugation at 15,000 x g for 30 minutes at 4°C and subsequent passage of the supernatant fraction through a 0.2 µ filter unit (Nalgene).

The HLA-A antigen purification was achieved using affinity columns prepared with mAb-conjugated Sepharose beads. For antibody production, cells were grown in RPMI with 10% FBS in large tissue culture flasks (Corning 25160-225). Antibodies were purified from clarified tissue culture medium by ammonium sulfate fractionation followed by affinity chromatography on protein-A-Sepharose (Sigma). Briefly, saturated ammonium sulfate was added slowly with stirring to the tissue culture supernatant to 45% (volume to volume) overnight at 4°C to precipitate the immunoglobulins. The precipitated proteins were harvested by centrifugation at 10,000 x g for 30 minutes. The precipitate was then dissolved in a minimum volume of PBS and transferred to dialysis tubing (Spectro/Por 2, Mol. wt. cutoff 12,000-14,000, Spectum Medical Ind.). Dialysis was against PBS (≥20 times the protein solution volume) with 4-6 changes of dialysis buffer over a 24-48 hour period at 4°C. The dialyzed protein solution was clarified by centrifugation (10,000 x g for 30 minutes) and the pH of the solution adjusted to pH 8.0 with 1N NaOH. Protein-A-Sepharose (Sigma) was hydrated according to the manufacturer's instructions, and a protein-A-Sepharose column was prepared. A column of 10 ml bed volume typically binds 50-100 mg of mouse IgG.

The protein sample was loaded onto the protein-A-Sepharose column using a peristaltic pump for large loading volumes or by gravity for smaller volumes (<100 ml). The column was washed with several volumes of PBS, and the eluate was monitored at A280 in a spectrophotometer until base line was reached. The bound antibody was eluted using 0.1 M citric acid at suitable pH (adjusted to the appropriate pH with 1N NaOH). For mouse IgG-1 pH 6.5 was used for IgG2a pH 4.5 was used and for IgG2b and IgG3 pH 3.0 was used. 2 M Tris base was used to neutralize the eluate. Fractions containing the antibody (monitored by A280) were pooled, dialyzed against PBS and further concentrated using an Amicon Stirred Cell system (Amicon Model 8050 with YM30 membrane). The anti-A2 mAb, BB7.2, was useful for affinity purification.

The HLA-A antigen was purified using affinity columns prepared with mAb-conjugated Sepharose beads. The affinity columns were prepared by incubating protein-A-Sepharose beads (Sigma) with affinity-purified mAb as described above. Five to 10 mg of mAb per ml of bead is the preferred ratio. The mAb bound beads were washed with borate buffer (borate buffer: 100 mM sodium tetraborate, 154 mM NaCl, pH 8.2) until the washes show A280 at based line. Dimethyl pimelimidate (20 mM) in 200 mM triethanolamine was added to covalently crosslink the bound mAb to the protein-A-Sepharose (Schneider et al., J. Biol. Chem. 257:10766 (1982). After incubation for 45 minutes at room temperature on a rotator, the excess crosslinking reagent was removed by washing the beads twice with 10-20 ml of 20 mM ethanolamine, pH 8.2. Between each one the slurry was placed on a rotator for 5 minutes at room temperature. The beads were washed with borate buffer and with PBS plus 0.02% sodium azide.

The cell lysate (5-10 x 10⁹ cell equivalents) was then slowly passed over a 5-10 ml affinity column (flow rate of 0.1-0.25 ml per minute) to allow the binding of the antigen to the immobilized antibody. After the lysate was allowed to pass through the column, the column was washed sequentially with 20 column volumes of detergent stock solution plus 0.1% sodium dodecyl sulfate, 20 column volumes of 0.5 M NaCl, 20 mM Tris, pH 8.0, and 10 column volumes of 20 mM Tris, pH 8.0. The HLA-A antigen bound to the mAb was eluated with a basic buffer solution (50 mM diethylamine in water). As an alternative, acid solutions such as 0.15-0.25 M acetic acid were also used to elute the bound antigen. An aliquot of the eluate (1/50) was removed for protein quantification using either a colorimetric assay (BCA assay, Pierce) or by SDS-PAGE, or both. SDS-PAGE analysis was performed as described by Laemmli (Laemmli, U.K., Nature 227:680 (1970)) using known amounts of bovine serum albumin (Sigma) as a protein standard. Allele specific antibodies were used to purify the specific MHC molecule. In the case of HLA-A2, the mAb BB7.2 was used.

### Example 2

### Isolation and sequencing of naturally processed peptides

For the HLA-A preparations derived from the base (50 mM diethylamine) elution protocol, the eluate was immediately neutralized with 1 N acetic acid to pH 7.0-7.5. The neutralized eluate was concentrated to a volume of 1-2 ml in an Amicon stirred cell [Model 8050, with YM3 membranes (Amicon)]. Ten ml of ammonium acetate (0.01 M, pH 8.0> was added to the concentrator to remove the non-volatile salts, and the sample was concentrated to approximately 1 ml. A small sample (1/50) was removed for protein quantitation as described above. The remainder was recovered into a 15 ml polypropylene conical centrifuge tube (Falcon, 2097) (Becton Dickinson). Glacial acetic acid was added to obtain a final concentration of 10% acetic acid. The acidified sample was placed in a boiling water bath for 5 minutes to allow for the dissociation of the bound peptides. The sample was cooled on ice, returned to the concentrator and the filtrate was collected. Additional aliquots of 10% acetic acid (1-2 ml) were added to the concentrator, and this filtrate was pooled with the original filtrate. Finally, 1-2 ml of distilled water was added to the concentrator, and this filtrate was pooled as well.

The retentate contains the bulk of the HLA-A heavy chain and β₂-microglobulin, while the filtrate contains the naturally processed bound peptides and other components with molecular weights less than about 3000. The pooled filtrate material was lyophilized in order to concentrate the peptide fraction. The sample was then ready for further analysis.

For HPLC (high performance liquid chromatography) separation of the peptide fractions, the lyophilized sample was dissolved in 50 µl of distilled water, or into 0.1% trifluoracetic acid (TFA) (Applied Biosystems) in water and injected to a C18 reverse-phase narrow bore column (Beckman C18 Ultrasphere, 10 x 250 mm), using a gradient system described by Stone and Williams (Stone, K.L. and Williams K.R., in, Macromolecular Sequencing and Synthesis; Selected Methods and Applications, A.R. Liss, New York, 1988, pp. 7-24. Buffer A was 0.06% TFA in water (Burdick-Jackson) and buffer B was 0.052% TFA in 80% acetonitrile (Hurdick-Jackson). The flow rate was 0.250 ml/minute with the following gradient: 0-60 min., 2-37.5% B; 60-95 min., 37.5-75% B; 95-105 min., 75-98% B. The Gilson narrow bore HPLC configuration is particularly useful for this purpose, although other configurations work equally well.

A large number of peaks were detected by absorbance at 214 nm, many of which appear to be of low abundance. Whether a given peak represents a single peptide or a peptide mixture was not determined. Pooled fractions were then sequenced to determine motifs specific for each allele as described below.

Pooled peptide fractions, prepared as described above were analyzed by automated Edman sequencing using the Applied Biosystems Model 477A automated sequencer. The sequencing method is based on the technique developed by Pehr Edman in the 1950s for the sequential degradation of proteins and peptides to determine the sequence of the constituent amino acids.

The protein or peptide to be sequenced was held by a 12-mm diameter porous glass fiber filter disk in a heated, argon-purged reaction chamber. The filter was generally pre-treated with BioBrene Plus™ and then cycled through one or more repetitions of the Edman reaction to reduce contaminants and improve the efficiency of subsequent sample sequencing. Following the pre-treatment of the filter, a solution of the sample protein or peptide (10 pmol-5 nmol range) was loaded onto the glass filter and dried. Thus, the sample was left embedded in the film of the pre-treated disk. Covalent attachment of the sample to the filter was usually not necessary because the Edman chemistry utilized relatively apolar solvents, in which proteins and peptides are poorly soluble.

Briefly, the Edman degradation reaction has three steps: coupling, cleavage, and conversion. In coupling step, phenylisothiocyanate (PITC) is added. The PITC reacts quantitatively with the free amino-terminal amino acid of the protein to form the phenylthiocarbamyl-protein in a basic environment. After a period of time for the coupling step, the excess chemicals are extracted and the highly volatile organic acid, trifluoroacetic acid, TFA, is used to cleave the PITC-coupled amino acid residue from the amino terminus of the protein yielding the anilinothiazolinone (ATZ) derivative of the amino acid. The remaining protein/peptide is left with a new amino terminus and is ready for the next Edman cycle. The ATZ amino acid is extracted and transferred to a conversion flask, where upon addition of 25% TFA in water, the ATZ amino acid is converted to the more stable phenylthiohydantoin (PTH) amino acid that can be identified and quantified following automatic injection into the Model 120 PTH Analyzer which uses a microbore C-18 reverse-phase HPLC column for the analysis.

In the present procedures, peptide mixtures were loaded onto the glass filters. Thus, a single amino acid sequence usually does not result. Rather, mixtures of amino acids in different yield are found. When the particular residue is conserved among the peptides being sequenced, increased yield for that amino acid is observed.

### Example 3

### Definition of an A2.1 specific motif

In one case, pooled peptide fractions prepared as described in Example 2 above were obtained from HLA-A2.1 homozygous cell lines, for example, JY. The pooled fractions were HPLC fractions corresponding to 7% to 45% CH₃CN. For this class I molecule, this region of the chromatogram was most abundant in peptides. Data from independent experiments were averaged as described below.

The amino acid sequence analyses from four independent experiments were analyzed and the results are shown in Table 3. For each position except the first, the data were analyzed by modifying the method described by Falk et al., supra, to allow for comparison of experiments from different HLA types. This modified procedure yielded quantitative yet standardized values while allowing the averaging of data from different experiments involving the same HLA type.

The raw sequenator data was converted to a simple matrix of 10 rows (each representing one Edman degradation cycle) and 16 columns (each representing one of the twenty amino acids; W, C, R and H were eliminated for technical reasons. The data corresponding to the first row (first cycle) was not considered further because, this cycle is usually heavily contaminated by free amino acids.). The values of each row were summed to yield a total pmoles value for that particular cycle. For each row, values for each amino acid were then divided by the corresponding total yield value, to determine what fraction of the total signal is attributable to each amino acid at each cycle. By doing so, an "Absolute Frequency" table was generated. This absolute frequency table allows correction for the declining yields of each cycle.

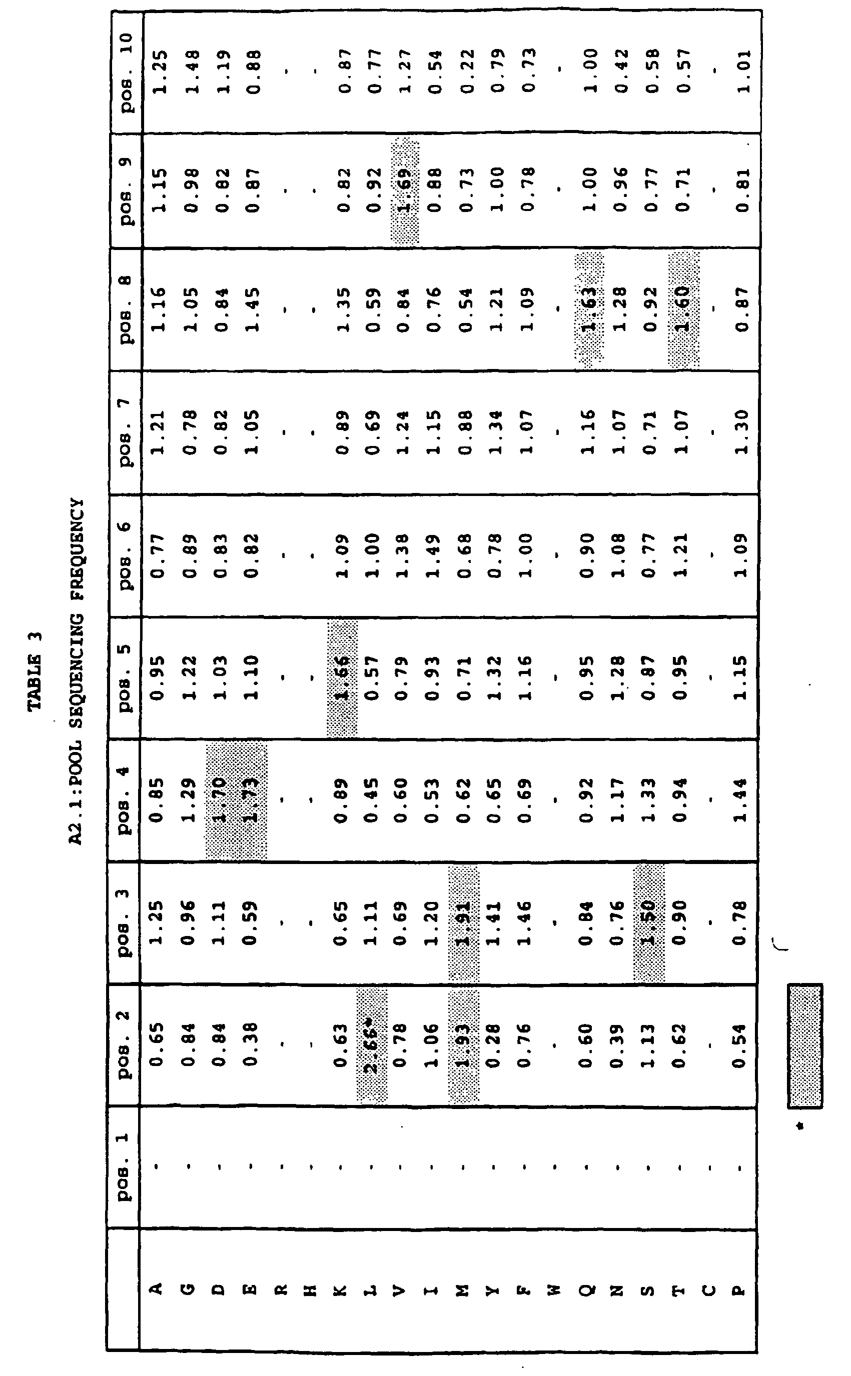

Starting from the absolute frequency table, a "relative frequency" table was then generated to allow comparisons among different amino acids. To do so the data from each column was summed, and then averaged. Then, each value was divided next by the average column value to obtain relative frequency values. These values quantitate, in a standardized manner, increases and decreases per cycle, for each of the different sixteen amino acid types. Tables generated from data from different experiments can thus be added together to generate average relative frequency values (and their standard deviations). All standard deviations can then be averaged, to estimate a standard deviation value applicable to the samples from each table. Any particular value exceeding 1.00 by more than two standard deviations is considered to correspond to a significant increase.

### Example 4

### Quantitative Binding Assays

Using isolated MHC molecules prepared as described in Example 2, above, quantitative binding assays were performed. Briefly, indicated amounts of MHC as isolated above were incubated in 0.05% NP40-PBS with ^{~}5 nM of radiolabeled peptides in the presence of 1-3 µM β₂M and a cocktail of protease inhibitors (final concentrations 1 mM PMSF, 1.3 mM 1.10 Phenanthroline, 73 µm Pepstatin A, 8 mM EDTA, 200 µM N-α-p-tosyl-L-Lysine Chloromethyl ketone). After various times, free and bound peptides were separated by TSK 2000 gel filtration, as described previously in A. Sette et al., J. Immunol. 148:844 (1992), which is incorporated herein by reference. Peptides were labeled by the use of the Chloramine T method Buus et al., Science 235:1352 (1987), which is incorporated herein by reference.

The HBc 18-27 peptide HLA binding peptide was radiolabeled and offered (5-10 nM) to 1 µM purified HLA A2.1. After two days at 23°C in presence of a cocktail of protease inhibitors and 1-3 µM purified human β₂M, the percent of MHC class I bound radioactivity was measured by size exclusion chromatography, as previously described for class II peptide binding assays in Sette et al., in Seminars in Immunology, Vol. 3, Gefter, ed. (W.B. Saunders, Philadelphia, 1991), pp 195-202, which is incorporated herein by reference. Using this protocol, high binding (95%) was detected in all cases in the presence of purified HLA A2.1 molecules.

To explore the specificity of binding, we determined whether the binding was inhibitable by excess unlabeled peptide, and if so, what the 50% inhibitory concentration (IC50%) might be. The rationale for this experiment was threefold. First, such an experiment is crucial in order to demonstrate specificity. Second, a sensitive inhibition assay is the most viable alternative for a high throughput quantitative binding assay. Third, inhibition data subjected to Scatchard analysis can give quantitative estimates of the equilibrium constant (K) of interaction and the fraction of receptor molecules capable of binding ligand (% occupancy). For instance, in analysis of an inhibition curve for the interaction of the peptide HBc 18-27 with A2.1, the IC50% was determined to be 25 nM. Further experiments were conducted to obtain Scatchard plots. For HBc 18-27/A2.1, six different experiments using six independent MHC preparations yielded a K_{D} of 15.5 ± 9.9 x 10⁻⁹ M and occupancy values of 6.2% (±1.4).

Several reports have demonstrated that class I molecules, unlike class II, are highly selective with regard to the size of the peptide epitope that they recognize. The optimal size varies between 8 and 10 residues for different peptides and different class I molecules, although MHC binding peptides as long as 13 residues have been identified. To verify the stringent size requirement, a series of N- and C-terminal truncation/extension analogs of the peptide HBc 18-27 were synthesized and tested for A2.1 binding. Previous studies had demonstrated that the optimal size for CTL recognition of this peptide was the 10-mer HBc18-27 (Sette et al. supra). It was found that removal or addition of a residue at the C terminus of the molecule resulted in a 30 to 100-fold decrease in binding capacity. Further removal or addition of another residue completely obliterated binding. Similarly, at the N-terminus of the molecule, removal or deletion of one residue from the optimal HBc 18-27 peptide completely abrogated A2.1 binding.

Throughout this disclosure, results have been expressed in terms of IC50's. Given the conditions in which our assays are run (i.e., limiting MHC and labeled peptide concentrations), these values approximate K_{D} values. It should be noted that IC50 values can change, often dramatically, if the assay conditions are varied, and depending on the particular reagents used (e.g., Class I preparation, etc.). For example, excessive concentrations of MHC will increase the apparent measured IC50 of a given ligand.

An alternative way of expressing the binding data, to avoid these uncertainties, is as a relative value to a reference peptide. The reference peptide is included in every assay. As a particular assay becomes more, or less, sensitive, the IC50's of the peptides tested may change somewhat. However, the binding relative to the reference peptide will not change. For example, in an assay run under conditions such that the IC50 of the reference peptide increases 10-fold, all IC50 values will also shift approximately ten-fold. Therefore, to avoid ambiguities, the assessment of whether a peptide is a good, intermediate, weak, or negative binder should be based on its IC50, relative to the IC50 of the standard peptide.

The reference peptide for the HLA-A2.1 assays described herein is referred to as 941.01 having a sequence of FLPSDYFPSV. An average IC50 of 5 (nM) was observed under the assay conditions utilized.

If the IC50 of the standard peptide measured in a particular assay is different from that reported in the table, then it should be understood that the threshold values used to determine good, intermediate, weak, and negative binders should be modified by a corresponding factor. For example, if in an A2.1 binding assay, the IC50 of the A2.1 standard (941.01) were to be measured as 8 nM instead of 5 nM, then a peptide ligand would be called a good binder only if it had an IC50 of less than 80 nM (i.e., 8nM x 0.1), instead of the usual cut-off value of 50 nM.

### Example 5

### HLA-A2.1 Binding Motif and algorithm

The structural requirements for peptide binding to A2.1 have been defined for both, 9-mer and 10-mer peptides. Two approaches have been used. The first approach referred to as the "poly-A approach" uses a panel of single amino acid substitutions of a 9-mer prototype poly-A binder (ALAKAAAAV) that is tested for A2.1 binding using the methods of Example 4 above to examine the degree of degeneracy of the anchor-positions and the possible influence of non-anchor positions on A2.1 binding.

The second approach, the "Motif-Library approach", uses a large library of peptides selected from sequences of potential target molecules of viral and tumor origin and tested for A2.1 binding using the methods in Example 4 above. The frequencies by which different amino-acids occured at each position in good binders and non-binders were analysed to further define the role of non-anchor positions in 9-mers and 10-mers.

### A2.1 binding of peptide 9-mers

Poly A Approach A poly-A 9-mer peptide, containing the A2.1 motif L (Leu) in position 2 and V (Val) in position 9 was chosen as a prototype binder. A K (Lys) residue was included in position 4 to increase solubility. A panel of 91 single amino-acid substitution analogues of the prototype parental 9-mer was synthesized and tested for A2.1 binding (Table 4). Shaded areas mark analogs with a greater than 10-fold reduction in binding capacity relative to the parental peptide. A reduction in binding greater than 100-fold is indicated by hyphenation.

Anchor-Positions 2 and 9 in poly-A Analogs The effect of single-amino-acid substitutions at the anchor positions 2 and 9 was examined first. Most substitutions in these positions had profound detrimental effects on binding capacity, thus confirming their role for binding. More specifically, in position 2 only L and M bound within a 10-fold range ("preferred resides"). Residues with similar characteristics, such as I, V, A, and T were tolerated, but bound 10 to 100-fold less strongly than the parental peptide. All the remaining substitutions (residues S, N, D, F, C, K, G, and P) were not tolerated and decreased binding by more than 100-fold. Comparably stringent requirements were observed for position 9, where V, L and I were preferred and A and M are tolerated, while the residues T, C, N, F, and Y virtually abolished binding. According to this set of peptides, an optimal 2-9 motif could be defined with L, M in position 2 and V, I, or L in position 9.

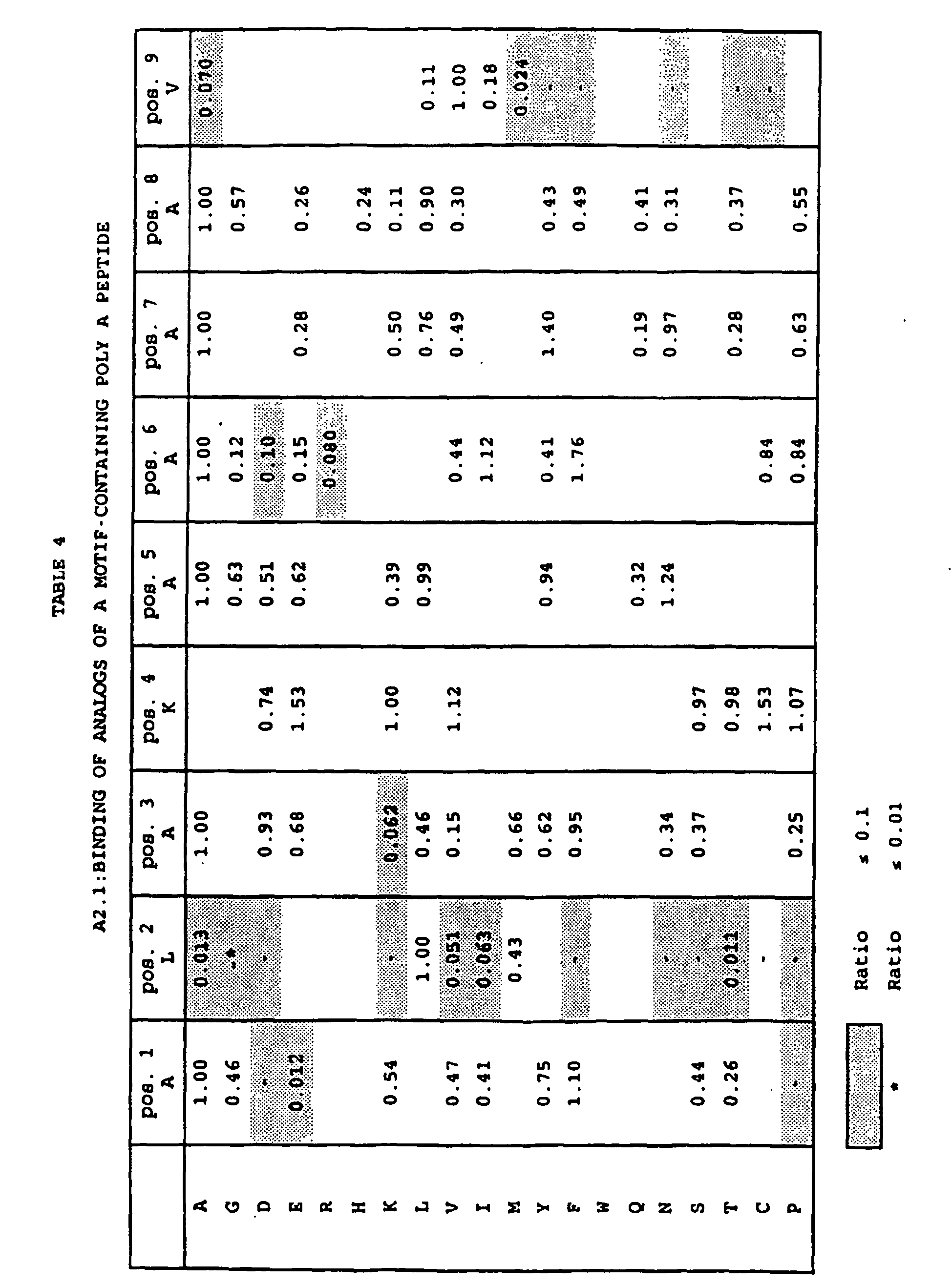

Non-Anchor Positions 1 and 3-8 in poly-A Analogs All non-anchor positions were more permissive to different substitutions than the anchor-positions 2 and 9, i.e most residues were tolerated. Significant decreases in binding were observed for some substitutions in distinct positions. More specifically, in position 1 a negative charge (residues D and E) or a P greatly reduced the binding capacity. Most substitutions were tolerated in position 3 with the exception of the residue K. Significant decreases were also seen in position 6 upon introduction of either a negative charge (D, E) or a positively charged residue (R). A summary of these effects by different single amino acid substitutions is given in Table 5.

**TABLE 5**

| Summary | A2.1 | Poly-A | |
|---|---|---|---|
| AA position | (+) | (+/-) | (-) |
| 1 | FAYKVGSIT | | EDP |
| 2 | LM | VITA | SNDFCKGP |
| 3 | AFDEMYLSNPV | K | |
| 4 | CEVPATSD | | |
| 5 | NALYGEDKQ | | |
| 6 | FIAPCVYEG | DR | |
| 7 | YANLPVETQ | | |
| 8 | ALGPFYQTNVEHK | | |
| 9 | VIL | AM | TCNFY |

| | | | |
|---|---|---|---|
| Ratio > 0.1 Ratio 0.01-0.1 Ratio < 0.01 | | | |

The Motif-Library Approach To further evaluate the importance of non-anchor positions for binding, peptides of potential target molecules of viral and tumor origin were scanned for the presence of sequences containing optimal 2-9 anchor motifs. A set of 161 peptides containing a L or M in position 2 and a V, L or I in position 9 was selected, synthesized and tested for binding (see Example 6). Only 11.8% of these peptides bind with high affinity (ratio ≥0.10; 22.4% were intermediate binders (ratio ≥0.1). As many as 36% were weak binders (ratio <0.01 - 0.0001), and 31% were non-binders (ratio<0.0001). The high number of non-binders containing optimal anchor-motifs indicates that in this set of peptides positions other than the 2-9 anchors influence A2.1 binding capacity. Appendix 1 sets forth all of the peptides having the 2-9 motif used for this analysis and the binding data for those peptides.

To define the influence on non-anchor positions more specifically, the frequency of occurrence of each amino acid in each of the non-anchor positions was calculated for the good and intermediate binders on one hand and non-binders on the other hand. Amino acids of similar chemical characteristic were grouped together. Weak binders were not considered for the following analysis. The frequency of occurence of each amino acid in each of the non-anchor positions was calculated for the good binders and non-binders (Table 6).

Several striking trends become apparent. For example in position 1, only 3.6% of the A2.1 binders and as much as 35% of the non-binders carried a negative charge (residues D and E). This observation correlates well with previous findings in the set of poly-A analogs, where a D or E substitution greatly affected binding. Similarly, the residue P was 8 times more frequent in non-binders than in good binders. Conversely, the frequencies of aromatic residues (Y, F, W) were greatly increased in A2.1 binders as compared to non-binders.

**TABLE 6**

| | | | | | | | | | | | | | | | | | | |
|---|---|---|---|---|---|---|---|---|---|---|---|---|---|---|---|---|---|---|
| A.2.1 9-mer PEPTIDES | | | | | | | | | | | | | | | | | | |
| NUMBER OF PEPTIDES | | | | 161 | | | | | | | | | | | | | | |
| GOOD BINDERS | | | | 19 | 11.8% | | | | | | | | | | | | | |
| INTERMEDIATE BINDERS | | | | 36 | 22.4% | | | | | | | | | | | | | |
| WEAK BINDERS | | | | 58 | 36.0% | | | | | | | | | | | | | |
| NON-BINDERS | | | | 48 | 29.8% | | | | | | | | | | | | | |

| | 1+ | 1- | 2+ | 2- | 3+ | 3- | 4+ | 4- | 5+ | 5- | 6+ | 6- | 7+ | 7- | 8+ | 8- | 9+ | 9- |
|---|---|---|---|---|---|---|---|---|---|---|---|---|---|---|---|---|---|---|
| A | 5.5 | 2.1 | 0.0 | 0.0 | 3.6 | 4.2 | 5.6 | 8.3 | 5.5 | 8.3 | 5.5 | 6.3 | 9.1 | 2.1 | 3.6 | 12.5 | 0.0 | 0.0 |
| G | 7.3 | 2.1 | 0.0 | 0.0 | 3.6 | 8.3 | 9.1 | 8.3 | 9.1 | 8.3 | 10.9 | 8.3 | 5.5 | 12.5 | 3.6 | 8.3 | 0.0 | 0.0 |
| D,E | 3.6 | 35.4 | 0.0 | 0.0 | 0.0 | 12.5 | 10.9 | 16.7 | 3.6 | 12.5 | 5.5 | 8.3 | 1.8 | 16.7 | 9.1 | 10.4 | 0.0 | 0.0 |
| R,H,K | 12.7 | 4.2 | 0.0 | 0.0 | 3.6 | 16.7 | 16.4 | 16.9 | 9.1 | 10.4 | 1.8 | 20.8 | 0.0 | 10.4 | 16.4 | 12.5 | 0.0 | 0.0 |
| L,V,I,M | 38.2 | 12.5 | 100.0 | 100.0 | 34.5 | 18.8 | 9.1 | 16.7 | 25.5 | 29.2 | 30.9 | 22.9 | 30.9 | 25.0 | 32.7 | 18.8 | 100.0 | 100.0 |
| Y, F, W | 14.5 | 2.1 | 0.0 | 0.0 | 21.8 | 4.2 | 7.3 | 8.3 | 18.2 | 2.1 | 16.4 | 8.3 | 14.5 | 8.3 | 5.5 | 8.3 | 0.0 | 0.0 |
| Q,N | 7.3 | 14.8 | 0.0 | 0.0 | 5.5 | 14.6 | 12.7 | 10.4 | 9.1 | 10.4 | 10.9 | 10.4 | 5.5 | 8.3 | 5.5 | 16.7 | 0.0 | 0.0 |
| S,T,C | 9.1 | 12.5 | 0.0 | 0.0 | 20.0 | 10.4 | 20.0 | 4.2 | 14.5 | 16.7 | 14.5 | 12.5 | 14.5 | 12.5 | 20.0 | 18.8 | 0.0 | 0.0 |
| P | 1.8 | 14.6 | 0.0 | 0.0 | 7.3 | 10.4 | 9.1 | 12.5 | 5.5 | 2.1 | 3.6 | 2.1 | 18.2 | 6.3 | 3.6 | 0.0 | 0.0 | 0.0 |
| | | | | | | | | | | | | | | | | | | |
| | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 |

Following this approach, amino acids of similar structural characteristics were grouped together. Then, the frequency of each amino acid group in each position was calculated for binders versus non-binders (Table 7). Finally, the frequency in the binders group was divided by the frequency in the non-binders to obtain a "frequency ratios". This ratio indicates whether a given amino-acid or group of residues occurs in a given position preferentially in good binders (ratio >1) or in non-binders (ratio <1).
TABLE 7

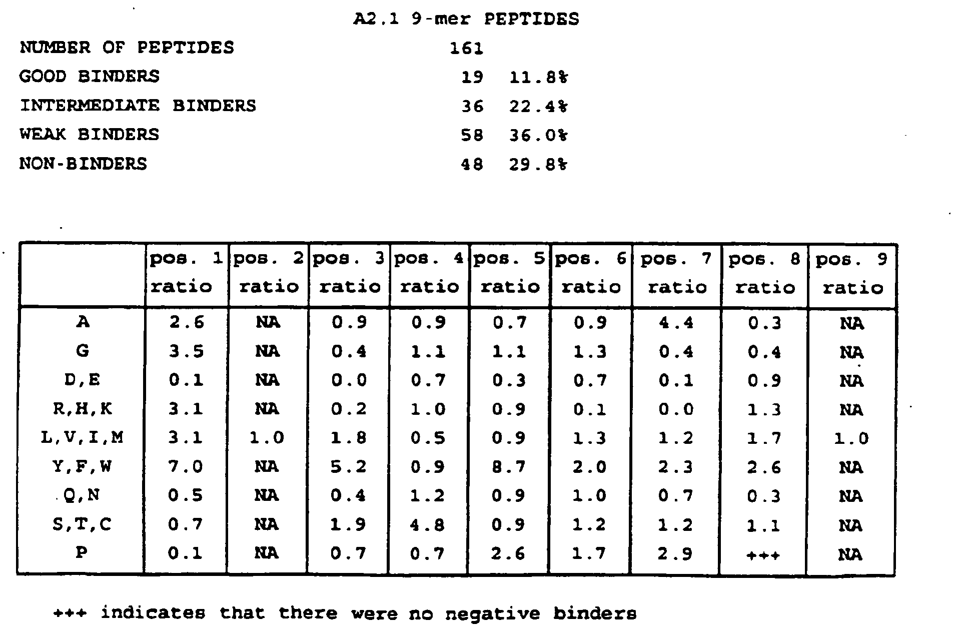

Different Residues Influence A2.1 Binding In order to analyse the most striking influences of certain residues on A2.1 binding, a threshold level was set for the ratios described in Table 7. Residues showing a more than 4-fold greater frequency in good binders were regarded as preferred residues (+). Residues showing a 4-fold lower frequency in A2.1 binders than in non-binders were regarded as disfavored residues (-). Following this approach, residues showing the most prominent positive or negative effects on binding are listed in Table 8.

This table identifies the amino acid groups which influence binding most significantly in each of the non-anchor positions. In general, the most negative effects were observed with charged amino acids. In position 1, negatively, charged amino acids were not observed in good binders, i.e., those amino acids were negative binding residues at position 1. The opposite was true for position 6 where only basic amino acids were detrimental for binding i.e., were negative binding residues. Moreover, both acidic and basic amino acids were not observed in A2.1 binders in positions 3 and 7. A greater than 4-fold increased frequency of non-binders was found when P was in position 1.
TABLE 8

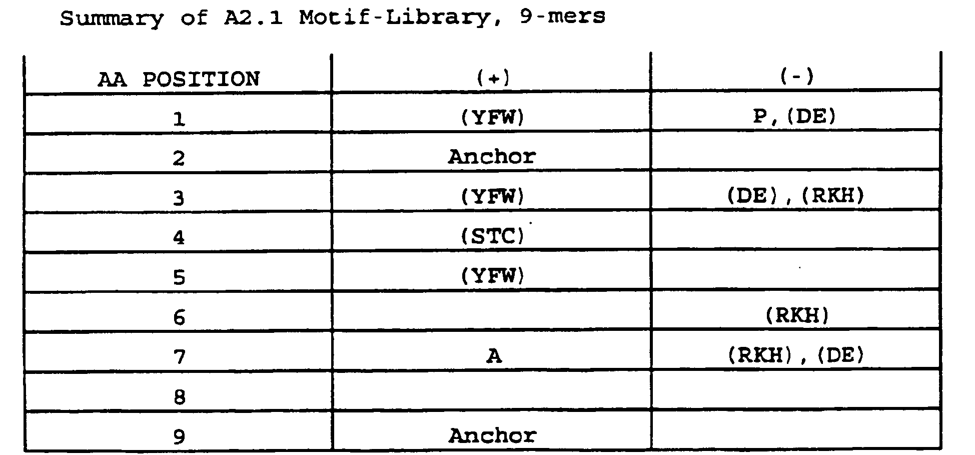

(+) = Ratio ≥ 4-fold (-) = Ratio s 0.25

Aromatic residues were in general favored in several of the non-anchor positions, particularly in positions 1, 3, and 5. Small residues like S, T, and C were favored in position 4 and A was favored in position 7.

An Improved A2.1 9-mer Motif The data described above was used to derive a stringent A2.1 motif. This motif is based in significant part on the effects of the non-anchor positions 1 and 3-8. The uneven distribution of amino acids at different positions is reflective of specific dominant negative binding effects of certain residues, mainly charged ones, on binding affinity. A series of rules were derived to identify appropriate anchor residues in positions 2 and 9 and negative binding residues at positions 1 and 3-8 to enable selection of a high affinity binding immunogenic peptide. These rules are summarized in Table 9.

To validate the motif defined above and shown in Table 9 published sequences of peptides that have been naturally processed and presented by A2.1 molecules were analysed (Table 10). Only 9-mer peptides containing the 2-9 anchor residues were considered.

When the frequencies of these peptides were analysed, it was found that in general they followed the rules summarized in Table 9. More specifically, neither acidic amino acids nor P were found in position 1. Only one acidic amino acid and no basic amino acids were found in position 3. Positions 6 and 7 showed no charged residues. Acidic amino acids, however, were frequently found in position 8, where they are tolerated, according to our definition of the A2.1 motif. The analysis of the sequences of naturally processed peptides therefore reveals that >90% of the peptides followed the defined rules for a complete motif.

Thus the data confirms a role of positions other than the anchor positions 2 and 9 for A2.1 binding. Most of the deleterious effects on binding are induced by charged amino acids in non-anchor positions, i.e. negative binding residues occupying positions 1, 3, 6 or 7.

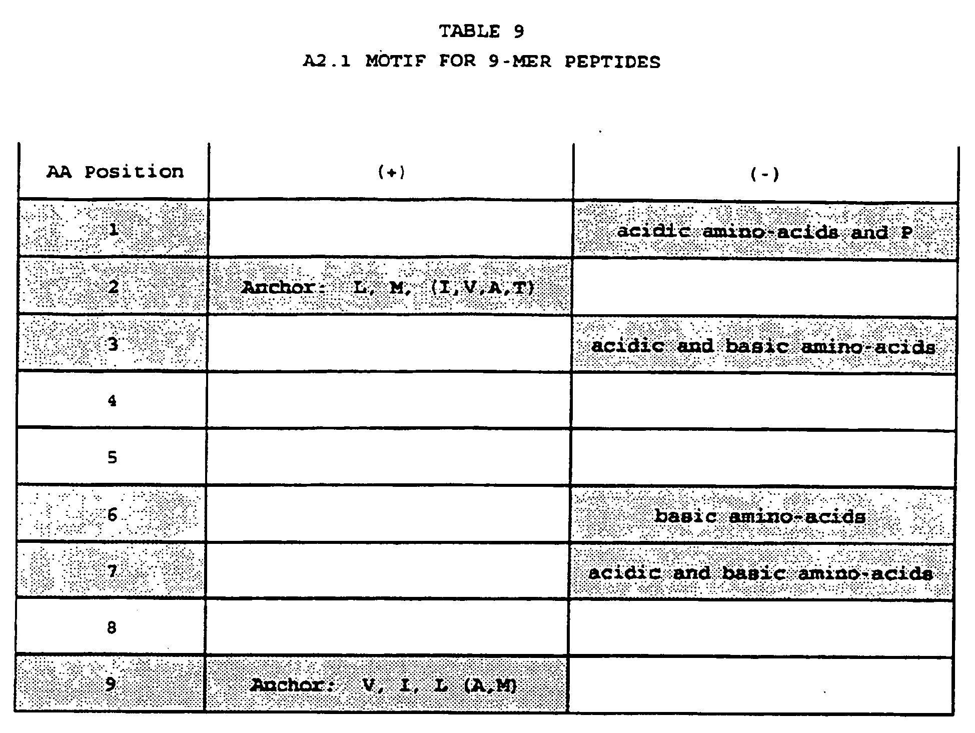

**TABLE 10**

| A2.1 naturally processed peptides | | | | | | | | | |
|---|---|---|---|---|---|---|---|---|---|
| 1 | 2 | 3 | 4 | 5 | 6 | 7 | 8 | 9 | A2.1 binding |
| A | L | X | G | G | X | V | N | V | ND |
| L | L | D | V | P | T | A | A | V | ND |
| G | X | V | P | F | X | V | S | V | 0.41 |
| S | L | L | P | A | I | V | E | L | 0.19 |
| S | X | X | V | R | A | X | E | V | ND |
| Y | M | N | G | T | M | S | Q | V | ND |
| K | X | N | E | P | V | X | X | X | ND |
| Y | L | L | P | A | I | V | H | I | 0.26 |
| A | X | W | G | F | F | P | V | X | ND |
| T | L | W | V | D | P | Y | E | V | 0.23 |
| G | X | V | P | F | X | V | S | V | 0.41 |

### A2.1 Binding of Peptide 10-mers

The "Motif-Library" Approach Previous data clearly indicated that 10-mers can also bind to HLA molecules even if with a somewhat lower affinity than 9-mers. For this reason we expanded our analysis to 10-mer peptides.

Therefore, a "Motif-Library" set of 170 peptide 10-mers containing optimal motif-combinations was selected from known target molecule sequences of viral and tumor origin and analysed as described above for 9-mers. In this set we found 5.9% good binders, 17.1% intermediate binders, 41.2% weak binders and 35.9% non-binders. The actual sequences, origin and binding capacities of this set of peptides are included as Appendix 2. This set of 10-mers was used to determine a) the rules for 10-mer peptide binding to A2.1, b) the similarities or differences to rules defined for 9-mers, and c) if an insertion point can be identified that would allow for a superimposable common motif for 9-mers and 10-mers.

Amino-acid frequencies and frequency ratios for the various amino-acid groups for each position were generated for 10-mer peptides as described above for 9-mer peptides and are also shown in Tables 11 and 12, respectively for grouped residues.

A summary of preferred versus disfavored residues and of the rules derived for the 10-mers in a manner analogous to that used for 9-mers, is also listed in Tables 13 and 14, respectively.

When the frequency-ratios of different amino-acid groups in binders and non-binders at different positions were analysed and compared to the corresponding ratios for the 9-mers, both striking similarities and significant differences emerged (Table 15). At the N-terminus and the C-termini of 9-mers and 10-mers, similarities predominate. In position 1 for example, in 10-mers again the P residue and acidic amino acids were not tolerated. In addition at position 1 in 10-mers aromatic residues were frequently observed in A2.1 binders. In position 3, acidic amino acids were frequently associated with poor binding capacity in both 9-mers and 10-mers. Interestingly, however, while in position 3 aromatic residues

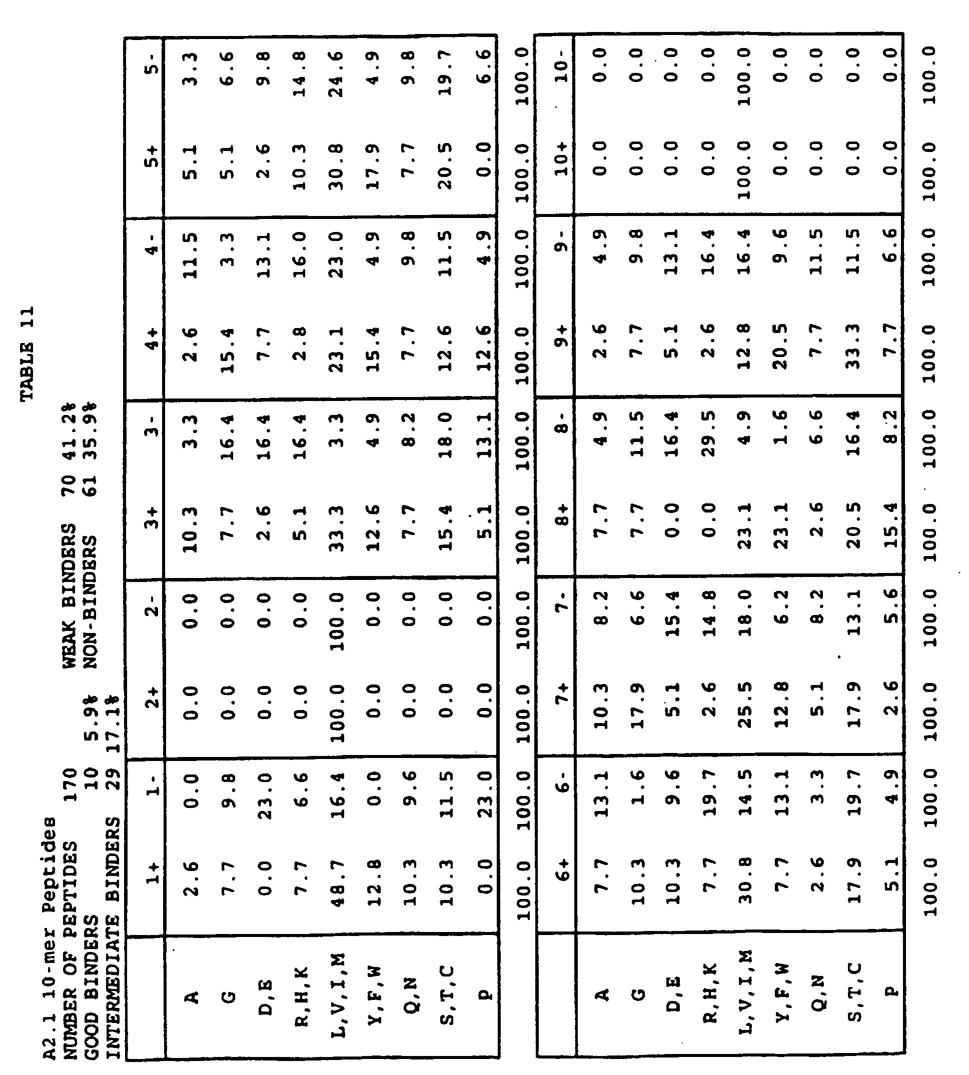

**TABLE 12**

| | | | | | | | | | | |
|---|---|---|---|---|---|---|---|---|---|---|
| A2.1 10-mer Peptides | | | | | | | | | | |
| NUMBER OF PEPTIDES | | | 170 | | | | | | | |
| GOOD BINDERS | | | 10 | 5.9% | | | | | | |
| INTERMEDIATE BINDERS | | | 29 | 17.1% | | | | | | |
| WEAK BINDERS | | | 70 | 41.2% | | | | | | |
| NON-BINDERS | | | 61 | 35.9% | | | | | | |

| | pos. 1 ratio | pos. 2 ratio | pos. 3 ratio | pos. 4 ratio | pos. 5 ratio | pos. 6 ratio | pos. 7 ratio | pos. 8 ratio | pos. 9 ratio | pos. 10 ratio |
|---|---|---|---|---|---|---|---|---|---|---|
| A | +++ | NA | 3.1 | 0.2 | 1.8 | 0.6 | 1.3 | 1.6 | 0.5 | NA |
| G | 0.8 | NA | 0.5 | 4.7 | 0.8 | 6.3 | 2.7 | 0.7 | 0.8 | NA |
| D,E | 0.0 | NA | 0.2 | 0.6 | 0.3 | 1.0 | 0.3 | 0.0 | 0.4 | NA |
| R,H,K | 1.2 | NA | 0.3 | 0.1 | 0.7 | 0.4 | 0.2 | 0.0 | 0.2 | NA |
| L,V,I,M | 3.0 | 1.0 | 10.2 | 1.0 | 1.3 | 2.1 | 1.4 | 4.7 | 0.8 | 1.0 |
| Y,F,W | +++ | NA | 2.6 | 3.1 | 3.6 | 0.6 | 1.6 | 14.1 | 2.1 | NA |
| Q,N | 1.0 | NA | 0.9 | 0.8 | 0.8 | 0.8 | 0.6 | 0.4 | 0.7 | NA |
| S,T,C | 0.9 | NA | 0.9 | 1.1 | 1.0 | 0.9 | 1.4 | 1.3 | 2.9 | NA |
| P | 0.0 | NA | 0.4 | 2.6 | 0.0 | 1.0 | 0.4 | 1.9 | 1.2 | NA |

| | | | | | | | | | | |
|---|---|---|---|---|---|---|---|---|---|---|
| +++ Indicates that there were no negative binders. | | | | | | | | | | |

**TABLE 13**

| Summary of A2.1 Motif-Library 10-mers | | |
|---|---|---|
| AA position | (+) | (-) |
| 1 | (YFW), A | (DE), P |
| 2 | Anchor | |
| 3 | (LVIM) | (DE) |
| 4 | G | A, (RKH) |
| 5 | | P |
| 6 | G | |
| 7 | | (RKH) |
| 8 | (YFW), (LVIM) | (DE), (RKH) |
| 9 | | (RKH) |
| 10 | Anchor | |

| | | |
|---|---|---|
| (+) = Ratio ≥ 4-fold (-) = Ratio s 0.25 | | |

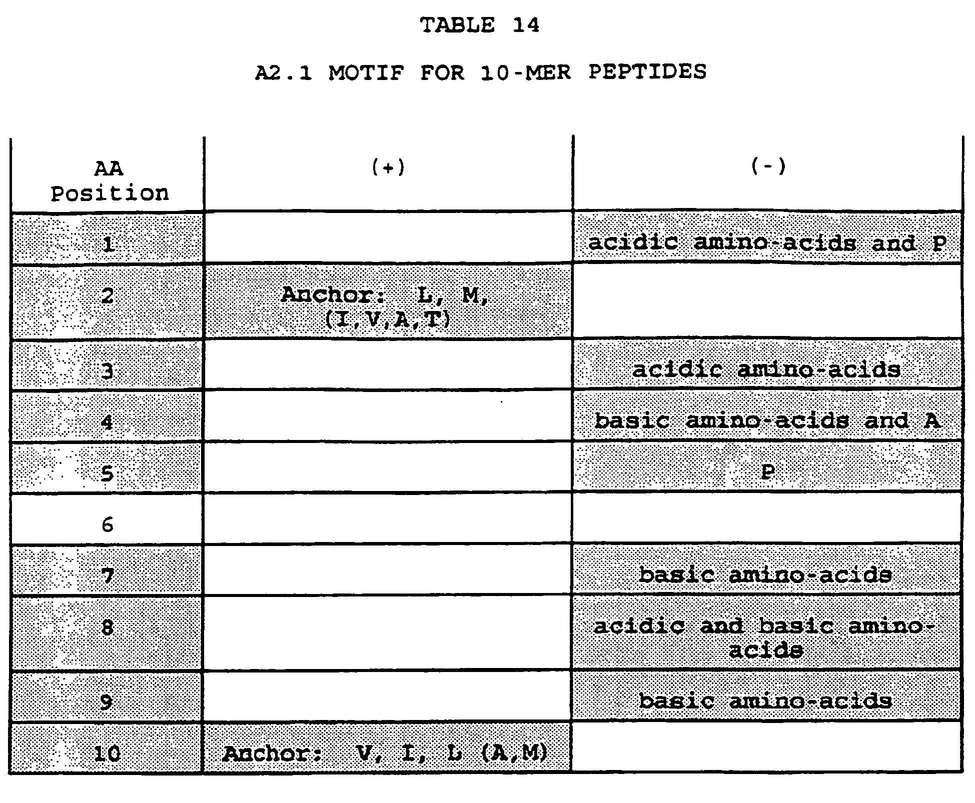

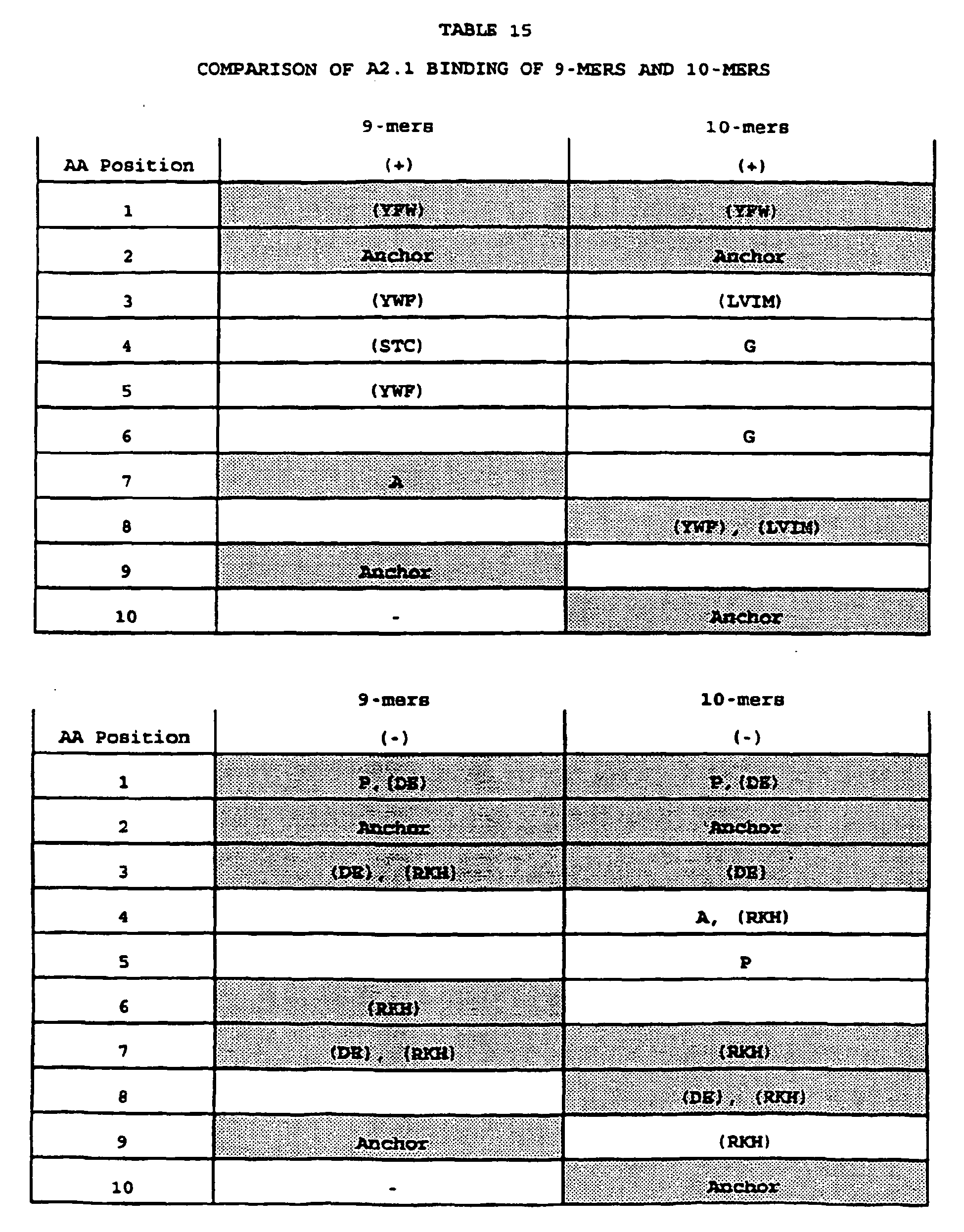

were preferred in 9-mers, aliphatic residues (L, V, I, M) were preferred in 10-mers.

At the C-terminus of the peptides, basic amino acids are not favored in position 7, and both acidic and basic amino acids are not favored in position 8 for 10-mers. This is in striking agreement with the observation that the same pattern was found in 9-mers at positions 6 and 7. Interestingly, again the favored residues differ between two peptides sizes. Aromatic (Y, F, W) or aliphatic (L, V, I, M) residues were preferred in 10-mers at position 8, while the A residue was preferred by 9-mers in the corresponding position 7.

By contrast, in the center of the peptide no similarities of frequency preferences were observed at positions 4, 5, and 6 in 10-mers and positions 4 and 5 in the 9-mers.

Most interestingly, among the residues most favored in the center of the tested peptides were G in position 4 and 6, P in position 5 was not observed in binders. All of these residues are known to dramatically influence the overall secondary structure of peptides, and in particular would be predicted to strongly influence the propensity of a 10-mer to adopt a "kinked" or "bulged" conformation.

Charged residues are predominantly deleterious for binding and are frequently observed in non-binders of 9-mers and 10-mers.

However, favored residues are different for 9-mers and 10-mers. Glycine is favored while Proline is disfavored in the center of 10-mer peptides but this is not the case for 9-mers.

These data establish the existence of an "insertion area" spanning two positions (4, 5) in 9-mers and 3 positions (4, 5, 6) in 10-mers. This insertion area is a more permissive region where few residue similarities are observed between the 9-mer and 10-mer antigenic peptides. Furthermore, in addition to the highly conserved anchor positions 2 and 9, there are "anchor areas" for unfavored residues in positions 1 and 3 at the N-terminus for both 9-mer and 10-mer and positions 7-10 or 6-9 at the C-terminus for 10-mers and 9-mers, respectively.

### Example 6

### Algorithm to Predict Binding of 9-mer Peptides to HLA-A2.1

Within the population of potential A2.1 binding peptides identified by the 2,9 motif, as shown in the previous example, only a few peptides are actually good or intermediate binders and thus potentially immunogenic. It is apparent from the data previously described that the residues present in positions other than 2 and 9 can influence, often profoundly, the binding affinity of a peptide. For example, acidic residues at position 1 for A2.1 peptides do not appear to be tolerated. Therefore, a more exact predictor of binding could be generated by taking into account the effects of different residues at each position of a peptide sequence, in addition to positions 2 and 9.

More specifically, we have utilized the data bank obtained during the screening of our collection of A2.1 motif containing 9-mer peptides to develop an algorithm which assigns a score for each amino acid, at each position along a peptide. The score for each residue is taken as the ratio of the frequency of that residue in good and intermediate binders to the frequency of occurrence of that residue in non-binders.

In the present "Grouped Ratio" algorithm residues have been grouped by similarity. This avoids the problem encountered with some rare residues, such as tryptophan, where there are too few occurrences to obtain a statistically significant ratio. Table 16 is a listing of scores obtained by grouping for each of the twenty amino acids by position for 9-mer peptides containing perfect 2/9 motifs. A peptide is scored in the "Grouped Ratio" algorithm as a product of the scores of each of its residues. In the case of positions other than 2 and 9, the scores have been derived using a set of peptides which contain only preferred residues in positions 2 and 9. To enable us to extend our "Grouped Ratio" algorithm

**TABLE 16**

| | 1 | 2 | 3 | 4 | 5 | 6 | 7 | 8 | 9 |
|---|---|---|---|---|---|---|---|---|---|
| | | | | | | | | | |
| A | 2.6 | 0.03 | 0.87 | 0.87 | 0.65 | 0.87 | 4.4 | 0.29 | 0.16 |
| C | 0.73 | 0.01 | 1.9 | 4.8 | 0.87 | 1.2 | 1.2 | 1.1 | 0.01 |
| D | 0.10 | 0.01 | 0.10 | 0.65 | 0.29 | 0.65 | 0.11 | 0.87 | 0.01 |
| E | 0.10 | 0.01 | 0.10 | 0.65 | 0.29 | 0.65 | 0.11 | 0.87 | 0.01 |
| F | 7.0 | 0.01 | 5.2 | 0.87 | 8.7 | 2.0 | 2.3 | 2.6 | 0.01 |
| G | 3.5 | 0.01 | 0.44 | 1.1 | 1.1 | 1.3 | 0.44 | 0.44 | 0.01 |
| H | 3.1 | 0.01 | 0.22 | 1.0 | 0.87 | 0.09 | 0.10 | 1.3 | 0.01 |
| I | 3.1 | 0.14 | 1.8 | 0.55 | 0.87 | 1.4 | 1.2 | 1.8 | 0.40 |
| K | 3.1 | 0.01 | 0.22 | 1.0 | 0.87 | 0.09 | 0.10 | 1.3 | 0.01 |
| L | 3.1 | 1.00 | 1.8 | 0.55 | 0.87 | 1.4 | 1.2 | 1.8 | 0.09 |
| M | 3.1 | 2.00 | 1.8 | 0.55 | 0.87 | 1.4 | 1.2 | 1.8 | 0.06 |
| N | 0.50 | 0.01 | 0.37 | 1.2 | 0.87 | 1.1 | 0.65 | 0.33 | 0.01 |
| P | 0.12 | 0.01 | 0.70 | 0.73 | 2.6 | 1.8 | 2.9 | 0.10 | 0.01 |
| Q | 0.50 | 0.01 | 0.37 | 1.2 | 0.87 | 1.1 | 0.65 | 0.33 | 0.01 |
| R | 3.1 | 0.01 | 0.22 | 1.0 | 0.87 | 0.09 | 0.10 | 1.3 | 0.01 |
| S | 0.73 | 0.01 | 1.9 | 4.8 | 0.87 | 1.2 | 1.2 | 1.1 | 0.01 |
| T | 0.73 | 0.01 | 1.9 | 4.8 | 0.87 | 1.2 | 1.2 | 1.1 | 0.01 |
| V | 3.1 | 0.08 | 1.8 | 0.55 | 0.87 | 1.4 | 1.2 | 1.8 | 1.00 |
| W | 7.0 | 0.01 | 5.2 | 0.87 | 8.7 | 2.0 | 2.3 | 2.6 | 0.01 |
| Y | 7.0 | 0.01 | 5.2 | 0.87 | 8.7 | 2.0 | 2.3 | 2.6 | 0.01 |

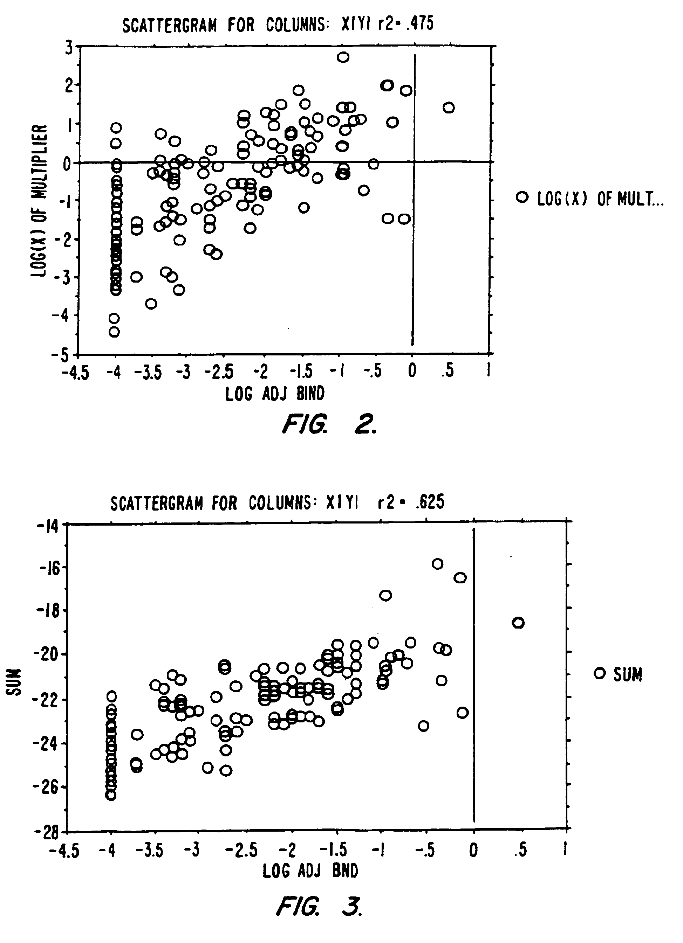

to peptides which may have residues other than the preferred ones at 2 and 9, scores for 2 and 9 have been derived from a set of peptides which are single amino acid substitutions at positions 2 and 9. Figure 2 shows a scattergram of the log of relative binding plotted against "Grouped Ratio" algorithm score for our collection of 9-mer peptides from the previous example.

The present "Grouped Ratio" algorithm can be used to predict a population of peptides with the highest occurrence of good binders. If one were to rely, for example, solely on a 2(L,M) and 9(V) motif for predicting A2.1 binding 9-mer peptides, it would have been predicted that all 160 peptides in our database would be good binders. In fact, as has already been described, only 12% of these peptides would be described as good binders and only 22% as intermediate binders; 66% of the peptides predicted by such a 2,9 motif are either weak or non-binding peptides. In contrast, using the "Grouped Ratio" algorithm described above, and selecting a score of 1.0 as threshold, 41 peptides were selected. Of this set, 27% are good binders, and 49% are intermediate, while only 20% are weak and 5% are non-binders (Table 17).

The present example of an algorithm has used the ratio of binders/non-binders to measure the impact of a particular residue at each position of a peptide. It is immediately apparent to one of ordinary skill that there are alternative ways of creating a similar algorithm.

An algorithm using the average binding affinity of all the peptides with a certain amino acid (or amino acid type) at a certain position has the advantage of including all of the peptides in the analysis, and not just good/intermediate binders and non-binders. Moreover, it gives a more quantitative measure of affinity than the simpler "Grouped Ratio" algorithm. We have created such an algorithm by calculating for each amino acid, by position, the average log of binding when that particular residue occurs in our set of 160 2,9 motif containing peptides. These values are shown in Table 18. The algorithm score for a peptide is then taken as the sum of the scores by position for each residues.
Figure 3 shows a scattergram of the log of relative binding against the average "Log of Binding" algorithm score. Table 17 shows the ability of the two algorithms to predict peptide binding at various levels, as a function of the cut-off score used. The ability of a 2,9 motif to predict binding in the same peptide set is also shown for reference purposes. It is clear from this comparison that both algorithms of this invention have a greater ability to predict populations with higher frequencies of good binders than a 2,9 motif alone. Differences between the "Grouped Ratio" algorithm and the "Log of Binding" algorithm are small in the set of peptides analyzed here, but do suggest that the "Log of Binding" algorithm is a better, if only slightly, predictor than the "Grouped Ratio" algorithm.

The log of binding algorithm was further revised in two ways. First, poly-alanine (poly-A) data were incorporated into the algorithms at the anchor positions for residues included in the expanded motifs where data obtained by screening a large library of peptides were not available. Second, an "anchor requirement screening filter" was incorporated into the algorithm. The poly-A approach is described in detail, above. The "anchor requirement screening filter" refers to the way in which residues are scored at the anchor positions, thereby providing the ability to screen out peptides which do not have preferred or tolerated residues in the anchor positions. This is accomplished by assigning a score for unacceptable residues at the anchor positions which are so high as to preclude any peptide which contains them from achieving an overall score which would allow it to be considered as a potential binder.

The results for 9-mers and 10-mers are presented in Tables 26 and 27, below. In these tables, values are group values as follows: A; G; P; D,E; R,H,K; L,I,V,M; F, Y, W; S,T,C; and Q,N, except where noted in the tables.

**TABLE 17**

| Criteria | Cut-off | Good Binders | Intermediate Binders | Weak Binders | Negative Binders | Totals |
|---|---|---|---|---|---|---|
| 2.9 motif | | 19 (12%) | 36 (22%) | 58 (36%) | 48 (30%) | 161 (100%) |
| | | | | | | |
| Grouped Ratio Algorithm | 1.5 | 5 (83%) | 1 (17%) | 0 (0%) | 0 (0%) | 6 (100%) |
| | 1.25 | 8 (67%) | 4 (33%) | 0 (0%) | 0 (0%) | 12 (100%) |
| | 1 | 10 (50%) | 9 (45%) | 1 (5%) | 0 (0%) | 20 (100%) |
| | 0.5 | 12 (32%) | 17 (46%) | 7 (19%) | 1 (3%) | 37 (100%) |
| | 0 | 12 (23%) | 26 (49%) | 12 (23%) | 3 (6%) | 53 (100%) |
| | -1 | 17 (18%) | 35 (37%) | 33 (35%) | 10 (11%) | 95 (100%) |
| | -2 | 19 (15%) | 36 (29%) | 50 (40%) | 21 (17%) | 126 (100%) |
| | -3 | 19 (13%) | 36 (24%) | 56 (38%) | 38 (26%) | 149 (100%) |
| | no cut | 19 (12%) | 36 (22%) | 58 (36%) | 48 (30%) | 161 (100%) |
| | | | | | | |
| Log of Binding Algorithm | -19 | 5 (100%) | 0 (0%) | 0 (0%) | 0 (0%) | 5 (100%) |
| | -20 | 8 (73%) | 3 (27%) | 0 (0%) | 0 (0%) | 11 (100%) |
| | -21 | 15 (43%) | 15 (43%) | 5 (14%) | 0 (0%) | 35 (100%) |
| | -22 | 17 (26%) | 27 (41%) | 21 (32%) | 1 (2%) | 68 (100%) |
| | -23 | 18 (19%) | 35 (37%) | 34 (36%) | 7 (7%) | 94 (100%) |
| | -24 | 18 (16%) | 36 (30%) | 47 (39%) | 17 (14%) | 119 (100%) |
| | -25 | 19 (14%) | 36 (26%) | 55 (39%) | 30 (21%) | 140 (100%) |
| | no cut | 19 (12%) | 36 (22%) | 58 (36%) | 48 (30%) | 161 (100%) |

**TABLE 18**

| | 1 | 2 | 3 | 4 | 5 | 6 | 7 | 8 | 9 |
|---|---|---|---|---|---|---|---|---|---|
| | | | | | | | | | |
| A | -2.38 | -3.22 | -2.80 | -2.68 | -2.89 | -2.70 | -2.35 | -3.07 | -2.49 |
| C | -2.94 | -4.00 | -2.58 | -1.96 | -3.29 | -2.22 | -2.97 | -2.37 | -4.00 |
| D | -3.69 | -4.00 | -3.46 | -2.71 | -2.26 | -2.63 | -3.61 | -3.03 | -4.00 |
| E | -3.64 | -4.00 | -3.51 | -2.65 | -3.39 | -3.41 | -3.21 | -2.63 | -4.00 |
| F | -1.89 | -4.00 | -2.35 | -2.50 | -1.34 | -2.43 | -2.18 | -1.71 | -4.00 |
| G | -2.32 | -4.00 | -3.04 | -2.63 | -2.56 | -2.30 | -3.13 | -2.96 | -4.00 |
| H | -2.67 | -4.00 | -2.58 | -2.58 | -2.05 | -3.32 | -3.13 | -2.16 | -4.00 |
| I | -1.65 | -2.55 | -2.80 | -3.44 | -2.74 | -2.79 | -2.20 | -2.69 | -2.10 |
| K | -2.51 | -4.00 | -3.65 | -2.93 | -3.34 | -3.77 | -3.13 | -3.27 | -4.00 |
| L | -2.32 | -1.70 | -2.02 | -2.49 | -2.71 | -2.63 | -2.62 | -2.01 | -2.74 |
| M | -0.39 | -1.39 | -1.79 | -3.07 | -3.43 | -1.38 | -1.33 | -0.97 | -2.96 |
| N | -3.12 | -4.00 | -3.52 | -2.22 | -2.36 | -2.30 | -3.14 | -3.31 | -4.00 |
| P | -3.61 | -4.00 | -2.97 | -2.64 | -2.42 | -2.31 | -1.83 | -2.42 | -4.00 |
| Q | -2.76 | -4.00 | -2.81 | -2.63 | -3.06 | -2.84 | -2.12 | -3.05 | -4.00 |
| R | -1.92 | -4.00 | -3.41 | -2.61 | -3.05 | -3.76 | -3.43 | -3.02 | -4.00 |
| S | -2.39 | -3.52 | -2.04 | -2.12 | -2.83 | -3.04 | -2.73 | -2.02 | -4.00 |
| T | -2.92 | -4.00 | -2.60 | -2.48 | -2.17 | -2.58 | -2.67 | -3.14 | -3.70 |
| V | -2.44 | -2.64 | -2.68 | -3.29 | -2.49 | -2.24 | -2.68 | -2.83 | -1.70 |
| W | -0.14 | -4.00 | -1.01 | -2.94 | -1.63 | -2.77 | -2.85 | -2.13 | -4.00 |
| X | -1.99 | -2.13 | -2.41 | -2.97 | -2.72 | -2.70 | -2.41 | -2.35 | -2.42 |
| Y | -1.46 | -4.00 | -1.67 | -2.70 | -1.92 | -2.39 | -1.35 | -3.37 | -4.00 |

### Example 7

### Use of an Algorithm to Predict Binding of 10-mer Peptides to HLA-A2.1

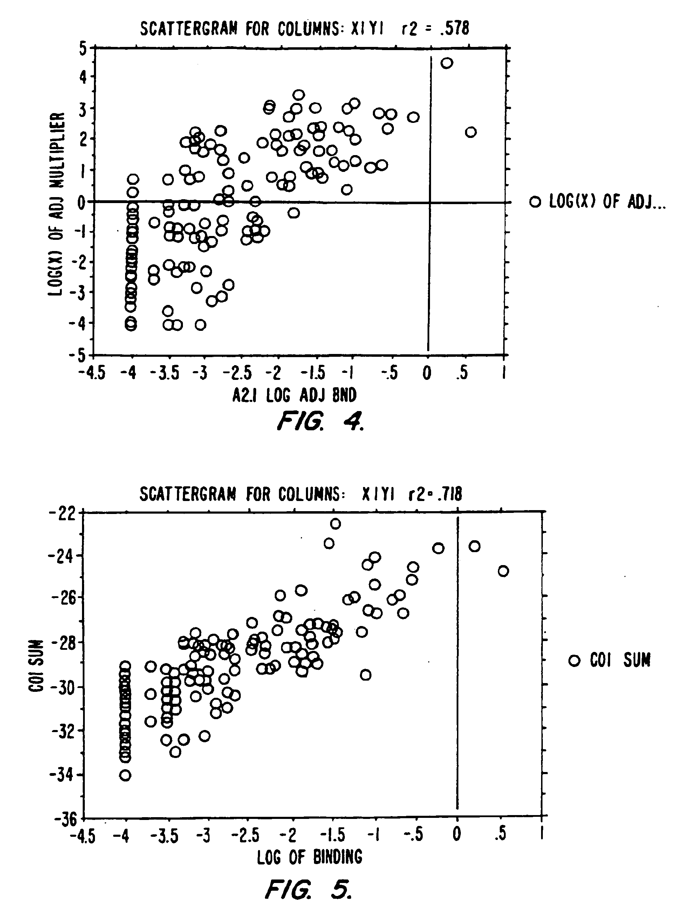

Using the methods described in the proceeding example, an analogous set of algorithms has been developed for predicting the binding of 10-mer peptides. Table 19 shows the scores used in a "Grouped Ratio" algorithm, and Table 20 shows the "Log of Binding" algorithm scores, for 10-mer peptides. Table 21 shows a comparison of the application of the two different algorithmic methods for selecting binding peptides. Figures 4 and 5 show, respectively, scattergrams of a set of 10-mer peptides containing preferred residues in positions 2 and 10 as scored by the "Grouped Ratio" and "Log of Binding" algorithms.

**TABLE 19**

| | 1 | 2 | 3 | 4 | 5 | 6 | 7 | 8 | 9 | 10 |
|---|---|---|---|---|---|---|---|---|---|---|
| | | | | | | | | | | |
| A | 3.00 | 0.01 | 3.10 | 0.20 | 1.60 | 0.60 | 1.30 | 1.60 | 0.50 | 0.01 |
| C | 0.90 | 0.01 | 0.90 | 1.10 | 1.00 | 0.90 | 1.40 | 1.30 | 2.90 | 0.01 |
| D | 0.01 | 0.01 | 0.20 | 0.60 | 0.30 | 1.00 | 0.30 | 0.01 | 0.40 | 0.01 |
| E | 0.01 | 0.01 | 0.20 | 0.60 | 0.30 | 1.00 | 0.30 | 0.01 | 0.40 | 0.01 |
| F | 3.00 | 0.01 | 2.60 | 3.10 | 3.60 | 0.60 | 1.60 | 14.1 | 2.10 | 0.01 |
| G | 0.80 | 0.01 | 0.50 | 4.70 | 0.80 | 6.30 | 2.70 | 0.70 | 0.80 | 0.01 |
| H | 1.20 | 0.01 | 0.30 | 0.10 | 0.70 | 0.40 | 0.20 | 0.01 | 0.20 | 0.01 |
| I | 3.00 | 0.50 | 10.2 | 1.00 | 1.30 | 2.10 | 1.40 | 4.70 | 0.80 | 1.00 |
| K | 1.20 | 0.01 | 0.30 | 0.10 | 0.70 | 0.40 | 0.20 | 0.01 | 0.20 | 0.01 |
| L | 3.00 | 1.10 | 10.2 | 1.00 | 1.30 | 2.10 | 1.40 | 4.70 | 0.80 | 0.50 |
| M | 3.00 | 0.60 | 10.2 | 1.00 | 1.30 | 2.10 | 1.40 | 4.70 | 0.80 | 0.01 |
| N | 1.00 | 0.01 | 0.90 | 0.80 | 0.80 | 0.80 | 0.60 | 0.40 | 0.70 | 0.01 |
| P | 0.00 | 0.01 | 0.40 | 2.60 | 0.01 | 1.00 | 0.40 | 1.90 | 1.20 | 0.01 |
| Q | 1.00 | 0.01 | 0.90 | 0.80 | 0.80 | 0.80 | 0.60 | 0.40 | 0.70 | 0.01 |
| R | 1.20 | 0.01 | 0.30 | 0.10 | 0.70 | 0.40 | 0.20 | 0.01 | 0.20 | 0.01 |
| S | 0.90 | 0.01 | 0.90 | 1.10 | 1.00 | 0.90 | 1.40 | 1.30 | 2.90 | 0.01 |
| T | 0.90 | 0.01 | 0.90 | 1.10 | 1.00 | 0.90 | 1.40 | 1.30 | 2.90 | 0.01 |
| V | 3.00 | 0.10 | 10.2 | 1.00 | 1.30 | 2.10 | 1.40 | 4.70 | 0.80 | 2.30 |
| W | 3.00 | 0.01 | 2.60 | 3.10 | 3.60 | 0.60 | 1.60 | 14.1 | 2.10 | 0.01 |
| Y | 3.00 | 0.01 | 2.60 | 3.10 | 3.60 | 0.60 | 1.60 | 14.1 | 2.10 | 0.01 |

**TABLE 20**

| | 1 | 2 | 3 | 4 | 5 | 6 | 7 | 8 | 9 | 10 |
|---|---|---|---|---|---|---|---|---|---|---|
| | | | | | | | | | | |
| A | -2.40 | -4.00 | -2.54 | -3.42 | -3.07 | -3.30 | -2.98 | -2.69 | -3.29 | -4.00 |
| C | -3.64 | -4.00 | -2.47 | -2.48 | -1.78 | -3.94 | -1.28 | -3.10 | -2.43 | -4.00 |
| D | -3.65 | -4.00 | -2.76 | -3.26 | -2.76 | -3.03 | -3.43 | -3.68 | -3.63 | -4.00 |
| E | -3.92 | -4.00 | -3.63 | -3.34 | -3.73 | -2.82 | -3.54 | -3.71 | -2.95 | -4.00 |
| F | -1.52 | -4.00 | -1.96 | -3.03 | -2.01 | -3.11 | -2.67 | -1.61 | -2.43 | -4.00 |
| G | -2.91 | -4.00 | -3.40 | -2.63 | -2.98 | -2.45 | -2.52 | -3.18 | -3.03 | -4.00 |
| H | -3.61 | -4.00 | -3.10 | -3.03 | -2.33 | -2.99 | -3.70 | -3.55 | -4.00 | -4.00 |
| 1 | -2.26 | -4.00 | -2.82 | -3.05 | -2.38 | -2.61 | -2.38 | -3.34 | -3.18 | -1.47 |
| K | -2.53 | -4.00 | -3.65 | -3.42 | -3.14 | -3.58 | -3.50 | -3.53 | -4.00 | -4.00 |
| L | -2.00 | -2.93 | -2.21 | -2.48 | -2.88 | -2.53 | -2.57 | -1.83 | -3.23 | -3.20 |
| M | -2.41 | -3.11 | -2.00 | -3.33 | -3.70 | -2.56 | -3.27 | -2.25 | -3.00 | -4.00 |
| N | -3.21 | -4.00 | -3.09 | -2.61 | -2.93 | -2.89 | -3.52 | -3.01 | -2.88 | -4.00 |
| P | -3.90 | -4.00 | -3.21 | -2.27 | -3.72 | -3.06 | -3.35 | -2.58 | -2.94 | -4.00 |
| Q | -2.92 | -4.00 | -2.97 | -4.00 | -2.98 | -3.46 | -2.20 | -3.23 | -3.45 | -4.00 |
| R | -3.01 | -4.00 | -3.44 | -3.50 | -3.23 | -3.32 | -3.72 | -3.59 | -2.97 | -4.00 |
| S | -2.47 | -4.00 | -3.17 | -3.11 | -3.23 | -2.64 | -3.19 | -2.79 | -2.26 | -4.00 |
| T | -3.59 | -4.00 | -3.07 | -2.88 | -2.89 | -3.16 | -2.43 | -3.11 | -2.58 | -4.00 |
| V | -2.97 | -4.00 | -2.46 | -3.14 | -3.27 | -2.53 | -3.14 | -3.02 | -2.90 | -2.61 |
| W | -2.10 | -4.00 | -2.72 | -1.79 | -2.65 | -1.92 | -1.80 | -2.24 | -2.11 | -4.00 |
| Y | -2.37 | -4.00 | -2.42 | -2.85 | -3.03 | -3.76 | -2.82 | -2.34 | -2.74 | -4.00 |

**TABLE 21**

| Criteria | Cut-off | Good | Intermediate | Weak | Negative | Totals |
|---|---|---|---|---|---|---|
| 2,10 motif | | 10 (6%) | 29 (17%) | 70 (41%) | 61 (36%) | 170 (100%) |
| | | | | | | |
| Grouped Ratio Algorithm | 4 | 1 (100%) | 0 (0%) | 0 (0%) | (0%) | 1 (100%) |
| | 3 | 1 (25%) | 2 (50%) | 1 (25%) | 0 (0%) | 4 (100%) |
| | 2 | 6 (24%) | 13 (52%) | 6 (24%) | 0 (0%) | 25 (100%) |
| | 1 | 10 (21%) | 21 (45%) | 16 (34%) | 0 (0%) | 47 (100%) |
| | 0 | 10 (15%) | 28 (42%) | 26 (39%) | 2 (3%) | 66 (100%) |
| | -1 | 10 (11%) | 29 (32%) | 42 (46%) | 11 (12%) | 92 (100%) |
| | -2 | 10 (9%) | 29 (25%) | 54 (47%) | 23 (20%) | 116 (100%) |
| | -3 | 10 (7%) | 29 (22%) | 63 (47%) | 32 (24%) | 134 (100%) |
| | no cut | 10 (6%) | 29 (17%) | 70 (41%) | 61 (36%) | 170 (100%) |
| | | | | | | |
| Log of Binding Algorithm | -24 | 2 (50%) | 2 (50%) | 0 (0%) | 0 (0%) | 4 (100%) |
| | -25 | 5 (56%) | 3 (33%) | 1 (11%) | 0 (0%) | 9 (100%) |
| | -26 | 7 (47%) | 5 (33%) | 3 (20%) | 0 (0%) | 15 (100%) |
| | -27 | 10 (32%) | 9 (29%) | 12 (39%) | 0 (0%) | 31 (100%) |
| | -28 | 10 (17%) | 19 (33%) | 29 (50%) | 0 (0%) | 58 (100%) |
| | -29 | 10 (12%) | 25 (30%) | 48 (58%) | 0 (0%) | 83 (100%) |
| | -30 | 10 (10%) | 29 (28%) | 59 (57%) | 5 (5%) | 103 (100%) |
| | -31 | 10 (8%) | 28 (22%) | 66 (51%) | 24 (19%) | 129 (100%) |
| | -32 | 10 (7%) | 29 (19%) | 70 (47%) | 40 (27%) | 149 (100%) |
| | no cut | 10 (6%) | 29 (17%) | 70 (41%) | 61 (36%) | 170 (100%) |

### Example 8

### Binding of A2.1 Algorithm Predicted Peptides

The results of Examples 6 and 7 indicate that an algorithm can be used to select peptides that bind to HLA-A2.1 sufficiently to have a high probability of being immunogenic.

To test this result, we tested our algorithm on a large (over 1300) non-redundant, independent set of peptides derived from various sources. After scoring this set with our algorithm, we selected 41 peptides (Table 21) for synthesis, and tested them for A2.1 binding. This set of peptides was comprised of 21 peptides with high algorithm scores, and 20 peptides with low algorithm scores.

The binding data and categorization profile are shown in Tables 22 and 23 respectively. The correlation between binding and algorithm score was 0.69. It is immediately apparent from Table 23 the striking difference between peptides with high algorithm scores, and those with low algorithm scores. Respectively, 76% of the high scorers and none of the low scorers were either good or intermediate binders. This data demonstrates the utility of the algorithm of this invention.

**TABLE 22**

| SEQUENCE | SOURCE | A2.1 Binding | Algorithm Score |
|---|---|---|---|
| | | | |
| MMWFVVLTV | CMV | 0.76 | 346 |
| YLLLYFSPV | CMV | 0.75 | 312 |
| YLYRLNFCL | CMV | 0.72 | 169 |
| FMWTYLVTL | CMV | 0.68 | 336 |
| LLWWITILL | CMV | 0.49 | 356 |
| GLWCVLFFV | CMV | 0.47 | 1989 |
| LMIRGVLEV | CMV | 0.45 | 296 |
| LLLCRLPFL | CMV | 0.42 | 1356 |
| RLLTSLFFL | HSV | 0.34 | 859 |
| LLLYYDYSL | HSV | 0.28 | 390 |
| AMSRNLFRV | CMV | 0.15 | 1746 |
| AMLTACVEV | CMV | 0.089 | 411 |
| RLQPNVPLV | CMV | 0.048 | 392 |
| VLARTFTPV | CMV | 0.044 | 196 |
| RLLRGURL | CMV | 0.037 | 494 |
| WMWFPSVLL | CMV | 0.036 | 362 |
| YLCCGITLL | CMV | 0.021 | 1043 |
| DMLGRVFFV | HSV | 0.011 | 1422 |
| ALGRYQQLV | CMV | 0.0089 | 184 |
| LMPPPVAEL | CMV | 0.0066 | 416 |
| LMCRYTPRL | CMV | 0.0055 | 414 |
| RLTWRLTWL | CMV | 0.0052 | 250 |
| AMPRRVLHV | CMV | 0.0014 | 628 |
| ALLLVLALL | CMV | 0.0014 | 535 |
| AMSGTGTTL | CMV | 0.0005 | 602 |
| MLNVMKEAV | CMV | 0.0039 | 0.00031 |
| TMELMIRTV | CMV | 0.0029 | 0.0013 |
| TLAAMHSKL | HSV | 0.0008 | 0.0019 |
| TLNIVRDHV | CMV | 0.0005 | 0.00021 |
| ELSIFRERL | HSV | 0.0002 | 0.0020 |
| FLRVQQKAL | HSV | 0.0002 | 0.00099 |
| ELQMMQDWV | CMV | 0.0001 | 0.0020 |
| QLNAMKPDL | MT | 0.0001 | 0.0017 |
| GLRQLKGAL | CMV | 0.0001 | 0.0010 |
| TLRMSSKAV | HSV | 0.0001 | 0.00085 |
| SLRIKRELL | CMV | 0 | 0.00041 |
| DLKQMERVV | CMV | 0 | 0.00026 |
| PLRVTPSDL | CMV | 0 | 0.0019 |
| QLDYEKQVL | CMV | 0 | 0.0012 |
| WLKLLRDAL | CMV | 0 | 0.0012 |
| PMEAVRHPL | CMV | 0 | 0.0011 |
| ELKQTRVNL | CMV | 0 | 0.00053 |
| NLEVIHDAL | CMV | 0 | 0.00050 |
| ELKKVKSVL | HSV | 0 | 0.00033 |
| PLAYERDKL | CMV | 0 | 0.00017 |

**TABLE 23**

| Set | Good Binders | Intermediate Binders | Weak Binders | Negative Binders | Totals |
|---|---|---|---|---|---|
| HI Scorers | 11 (52.4%) | 5 (23.8%) | 5 (23.8%) | 0 (0.0%) | 21 (100%) |
| Low Scorers | 0 (0.0%) | C (0.0%) | 10 (50.0%) | 10 (50.0%) | 20 (100%) |
| Totals | 11 (26.6%) | 5 (12.2%) | 15 (36.6%) | 10 (24.4%) | 41 (100%) |

### Example 9

### Ex vivo induction of Cytotoxic T Lymphocytes (CTL)

Peripheral blood mononuclear cells (PBMC) are isolated from an HLA-typed patient by either venipuncture or apheresis (depending upon the initial amount of CTLp required), and purified by gradient centrifugation using Ficoll-Paque (Pharmacia). Typically, one can obtain one million PBMC for every ml of peripheral blood, or alternatively, a typical apheresis procedure can yield up to a total of 1-10 X 10¹⁰ PBMC.

The isolated and purified PBMC are co-cultured with an appropriate number of antigen presenting cell (APC), previously incubated ("pulsed") with an appropriate amount of synthetic peptide (containing the HLA binding motif and the sequence of the antigen in question). PBMC are usually incubated at 1-2 X 10⁶ cells/ml in culture medium such as RPMI-1640 (with autologous serum or plasma) or the serum-free medium AIM-V (Gibco).

APC are usually used at concentrations ranging from 1X10⁴ to 2X10⁵ cells/ml, depending on the type of cell used. Possible sources of APC include: 1) autologous dendritic cells (DC), which are isolated from PBMC and purified as described (Inaba, et al. , J. Exp. Med. 166:182 (1987)) ; and 2) mutant and genetically engineered mammalian cells that express "empty" HLA molecules (which are syngeneic [genetically identical] to the patient's allelic HLA form), such as the, mouse RMA-S cell line or the human T2 cell line. APC containing empty HLA molecules are known to be potent inducers of CTL responses, possibly because the peptide can associate more readily with empty MHC molecules than with MHC molecules which are occupied by other peptides (DeBruijn, et al., Eur. J. Immunol. 21:2963-2970 (1991)).

In those cases when the APC used are not autologous, the cells will have to be gamma irradiated with an appropriate dose (using, e.g., radioactive cesium or cobalt) to prevent their proliferation both ex vivo, and when the cells are re-introduced into the patients.

The mixture cultures, containing PBMC, APC and peptide are kept in an appropriate culture vessel such as plastic T-flasks, gas-permeable plastic bags, or roller bottles, at 37° centigrade in a humid air/CO₂ incubator. After the activation phase of the culture, which usually occurs during the first 3-5 days, the resulting effector CTL can be further expanded, by the addition of recombinant DNA-derived growth factors such as interleukin-2 (IL-2), interleukin-4 (IL-4), or interleukin-7 (IL-7) to the cultures. An expansion culture can be kept for an additional 5 to 12 days, depending on the numbers of effector CTL required for a particular patient. In addition, expansion cultures may be performed using hollow fiber artificial capillary systems (Cellco), where larger numbers of cells (up to 1X10¹¹) can be maintained.

Before the cells are infused into the patient, they are tested for activity, viability, toxicity and sterility. The cytotoxic activity of the resulting CTL can be determined by a standard ^{5I}Cr-release assay (Biddison, W.E. 1991, Current Protocols in Immunology, p7,17.1-7.17.5, Ed. J. Coligan et al., J. Wiley and Sons, New York), using target cells that express the appropriate HLA molecule, in the presence and absence of the immunogenic peptide. Viability is determined by the exclusion of trypan blue dye by live cells. Cells are tested for the presence of endotoxin by conventional techniques. Finally, the presence of bacterial or fungal contamination is determined by appropriate microbiological methods (chocolate agar, etc.). Once the cells pass all quality control and safety tests, they are washed and placed in the appropriate infusion solution (Ringer/glucose lactate) and infused intravenously into the patient.

### Example 10

### Assays for CTL Activity

1. Peptide synthesis: Peptide syntheses were carried out by sequential coupling of N-α-Fmoc-protected amino acids on an Applied Biosystems (Foster City, CA) 430A peptide synthesizer using standard Fmoc coupling cycles (software version 1.40). All amino acids, reagents, and resins were obtained from Applied Biosystems or Bachem. Solvents were obtained from Burdick & Jackson. Solid-phase synthesis was started from an appropriately substituted Fmoc-amino acid-Sasrin resin. The loading of the starting resin was 0.5-0.7 mmol/g polystyrene, and 0.1 or 0.25 meq were used in each synthesis. A typical reaction cycle proceeded as follows: 1) The N-terminal Fmoc group was removed with 25% piperidine in dimethylformamide (DMF) for 5 minutes, followed by another treatment with 25% piperdine in DMF for 15 minutes. The resin was washed 5 times with DMF. An N-methylpyrolidone (NMP) solution of a 4 to 10 fold excess of a pre-formed 1-hydroxybenzotriazole ester of the appropriate Fmoc-amino acid was added to the resin and the mixture was allowed to react for 30-90 min. The resin was washed with DMF in preparation for the next elongation cycle. The fully protected, resin bound peptide was subjected to a piperidine cycle to remove the terminal Fmoc group. The product was washed with dichloromethane and dried. The resin was then treated with trifluoroacetic acid in the presence of appropriate scavengers [e.g. 5% (v/v) water] for 60 minutes at 20 °C. After evaporation of excess trifluoroacetic acid, the crude peptide was washed with dimethyl ether, dissolved in water and lyophilized. The peptides wee purified to >95% homogeneity by reverse-phase HPLC using H₂O/CH₃CN gradients containing 0.2% TFA modifier on a Vydac, 300Å pore-size, C-18 preparative column. The purity of the synthetic peptides was assayed on an analytical reverse-phase column, and their composition ascertained by amino acid analysis and/or sequencing. Peptides were routinely dissolved in DMSO at the concentration of 20 mg/ml.
2. Media: RPMI-1640 containing 10% fetal calf serum (FCS) 2 mM Glutamine, 50 µg/ml Gentamicin and 5x10⁻⁵M 2-mercaptoethanol served as culture medium and will be referred to as R10 medium.
RPMI-1640 containing 25 mM Hepes buffer and supplemented with 2% FCS was used as cell washing medium.
3. Rat Concanavalin A supernatant: The spleen cells obtained from Lewis rats (Sprague-Dawley) were resuspended at a concentration of 5x10⁶ cells/ml in R¹⁰ medium supplemented with 5 µg/ml of ConA in 75 cm2 tissue culture flasks. After 48 hr at 37°C, the supernatants were collected, supplemented with 1% α-methyl-D-mannoside and filter sterilized (.45 µm filter). Aliquots were stored frozen at -20°C.
4. LPS-activated lymphoblasts: Murine splenocytes were resuspended at a concentration of 1-1.5x10⁶/ml in R10 medium supplemented with 25 µg/ml LPS and 7 µg/ml dextran sulfate in 75 cm² tissue culture flasks. After 72 hours at 37°C, the lymphoblasts were collected for use by centrifugation.
5. Peptide coating of lymphoblasts: Coating of the LPS activated lymphoblasts was achieved by incubating 30x10⁶ lymphoblasts with 100 µg of peptide in 1 ml of R10 medium for 1 hr at 37°C. Cells were then washed once and resuspended in R10 medium at the desired concentration for use in in vitro CTL activation.
6. Peptide coating of Jurkat A2/K^{b} cells: Peptide coating was achieved by incubating 10x10⁶ irradiated (20,000 rads) Jurkat A2.1/K^{b} cells with 20 µg of peptide in 1 ml of R10 medium for 1 hour at 37°C. Cells were washed three times and resuspended at the required concentration in R10 medium.
7. In Vitro CTL activation: One to four weeks after priming spleen cells (5 x 10⁶ cells/well or 30x10⁶ cells/T25 flask) were concultured at 37°C with syngeneic, irradiated (3,000 rads), peptide coated lymphoblasts (2x10⁶ cells/well or 10x10⁶ cells/T25 flask) in R10 medium to give a final volume of 2 ml in 24-well plates or 10 ml in T25 flasks.
8. Restimulation of effector cells: Seven to ten days after the initial in vitro activation, described in paragraph 7 above, a portion of the effector cells were restimulated with irradiated (20,000 rads), peptide-coated Jurkat A2/K^{b} cells (0.2x10⁶ cells/well) in the presence of 3x10⁶ "feeder cells"/well (C57B1/6 irradiated spleen cells) in R10 medium supplemented with 5% rat ConA supernatant to help provide all of the cytokines needed for optimal effector cell growth.
9. Assay for cytotoxic activity: Target cells (3x10⁶) were incubated at 37°C in the presence of 200 µl of sodium ⁵¹Cr chromate. After 60 minutes, cells were washed three times and resuspended in R10 medium. Peptides were added at the required concentration. For the assay, 10^{4 51}Cr-labeled target cells wee added to different concentrations of effector cells (final volume of 200 µl) in U-bottom 96-2311 plates. After a 6-hour incubation period at 37°C, 0.1 ml aliquots of supernatant were removed from each well and radioactivity was determined in a Micromedic automatic gamma counter. The percent specific lysis was determined by the formula: percent specific release = 100x(experimental release - spontaneous release)/(maximum release - spontaneous release). Where peptide titrations wee performed, the antigenicity of a given peptide (for comparison purposes) was expressed as the peptide concentration required to induce 40% specific ⁵¹Cr release at a given E:T.
Transgenic mice were injected subcutaneously in the base of the tail with an incomplete Freund's adjuvant emulsion containing 50 nM of the putative CTL epitopes containing the A2.1 motifs, and 50 nM of a hepatitis B core T helper epitope. Eight to 20 days later, animals were sacrificed and spleen cells were restimulated in vitro with syngeneic LPS lymphoblasts coated with the putative CTL epitope. A source of IL-2 (rat con A supernatant) was added at day 6 of the assay to a final concentration of 5% and CTL activity was measured on day 7. The capacity of these effector T cells to lyse peptide-coated target cells that express the A2 KB molecule (Jurkat A2 KB) was measured as lytic units. The results are presented in Table 24.
The results of this experiment indicate that those peptides having a binding of at least 0.01 are capable of inducing CTL. All of the peptides in Appendices 1 and 2 having a binding of at least about 0.01 would be immunogenic.

**TABLE 24**

| Binding and Immunogenicity HBV Polymerase (ayw) | | | |
|---|---|---|---|
| Peptide | Binding** | CTL Activity | Algorithm |
| 1 2 3 4 5 6 7 8 9 | | | |
| F L L S L G I H L | 0.52 | 63 | -20.8 |
| G L Y S S T V P V | 0.15 | 10 | -21.9 |
| H L Y S H P I I L | 0.13 | 10 | -21.1 |
| W I L R G T S F V | 0.018 | -+ | -20.9 |
| N L S W L S L D V | 0.013 | 6 | -24.7 |
| L L S S N L S W L | 0.005 | - | -21.7 |
| N L Q S L T N L L | 0.003 | - | -23.9 |
| H L L V G S S G L | 0.002 | - | -24.7 |
| L L D D E A G P L | 0.0002 | - | -25.5 |
| P L E E E L P R L | 0.0001 | - | -26.1 |
| D L N L G N L N V | -* | - | -25.7 |
| N L Y V S L L L L | - | - | -23.6 |
| P L P I H T A E L | - | - | -25.04 |

| | | | |
|---|---|---|---|
| *-=<0.0001 ** Relative blinding capacity compared to std with IC₅₀ = 52mM xxx Lytic units/10⁶ cells; 1 lytic unit = the number of effector cells required to give 30% Cr⁵¹ release. -,-+ no measurable cytotoxic activity. | | | |

### Example 11

### Identification of immunogenic peptides

Using the motifs identified above for HLA-A2.1 allele amino acid sequences from a tumor-related protein, Melanoma Antigen-1 (MAGE-1), were analyzed for the presence of these motifs. Sequences for the target antigen are obtained from the GenBank data base (Release No. 71.0; 3/92). The identification of motifs is done using the "FINDPATTERNS" program (Devereux et al., Nucleic Acids Research 12:387-395 (1984) ) .

Other viral and tumor-related proteins can also be analyzed for the presence of these motifs. The amino acid sequence or the nucleotide sequence encoding products is obtained from the GenBank database in the cases of Human Papilloma Virus (HPV), Prostate Specific antigen (PSA), p53 oncogene, Epstein Barr Nuclear Antigen-1 (EBNA-1), and c-erb2 oncogene (also called HER-2/neu).

In the cases of Hepatitis B Virus (HBV), Hepatitis C Virus (HCV), and Human Immunodeficiency Virus (HIV) several strains/isolates exist and many sequences have been placed in GenBank.

For HBV, binding motifs are identified for the adr, adw and ayw types. In order to avoid replication of identical sequences, all of the adr motifs and only those motifs from adw and ayw that are not present in adr are added to the list of peptides.

In the case of HCV, a consensus sequence from residue 1 to residue 782 is derived from 9 viral isolates. Motifs are identified on those regions that have no or very little (one residue) variation between the 9 isolates. The sequences of residues 783 to 3010 from 5 viral isolates were also analyzed. Motifs common to all the isolates are identified and added to the peptide list.

Finally, a consensus sequence for HIV type 1 for North American viral isolates (10-12 viruses) was obtained from the Los Alamos National Laboratory database (May 1991 release) and analyzed in order to identify motifs that are constant throughout most viral isolates. Motifs that bear a small degree of variation (one residue, in 2 forms) were also added to the peptide list.

Appendices 1 and 2 provide the results of searches of the following antigens cERB2, EBNA1, HBV, HCV, HIV, HPV, MAGE, p53, and PSA. Only peptides with binding affinity of at least 1% as compared to the standard peptide in assays described in Example 5 are presented. Binding as compared to the standard peptide is shown in the far right column. The column labeled "Pos." indicates the position in the antigenic protein at which the sequence occurs.

### Example 12

### Identification of immunogenic peptides

Using the motifs disclosed here, amino acid sequences from various antigens were screened for further motifs. Screening was carried out as described in Example 11. Tables 25 and 26 provide the results of searches of the following antigens cERB2, CMV, Influenza A, HBV, HIV, HPV, MAGE, p53, PSA, Hu S3 ribosomal protein, LCMV, and PAP. Only peptides with binding affinity of at least 1% as compared to the standard peptide in assays described in Example 5 are presented. Binding as compared to the standard peptide is shown for each peptide.

**TABLE 25**

| Sequence | Antigen | Molecule | A2 Bind. |
|---|---|---|---|
| KIFGSLAFL | c-ERB2 | | 0.1500 |
| RILHNGAYSL | c-ERB2 | | 0.0180 |
| IISAVVGILL | c-ERB2 | | 0.0120 |
| MMWFVVLTV | CMV | | 0.7600 |
| YLLLYFSPV | CMV | | 0.7500 |
| YLYRLNFCL | CMV | | 0.7200 |
| FMWTYLVTL | CMV | | 0.6800 |
| LLWWITILL | CMV | | 0.4900 |
| GLWCVLFFV | CMV | | 0.4700 |
| LMIRGVLEV | CMV | | 0.4500 |
| LLLCRLPFL | CMV | | 0.4200 |
| AMSRNLFRV | CMV | | 0.1500 |
| AMLTACVBV | CMV | | 0.1000 |
| RLQPNVPLV | CMV | | 0.0480 |
| VLARTFTPV | CMV | | 0.0440 |
| RLLRGLIRL | CMV | | 0.0370 |
| WMWFPSVLL | CMV | | 0.0360 |
| YLCCGITLL | CMV | | 0.0210 |
| SLLTEVETYV | FLU-A | M1 | 0.0650 |
| LLTEVETYV | FLU-A | M1 | 0.2000 |
| LLTEVETYVL | FLU-A | M1 | 0.0130 |
| GILGFVFTL | FLU-A | M1 | 0.1900 |
| GILGFVFTLT | FLU-A | M1 | 0.0150 |
| ILGFVFTLT | FLU-A | M1 | 0.2600 |
| ILGFVFTLTV | FLU-A | M1 | 0.0550 |
| ALASCMGLI | FLU-A | M1 | 0.0110 |
| RMGAVTTEV | FLU-A | M1 | 0.0200 |
| VTTEVAFGL | FLU-A | M1 | 0.0360 |
| MVTTTNPLI | FLU-A | M1 | 0.0150 |
| FTFSPTYKA | HBV | POL | 0.0190 |
| YLHTLWKAGI | HBV | POL | 0.0280 |
| LMLQAGFFLV | HBV(a) | ENV(a) | 0.6300 |
| RMLTIPQSV | HBV(a) | ENV(a) | 0.0580 |
| SLDSWWTSV | HBV(a) | ENV(a) | 0.1000 |
| FMLLLCLIFL | HBV(a) | ENV(a) | 0.0450 |
| LLPFVQWFV | HBV(a) | ENV(a) | 0.6500 |
| LMPFVQWFV | HBV(a) | ENV(a) | 0.8300 |
| FLGLSPTVWV | HBV(a) | ENV(a) | 0.0300 |
| SMLSPFLPLV | HBV(a) | ENV(a) | 0.9700 |
| GLWIRTPPV | HBV(a) | ENV(a) | 0.3600 |
| NLGNLNVSV | HBV(a) | ENV(a) | 0.0160 |
| YLHTLWKAGV | HBV(a) | POL(a) | 0.1500 |
| RLTGGVFLV | HBV(a) | POL(a) | 0.1600 |
| RMTGGVFLV | HBV(a) | POL(a) | 0.1500 |
| RLTGGVFLV | HBV(a) | ENV(a) | 0.1600 |
| ILGLLGFAV | HBV(a) | ENV(a) | 0.0600 |
| GLCQVFADV | HBV(a) | ENV(a) | 0.0300 |
| WLLRGTSFV | HBV(a) | ENV(a) | 0.1000 |
| YLPSALNPV | HBV(a) | ENV(a) | 0.3200 |
| LLVPFVQWFA | HBV adr | | 0.2600 |
| FLPSDFFPSI | HBV adr | | 0.2100 |
| VVSYVNVNM | HBV adr | | 0.0100 |
| HLPDRVHFA | HBV adr | | 0.0160 |
| SLAFSAVPA | HBV adr | | 0.0340 |
| FLLTKILTI | HBV adw | | 0.6300 |
| SLYNILSPFM | HBV adw | | 0.0440 |
| CLFHIVNLI | HBV adw | | 0.2100 |
| RLPDRVHFA | HBV adw | | 0.0940 |
| ALPPASPSA | HBV adw | | 0.0710 |
| GLLGWSPQA | HBV ayw | | 0.8650 |
| FLGPLLVLQA | HBV ayw | | 0.0190 |
| FLLTRILTI | HBV ayw | | 0.9300 |
| GMLPVCPLI | HBV ayw | | 0.0520 |
| QLFHLCLII | HBV ayw | | 0.0390 |
| KLCLGWLWGM | HBV ayw | | 0.0210 |
| LLWFFIISCLI | HBV ayw | | 0.0130 |
| YLVSFGVWI | HBV ayw | | 2.7000 |
| LLEDWGPCA | HBV ayw | | 0.0180 |
| KLHLYSHPI | HBV ayw | | 0.2900 |
| FLLAQFTSA | HBV ayw | | 0.6600 |
| LLAQFTSAI | HBV ayw | | 9.6000 |
| YMDDWLGA | HBV ayw | | 0.1600 |
| ALMPLYACI | HBV ayw | | 0.2000 |
| GLCQVFADA | HBV ayw | | 0.0180 |
| HLPDLVHFA | HBV ayw | | 0.1100 |
| RLCCQLDPA | HBV ayw | | 0.0290 |
| ALMPLYACI | HBV ayw polymerase | | 0.5000 |
| FLCKQYLNL | HBV ayw polymerase 665-673 | | 0.0210 |
| SLYADSPSV | HBV polymerase | | 0.3500 |
| ALMPLYASI | HBV polymerase | | 0.0760 |
| NINNLNVSI | HBV polymerase | | 0.0660 |
| ALSLIVNLL | HBV polymerase | | 0.0470 |
| KLHLYSHPI | HBV polymerase | | 0.2900 |
| WILRGTSFV | HBV polymerase 1344-1352 | | 0.0270 |
| LVLQAGFFLL | HBVadr | ENV | 0.0150 |
| FILLLCLIFL | HBVadr | ENV | 0.0280 |
| WILRGTSFV | HBVadr | POL | 0.0180 |
| IISCTCPTV | HBVadw | PreCore | 0.0190 |
| LVPFVQWFV | HBVadw | ENV | 0.0200 |
| LIISCSCPTV | HBVadw | CORE | 0.0290 |
| FLPSDFFPSI | HBVayr | PreCore | 0.2100 |
| LLCLGWLWGM | HBVayr | PreCore | 0.0220 |
| QLFHLCLII | HBVayw | PreCore | 0.0390 |
| CLGWLTGMDI | HBVayw | PreCore | 0.0190 |
| FLGGTTVCL | HBVayw | ENV | 0.1700 |
| SLYSILSPFL | HBVayw | ENV | 0.2000 |
| FLPSDFFPSV | HBVayw | CORE | 1.5000 |
| ILCWGELMTL | HBVayw | CORE | 0.1900 |
| LMTLATWVGV | HBVayw | CORE | 0.6800 |
| TLATWVGVNL | HBVayw | CORE | 0.5700 |
| GLSRYVARL | HBVayw | POL | 0.1200 |
| FLCKQYLNL | HBVayw | POL | 0.1700 |
| RMRGTFSAPL | HBVayw | POL | 0.0110 |
| SLYADSPSV | HBVayw | POL | 0.3500 |
| YLYGVGSAV | HCV | | 0.1600 |
| LLSTTEWQV | HCV | | 0.0480 |
| IIGAETFYV | HIV | POL | 0.0260 |
| QLWVTVYYGV | HIV | ENV | 0.0250 |
| NLWVTVYYGV | HIV | ENV | 0.0160 |
| KLWVTVYYGV | HIV | ENV | 0.0150 |
| KLWVTVYYGV | HIV.MN gp160 | | 0.0150 |
| YMLDLQPET | HPV16 | E7 | 1.4000 |
| TLGIVCPI | HPV16 | E7 | 0.6500 |
| YLLDLQEPV | HPV16(a) | E7 (a) | 0.2200 |
| YMLDLQPEV | HPV16(a) | E7 (a) | 1.9000 |
| MLDLQPETT | HPV16E7 | E7 | 0.0130 |
| SLQDIEITCVYCKTV | HPV18 | E6 | 0.0100 |
| RLLTSLFFL | HSV | | 0.3400 |
| RLLTSLFFL | HSV | | 0.3400 |
| LLLYYDYSL | HSV | | 0.2800 |
| DMLGRVFFV | HSV | | 0.0110 |
| TMFEALPHI | LCMV | Gp | 0.2000 |
| ALISFLLLA | LCMV | Gp | 0.2200 |
| TLMSIVSSL | LCMV | Gp | 0.2000 |
| NISGYNFSL | LCMV | Np | 0.0280 |
| ALLDGGNML | LCMV | Np | 0.0320 |
| ALHLFKTTV | LCMV | Gp | 0.0170 |
| SLISDQLLM | LCMV | Gp | 0.0540 |
| WLVTNGSYL | LCMV | Gp | 0.0180 |
| ALMDLLMFS | LCMV | Gp | 0.4300 |
| LMDLLMFST | LCMV | Gp | 0.0460 |
| LMFSTSAYL | LCMV | Gp | 0.3600 |
| YLVSIFLHL | LCMV | Gp | 0.4200 |
| SLHCKPEEA | MAGE1 | | 0.0130 |
| ALGLVCVQA | MAGE1 | | 0.0150 |
| LVLGTLEEV | MAGE1 | | 0.0320 |
| GTLEEVPTA | MAGR1 | | 0.0130 |
| CILESLFRA | MRGE1 | | 0.0460 |
| KVADLVGFLL | MAGR1 | | 0.0560 |
| KVADLVGFLLL | MAGE1 | | 0.0200 |
| VMIAMEGGHA | MAGE1 | | 0.0360 |
| SMHCKPEEV | MAGE1 (a) | | 0.0180 |
| AMGLVCVQV | MAGE1 (a) | | 0.0120 |
| LMLGTLEEV | MAGE1 (a) | | 0.1300 |
| KMADLVGFLV | MAGE1 (a) | | 1.5000 |
| VMVTCLGLSV | MAGE1 (a) | | 0.3000 |
| LLGDNQIMV | MAGE1 (a) | | 0.0430 |
| QMMPKTGFLV | MAGE1 (a) | | 0.0500 |
| VMIAMEGGHV | MAGE1 (a) | | 0.0530 |
| WMELSVMEV | MAGE1 (a) | | 0.0410 |
| FLWGPRALA | MAGE1N | | 0.0420 |
| RALAETSYV | MAGE1N | | 0.0100 |
| ALAETSYVKVL | MAGE1N | | 0.0120 |
| ALAETSYVKV | MAGE1N | | 0.0150 |
| KVLEYVIKV | MAGE1N | | 0.0900 |
| YVIKVSARV | MAGE1N | | 0.0140 |
| ALREEEEGV | MAGE1N | | 0.0210 |
| YMFLWGPRV | MAGE1N(a) | | 0.2200 |
| KMVELVHFLLL | MAGE2 | | 0.6700 |
| KMVELVHFL | MAGE2 | | 0.1600 |
| KMVELVHFLL | MAGE2 | | 0.1100 |
| KASBYLQLV | MAGE2 | | 0.0110 |
| YLQLVFGIEV | MAGE2 | | 0.3700 |
| LVFGIEVVEV | MANGE2 | | 0.0120 |
| QLVFGIELMEV | MAGE3 | | 0.3400 |
| KVAELVHFL | MANGE3 | | 0.0550 |
| KVAELVHFLL | MARGE3 | | 0.0120 |
| ELMEVDPIGHL | MAGE3 | | 0.0260 |
| HLYIFATCLGL | MAGE3 | | 0.0410 |
| IMPKAGLLIIV | MAGE3 | | 0.0130 |
| LVFGIELMEV | MAGE3 | | 0.1100 |
| ALGRNSFEV | p53 264-272 A8 (A1) | | 0.0570 |
| LLGANSFEV | p53 264-272 A8 (A4) | | 0.1100 |
| LLGRASFEV | p53 264-272 A8 (A5) | | 0.2200 |
| LLGRNAFEV | p53 264-272 A8 (A6) | | 0.0390 |
| LLGRNSFAV | p53 264-272 A8 (A8) | | 0.0420 |
| RLGRNSFEV | p53 264-272 A8 (R1) | | 0.0190 |
| LLGRRSFEV | p53 264-272 A8 (R5) | | 0.0540 |
| LLGRNSFRV | p53 264-272 A8 (R8) | | 0.0250 |
| LLFFWLDRSV | PAP | | 0.6000 |
| VLAKELKFV | PAP | | 0.0590 |
| ILLWQPIPV | PAP | | 1.3000 |
| IMYSAHDTTV | PAP | | 0.0610 |
| FLTLSVTWI | PSA | | 0.0150 |
| FLTLSVTWIGA | PSA | | 0.0160 |
| FLTLSVTWI | PSA | | 0.0150 |
| VLVHPQWVLTA | PSA | | 0.0130 |
| SLFHPEDTGQV | PSA | | 0.0190 |
| MLLRLSEPAEL | PSA | | 0.1400 |
| ALGTTCYA | PSA | | 0.0230 |
| KLQCVDLHVI | PSA | | 0.0370 |
| FLPSDYFPSV | HBVc18-27 analog | | 1.0000 |
| YSFLPSDFFPSV | HBVc18-27 analog | | 0.0190 |

**Table 26**

| **Sequence** | **Antigen** | **Molecule** | **A2 Bind.** |
|---|---|---|---|
| ALFLGFLGAA | HIV | gp160 | 0.4950 |
| MLQLTVWGI | HIV | gp160 | 0.2450 |
| RVIEVLQRA | HIV | gp160 | 0.1963 |
| KLTPLCVTL | HIV | gp160 | 0.1600 |
| LLIAARIVEL | HIV | gp160 | 0.1550 |
| SLLNATDIAV | HIV | gp160 | 0.1050 |
| ALFLGFLGA | HIV | gp160 | 0.0945 |
| HMLQLTVWGI | HIV | gp160 | 0.0677 |
| LLNATDIAV | HIV | gp160 | 0.0607 |
| ALLYKLDIV | HIV | gp160 | 0.0362 |
| WLWYIKIFI | HIV | gp160 | 0.0355 |
| TIIVHLNESV | HIV | gp160 | 0.0350 |
| LLQYWSQEL | HIV | gp160 | 0.0265 |
| IMIVGGLVGL | HIV | gp160 | 0.0252 |
| LLYKLDIVSI | HIV | gp160 | 0.0245 |
| FLAIIWVDL | HIV | gp160 | 0.0233 |
| TLQCKIKQII | HIV | gp160 | 0.0200 |
| GLVGLRIVFA | HIV | gp160 | 0.0195 |
| FLGAAGSTM | HIV | gp160 | 0.0190 |
| IISLWDQSL | HIV | gp160 | 0.0179 |
| TVWGIKQLQA | HIV | gp160 | 0.0150 |
| LLGRRGWEV | HIV | gp160 | 0.0142 |
| AVLSIVNRV | HIV | gp160 | 0.0132 |
| FIMIVGGLV | HIV | gp160 | 0.0131 |
| LLNATDIAVA | HIV | gp160 | 0.0117 |
| FLYGALLLA | PLP | | 1.9000 |
| SLLTFMIAA | PLP | | 0.5300 |
| FMIAATYNFAV | PLP | | 0.4950 |
| RMYGVLPWI | PLP | | 0.1650 |
| IAATYNFAV | PLP | | 0.0540 |
| GLLECCARCLV | PLP | | 0.0515 |
| YALIVVWLL | PLP | | 0.0415 |
| ALTVVWLLV | PLP | | 0.0390 |
| FLYGALLL | PLP | | 0.0345 |
| SLCADARMYGV | PLP | | 0.0140 |
| LLVFACSAV | PLP | | 0.0107 |
| KMVELVHFLL | MANGE2 | 0.2200 | |
| KVAELVHFL | MANGE3 | 0.0550 | |
| RALAETSYV | MAGE1N | 0.0100 | |
| LVFGIELMEV | MANGE3 | 0.1100 | |
| FLWGPRALA | MAGE1N | 0.0420 | |
| ALAETSYVKV | MAGE1 | 0.0150 | |
| LVLGTLEEV | HIV | 0.0320 | |
| LLWKGEGAVV | HIV | 0.0360 | |
| IIGAETFYV | HIV | 0.0260 | |
| LMVTVYYGV | HIV | 0.4400 | |
| LLFNILGGWV | HCV | 3.5000 | |
| LLALLSCLTV | HCV | 0.6100 | |
| YLVAYQATV | HCV | 0.2500 | |
| FLLLADARV | HCV | 0.2300 | |
| ILAGYGAGV | HCV | 0.2200 | |
| YLLPRRGPRL | HCV | 0.0730 | |
| GLLGCIITSL | HCV | 0.0610 | |
| DLMGYIPLV | HCV | 0.0550 | |
| LLALLSCLTI | HCV | 0.0340 | |
| VLAALAAYCL | HCV | 0.0110 | |
| LLVPFVQWFV | HBV | 1.6000 | |
| FLIAQFTSA | HBV | 0.6600 | |
| FLLSLGIHL | HBV | 0.5200 | |
| ALMPLYACI | HBV | 0.5000 | |
| ILLLCLIFLL | HBV | 0.3000 | |
| LLPIFFCLWV | HBV | 0.1000 | |
| YLHTLWKAGI | HBV | 0.0560 | |
| YLHTLWKAGV | HBV | 0.1300 | |

### Example 13

### Identification of immunogenic peptides in autoantigens

As noted above, the motifs of the present invention can also be screened in antigens associated with autoimmune diseases. Using the motifs identified above for HLA-A2.1 allele amino acid sequences from myelin proteolipid (PLP), myelin basic protein (MBP), glutamic acid decarboxylase (GAD), and human collagen types II and IV were analyzed for the presence of these motifs. Sequences for the antigens were obtained from Trifilieff et al., C.R. Sceances Acad. Sci. 300:241 (1985); Eyler at al., J. Biol. Chem. 246:5770 (1971); Yamashita et al. Biochiem. Biophys. Res. Comm. 192:1347 (1993); Su et al., Nucleic Acids Res. 17:9473 (1989) and Pihlajaniemi et al. Proc. Natl. Acad. Sci. USA 84:940 (1987). The identification of motifs was done using the approach described in Example 5 and the algorithms of Examples 6 and 7. Table 27 provides the results of the search of these antigens.

Using the quantitative binding assays of Example 4, the peptides are next tested for the ability to bind MHC molecules. The ability of the peptides to suppress proliferative responses in autoreactive T cells is carried out using standard assays for T cell proliferation. For instance, methods as described by Miller et al. Proc. Natl. Acad. Sci. USA, 89:421 (1992) are suitable.

For further study, animal models of autoimmune disease can be used to demonstrate the efficacy of peptides of the invention. For instance, in HLA transgenic mice, autoimmune model diseases can be induced by injection of MBP, PLP or spinal cord homogenate (for MS), collagen (for arthritis). In addition, some mice become spontaneously affected by autoimmune disease (e.g., NOD mice in diabetes). Peptides of the invention are injected into the appropriate animals, to identify preferred peptides.

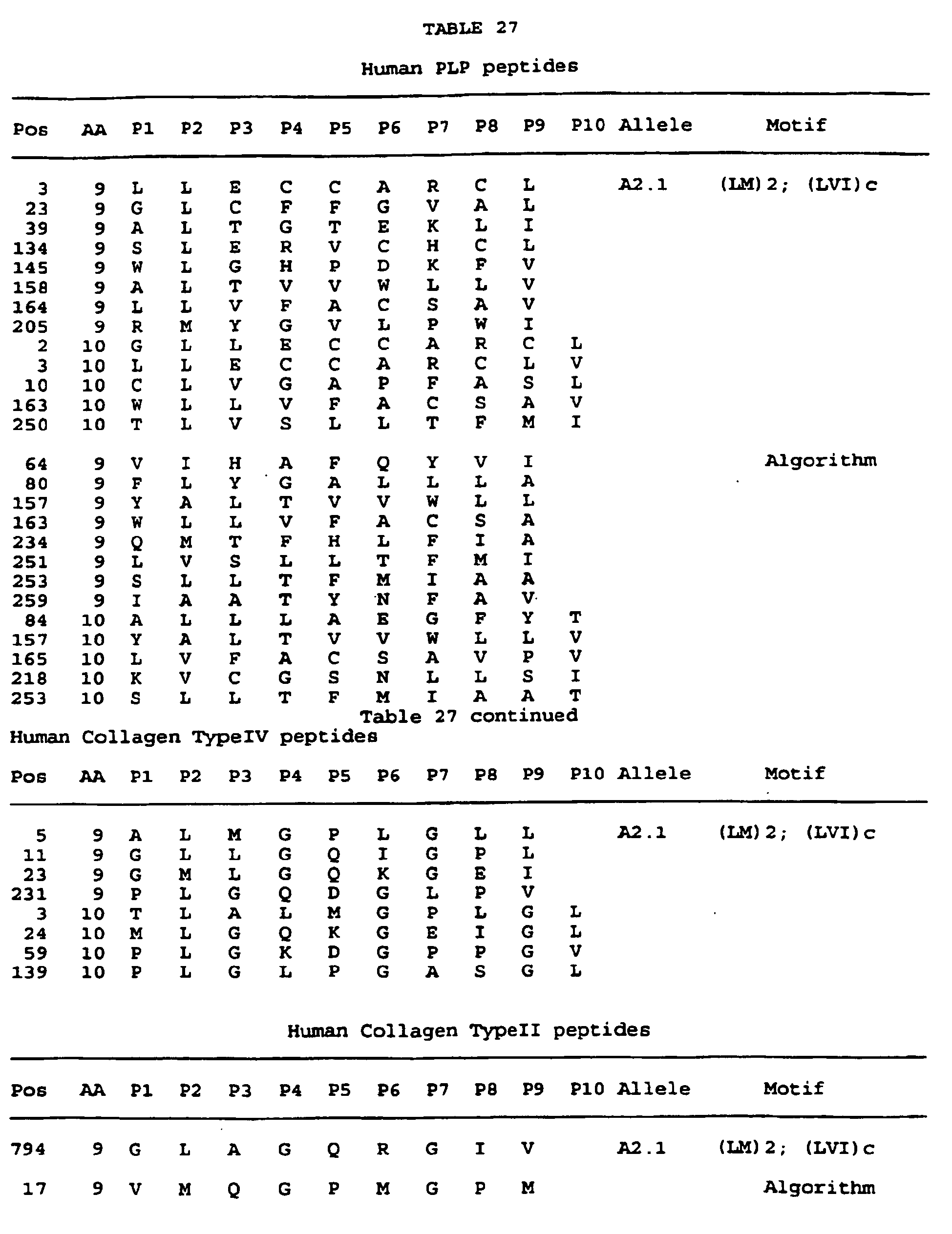

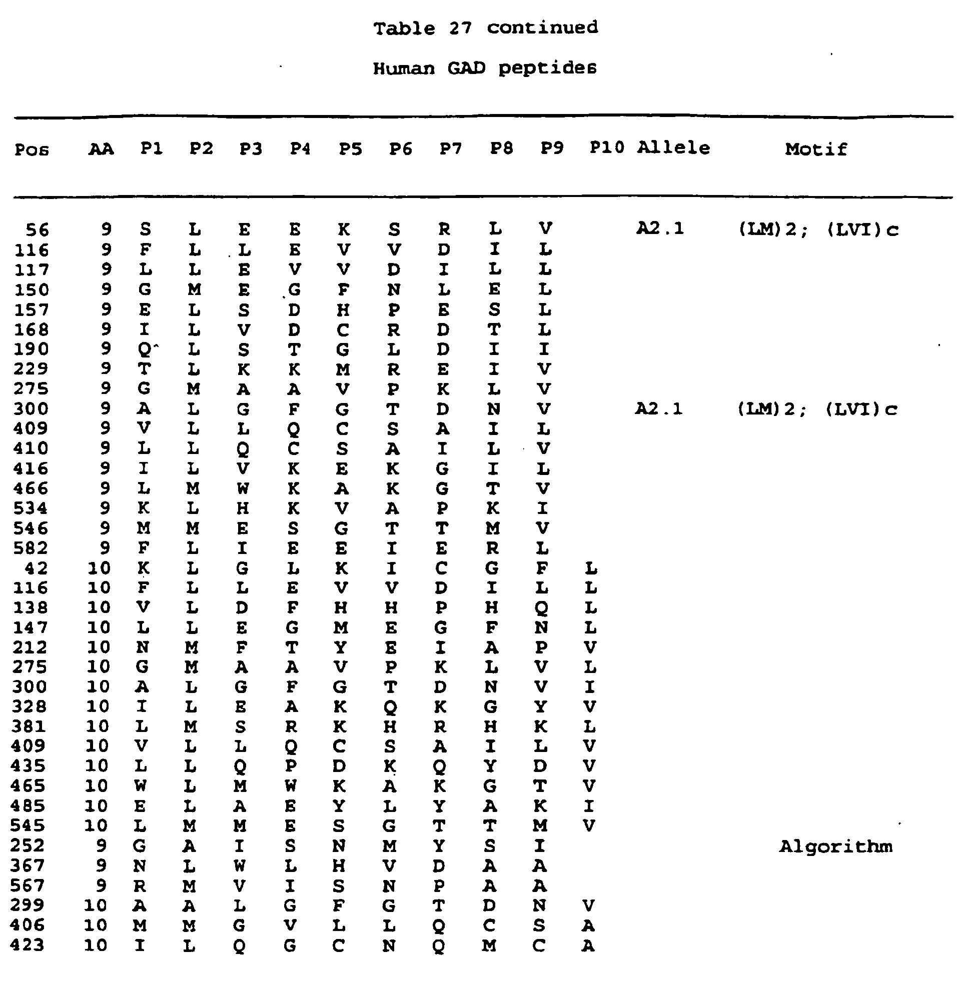

### Example 14

### Immunogenicity of HPV peptides in A2.1 transgenic mice

A group of 14 HPV peptides, including 9 potential epitopes plus 3 low binding and one non-binding peptides as controls was screened for immunogenicity in HLA-A2.1 transgenic mice using the methods described in Example 10. To test the immunogenic potential of the peptides, HLA A2.1 transgenic mice were injected with 50 µg/mouse of each HPV peptide together with 140 µg/mouse of helper peptide (HBV core 128-140 (TPPAYRPPNAPIL). The peptides were injected in the base of the tail in a 1:1 emulsion IFA. Three mice per group were used. As a positive control, the HBV polymerase 561-570 peptide, which induced a strong CTL response in previous experiments, was utilized.

Based on these results (Table 28), four unrelated peptides were considered to be the most immunogenic: TLGIVCPI , LLMGTLGIV, YMLDLQPETT, and TIHDIILECV. TLGIVCPI and YMLDLQPETT were found to be good HLA-A2.1 binders, while LLMGTLGIV and TIHDIILECV were found to be intermediate binders in previous binding assays.

**TABLE 28**

| HPV-16 Peptides for possible use in clinical trial | | | | | |
|---|---|---|---|---|---|
| Peptide Position/ Cytel ID | Sequence | AA | A2.1 binding | Immunogenicity Experiment 1 | Immunogenicity Experiment 2 |
| E7.86/1088.01 | TLGIVCPI | 8 | 0.15 | 94.4 (1.34) | 54.2 (1.43)* |
| E7.86/1088.06 | TLGIVCPIC | 9 | 0.075 | 2.05 (4.93) | 1.3 (3.74) |
| E7.85/1088.08 | GTLGIVCPI | 9 | 0.021 | 9/08 (3.93) | -** |
| B7.11/1088.03 | YMLDLQPETT | 10 | 0.15 | 10.32 (1.66) | 5.7 (2.39) |
| E7.11/1088.04 | YMLDLQPET | 9 | 0.14 | 5.0 (3.70) | 2.6 (15.5) |
| E7.12/1088.09 | MLDLQPETT | 9 | 0.0028 | - | - |
| E6.52/1088.05 | FAFRDLCIV | 9 | 0.057 | - | ND |
| E7.82/1088.02 | LLMGTLGIV | 9 | 0.024 | 9.62 (2.53) | 8.93 (1.91) |
| E6.29/1088.10 | TIHDIILECV | 10 | 0.021 | 22.13 (3.71) | 0.4 (3.52) |
| E7.7/1088.07 | TLHEYMLDL | 9 | 0.0070 | - | 1.2 (3.88 |
| E6.18/1088.15 | KLPQLCTEL | 9 | 0.0009 | - | 0.3 (5.64) |
| E6.7/1088.11 | AMFQDPQER | 10 | 0.0002 | - | ND |
| E6.26/1088.12 | LQTTIHDII | 9 | 0.0002 | - | - |
| E7.73/1088.13 | HVDIRTLED | 9 | 0 | - | ND |

| | | | | | |
|---|---|---|---|---|---|
| * A Lytic Units, geometric mean x+ SD (3 mice/peptide) ** a dash indicates Δ Lytic Units with a geometric mean ≤0.2 | | | | | |

### Mixtures of selected HPV epitopes

A combination of CTL peptides and a helper peptide were tested for the ability to provide an increased immune response. The four single peptides were injected separately in order to compare their immunogenicity to injections containing only the two good binders or only the two intermediate binders. In addition all four peptide were injected together. To further evaluate the immunogenicity of a combination of peptides with different binding affinity decreases, another control was introduced in this experiment. A mixture of the two good binders was injected in a different site than the mixture of the two intermediate binders into the base of the tail of the same mouse. All groups of CTL epitopes were injected together with the HBVc helper epitope, with the exception of two groups in which all four HPV coinjected with two different doses of a PADRE helper peptide (aKXVAAWTLKAAa, where a is d-alanine and X is cyclohexylalanine) either 1µg or 0.05µg per mouse.

All four peptides induced a strong CTL response when injected alone and tested using target cells labeled with the appropriate peptide (Table 29). TLGIVCPI proved to be the strongest epitope, an observation confirming the results described above. When mixtures of all four peptides were injected and the responses were stimulated in vitro and tested with target cells pulsed with each single peptide, all combinations showed a strong CTL response. No significant difference was observed when the two helper epitopes were compared. This might in part be due to the fact that the highest dose of PADRE used in this experiment was 140-fold lower than the one for the HBV helper peptide.

Injection of mixtures of the two good binders together or the two intermediate binders resulted in a very low CTL response in both cases even though the single peptides were highly effective. These results, however, are due to a very low number of cell recovery after splenocyte culture of 6 days and are therefore regarded as preliminary.

**TABLE 29**

| HPV Peptides single and in combinations | | | | | | | | |
|---|---|---|---|---|---|---|---|---|
| A | | | | | | | | |
| | Peptides in restimulation and CTL assay | | | | | | | |
| Peptide/s injected | 1088.01 | | 1088.02 | | 1088.03 | | 1088.10 | |
| same as in vitro | 116.1 | (3.49)* | 55.98 | (2.49) | 5.56 | (1.75) | 16.4 | (1.49) |
| 1088.01 + 1088.03 + 875.23 | 1.37 | (16.56) | | | 0 (0) | | | |
| 1088.02 + 1088.10 + 875.23 | | | 1.11 | (2.9) | | | 1.62 | (13.1) |
| 1088.01/.03 + 1088.02/.10 + 875.23 | 19.5 | (4.1) | 4.68 | (2.3) | 1.13 | (21.9) | 1.17 | (2.58) |
| 1088.a11 + 875.23 | 107.9 | (4.77) | 13.52 | (1.4) | 2.58 | (5.07) | 102.3 | (1.32) |
| 1088.a11 + PADRE 1 µg | 73.11 | (4.48) | 16.83 | (2.54) | 3.55 | (2.9) | 20.13 | (1.05) |
| 1088.a11 + PADRE 0.05 µg | 37.15 | (2.25) | 26.79 | (2.09) | 6.5 | (1.64) | 4.45 | (4.14) |

| | | | | | | | | |
|---|---|---|---|---|---|---|---|---|
| * Δ Lytic Units 30% geometric mean (+x deviation) | | | | | | | | |

Peptides were dissolved in 50%DMSO/H2O to reach a stock concentration of 20mg/ml and were further dissolved in sterile PBS. For subcutaneous injection in the base of the tail of A2.1 transgenic mice, the peptide solution was mixed 1:1 with IFA. The injected amount of HPV-CTL peptides was 50 µg/mouse coinjected with 140 µg/mouse of the HBVcore peptide 875.23 or the indicated dose of PADRE (3 mice/group). Spleens were removed on day 11 and splenocytes were restimulated in vitro with irradiated LPS-Blasts pulsed with the indicated HPV-CTL epitopes at 1µg/ml. After six days, the cytotoxic assay was performed using Jurkat JA2Kb cells (A) or MBB17 (B) as target cells labelled with 51Cr in the presence or absence of the appropriate HPV epitope peptides.

The above examples are provided to illustrate the invention but not to limit its scope. Other variants of the invention will be readily apparent to one of ordinary skill in the art and are encompassed by the appended claims. All publications, patents, and patent applications cited herein are hereby incorporated by reference.

| APPENDIX I: 9-MER PEPTIDES | | | | | | | |
|---|---|---|---|---|---|---|---|
| **Peptide** | **Sequence** | **AA** | **Virus** | **Strain** | **Molecule** | **Pos.** | **A2. 1** |
| 1.0841 | ILSPFLPLL | 9 | HBV | adr | ENV | 371 | 2.9 |
| 1.0240 | TLQDIVLHL | 9 | HPV | 18 | E7 | 7 | 0.76 |
| 1.0838 | WLSLLVPFV | 9 | HBV | adr | ENV | 335 | 0.72 |
| 1.0851 | FLLSLGIHL | 9 | HBV | adr | POL | 1147 | 0.52 |
| 1.0306 | QLFEDNYAL | 9 | c-ERB2 | | | 106 | 0.46 |
| 1.0814 | LMVTVYYGV | 9 | HIV | | ENV | 2182 | 0.44 |
| 1.0878 | MMWFWGPSL | 9 | HBV | adw | ENV | 360 | 0.41 |
| 1.0839 | MMWYWGPSL | 9 | HBV | adr | ENV | 360 | 0.41 |
| 1.0384 | FLTKQYLNL | 9 | HBV | adw | POL | 1279 | 0.29 |
| 1.0321 | ILHNGAYSL | 9 | C-ERB2 | | | 435 | 0.21 |
| 1.0834 | LLLCLIFLL | 9 | HBV | adr | ENV | 250 | 0.19 |
| 1.0167 | GLYSSTVPV | 9 | HBV | adr | POL | 635 | 0.15 |
| 1.0849 | HLYSHPIIL | 9 | HBV | adr | POL | 1076 | 0.13 |
| 1.0275 | RMPEAAPPV | 9 | p53 | | | 65 | 0.12 |
| 1.0854 | LLMGTLGIV | 9 | HPV | 16 | E7 | 82 | 0.11 |
| 1.0880 | ILSPFMPLL | 9 | HBV | adw | ENV | 371 | 0.11 |
| 1.0127 | YLVAYQATV | 9 | HCV | | LORF | 1585 | 0.11 |
| 1.0151 | VLLDYQGML | 9 | HBV | adr | ENV | 259 | 0.11 |
| 1.0018 | VLAEAMSQV | 9 | HIV | | GAG | 367 | 0.11 |
| 1.0330 | RLLQETELV | 9 | c-ERB2 | | | 689 | 0.091 |
| 1.0209 | SLYAVSPSV | 9 | HBV | adr | POL | 1388 | 0.078 |
| 1.0816 | DLMGYIPLV | 9 | HCV | | CORE | 132 | 0.055 |
| 1.0835 | LLCLIFLLV | 9 | HBV | adr | ENV | 251 | 0.049 |
| 1.0852 | FLCQQYLHL | 9 | HBV | adr | POL | 1250 | 0.048 |
| 1.0882 | NLYVSLMLL | 9 | HBV | adw | POL | 1088 | 0.046 |
| 1.0837 | GMLPVCPLL | 9 | HBV | adr | ENV | 265 | 0.046 |
| 1.0819 | ILPCSFTTL | 9 | HCV | | NS1/ENV2 | 676 | 0.045 |
| 1.0109 | ALSTGMHL | 9 | HCV | | NS1/ENV2 | 686 | 0.042 |
| 1.0833 | ILLLCLIFL | 9 | HBV | adr | ENV | 249 | 0.035 |
| 1.0301 | HLYQGCQW | 9 | c-ERB2 | | | 48 | 0.034 |
| 1.0337 | CLTSTVQLV | 9 | c-ERB2 | | | 789 | 0.034 |
| 1.0842 | PLLPIFFCL | 9 | HBV | adr | ENV | 377 | 0.031 |
| 1.0861 | ALCRWGLLL | 9 | c-ERB2 | | | 5 | 0.031 |
| 1.0309 | VLIQRNPQL | 9 | c-ERB2 | | | 153 | 0.029 |
| 1.0828 | VLQAGFFLL | 9 | HBV | adr | ENV | 177 | 0.024 |
| 1.0844 | LLWFHISCL | 9 | HBV | adr | CORE | 490 | 0.024 |
| 1.0135 | ILAGYGAGV | 9 | HCV | | LORF | 1851 | 0.024 |
| 1.0870 | QLMPYGCLL | 9 | c-ERB2 | | | 799 | 0.023 |
| 1.0075 | LLWKGEGAV | 9 | HIV | | POL | 1496 | 0.023 |
| 1.0873 | FLGGTPVCL | 9 | HBV | adw | ENV | 204 | 0.021 |
| 1.0323 | ALIHHNTHL | 9 | c-BRB2 | | | 466 | 0.021 |
| 1.0859 | VLVHPQWVL | 9 | PSA | | | 49 | 0.020 |
| 1.0267 | KLQCVDLHV | 9 | PSA | | | 166 | 0.019 |
| 1.0820 | VLPCSFTTL | 9 | HCV | | NS1/ENV2 | 676 | 0.017 |
| 1.0111 | HLHQNIVDV | 9 | HCV | | NS1/ENV2 | 693 | 0.016 |
| 1.0103 | SMVGNWAKV | 9 | HCV | | ENV1 | 364 | 0.016 |
| 1.0283 | LLGRNSFEV | 9 | p53 | | | 264 | 0.014 |
| 1.0207 | GLYRPLLSL | 9 | HBV | adr | POL | 1370 | 0.014 |
| 1.0389 | GLYRPLLRL | 9 | HBV | adw | POL | 1399 | 0.014 |
| 1.0185 | NLSWLSLDV | 9 | HBV | adr | POL | 996 | 0.013 |
| 1.0113 | FLLLADARV | 9 | HCV | | NS1/ENV2 | 725 | 0.013 |
| 1.0119 | YLVTRHADV | 9 | HCV | | LORF | 1131 | 0.011 |
| 1.0846 | CLTHIVNLL | 9 | HBV | adr | POL | 912 | 0.010 |
| 1.0156 | ELMNLATWV | 9 | HBV | adr | CORE | 454 | 0.010 |
| 1.0236 | KLPDLCTEL | 9 | HPV | 18 | E6 | 13 | 0.010 |
| 1.0056 | ALQDSGLEV | 9 | HIV | | POL | 1180 | 0.0083 |
| 1.0375 | LLSSDLSWL | 9 | HBV | adw | POL | 1021 | 0.0081 |
| 1.0094 | ALAHGVRVL | 9 | HCV | | CORE | 150 | 0.0072 |
| 1.0129 | TLHGPTPLL | 9 | HCV | | LORF | 1617 | 0.0070 |
| 1.0041 | KLLRGTKAL | 9 | HIV | | POL | 976 | 0.0069 |
| 1.0131 | CMSADLEVV | 9 | HCV | | LORF | 1648 | 0.0067 |
| 1.0872 | GLLGPLLVL | 9 | HBV | adw | ENV | 170 | 0.0066 |
| 1.0228 | TLHEYMLDL | 9 | HPV | 16 | E7 | 7 | 0.0059 |
| 1.0274 | KLLPENNVL | 9 | p53 | | | 24 | 0.0058 |
| 1.0043 | ILKEPVHGV | 9 | HIV | | POL | 1004 | 0.0055 |
| 1.0206 | RLGLYRPLL | 9 | HBV | adr | POL | 1368 | 0.0050 |
| 1.0188 | GLPRYVARL | 9 | HBV | adr | POL | 1027 | 0.0050 |
| 1.0202 | ICLIGTDNSV | 9 | HBV | adr | POL | 1317 | 0.0050 |
| 1.0818 | FLLALLSCL | 9 | HCV | | CORE | 177 | 0.0046 |
| 1.0184 | LLSSNLSWL | 9 | HBV | adr | POL | 992 | 0.0046 |
| 1.0102 | QLLRIPQAV | 9 | HCV | | ENV1 | 337 | 0.0039 |
| 1.0114 | GLRDLAVAV | 9 | HCV | | LORF | 963 | 0.0034 |
| 1.0005 | TLNAWVKVI | 9 | HIV | | GAG | 156 | 0.0032 |
| 1.0183 | NLQSLTNLL | 9 | HBV | adr | POL | 985 | 0.0025 |
| 1.0359 | QLGRKPTPL | 9 | HBV | adw | ENV | 89 | 0.0025 |
| 1.0150 | SLDSWWTSL | 9 | HBV | adr | ENV | 194 | 0.0023 |
| 1.0362 | ILSKTGDPV | 9 | HBV | adw | ENV | 153 | 0.0021 |
| 1.0866 | ILLVVVLGV | 9 | c-ERB2 | | | 661 | 0.0020 |
| 1.0214 | LLHKRTLGL | 9 | HBV | adr | "X" | 1510 | 0.0019 |
| 1.0216 | CLFKDWEEL | 9 | HBV | adr | "X" | 1533 | 0.0019 |
| 1.0862 | GLGISWLGL | 9 | c-ERB2 | | | 447 | 0.0018 |
| 1.0187 | HLLVGSSGL | 9 | HBV | adr | POL | 1020 | 0.0018 |
| 1.0318 | TLEEITGYL | 9 | c-ERB2 | | | 402 | 0.0018 |
| 1.0328 | PLTSIISAV | 9 | c-ERB2 | | | 650 | 0.0015 |
| 1.0822 | LLGCZITSL | 9 | HCV | | LORF | 1039 | 0.0015 |
| 1.0277 | ALNKMFCQL | 9 | p53 | | | 129 | 0.0013 |
| 1.0066 | HLEGKIILV | 9 | HIV | | POL | 1322 | 0.0010 |
| 1.0308 | QLRSLTEIL | 9 | c-ERB2 | | | 141 | 0.0008 |
| 1.0115 | DLAVAVEPV | 9 | HCV | | LORF | 966 | 0.0008 |
| 1.0391 | VLHKRTLGL | 9 | HBV | adw | "X" | 1539 | 0.0007 |
| 1.0876 | FLCILLLCL | 9 | HBV | adw | ENV | 246 | 0.0007 |
| 1.0148 | LLDPRVRGL | 9 | HBV | adr | ENV | 120 | 0.0006 |
| 1.0221 | KLPQLCTEL | 9 | HPV | 16 | E6 | 18 | 0.0006 |
| 1.0065 | HLEGKVILV | 9 | HIV | | POL | 1322 | 0.0006 |
| 1.0017 | HMMTACQGV | 9 | HIV | | GAG | 350 | 0.0006 |
| 1.0055 | HLALQDSGL | 9 | HIV | | POL | 1178 | 0.0005 |
| 1.0868 | VLGVVFGIL | 9 | c-ERB2 | | | 666 | 0.0005 |
| 1.0004 | TLNAWVKVV | 9 | HIV | | GAG | 156 | 0.0005 |
| 1.0381 | HLESLYAAV | 9 | HBV | adw | POL | 1165 | 0.0005 |
| 1.0128 | CLIRLKPTL | 9 | HCV | | LORF | 1610 | 0.0004 |
| 1.0255 | CLGLSYDGL | 9 | MAGE | 1/3 | | 174 | 0.0004 |
| 1.0212 | HLSLRGLPV | 9 | HBV | adr | "X" | 1470 | 0.0004 |
| 1.0247 | ILESLFRAV | 9 | MAGE | 1 | | 93 | 0.0004 |
| 1.0092 | TLTCGFADL | 9 | HCV | | CORE | 125 | 0.0003 |
| 1.0108 | TLPALSTGL | 9 | HCV | | NS1/ENV2 | 683 | 0.0003 |
| 1.0294 | ALAIPQCRL | 9 | ETNA1 | | | 525 | 0.0003 |
| 1.0101 | DLCGSVFLV | 9 | HCV | | ENV1 | 280 | 0.0003 |
| 1.0231 | RLCVQSTHV | 9 | HPV | 16 | E7 | 66 | 0.0003 |
| 1.0162 | LLDDEAGPL | 9 | HBV | adr | POL | 587 | 0.0002 |
| 1.0829 | CLRRFIIFL | 9 | HBV | adr | ENV | 239 | 0.0002 |
| 1.0126 | GLPVCQDHL | 9 | HCV | | LORF | 1547 | 0.0001 |
| 1.0163 | PLEEELPRL | 9 | HBV | adr | POL | 594 | 0.0001 |
| 1.0130 | PLLYRLGAV | 9 | HCV | | LORF | 1623 | 0.0001 |
| 1.0042 | ELAENREIL | 9 | HIV | | POL | 997 | 0 |
| 1.0054 | ELQAIHLAL | 9 | HIV | | POL | 1173 | 0 |
| 1.0089 | LIPRRGPRL | 9 | HCV | | CORE | 36 | 0 |
| 1.0091 | NLGKVIDTL | 9 | HCV | | CORE | 118 | 0 |
| 1.0093 | PLGGAARAL | 9 | HCV | | CORE | 143 | 0 |
| 1.0154 | DLLDTASAL | 9 | HBV | adr | CORE | 419 | 0 |
| 1.0178 | QLKQSRLGL | 9 | HBV | adr | POL | 791 | 0 |
| 1.0179 | GLQPQQGSL | 9 | HBV | adr | POL | 798 | 0 |
| 1.0286 | PLDGEYFTL | 9 | p53 | | | 322 | 0 |
| 1.0296 | VLKDAIKDL | 9 | EBNA1 | | | 574 | 0 |
| 1.0310 | QLCYQDTIL | 9 | c-ERB2 | | | 160 | 0 |
| 1.0007 | DLNTMLNTV | 9 | HIV | | GAG | 188 | 0 |
| 1.0037 | ELHPDKWTV | 9 | HIV | | POL | 928 | 0 |
| 1.0070 | BLKKIIGQV | 9 | HIV | | POL | 1412 | 0 |
| 1.0157 | ELVVSYVNV | 9 | HBV | adr | CORE | 473 | 0 |
| 1.0160 | CLTFGRETV | 9 | HBV | adr | CORE | 497 | 0 |
| 1.0164 | DLNLGNLNV | 9 | HBV | adr | POL | 614 | |
| 1.0867 | LLVVVLGVV | 9 | c-ERB2 | | | 662 | 0 |
| 1.0159 | NMGLKIRQL | 9 | HBV | adr | CORE | 482 | 0 |
| 1.0322 | SLRELGSGL | 9 | c-ERB2 | | | 457 | <0.0002 |
| 1.0350 | DLLEKGERL | 9 | c-ERB2 | | | 933 | <0.0002 |
| 1.0352 | DLVDAEEYL | 9 | c-ERB2 | | | 1016 | <0.0002 |
| 1.0366 | PLEEELPHL | 9 | HBV | adw | POL | 623 | <0.0002 |
| 1.0372 | DLQHGRLVL | 9 | HBV | adw | POL | 781 | <0.0002 |
| 1.0390 | PLPGPLGAL | 9 | HBV | adw | "X" | 1476 | <0.0002 |
| 1.0811 | LLTQIGCTL | 9 | HIV | | POL | 685 | <0.0002 |
| 1.0812 | PLVKLWYQL | 9 | HIV | | POL | 1116 | <0.0002 |
| 1.0832 | FLFILLLCL | 9 | HBV | adr | ENV | 246 | <0.0002 |
| 1.0847 | NLYVSLLLL | 9 | HBV | adr | POL | 1059 | <0.0002 |
| 1.0316 | PLQPEQLQV | 9 | c-ERB2 | | | 391 | <0.0002 |
| 1.0342 | DLAARNVLV | 9 | c-ERB2 | | | 845 | <0.0002 |
| 1.0343 | VLVKSPNHV | 9 | c-ERB2 | | | 851 | <0.0002 |
| 1.0356 | TLSPGKNGV | 9 | c-ERB2 | | | 1172 | <0.0002 |
| 1.0376 | DLSWLSLDV | 9 | HBV | adw | POL | 1025 | <0.0002 |
| 1.0363 | NMENIASGL | 9 | HBV | adw | ENV | 163 | <0.0002 |
| 1.0195 | TLPQEHIVL | 9 | HBV | adr | POL | 1179 | <0.0003 |
| 1.0196 | KLKQCFRKL | 9 | HBV | adr | POL | 1188 | <0.0003 |
| 1.0201 | PLPIHTAEL | 9 | HBV | adr | POL | 1296 | <0.0003 |
| 1.0210 | QLDPARDVL | 9 | HBV | adr | "X" | 1426 | <0.0003 |
| 1.0220 | VLGGCRHKL | 9 | HBV | adr | "X" | 1551 | <0.0003 |
| 1.0229 | DLQPETTDL | 9 | HPV | 16 | E7 | 14 | <0.0003 |
| 1.0245 | ALEAQQEAL | 9 | MAGE | 1 | | 15 | <0.0003 |
| 1.0266 | DLPTQEPAL | 9 | PSA | | | 136 | <0.0003 |
| 1.0279 | HLIRVEGNL | 9 | p53 | | | 193 | <0.0003 |
| 1.0282 | TLEDSSGNL | 9 | p53 | | | 256 | <0.0003 |
| 1.0238 | ELRHYSDSV | 9 | HPV | 18 | E6 | 77 | <0.0003 |
| 1.0268 | DLHVISNDV | 9 | PSA | | | 171 | <0.0003 |
| 1.0836 | CLIFLLVLL | 9 | HBV | adr | ENV | 253 | <0.0006 |

| APPENDIX II: 10-MER PEPTIDES | | | | | | | |
|---|---|---|---|---|---|---|---|
| **Peptide** | **Sequence** | **AA** | **Virus** | **Strain** | **Molecule** | **Pos.** | **A2.1** |
| 1.0890 | LLFNILGGWV | 10 | HCV | | LORF | 1807 7 | 3.5 |
| 1.0930 | LLVPFVQWFV | 10 | HBV | adw | ENV | 338 | 1.6 |
| 1.0884 | LLALLSCLTV | 10 | HCV | | CORE | 178 | 0.61 |
| 1.0896 | ILLLCLIFLL | 10 | HBV | adr | ENV | 249 | 0.30 |
| 1.0518 | GLSPTVWLSV | 10 | HBV | adr | ENV | 348 | 0.28 |
| 1.0902 | SLYNILSPFL | 10 | HBV | adr | ENV | 367 | 0.23 |
| 1.0892 | LLVLQAGFFL | 10 | HBV | adr | ENV | 175 | 0.21 |
| 1.0686 | FLQTHIFAEV | 10 | EBNA1 | | | 565 | 0.17 |
| 1.0628 | QLFLNTLSFV | 10 | HPV | 18 | E7 | 88 | 0.11 |
| 1.0904 | LLPIFFCLWV | 10 | HBV | adr | ENV | 378 | 0.10 |
| 1.0897 | LLLCLIFLLV | 10 | HBV | adr | ENV | 250 | 0.099 |
| 1.0516 | LLDYQGMLPV | 10 | HBV | adr | ENV | 260 | 0.085 |
| 1.0901 | WMMWYWGPSL | 10 | HBV | adr | ENV | 359 | 0.084 |
| 1.0533 | GLYSSTVPVL | 10 | HBV | adr | POL | 635 | 0.080 |
| 1.0469 | YLLPRRGPRL | 10 | HCV | | CORE | 35 | 0.073 |
| 1.0888 | GLLGCIITSL | 10 | HCV | | LORF | 1038 | 0.061 |
| 1.0907 | ILCWGELMNL | 10 | HBV | adr | CORE | 449 | 0.052 |
| 1.0927 | LLGICLTSTV | 10 | c-ERB2 | | | 785 | 0.049 |
| 1.0452 | LLWKGEGAVV | 10 | HIV | | POL | 1496 | 0.036 |
| 1.0885 | LLALLSCLTI | 10 | HCV | | CORE | 178 | 0.034 |
| 1.0620 | KLTNTGLYNL | 10 | HPV | 18 | E6 | 92 | 0.032 |
| 1.0502 | RLIVFPDLGV | 10 | HCV | | LORF | 2578 | 0.032 |
| 1.0659 | FLTPKKLQCV | 10 | PSA | | | 161 | 0.031 |
| 1.0932 | WMMWFWGPSL | 10 | HBV | adw | ENV | 359 | 0.029 |
| 1.0772 | SLNFLGGTPV | 10 | HBV | adw | ENV | 201 | 0.027 |
| 1.0609 | SLQDIEITCV | 10 | HPV | 18 | E6 | 24 | 0.025 |
| 1.0526 | ILSTLPETTV | 10 | HBV | adr | CORE | 529 | 0.022 |
| 1.0508 | RLHGLSAFSL | 10 | HCV | | LORF | 2885 | 0.020 |
| 1.0493 | ILGGWVAAQL | 10 | HCV | | LORF | 1811 | 0.018 |
| 1.0738 | VMAGVGSPYV | 10 | c-ERB2 | | | 773 | 0.018 |
| 1.0460 | QLMVTVYYGV | 10 | HIV | | ENV | 2181 | 0.017 |
| 1.0573 | ILRGTSFVYV | 10 | HBV | adr | POL | 1345 | 0.016 |
| 1.0703 | SLTEILKGGV | 10 | c-ERB2 | | | 144 | 0.015 |
| 1.0912 | LLGCAANWIL | 10 | HBV | adr | POL | 1337 | 0.014 |
| 1.0798 | ALPPASPSAV | 10 | HBV | adw | "X" | 1483 | 0.013 |
| 1.0908 | QLLWFHISCL | 10 | HBV | adr | CORE | 489 | 0.013 |
| 1.0677 | NLLGRNSFEV | 10 | p53 | | | 263 | 0.013 |
| 1.0889 | VLAALAAYCL | 10 | HCV | | LORF | 1666 | 0.011 |
| 1.0528 | LLLDDEAGPL | 10 | HBV | adr | POL | 586 | 0.011 |
| 1.0500 | IMAKNEVFCV | 10 | HCV | | LORF | 2558 | 0.0088 |
| 1.0992 | VLVCGVLAAAL | 10 | HCV | | LORF | 1661 | 0.0084 |
| 1.0898 | LLCLIFLLVL | 10 | HBV | adr | ENV | 251 | 0.0075 |
| 1.0458 | KLMVTVYYGV | 10 | HIV | | ENV | 2181 | 0.0069 |
| 1.0459 | NLMVTVYYGV | 10 | HIV | | ENV | 2181 | 0.0067 |
| 1.0530 | GLSPTVWLSA | 10 | HBV | adw | ENV | 348 | 0.0067 |
| 1.0759 | SLPTHDPSPL | 10 | c-ERB2 | | | 1100 | 0.0059 |
| 1.0419 | VLPEKDSWTV | 10 | HIV | | POL | 940 | 0.0056 |
| 1.0666 | FLHSGTAKSV | 10 | p53 | | | 113 | 0.0050 |
| 1.0473 | GLIHLHQNIV | 10 | HCV | | NS1/ENV2 | 690 | 0.0047 |
| 1.0792 | SLYAAVTNFL | 10 | HBV | adw | POL | 1168 | 0.0046 |
| 1.0780 | IMPARFYPNV | 10 | HBV | adw | POL | 713 | 0.0043 |
| 1.0507 | YLTRDPTTPL | 10 | HCV | | LORF | 2803 | 0.0042 |
| 1.0914 | GLYNLLIRCL | 10 | HPV | 18 | E6 | 97 | 0.0036 |
| 1.0649 | YLEYGRCRTV | 10 | MAGE | 1 | | 248 | 0.0034 |
| 1.0561 | SLFTSITNFL | 10 | HBV | adr | POL | 1139 | 0.0034 |
| 1.0788 | NLLSSDLSWL | 10 | HBV | adw | POL | 1020 | 0.0032 |
| 1.0753 | RMARDPQRFV | 10 | c-ERB2 | | | 978 | 0.0020 |
| 1.0568 | RMRGTFVVPL | 10 | HBV | adr | POL | 1288 | 0.0020 |
| 1.0642 | SLQLVFGIDV | 10 | MAGE | 1 | | 150 | 0.0020 |
| 1.0582 | KLLHKRTLGL | 10 | HBV | adr | 'X' | 1509 | 0.0019 |
| 1.0713 | GLGMEHLREV | 10 | c-ERB2 | | | 344 | 0.0017 |
| 1.0742 | GMSYLEDVRL | 10 | c-ERB2. | | | 832 | 0.0017 |
| 1.0549 | NLLSSNLSWL | 10 | HBV | adr | POL | 991 | 0.0016 |
| 1.0465 | QLTVWGIKQL | 10 | HIV | | ENV | 2760 | 0.0015 |
| 1.0524 | VLEYLVSFGV | 10 | HBV | adr | CORE | 505 | 0.0015 |
| 1.0483 | VLNPSVAATL | 10 | HCV | | LORF | 1253 | 0.0015 |
| 1.0548 | SLTNLLSSNL | 10 | HBV | adr | POL | 988 | 0.0014 |
| 1.0512 | ALLDPRVRGL | 10 | HBV | adr | ENV | 119 | 0.0011 |
| 1.0676 | TLEDSSGNLL | 10 | p53 | | | 256 | 0.0011 |
| 1.0719 | TLQGLGISWL | 10 | c-ERB2 | | | 444 | 0.0011 |
| 1.0627 | DLRAFQQLFL | 10 | HPV | 18 | E7 | 82 | 0.0010 |
| 1.0725 | VLQGLPREYV | 10 | c-5R82 | | | 546 | 0.0009 |
| 1.0918 | DLPPWFPPMV | 10 | EBNA1 | | | 605 | 0.0009 |
| 1.0499 | DLSDGSWSTV | 10 | HCV | | LORF | 2399 | 0.0008 |
| 1.0559 | CLAFSYMDDV | 10 | HBV | adr | POL | 1118 | 0.0008 |
| 1.0632 | PLVLGTLEEV | 10 | MAGE | 1 | | 37 | 0.0008 |
| 1.0520 | NLATWVGSNL | 10 | HBV | adr | CORE | 457 | 0.0008 |
| 1.0400 | NLLTQIGCTL | 10 | HIV | | POL | 684 | 0.0007 |
| 1.0488 | GLTHIDAHFL | 10 | HCV | | LORF | 1564 | 0.0007 |
| 1.0733 | VLGSGAFGTV | 10 | c-ERB2 | | | 725 | 0.0007 |
| 1.0434 | QLIKKEKVYL | 10 | HIV | | POL | 1219 | 0.0006 |
| 1.0451 | KLLWKGEGAV | 10 | HIV | | POL | 1495 | 0.0006 |
| 1.0470 | SMVGNWAKVL | 10 | HCV | | ENV1 | 364 | 0.0006 |
| 1.0570 | KLIGTDNSW | 10 | HBV | adr | POL | 1317 | 0.0006 |
| 1.0924 | ILLVVVLGVV | 10 | c-ERB2 | | | 661 | 0.0006 |
| 1.0397 | LLDTGADDTV | 10 | HIV | | POL | 619 | 0.0005 |
| 1.0446 | HLKTAVQMAV | 10 | HIV | | POL | 1426 | 0.0005 |
| 1.0604 | DLLMGTLGIV | 10 | HPV | 16 | E7 | 81 | 0.0005 |
| 1.0443 | LLKLAGRG1PV | 10 | HIV | | POL | 1356 | 0.0004 |
| 1.0461 | DLMVTVYYGV | 10 | HIV | | ENV | 2181 | 0.0004 |
| 1.0619 | TLEKLTNTGL | 10 | HPV | 18 | E6 | 89 | 0.0004 |
| 1.0787 | SLTNLLSSDL | 10 | HBV | adw | POL | 1017 | 0.0004 |
| 1.0521 | NLEDPASREL | 10 | HBV | adr | CORE | 465 | 0.0003 |
| 1.0583 | GLSAMSTTDL | 10 | HBV | adr | 'X' | 1517 | 0.0003 |
| 1.0652 | VLVASRGRAV | 10 | PSA | | | 36 | 0.0003 |
| 1.0716 | DLSVFQNLQV | 10 | c-ERB2 | | | 421 | 0.0003 |
| 1.0723 | QLFRNPHQAL | 10 | c-ERB2 | | | 484 | 0.0003 |
| 1.0727 | PLTSIISAVV | 10 | c-ERB2 | | | 650 | 0.0003 |
| 1.0479 | YLICGSSGGPL | 10 | HCV | | LORF | 1160 | 0.0002 |
| 1.0497 | QLPCEPEPDV | 10 | HCV | | LORF | 2159 | 0.0002 |
| 1.0523 | CLTFGRETVL | 10 | HBV | adr | CORE | 497 | 0.0002 |
| 1.0603 | TLEDLLMGTL | 10 | HPV | 16 | E7 | 78 | 0.0002 |
| 1.0631 | SLHCKPEEAL | 10 | MAGE | 1 | | 7 | 0.0002 |
| 1.0680 | EMFRELNEAL | 10 | p53 | | | 339 | 0.0002 |
| 1.0689 | VLKDAIKDLV | 10 | BBNA1 | | | 574 | 0.0002 |
| 1.0757 | DLVDAEEYLV | 10 | c-ERB2 | | | 1016 | 0.0002 |
| 1.0796 | RMRGTFVSPL | 10 | HBV | adw | POL | 1317 | 0.0002 |
| 1.0669 | QLAKTCPVQL | 10 | p53 | | | 136 | 0.0001 |
| 1.0717 | NLQVIRGRIL | 10 | c-ERB2 | | | 427 | 0.0001 |
| 1.0721 | WLGLRSLREL | 10 | c-ERB2 | | | 452 | 0.0001 |
| 1.0522 | NMGLKIRQLL | 10 | HBV | adr | CORE | 482 | 0 |
| 1.0527 | PLSYQHFRKL | 10 | HBV | adr | POL | 576 | 0 |
| 1.0529 | ELPRLADEGL | 10 | HBV | adr | POL | 598 | 0 |
| 1.0531 | GLNRRVAEDL | 10 | HBV | adr | POL | 606 | 0 |
| 1.0536 | PLTVNEKRRL | 10 | HBV | adr | POL | 672 | 0 |
| 1.0539 | IMPARFYPNL | 10 | HBV | adr | POL | 684 | 0 |
| 1.0550 | PLHPAAMPHL | 10 | HBV | adr | POL | 1012 | 0 |
| 1.0552 | DLHDSCSRNL | 10 | HBV | adr | POL | 1051 | 0 |
| 1.0555 | LLYKTFGRKL | 10 | HBV | adr | POL | 1066 | 0 |
| 1.0557 | PMGVGLSPFL | 10 | HBV | adr | POL | 1090 | 0 |
| 1.0560 | VLGAKSVQHL | 10 | HBV | adr | POL | 1128 | 0 |
| 1.0569 | PLPIHTAELL | 10 | HBV | adr | POL | 1296 | 0 |
| 1.0579 | PLPSLAFSAV | 10 | HBV | adr | 'X' | 1454 | 0 |
| 1.0585 | DLEAYFKDCL | 10 | HBV | adr | 'X' | 1525 | 0 |
| 1.0587 | ELGEEIRLKV | 10 | HBV | adr | 'X' | 1540 | 0 |
| 1.0589 | VLGGCRHKLV | 10 | HBV | adr | 'X' | 1551 | 0 |
| 1.0597 | TLEQQYNKPL | 10 | HPV | 16 | E6 | 94 | 0 |
| 1.0608 | DLCTELNTSL | 10 | HPV | 18 | E6 | 16 | 0 |
| 1.0616 | RLQRRRETQV | 10 | HPV | 18 | E6 | 49 | 0 |
| 1.0621 | HLEPQNEIPV | 10 | HPV | 18 | E7 | 14 | 0 |
| 1.0639 | LLKYRARSPV | 10 | MAGE | 1/3 | | 114 | 0 |
| 1.0693 | CLGLSYDGLL | 10 | MARGE | 1/3 | | 174 | 0 |
| 1.0657 | DMSLLKNRFL | 10 | PSA | | | 98 | 0 |
| 1.0658 | LLRLSEPAEL | 10 | PSA | | | 119 | 0 |
| 1.0663 | PLSQETFSDL | 10 | p53 | | | 13 | 0 |
| 1.0669 | PLPSQAMDDL | 10 | p53 | | | 34 | 0 |
| 1.0690 | ELAALCRWGL | 10 | c-ERB2 | | | 2 | 0 |
| 1.0692 | RLPASPETHL | 10 | c-ERB2 | | | 34 | 0 |
| 1.0699 | RLRIVRGTQL | 10 | c-ERB2 | | | 98 | 0 |
| 1.0701 | GLRELQLRSL | 10 | c-ERB2 | | | 136 | 0 |
| 1.0730 | QMRILKETEL | 10 | c-ERB2 | | | 711 | 0 |
| 1.0732 | ILKETELRKV | 10 | c-ERB2 | | | 714 | 0 |
| 1.0754 | PLDSTFYRSL | 10 | c-ERB2 | | | 999 | 0 |
| 1.0755 | LLEDDDMGDL | 10 | c-ERB2 | | | 1008 | 0 |
| 1.0758 | DLGMGAAKGL | 10 | c-ERB2 | | | 1089 | 9 |
| 1.0761 | PLPSETDGYV | 10 | c-ERB2 | | | 1119 | 0 |
| 1.0763 | TLSPGKNGVV | 10 | c-ERB2 | | | 1172 | 0 |
| 1.0765 | TLQDPRVRAL | 10 | HBV | adw | ENV | 119 | 0 |
| 1.0768 | NMENIASGLL | 10 | HBV | adw | ENV | 163 | 0 |
| 1.0775 | ELPHLADEGL | 10 | HBV | adw | POL | 627 | 0 |
| 1.0776 | GLNRPVAEDL | 10 | HBV | adw | POL | 635 | 0 |
| 1.0777 | PLTVNENRRL | 10 | HBV | adw | POL | 701 | 0 |
| 1.0790 | LLYKTYGRKL | 10 | HBV | adw | POL | 1095 | 0 |
| 1.0801 | GLSAMSPTDL | 10 | HBV | adw | "X" | 1546 | 0 |
| 1.0802 | DLEAYFKDCV | 10 | HBV | adw | "X" | 1554 | 0 |
| 1.0803 | TLQDPRVRGL | 10 | HBV | ayw | ENV | 119 | 0 |
| 1.0804 | NMENITSGFL | 10 | HBV | ayw | ENV | 163 | 0 |
| 1.0891 | DLVNLLPAIL | 10 | HCV | | LORF | 1878 | 0 |
| 1.0404 | PLTEEKIKAL | 10 | HIV | | POL | 720 | <0.0002 |
| 1.0409 | QLGIPHPAGL | 10 | HIV | | POL | 786 | <0.0002 |
| 1.0411 | GLKKKKSVTV | 10 | HIV | | POL | 794 | <0.0002 |
| 1.0450 | PIWKGPAKLL | 10 | HIV | | POL | 1488 | <0.0002 |
| 1.0476 | DLAVAVEPVV | 10 | HCV | | LORF | 966 | <0.0002 |
| 1.0478 | SLTGRDKNQV | 10 | HCV | | LORF | 1046 | <0.0002 |
| 1.0490 | DLEVVTSTWV | 10 | HCV | | LORF | 1652 | <0.0002 |
| 1.0494 | GLGKVLIDIL | 10 | HCV | | LORF | 1843 | <0.0002 |
| 1.0505 | VLTTSCGNTL | 10 | HCV | | LORF | 2704 | <0.0002 |
| 1.0506 | ELITSCSSNV | 10 | HCV | | LORF | 2781 | <0.0002 |
| 1.0510 | CLRKLGVPPL | 10 | HCV | | LORF | 2908 | <0.0002 |
| 1.0511 | PLGFFPDHQL | 10 | HBV | adr | ENV | 10 | <0.0002 |
| 1.0514 | NMENTTSGFL | 10 | HBV | adr | ENV | 163 | <0.0002 |

| Appendix III PLP 8-mers | | | | | | | | | | | |
|---|---|---|---|---|---|---|---|---|---|---|---|
| Source | Peptide | AA | 1 | 2 | 3 | 4 | 5 | 6 | 7 | 8 | Algorithm Score (EO2) |
| Hu PLP | 10 | 8 | C | L | V | G | A | P | F | A | |
| Hu PLP | 13 | 8 | G | A | P | F | A | S | L | V | |
| Hu PLP | 23 | 8 | G | L | C | F | F | G | V | A | |
| Hu PLP | 39 | 8 | A | L | T | G | T | E | K | L | |
| Hu PLP | 40 | 8 | L | T | G | T | E | K | L | I | |
| Hu PLP | 60 | 8 | Y | L | I | N | V | I | H | A | |
| Ms PLP | 64 | 8 | V | I | H | A | F | Q | C | V | |
| Hu PLP | 64 | 8 | V | I | H | A | F | Q | Y | V | |
| Hu PLP | 74 | 8 | G | T | A | S | F | F | F | L | |
| Hu PLP | 80 | 8 | F | L | Y | G | A | L | L | L | |
| Hu PLP | 93 | 8 | T | T | G | A | V | R | Q | I | |
| Hu PLP | 106 | 8 | T | T | I | C | G | K | G | L | |
| Hu PLP | 131 | 8 | Q | A | H | S | L | E | R | V | |
| Hu PLP | 152 | 8 | F | V | G | I | T | Y | A | L | |
| Hu PLP | 154 | 8 | G | I | T | Y | A | L | T | V | |
| Hu PLP | 155 | 8 | I | T | Y | A | L | T | V | V | |
| Hu PLP | 157 | 8 | Y | A | L | T | V | V | W | L | |
| Hu PLP | 158 | 8 | A | L | T | V | V | W | L | L | |
| Hu PLP | 159 | 8 | L | T | V | V | W | L | L | V | |
| Hu PLP | 164 | 8 | L | L | V | F | A | C | S | A | |
| Hu PLP | 165 | 8 | L | V | F | A | C | S | A | V | |
| Hu PLP | 167 | 8 | F | A | C | S | A | V | P | V | |
| Hu PLP | 199 | 8 | S | L | C | A | D | A | R | M | |
| Hu PLP | 203 | 8 | D | A | R | M | Y | G | V | L | |
| Hu PLP | 212 | 8 | W | I | A | F | P | G | K | V | |
| Hu PLP | 218 | 8 | K | V | C | G | S | N | L | L | |
| Hu PLP | 224 | 8 | L | L | S | I | C | K | T | A | |
| Hu PLP | 234 | 8 | Q | M | T | F | H | L | F | I | |
| Hu PLP | 238 | 8 | H | L | F | I | A | A | F | V | |
| Hu PLP | 244 | 8 | F | V | G | A | A | A | T | L | |
| Hu PLP | 247 | 8 | A | A | A | T | L | V | S | L | |
| Hu PLP | 248 | 8 | A | A | T | L | V | S | L | L | |
| Hu PLP | 253 | 8 | S | L | L | T | F | M | I | A | |
| Hu PLP | 254 | 8 | L | L | T | F | M | I | A | A | |
| Hu PLP | 260 | 8 | A | A | T | Y | N | F | A | V | |
| Hu PLP | 261 | 8 | A | T | Y | N | F | A | V | L | |

| Appendix III MBP 8-mers | | | | | | | | | | | |
|---|---|---|---|---|---|---|---|---|---|---|---|
| Source | Peptide | AA | 1 | 2 | 3 | 4 | 5 | 6 | 7 | 8 | Algorithm Score (EO2) |
| Hu MBP | 14 | 8 | Y | L | A | T | A | S | T | M | |
| Hu MBP | 34 | 8 | D | T | G | I | L | D | S | I | |
| Hu MBP | 65 | 8 | R | T | A | H | Y | G | S | L | |
| Ms MBP | 70 | 8 | H | A | R | S | R | P | G | L | |
| Hu MBP | 79 | 8 | R | T | Q | D | E | N | P | V | |
| Hu MBP | 86 | 8 | V | V | H | F | F | K | N | I | |
| Ms MBP | 87 | 8 | R | T | T | H | Y | G | S | L | |
| Hu MBP | 143 | 8 | G | V | D | A | Q | G | T | L | |
| Hu MBP | 149 | 8 | T | L | S | K | I | F | K | L | |

| Appendix III PLP 9-mers | | | | | | | | | | | | |
|---|---|---|---|---|---|---|---|---|---|---|---|---|
| Source | Peptide | AA | 1 | 2 | 3 | 4 | 5 | 6 | 7 | 8 | 9 | Algorithm Score (EO2) |
| Hu PLP | 163 | 9 | W | L | L | V | F | A | C | S | A | -18.67 |
| Hu PLP | 205 | 9 | R | M | Y | G | V | L | P | W | I | -18.79 |
| Hu PLP | 145 | 9 | W | L | G | H | P | D | K | F | V | -19.05 |
| Hu PLP | 253 | 9 | S | L | L | T | F | M | I | A | A | -19.07 |
| Hu PLP | 251 | 9 | L | V | S | L | L | T | F | M | I | -20.03 |
| Hu PLP | 258 | 9 | M | I | A | A | T | Y | N | F | A | -20.32 |
| Hu PLP | 80 | 9 | F | L | Y | G | A | L | L | L | A | -20.53 |
| Ms PLP | 205 | 9 | R | M | Y | G | V | L | P | W | N | -20.69 |
| Hu PLP | 64 | 9 | V | I | H | A | F | Q | Y | V | I | -20.71 |
| Hu PLP | 23 | 9 | G | L | C | F | F | G | V | A | L | -21.23 |
| Ms PLP | 23 | 9 | G | L | C | F | F | G | V | A | L | -21.23 |
| Ms PLP | 179 | 9 | N | T | W | T | T | C | Q | S | I | -21.24 |
| Hu PLP | 233 | 9 | F | Q | M | T | F | H | L | F | I | -21.25 |
| Hu PLP | 234 | 9 | Q | M | T | F | H | L | F | I | A | -21.29 |
| Hu PLP | 259 | 9 | I | A | A | T | Y | N | F | A | V | -21.32 |
| Hu PLP | 157 | 9 | Y | A | L | T | V | V | W | L | L | -21.51 |
| Hu PLP | 76 | 9 | A | S | F | F | F | L | Y | G | A | -21.52 |
| Hu PLP | 158 | 9 | A | L | T | V | V | W | L | L | V | -21.56 |
| Hu PLP | 252 | 9 | V | S | L | L | T | F | M | I | A | -21.58 |
| Hu PLP | 237 | 9 | F | H | L | F | I | A | A | F | V | -21.61 |
| Ms PLP | 208 | 9 | G | V | L | P | W | N | A | F | P | -21.61 |
| Hu PLP | 164 | 9 | L | L | V | F | A | C | S | A | V | -21.81 |
| Hu PLP | 78 | 9 | F | F | F | L | Y | G | A | L | L | -22.05 |
| Hu PLP | 250 | 9 | T | L | V | S | L | L | T | F | M | -22.10 |
| Hu PLP | 208 | 9 | G | V | L | P | W | I | A | F | P | -22.10 |
| Hu PLP | 39 | 9 | A | L | T | G | T | E | K | L | I | -22.13 |
| Hu PLP | 240 | 9 | F | I | A | A | F | V | G | A | A | -22.19 |
| Hu PLP | 235 | 9 | M | T | F | H | L | F | I | A | A | -22.22 |
| Hu PLP | 244 | 9 | F | V | G | A | A | A | T | L | V | -22.22 |
| Ms PLP | 64 | 9 | V | I | H | A | F | Q | C | V | I | -22.33 |
| Hu PLP | 12 | 9 | V | G | A | P | F | A | S | L | V | -22.36 |
| Hu PLP | 45 | 9 | K | L | I | E | T | Y | F | S | K | -22.42 |
| Hu PLP | 30 | 9 | A | L | F | C | G | C | G | H | E | -22.46 |
| Hu PLP | 9 | 9 | R | C | L | V | G | A | P | F | A | -22.52 |
| Hu PLP | 189 | 9 | F | P | S | K | T | S | A | S | I | -22.54 |
| Hu PLP | 71 | 9 | V | I | Y | G | T | A | S | F | F | -22.60 |
| Hu PLP | 73 | 9 | Y | G | T | A | S | F | F | F | L | -22.63 |
| Hu PLP | 11 | 9 | L | V | G | A | P | F | A | S | L | -22.64 |
| Hu PLP | 86 | 9 | L | L | A | E | G | F | Y | T | T | -22.65 |
| Ms PLP | 63 | 9 | N | V | I | H | A | F | Q | C | V | -22.65 |
| Hu PLP | 212 | 9 | W | I | A | F | P | G | K | V | C | -22.67 |
| Hu PLP | 223 | 9 | N | L | L | S | I | C | K | T | A | -22.68 |
| Hu PLP | 199 | 9 | S | L | C | A | D | A | R | M | Y | -22.71 |
| Hu PLP | 179 | 9 | N | T | W | T | T | C | D | S | I | -22.73 |
| Hu PLP | 201 | 9 | C | A | D | A | R | M | Y | G | V | -22.74 |
| Hu PLP | 112 | 9 | G | L | S | A | T | V | T | G | G | -22.78 |
| Hu PLP | 161 | 9 | V | V | W | L | L | V | F | A | C | -22.78 |
| Hu PLP | 175 | 9 | Y | I | Y | F | N | T | W | T | T | -22.81 |
| Hu PLP | 56 | 9 | Q | D | Y | E | Y | L | I | N | V | -22.84 |
| Hu PLP | 241 | 9 | I | A | A | F | V | G | A | A | A | -22.87 |
| Hu PLP | 154 | 9 | G | I | T | Y | A | L | T | V | V | -22.89 |
| Hu PLP | 257 | 9 | F | M | I | A | A | T | Y | N | F | -22.89 |
| Hu PLP | 196 | 9 | S | I | G | S | L | C | A | D | A | -22.90 |
| Hu PLP | 18 | 9 | S | L | V | A | T | G | L | C | F | -22.91 |
| Hu PLP | 261 | 9 | A | T | Y | N | F | A | V | L | K | -23.00 |
| Hu PLP | 171 | 9 | A | V | P | V | Y | I | Y | F | N | -23.05 |
| Hu PLP | 70 | 9 | Y | V | I | Y | G | T | A | S | F | -23.11 |
| Hu PLP | 22 | 9 | T | G | L | C | F | F | G | V | A | -23.12 |
| Hu PLP | 134 | 9 | S | L | E | R | V | C | H | C | L | -23.16 |
| Hu PLP | 16 | 9 | F | A | S | L | V | A | T | G | L | -23.20 |
| Hu PLP | 74 | 9 | G | T | A | S | F | F | F | L | Y | -23.20 |
| Hu PLP | 79 | 9 | F | F | L | Y | G | A | L | L | L | -23.24 |
| Hu PLP | 246 | 9 | G | A | A | A | T | L | V | S | L | -23.26 |
| Hu PLP | 181 | 9 | W | T | T | C | D | S | I | A | F | -23.27 |
| Hu PLP | 28 | 9 | G | V | A | L | F | C | G | C | G | -23.31 |
| Hu PLP | 247 | 9 | A | A | A | T | L | V | S | L | L | -23.31 |
| Hu PLP | 219 | 9 | V | C | G | S | N | L | L | S | I | -23.33 |
| Hu PLP | 160 | 9 | T | V | V | W | L | L | V | F | A | -23.40 |
| Hu PLP | 54 | 9 | N | Y | Q | D | Y | E | Y | L | I | -23.43 |
| Hu PLP | 107 | 9 | T | I | C | G | K | G | L | S | A | -23.45 |
| Hu PLP | 166 | 9 | V | F | A | C | S | A | V | P | V | -23.53 |
| Hu PLP | 2 | 9 | G | L | L | E | C | C | A | R | C | -23.57 |
| Hu PLP | 167 | 9 | F | A | C | S | A | V | P | V | Y | -23.60 |
| Hu PLP | 260 | 9 | A | A | T | Y | N | F | A | V | L | -23.61 |
| Hu PLP | 152 | 9 | F | V | G | I | T | Y | A | L | T | -23.63 |
| Hu PLP | 187 | 9 | I | A | F | P | S | K | T | S | A | -23.64 |
| Hu PLP | 63 | 9 | N | V | I | H | A | F | Q | Y | V | -23.65 |
| Hu PLP | 60 | 9 | Y | L | I | N | V | I | H | A | F | -23.66 |
| Hu PLP | 85 | 9 | L | L | L | A | E | G | F | Y | T | -23.66 |
| Ms PLP | 210 | 9 | L | P | W | N | A | F | P | G | K | -23.66 |
| Hu PLP | 198 | 9 | G | S | L | C | A | D | A | R | M | -23.67 |
| Hu PLP | 20 | 9 | V | A | T | G | L | C | F | F | G | -23.71 |
| Hu PLP | 263 | 9 | Y | N | F | A | V | L | K | L | M | -23.71 |
| Ms PLP | 209 | 9 | V | L | P | W | N | A | F | P | G | -23.71 |
| Hu PLP | 84 | 9 | A | L | L | L | A | E | G | F | Y | -23.73 |
| Hu PLP | 206 | 9 | M | Y | G | V | L | P | W | I | A | -23.77 |
| Hu PLP | 153 | 9 | V | G | I | T | Y | A | L | T | V | -23.80 |
| Hu PLP | 269 | 9 | K | L | M | G | R | G | T | K | F | -23.92 |
| Hu PLP | 138 | 9 | V | C | H | C | L | G | K | W | L | -23.99 |
| Hu PLP | 3 | 9 | L | L | E | C | C | A | R | C | L | -24.02 |
| Hu PLP | 92 | 9 | Y | T | T | G | A | V | R | Q | I | -24.40 |
| Hu PLP | 21 | 9 | A | T | G | L | C | F | F | G | V | -24.47 |
| Hu PLP | 192 | 9 | K | T | S | A | S | I | G | S | L | -24.74 |
| Hu PLP | 38 | 9 | E | A | L | T | G | T | E | K | L | -25.72 |
| Hu PLP | 105 | 9 | K | T | T | I | C | G | K | G | L | -26.97 |

| Appendix III MBP 9-mers | | | | | | | | | | | | |
|---|---|---|---|---|---|---|---|---|---|---|---|---|
| Source | Peptide | AA | 1 | 2 | 3 | 4 | 5 | 6 | 7 | 8 | 9 | Algorithm Score (EO2) |
| Hu MBP | 110 | 9 | S | L | S | R | F | S | W | G | A | -21.42 |
| Hu MBP | 14 | 9 | Y | L | A | T | A | S | T | M | D | -22.01 |
| Ms MBP | 59 | 9 | W | L | K | Q | S | R | S | P | L | -22.60 |
| Hu MBP | 86 | 9 | V | V | H | F | F | K | N | I | V | -22.80 |
| Ms MBP | 52 | 9 | R | G | S | G | K | V | P | W | L | -22.87 |
| Hu MBP | 16 | 9 | A | T | A | S | T | M | D | H | A | -23.11 |
| Hu MBP | 37 | 9 | I | L | D | S | I | G | R | F | F | -23.11 |
| Hu MBP | 108 | 9 | G | L | S | L | S | R | F | S | W | -23.34 |
| Hu MBP | 93 | 9 | I | V | T | P | R | T | P | P | P | -23.41 |
| Ms MBP | 63 | 9 | S | R | S | P | L | P | S | H | A | -23.47 |
| Hu MBP | 79 | 9 | R | T | Q | D | E | N | P | V | V | -23.49 |
| Hu MBP | 129 | 9 | G | R | A | S | D | Y | K | S | A | -23.53 |
| Hu MBP | 21 | 9 | M | D | H | A | R | H | G | F | L | -23.60 |
| Hu MBP | 160 | 9 | D | S | R | S | G | S | P | M | A | -23.63 |
| Ms MBP | 75 | 9 | P | G | L | C | H | M | Y | K | D | -23.64 |
| Hu MBP | 112 | 9 | S | R | F | S | W | G | A | E | G | -23.77 |
| Hu MBP | 162 | 9 | R | S | G | S | P | M | A | R | R | -23.77 |
| Hu MBP | 159 | 9 | R | D | S | R | S | G | S | P | M | -23.81 |
| Hu MBP | 85 | 9 | P | V | V | H | F | F | K | N | I | -23.82 |
| Hu MBP | 136 | 9 | S | A | H | K | G | F | K | G | V | -23.90 |
| Hu MBP | 149 | 9 | T | L | S | K | I | F | K | L | G | -23.90 |
| Ms MSP | 162 | 9 | K | G | F | K | G | A | Y | D | A | -23.92 |
| Hu MBP | 64 | 9 | A | R | T | A | H | Y | G | S | L | -23.99 |
| Ms MBP | 166 | 9 | G | A | Y | D | A | Q | G | T | L | -24.66 |
| Hu MBP | 148 | 9 | G | T | L | S | K | I | F | K | L | -24.78 |
| Hu MBP | 145 | 9 | D | A | Q | G | T | L | S | K | I | -25.25 |

| Appendix III PLP 10-mers | | | | | | | | | | | | | |
|---|---|---|---|---|---|---|---|---|---|---|---|---|---|
| Source | Peptide | AA | 1 | 2 | 3 | 4 | 5 | 6 | 7 | 8 | 9 | 10 | Algorithm Score (EO2) |
| Ms PLP | 178 | 10 | F | N | T | W | T | T | C | Q | S | I | -24.68 |
| Hu PLP | 178 | 10 | F | N | T | W | T | T | C | D | S | I | -25.14 |
| Hu PLP | 204 | 10 | A | R | M | Y | G | V | L | P | W | I | -25.48 |
| Hu PLP | 163 | 10 | W | L | L | V | F | A | C | S | A | V | -25.66 |
| Hu PLP | 218 | 10 | K | V | C | G | S | N | L | L | S | I | -25.89 |
| Hu PLP | 250 | 10 | T | L | V | S | L | L | T | F | M | I | -26.00 |
| Hu PLP | 19 | 10 | L | V | A | T | G | L | C | F | F | G | -26.25 |
| Hu PLP | 78 | 10 | F | F | F | L | Y | G | A | L | L | L | -26.68 |
| Hu PLP | 157 | 10 | Y | A | L | T | V | V | W | L | L | V | -26.72 |
| Hu PLP | 84 | 10 | A | L | L | L | A | E | G | F | Y | T | -26.77 |
| Hu PLP | 233 | 10 | F | Q | M | T | F | H | L | F | I | A | -26.78 |
| Hu PLP | 80 | 10 | F | L | Y | G | A | L | L | L | A | E | -26.79 |
| Hu PLP | 167 | 10 | F | A | C | S | A | V | P | V | Y | I | -27.28 |
| Hu PLP | 165 | 10 | L | V | F | A | C | S | A | V | P | V | -27.32 |
| Hu PLP | 4 | 10 | L | E | C | C | A | R | C | L | V | G | -27.36 |
| Hu PLP | 253 | 10 | S | L | L | T | F | M | I | A | A | T | -27.42 |
| Hu PLP | 135 | 10 | L | E | R | V | C | H | C | L | G | K | -27.48 |
| Hu PLP | 176 | 10 | I | Y | F | N | T | W | T | T | C | D | -27.62 |
| Hu PLP | 24 | 10 | L | C | F | F | G | V | A | L | F | C | -27.74 |
| Hu PLP | 146 | 10 | L | G | H | P | D | K | F | V | G | I | -27.88 |
| Hu PLP | 237 | 10 | F | H | L | F | I | A | A | F | V | G | -27.95 |
| Hu PLP | 56 | 10 | Q | D | Y | E | Y | L | I | N | V | I | -27.99 |
| Ms PLP | 204 | 10 | A | R | M | Y | G | V | L | P | W | N | -28.01 |
| Hu PLP | 158 | 10 | A | L | T | V | V | W | L | L | V | F | -28.04 |
| Hu PLP | 137 | 10 | R | V | C | H | C | L | G | K | W | L | -28.15 |
| Hu PLP | 72 | 10 | I | Y | G | T | A | S | F | F | F | L | -28.16 |
| Hu PLP | 63 | 10 | N | V | I | H | A | F | Q | Y | V | I | -28.17 |
| Hu PLP | 208 | 10 | G | V | L | P | W | I | A | F | P | G | -28.17 |
| Hu PLP | 27 | 10 | F | G | V | A | L | F | C | G | C | G | -28.29 |
| Hu PLP | 85 | 10 | L | L | L | A | E | G | F | Y | T | T | -28.32 |
| Ms PLP | 62 | 10 | I | N | V | I | H | A | F | Q | C | V | -28.33 |
| Hu PLP | 222 | 10 | S | N | L | L | S | I | C | K | T | A | -28.40 |
| Hu PLP | 76 | 10 | A | S | F | F | F | L | Y | G | A | L | -28.43 |
| Ms PLP | 208 | 10 | G | V | L | P | W | N | A | F | P | G | -28.45 |
| Hu PLP | 207 | 10 | Y | G | V | L | P | W | I | A | F | P | -28.46 |
| Hu PLP | 79 | 10 | F | F | L | Y | G | A | L | L | L | A | -28.49 |
| Hu PLP | 236 | 10 | T | F | H | L | F | I | A | A | F | V | -28.50 |
| Hu PLP | 240 | 10 | F | I | A | A | F | V | G | A | A | A | -28.51 |
| Hu PLP | 181 | 10 | W | T | T | C | D | S | I | A | F | P | -28.56 |
| Hu PLP | 224 | 10 | L | L | S | I | C | K | T | A | E | F | -28.56 |
| Hu PLP | 10 | 10 | C | L | V | G | A | P | F | A | S | L | -28.62 |
| Hu PLP | 152 | 10 | F | V | G | I | T | Y | A | L | T | V | -28.64 |
| Hu PLP | 62 | 10 | I | N | V | I | H | A | F | Q | Y | V | -28.64 |
| Hu PLP | 214 | 10 | A | F | P | G | K | V | C | G | S | N | -28.65 |
| Hu PLP | 188 | 10 | A | F | P | S | K | T | S | A | S | I | -28.65 |
| Hu PLP | 99 | 10 | Q | I | F | G | D | Y | K | T | T | I | -28.69 |
| Hu PLP | 18 | 10 | S | L | V | A | T | G | L | C | F | F | -28.73 |
| Hu PLP | 3 | 10 | L | L | E | C | C | A | R | C | L | V | -28.75 |
| Hu PLP | 17 | 10 | A | S | L | V | A | T | G | L | C | F | -28.76 |
| Hu PLP | 144 | 10 | K | W | L | G | H | P | D | K | F | V | -28.78 |
| Ms PLP | 181 | 10 | W | T | T | C | Q | S | I | A | F | P | -28.78 |
| Hu PLP | 159 | 10 | L | T | V | V | W | L | L | V | F | A | -28.79 |
| Hu PLP | 174 | 10 | V | Y | I | Y | F | N | T | W | T | T | -28.80 |
| Hu PLP | 248 | 10 | A | A | T | L | V | S | L | L | T | F | -28.84 |
| Hu PLP | 23 | 10 | G | L | C | F | F | G | V | A | L | F | -28.87 |
| Hu PLP | 209 | 10 | V | L | P | W | I | A | F | P | G | K | -28.87 |
| Hu PLP | 29 | 10 | V | A | L | F | C | G | C | G | H | E | -28.90 |
| Hu PLP | 261 | 10 | A | T | Y | N | F | A | V | L | K | L | -28.92 |
| Ms PLP | 63 | 10 | N | V | I | H | A | F | Q | C | V | I | -28.93 |
| Hu PLP | 74 | 10 | G | T | A | S | F | F | F | L | Y | G | -28.93 |
| Hu PLP | 259 | 10 | I | A | A | T | Y | N | F | A | V | L | -29.06 |
| Hu PLP | 242 | 10 | A | A | F | V | G | A | A | A | T | L | -29.24 |
| Hu PLP | 2 | 10 | G | L | L | E | C | C | A | R | C | L | -29.30 |
| Hu PLP | 257 | 10 | F | M | I | A | A | T | Y | N | F | A | -29.37 |
| Hu PLP | 20 | 10 | V | A | T | G | L | C | F | F | G | V | -29.41 |
| Ms PLP | 205 | 10 | R | M | Y | G | V | L | P | W | N | A | -29.43 |
| Hu PLP | 155 | 10 | I | T | Y | A | L | T | V | V | W | L | -29.60 |
| Hu PLP | 30 | 10 | A | L | F | C | G | C | G | H | E | A | -29.70 |
| Hu PLP | 205 | 10 | R | M | Y | G | V | L | P | W | I | A | -29.74 |
| Hu PLP | 258 | 10 | M | I | A | A | T | Y | N | F | A | V | -30.06 |
| Hu PLP | 234 | 10 | Q | M | T | F | H | L | F | I | A | A | -30.29 |
| Hu PLP | 238 | 10 | H | L | F | I | A | A | F | V | G | A | -30.64 |
| Hu PLP | 246 | 10 | G | A | A | A | T | L | V | S | L | L | -30.64 |
| Hu PLP | 38 | 10 | E | A | L | T | G | T | E | K | L | I | -30.92 |
| Hu PLP | 230 | 10 | T | A | E | F | Q | M | T | F | H | L | -31.03 |
| Hu PLP | 11 | 10 | L | V | G | A | P | F | A | S | L | V | -31.25 |
| Hu PLP | 201 | 10 | C | A | D | A | R | M | Y | G | V | L | -31.73 |

| Appendix III PLP 11-mers | | | | | | | | | | | | | | |
|---|---|---|---|---|---|---|---|---|---|---|---|---|---|---|
| Source | Peptide | AA | 1 | 2 | 3 | 4 | 5 | 6 | 7 | 8 | 9 | 10 | 11 | Algorithm Score (EO2) |
| Hu PLP | 2 | 11 | G | L | L | E | C | C | A | R | C | L | V | |
| Hu PLP | 10 | 11 | C | L | V | G | A | P | F | A | S | L | V | |
| Hu PLP | 19 | 11 | L | V | A | T | G | L | C | F | F | G | V | |
| Hu PLP | 21 | 11 | A | T | G | L | C | F | F | G | V | A | L | |
| Hu PLP | 30 | 11 | A | L | F | C | G | C | G | H | E | A | L | |
| Hu PLP | 61 | 11 | L | I | N | V | I | H | A | F | Q | Y | V | |
| Ms PLP | 61 | 11 | L | I | N | V | I | H | A | F | Q | C | V | |
| Hu PLP | 71 | 11 | V | I | Y | G | T | A | S | F | F | F | L | |
| Hu PLP | 75 | 11 | T | A | S | F | F | F | L | Y | G | A | L | |
| Hu PLP | 86 | 11 | L | L | A | E | G | F | Y | T | T | G | A | |
| Hu PLP | 87 | 11 | L | A | E | G | F | Y | T | T | G | A | V | |
| Hu PLP | 107 | 11 | T | I | C | G | K | G | L | S | A | T | V | |
| Hu PLP | 145 | 11 | W | L | G | H | P | D | K | F | V | G | I | |
| Hu PLP | 152 | 11 | F | V | G | I | T | Y | A | L | T | V | V | |
| Hu PLP | 154 | 11 | G | I | T | Y | A | L | T | V | V | W | L | |
| Hu PLP | 155 | 11 | I | T | Y | A | L | T | V | V | W | L | L | |
| Hu PLP | 158 | 11 | A | L | T | V | V | W | L | L | V | F | A | |
| Hu PLP | 164 | 11 | L | L | V | F | A | C | S | A | V | P | V | |
| Hu PLP | 187 | 11 | I | A | F | P | S | K | T | S | A | S | I | |
| Hu PLP | 199 | 11 | S | L | C | A | D | A | R | M | Y | G | V | |
| Hu PLP | 203 | 11 | D | A | R | M | Y | G | V | L | P | W | I | |
| Hu PLP | 209 | 11 | V | L | P | W | I | A | F | P | G | K | V | |
| Ms PLP | 209 | 11 | V | L | P | W | N | A | F | P | G | K | V | |
| Hu PLP | 229 | 11 | K | T | A | E | F | Q | M | T | F | H | L | |
| Hu PLP | 235 | 11 | M | T | F | H | L | F | I | A | A | F | V | |
| Hu PLP | 238 | 11 | H | L | F | I | A | A | F | V | G | A | A | |
| Hu PLP | 241 | 11 | I | A | A | F | V | G | A | A | A | T | L | |
| Hu PLP | 242 | 11 | A | A | F | V | G | A | A | A | T | L | V | |
| Hu PLP | 244 | 11 | F | V | G | A | A | A | T | L | V | S | L | |
| Hu PLP | 249 | 11 | A | T | L | V | S | L | L | T | F | M | I | |
| Hu PLP | 250 | 11 | T | L | V | S | L | L | T | F | M | I | A | |
| Hu PLP | 257 | 11 | F | M | I | A | A | T | Y | N | F | A | V | |
| Hu PLP | 258 | 11 | M | I | A | A | T | Y | N | F | A | V | L | |
| Hu PLP | 260 | 11 | A | A | T | Y | N | F | A | V | L | K | L | |

| AppendixIII MBP 10-mers | | | | | | | | | | | | | |
|---|---|---|---|---|---|---|---|---|---|---|---|---|---|
| Source | Peptide | AA | 1 | 2 | 3 | 4 | 5 | 6 | 7 | 8 | 9 | 10 | Algorithm Score (EO2) |
| Hu MBP | 37 | 10 | I | L | D | S | I | G | R | F | F | G | -27.66 |
| Hu MBP | 28 | 10 | F | L | P | R | H | R | D | T | G | I | -27.85 |
| Ms MBP | 167 | 10 | A | Y | D | A | Q | G | T | L | S | K | -28.54 |
| Hu MSP | 89 | 10 | F | F | K | N | I | V | T | P | R | T | -28.68 |
| Hu MBP | 14 | 10 | Y | L | A | T | A | S | T | M | D | H | -28.75 |
| Hu MBP | 84 | 10 | N | P | V | V | H | F | F | K | N | I | -28.80 |
| Hu MBP | 32 | 10 | H | R | D | T | G | I | L | D | S | I | -28.83 |
| Hu MBP | 110 | 10 | S | L | S | R | F | S | W | G | A | E | -28.98 |
| Hu MBP | 85 | 10 | P | V | V | H | F | F | K | N | I | V | -30.82 |
| Ms MBP | 85 | 10 | H | T | R | T | T | H | Y | G | S | L | -31.29 |
| Hu MBP | 20 | 10 | T | M | D | H | A | R | H | G | F | L | -31.40 |
| Hu MBP | 63 | 10 | P | A | R | T | A | H | Y | G | S | L | -31.76 |
| Ms MBP | 48 | 10 | G | A | P | K | R | G | S | G | K | V | -32.21 |

| Appendix III MBP 11-mers | | | | | | | | | | | | | | |
|---|---|---|---|---|---|---|---|---|---|---|---|---|---|---|
| Source | Peptide | AA | 1 | 2 | 3 | 4 | 5 | 6 | 7 | 8 | 9 | 10 | 11 | Algorithm Score (EO2) |
| Hu MBP | 14 | 11 | Y | L | A | T | A | S | T | M | D | H | A | |
| Hu MBP | 19 | 11 | S | T | M | D | H | A | R | H | G | F | L | |
| Hu MBP | 28 | 11 | F | L | P | R | H | R | D | T | G | I | L | |
| Hu MBP | 108 | 11 | G | L | S | L | S | R | F | S | W | G | A | |
| Hu MBP | 143 | 11 | G | V | D | A | Q | G | T | L | S | K | I | |

## Claims

1. A method of making an immunogenic peptide comprising an epitope that bears an HLA-A2,1 structural motif, the method comprising:
providing an amino acid sequence of an antigen of interest;
identifying within said sequence an epitope consisting of 8-11 amino acid residues that comprises an HLA-A2.1 motif, said motif comprising a first amino acid residue at a position two relative to an amino terminus of the epitope, said first amino acid residue selected from the group consisting of V, A, and T; and a second amino acid residue at a carboxyl terminus of the epitope, said second amino acid residue selected from the group consisting of L, I, V, M and A;
obtaining a peptide that comprises the epitope;
testing a complex of the peptide and an HLA-A2.1 molecule for an ability to be recognized by HLA-A2.1-restricted cytotoxic T cells and to thereby induce a cytotoxic T cell response to the epitope; and,
selecting the peptide that induces a cytotoxic T cell response to the epitope.

2. A method of claim 1, further comprising:
determining binding affinity of the peptide for an HLA-A2.1 molecule; and
selecting the peptide that comprises a relative binding ratio of at least 0.01 for the HLA-A2.1 molecule.

3. A method according to claim 1 or 2, wherein the obtaining step comprises obtaining the peptide as a peptide of 8, 9, 10 or 11 amino acid residues.

4. A method according to claim 1 or 2, wherein the obtaining step comprises obtaining the peptide comprised by a peptide longer than 11 amino acid residues, with a proviso that the peptide longer than 11 amino acid residues is not an entire native antigen.

5. A method according to any of claims 1-4, wherein the antigen of interest is a cancer-associated antigen.

6. A method of claim 5, wherein the cancer-associated antigen is HER2/neu, p53, MAGE, or a prostate antigen.

7. A method according to any of claims 1-4, wherein the antigen of interest is from a pathogenic agent.

8. A method of claim 7, wherein the pathogenic agent is HIV, HBV, HCV or HPV.

9. A method according to any of claims 1-8, wherein the obtaining step comprises expressing in a cell a recombinant nucleic acid molecule that encodes the peptide.

10. An in vitro method of inducing an HLA-A2.1-restricted cytotoxic T cell response, said method comprising:
providing a peptide comprising an epitope of 8-11 amino acid residues that comprises an HLA-A2.1 motif, said motif comprising a first amino acid residue at a position two relative to an amino terminus of the epitope, said first amino acid residue selected from the group consisting of V, A, and T; and a second amino acid residue at a carboxyl terminus of the epitope, said second amino acid residue selected from the group consisting of L, I, V, M and A;
said peptide linked to another molecule to create a compound, with a proviso that neither said peptide, said another molecule, nor said compound comprise an entire native antigen;
complexing the peptide or an epitope-comprising fragment thereof with an HLA-A2.1 molecule whereby a complex is created; and,
contacting a cytotoxic T lymphocyte (CTL) with the complex, whereby a CTL response is induced to the complex.

11. The method of claim 10, wherein the providing step comprises providing a peptide that binds to the HLA-A2.1 molecule at a relative binding affinity ratio of at least 0.01.

12. A method of claim 10 or 11, wherein the another molecule is a CTL epitope.

13. A method of claim 10 or 11, wherein the another molecule is a helper T lymphocyte (HTL) epitope.

14. A method of claim 13, wherein the HTL epitope is a Pan-DR binding epitope.

15. A method of claim 10 or 11, wherein the another molecule is a lipid.

16. A method of claim 10 or 11, wherein the another molecule is a carrier molecule.

17. A method according to any of claims 10-16, wherein the providing step comprises providing a peptide 8, 9, 10 or 11 amino acid residues in length.

18. A method according to any of claims 10-16, wherein the providing step comprises providing a peptide greater than 11 amino acid residues in length.

19. A method of claim 10, wherein the antigen of interest is a cancer-associated antigen.

20. A method of claim 19, wherein the cancer-associated antigen is HER2-neu, p53, MAGE, or a prostate antigen.

21. A method according to any of claims 10-16, wherein the antigen of interest is from a pathogenic agent.

22. A method of claim 21, wherein the pathogenic agent is HIV, HBV, HCV or HPV.

23. A method according to any of claims 10-22, wherein the providing step comprises expressing a recombinant nucleic acid molecule that encodes the peptide and the another molecule, with a proviso that neither an additional peptide nor a combination of the peptide and an additional peptide comprise an entire native antigen.

## Patentansprüche

1. Verfahren zur Herstellung eines immunogenen Peptids, das ein Epitop umfasst, das ein HLA-A2.1-Strukturmotiv trägt, wobei das Verfahren umfasst:
Bereitstellen einer Aminosäuresequenz eines Antigens von Interesse;
Identifizieren innerhalb der Sequenz ein Epitop, das aus 8-11 Aminosäureresten besteht, das ein HLA-A2.1-Motiv umfasst, wobei das Motiv einen ersten Aminosäurerest an einer Position zwei relativ zu einem Aminoterminus des Epitops, wobei der erste Aminosäurerest aus der Gruppe, bestehend aus V, A und T, ausgewählt ist, und einen zweiten Aminosäurerest am Carboxylterminus des Epitops umfasst, wobei der zweite Aminosäurerest aus der Gruppe, bestehend aus L, I, V, M und A, ausgewählt ist;
Gewinnen eines Peptids, das das Epitop umfasst;
Untersuchen eines Komplexes des Peptids und eines HLA-A2.1-Moleküls auf die Fähigkeit, durch HLA-A2.1-restringierte cytotoxische T-Zellen erkannt zu werden, und **dadurch** eine cytotoxische T-Zellreaktion auf das Epitop zu induzieren, und
Auswählen des Peptids, das eine cytotoxische T-Zellantwort auf das Epitop induziert.

2. Verfahren gemäß Anspruch 1, außerdem umfassend:
Bestimmen der Bindungsaffinität des Peptids für ein HLA-A2.1-Molekül und
Auswählen des Peptids, das ein relatives Bindungsverhältnis von wenigstens 0,01 für das HLA-A2.1-Molekül hat.

3. Verfahren gemäß Anspruch 1 oder 2, wobei der Schritt des Gewinnens Gewinnen des Peptids als ein Peptid aus 8, 9, 10 oder 11 Aminosäureresten umfasst.

4. Verfahren gemäß Anspruch 1 oder 2, wobei der Schritt des Gewinnens ein Gewinnen des Peptids, bestehend aus einem Peptid, das länger als 11 Aminosäurereste ist, mit der Maßgabe, dass das Peptid, das länger als 11 Aminosäurereste ist, kein ganzes natives Antigen ist, umfasst.

5. Verfahren gemäß einem der Ansprüche 1 bis 4, wobei das Antigen von Interesse ein Krebs-assoziiertes Antigen ist.

6. Verfahren gemäß Anspruch 5, wobei das Krebs-assoziierte Antigen HER2/neu, p53, MAGE oder ein Prostataantigen ist.

7. Verfahren gemäß einem der Ansprüche 1 bis 4, wobei das Antigen von Interesse von einem pathogenen Agens ist.

8. Verfahren gemäß Anspruch 7, wobei das pathogene Agens HIV, HBV, HCV oder HPV ist.

9. Verfahren gemäß einem der Ansprüche 1 bis 8, wobei der Schritt des Gewinnens Exprimieren eines rekombinanten Nucleinsäuremoleküls, das das Peptid codiert, in einer Zelle umfasst.

10. In vitro-Verfahren zum Induzieren der Reaktion einer HLA-A2.1-restringierten cytotoxischen T-Zelle, wobei das Verfahren umfasst:
Bereitstellen eines Peptids, das ein Epitop aus 8 bis 11 Aminosäureresten umfasst, das ein HLA-A2.1-Motiv umfasst, wobei das Motiv einen ersten Aminosäurerest an einer Position zwei relativ zu einem Aminoterminus des Epitops, wobei der erste Aminosäurerest aus der Gruppe, bestehend aus V, A und T, ausgewählt ist, und einen zweiten Aminosäurerest am Carboxylterminus des Epitops umfasst, wobei der zweite Aminosäurerest aus der Gruppe, bestehend aus L, I, V, M und A, ausgewählt ist;
das Peptid unter Bildung einer Verbindung an ein anderes Molekül gebunden wird, mit der Maßgabe, dass weder das Peptid, das andere Molekül, noch die Verbindung ein ganzes natives Antigen umfasst;
Komplexieren des Peptids oder eines Epitop-umfassenden Fragments davon mit einem HLA-A2.1-Molekül, wodurch ein Komplex gebildet wird, und
Inkontaktbringen eines cytotoxischen T-Lymphozyten (CTL) mit dem Komplex, wodurch eine CTL-Reaktion auf den Komplex induziert wird.

11. Verfahren gemäß Anspruch 10, wobei der Schritt des Bereitstellens ein Bereitstellen eines Peptids umfasst, das an das HLA-A2.1-Molekül mit einem relativen Bindungsaffinitätsverhältnis von wenigstens 0,01 bindet.

12. Verfahren gemäß Anspruch 10 oder 11, wobei das andere Molekül ein CTL-Epitop ist.

13. Verfahren gemäß Anspruch 10 oder 11, wobei das andere Molekül ein Helfer-T-Lymphozyten (HTL)-Epitop ist.

14. Verfahren gemäß Anspruch 13, wobei das HTL-Epitop ein Pan-DR-Bindungsepitop ist.

15. Verfahren gemäß Anspruch 10 oder 11, wobei das andere Molekül ein Lipid ist.

16. Verfahren gemäß Anspruch 10 oder 11, wobei das andere Molekül ein Trägermolekül ist.

17. Verfahren gemäß einem der Ansprüche 10 bis 16, wobei der Schritt des Bereitstellens Bereitstellen eines Peptids mit einer Länge von 8, 9, 10 oder 11 Aminosäureresten umfasst.

18. Verfahren gemäß einem der Ansprüche 10 bis 16, wobei der Schritt des Bereitstellens ein Bereitstellen eines Peptids mit einer Länge von größer als 11 Aminosäureresten umfasst.

19. Verfahren gemäß Anspruch 10, wobei das Antigen von Interesse ein Krebs-assoziiertes Antigen ist.

20. Verfahren gemäß Anspruch 19, wobei das Krebs-assoziierte Antigen HER2-neu, p53, MAGE oder ein Prostataantigen ist.

21. Verfahren gemäß einem der Ansprüche 10 bis 16, wobei das Antigen von Interesse von einem pathogenen Agens ist.

22. Verfahren gemäß Anspruch 21, wobei das pathogene Agens HIV, HBV, HCV oder HPV ist.

23. Verfahren gemäß einem der Ansprüche 10 bis 22, wobei der Schritt des Bereitstellens ein Exprimieren eines rekombinanten Nucleinsäuremoleküls, das das Peptid und das andere Molekül codiert, umfasst, mit der Maßgabe, dass weder ein zusätzliches Peptid noch eine Kombination des Peptids und eines zusätzlichen Peptids ein ganzes natives Antigen umfasst.

## Revendications

1. Méthode pour préparer un peptide immunogène comprenant un épitope qui porte un motif structurel HLA-A2.1, la méthode comprenant:
fourniture d'une séquence d'acides aminés d'un antigène d'intérêt;
identification au sein de ladite séquence d'un épitope consistant en 8 à 11 résidus d'acide aminé qui comprend un motif HLA-A2.1, ledit motif comprenant un premier résidu d'acide aminé à une position deux par rapport à une terminaison amine de l'épitope, ledit premier résidu d'acide aminé étant sélectionné dans le groupe consistant en V, A, et T; et un second résidu d'acide aminé à une terminaison carboxyle de l'épitope, ledit second résidu d'acide aminé étant sélectionné dans le groupe consistant en L, I, V, M and A;
obtention d'un peptide qui comprend l'épitope;
analyse d'une capacité d'un complexe du peptide et d'une molécule HLA-A2.1 d'être reconnu par des cellules T cytotoxiques à restriction HLA-A2.1 et à de ce fait provoquer une réponse de cellule T cytotoxique à l'épitope; et
sélection du peptide qui provoque une réponse de cellule T cytotoxique à l'épitope.

2. Méthode selon la revendication 1, comprenant en outre:
détermination de l'affinité de liaison du peptide pour une molécule HLA-A2.1; et
sélection du peptide qui comprend un rapport de liaison relatif d'au moins 0,01 pour la molécule HLA-A2.1

3. Méthode selon la revendication 1 ou 2, dans laquelle l'étape d'obtention comprend l'obtention du peptide comme un peptide de 8, 9,10 ou 11 résidus d'acide aminé.

4. Méthode selon la revendication 1 ou 2, dans laquelle l'étape d'obtention comprend l'obtention du peptide compris dans un peptide ayant une longueur de plus de 11 résidus d'acide aminé, à condition que le peptide ayant une longueur de plus de 11 résidus d'acide aminé ne soit pas un antigène natif entier.

5. Méthode selon l'une quelconque des revendications 1 à 4, dans laquelle l'antigène d'intérêt est un antigène associé au cancer.

6. Méthode selon la revendication 5, dans laquelle l'antigène associé au cancer est HER2/neu, p53, MAGE, ou un antigène de prostate.

7. Méthode selon l'une quelconque des revendications 1 à 4, dans laquelle l'antigène d'intérêt provient d'un agent pathogène.

8. Méthode selon la revendication 7, dans laquelle l'agent pathogène est HIV, HBV, HCV ou HPV.

9. Méthode selon l'une quelconque des revendications 1 à 8, dans laquelle l'étape d'obtention comprend l'expression dans une cellule d'une molécule d'acide nucléique recombinante qui encode le peptide.

10. Méthode in-vitro pour provoquer une réponse de cellule T cytotoxique à restriction HLA-A2.1, ladite méthode comprenant:
fourniture d'un peptide comprenant un épitope de 8 à 11 résidus d'acide aminé qui comprend un motif HLA-A2.1, ledit motif comprenant un premier résidu d'acide aminé à une position deux par rapport à une terminaison amine de l'épitope, ledit premier résidu d'acide aminé étant sélectionné dans le groupe consistant en V, A, et T; et un second résidu d'acide aminé à une terminaison carboxyle de l'épitope, ledit second résidu d'acide aminé étant sélectionné dans le groupe consistant en L, I, V, M et A;
ledit peptide étant lié à une autre molécule pour créer un composé, à condition que ni ledit peptide, ni ladite autre molécule, ni ledit composé ne comprenne un antigène natif entier;
complexation du peptide ou d'un fragment de celui-ci comprenant épitope avec une molécule HLA-A2.1, créant ainsi un complexe; et
contact d'un lymphocyte T cytotoxique (CTL) avec le complexe, provoquant ainsi une réponse de CTL au complexe.

11. Méthode selon la revendication 10, dans laquelle l'étape de fourniture comprend la fourniture d'un peptide qui se lie à la molécule HLA-A2.1 à un rapport d'affinité de liaison relatif d'au moins 0,01.

12. Méthode selon la revendication 10 ou 11, dans laquelle l'autre molécule est un épitope de CTL.

13. Méthode selon la revendication 10 ou 11, dans laquelle l'autre molécule est un épitope de lymphocyte T auxiliaire (HTL).

14. Méthode selon la revendication 13, dans laquelle l'épitope de HTL est un épitope de liaison Pan-DR.

15. Méthode selon la revendication 10 ou 11, dans laquelle l'autre molécule est un lipide.

16. Méthode selon la revendication 10 ou 11, dans laquelle l'autre molécule est une molécule porteuse.

17. Méthode selon l'une quelconque des revendications 10 à 16, dans laquelle l'étape de fourniture comprend la fourniture d'un peptide ayant une longueur de 8, 9, 10 ou 11 résidus d'acide aminé.

18. Méthode selon l'une quelconque des revendications 10 à 16, dans laquelle l'étape de fourniture comprend la fourniture d'un peptide ayant une longueur de plus de 11 résidus d'acide aminé.

19. Méthode selon la revendication 10, dans laquelle l'antigène d'intérêt est un antigène associé au cancer.

20. Méthode selon la revendication 19, dans laquelle l'antigène associé au cancer est HER2-neu, p53, MAGE, ou un antigène de prostate.

21. Méthode selon l'une quelconque des revendications 10 à 16, dans laquelle l'antigène d'intérêt provient d'un agent pathogène.

22. Méthode selon la revendication 21, dans laquelle l'agent pathogène est HIV, HBV, HCV ou HPV.

23. Méthode selon l'une quelconque des revendications 10 à 22, dans laquelle l'étape de fourniture comprend l'expression d'une molécule d'acide nucléique recombinante qui encode le peptide et l'autre molécule, à condition que ni un peptide supplémentaire ni une combinaison du peptide et d'un peptide supplémentaire ne comprenne un antigène natif entier.


### SEQUENCE LISTING

(1) GENERAL INFORMATION:
   (i) APPLICANT:
      (A) NAME: Cytel Corporation
      (B) STREET: 3525 John Hopkins Court
      (C) CITY: San Diego
      (D) STATE: California.
      (E) COUNTRY: USA
      (F) POSTAL CODE (ZIP): CA 92121
   (ii) TITLE OF INVENTION: HLA-A2.1 binding peptides and their uses
   (iii) NUMBER OF SEQUENCES: 1043
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (v) CURRENT APPLICATION DATA:
      APPLICATION NUMBER: EP 94910837.7
   (vi) PRIOR APPLICATION DATA:
      (A) APPLICATION NUMBER: US 08/027,146 (B) FILING DATE: 05-MAR-1993
   (vi) PRIOR APPLICATION DATA:
      (A) APPLICATION NUMBER: US 08/073,205 (B) FILING DATE: 04-JUN-1993
   (vi) PRIOR APPLICATION DATA:
      (A) APPLICATION NUMBER: US 08/159,184 (B) FILING DATE: 29-NOV-1993
(2) INFORMATION FOR SEQ ID NO:1:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:1:
   Met Met Trp Phe Val Val Leu Thr Val
   1               5
(2) INFORMATION FOR SEQ ID NO:2:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:2:
   Tyr Leu Leu Leu Tyr Phe Ser Pro Val
   1               5
(2) INFORMATION FOR SEQ ID NO:3:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:3:
   Tyr Leu Tyr Arg Leu Asn Phe Cys Leu
   1 5
(2) INFORMATION FOR SEQ ID NO:4:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:4:
   Phe Met Trp Thr Tyr Leu Val Thr Leu
   1 5
(2) INFORMATION FOR SEQ ID NO:5:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:5:
   Leu Leu Trp Trp Ile Thr Ile Leu Leu
   1 5
(2) INFORMATION FOR SEQ ID NO:6:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:6:
   Gly Leu Trp Cys Val Leu Phe Phe Val
   1 5
(2) INFORMATION FOR SEQ ID NO:7:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:7:
   Leu Met Ile Arg Gly Val Leu Glu Val
   1               5
(2) INFORMATION FOR SEQ ID NO:8:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:8:
   Leu Leu Leu Cys Arg Leu Pro Phe Leu
   1               5
(2) INFORMATION FOR SEQ ID NO:9:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (x) SEQUENCE DESCRIPTION: SEQ ID NO:9:
   Arg Leu Leu Thr Ser Leu Phe Phe Leu
   1               5
(2) INFORMATION FOR SEQ ID NO:10:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:10:
   Leu Leu Leu Tyr Tyr Asp Tyr Ser Leu
   1 5
(2) INFORMATION FOR SEQ ID NO:11:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:11:
   Ala Met Ser Arg Asn Leu Phe Arg Val
   1 5
(2) INFORMATION FOR SEQ ID NO:12:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:12:
   Ala Met Leu Thr Ala Cys Val Glu Val
   1 5
(2) INFORMATION FOR SEQ ID NO:13:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:13:
   Arg Leu Gln Pro Asn Val Pro Leu Val
   1 5
(2) INFORMATION FOR SEQ ID NO:14:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:14:
   Val Leu Ala Arg Thr Phe Thr Pro Val
   1 5
(2) INFORMATION FOR SEQ ID NO:15:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:15:
   Arg Leu Leu Arg Gly Leu Ile Arg Leu
   1 5
(2) INFORMATION FOR SEQ ID NO:16:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:16:
   Trp Met Trp Phe Pro Ser Val Leu Leu
   1 5
(2) INFORMATION FOR SEQ ID NO:17:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:17:
   Tyr Leu Cys Cys Gly Ile Thr Leu Leu
   1 5
(2) INFORMATION FOR SEQ ID NO:18:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:18:
   Asp Met Leu Gly Arg Val Phe Phe Val
   1 5
(2) INFORMATION FOR SEQ ID NO:19:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:19:
   Ala Leu Gly Arg Tyr Gln Gln Leu Val
   1 5
(2) INFORMATION FOR SEQ ID NO:20:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:20:
   Leu Met Pro Pro Pro Val Ala Glu Leu
   1 5
(2) INFORMATION FOR SEQ ID NO:21:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:21:
   Leu Met Cys Arg Tyr Thr Pro Arg Leu
   1               5
(2) INFORMATION FOR SEQ ID NO:22:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:22:
   Arg Leu Thr Trp Arg Leu Thr Trp Leu
   1               5
(2) INFORMATION FOR SEQ ID NO:23:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:23:
   Ala Met Pro Arg Arg Val Leu His Val
   1               5
(2) INFORMATION FOR SEQ ID NO:24:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:24:
   Ala Leu Leu Leu Val Leu Ala Leu Leu
   1 5
(2) INFORMATION FOR SEQ ID NO:25:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:25:
   Ala Met Ser Gly Thr Gly Thr Thr Leu
   1 5
(2) INFORMATION FOR SEQ ID NO:26:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:26:
   Met Leu Asn Val Met Lys Glu Ala Val
   1 5
(2) INFORMATION FOR SEQ ID NO:27:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:27:
   Thr Met Glu Leu Met Ile Arg Thr Val
   1 5
(2) INFORMATION FOR SEQ ID NO:28:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:28:
   Thr Leu Ala Ala Met His Ser Lys Leu
   1 5
(2) INFORMATION FOR SEQ ID NO:29:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:29:
   Thr Leu Asn Ile Val Arg Asp His Val
   1 5
(2) INFORMATION FOR SEQ ID NO:30:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:30:
   Glu Leu Ser Ile Phe Arg Glu Arg Leu
   1 5
(2) INFORMATION FOR SEQ ID NO:31:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:31:
   Phe Leu Arg Val Gln Gln Lys Ala Leu
   1 5
(2) INFORMATION FOR SEQ ID NO:32:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:32:
   Glu Leu Gln Met Met Gln Asp Trp Val
   1 5
(2) INFORMATION FOR SEQ ID NO:33:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:33:
   Gln Leu Asn Ala Met Lys Pro Asp Leu
   1 5
(2) INFORMATION FOR SEQ ID NO:34:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:34:
   Gly Leu Arg Gln Leu Lys Gly Ala Leu
   1 5
(2) INFORMATION FOR SEQ ID NO:35:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:35:
   Thr Leu Arg Met Ser Ser Lys Ala Val
   1               5
(2) INFORMATION FOR SEQ ID NO:36:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:36:
   Ser Leu Arg Ile Lys Arg Glu Leu Leu
   1               5
(2) INFORMATION FOR SEQ ID NO:37:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:37:
   Asp Leu Lys Gln Met Glu Arg Val Val
   1               5
(2) INFORMATION FOR SEQ ID NO:38:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:38:
   Pro Leu Arg Val Thr Pro Ser Asp Leu
   1 5
(2) INFORMATION FOR SEQ ID NO:39:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:39:
   Gln Leu Asp Tyr Glu Lys Gln Val Leu
   1 5
(2) INFORMATION FOR SEQ ID NO:40:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:40:
   Trp Leu Lys Leu Leu Arg Asp Ala Leu
   1 5
(2) INFORMATION FOR SEQ ID NO:41:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:41:
   Pro Met Glu Ala Val Arg His Pro Leu
   1 5
(2) INFORMATION FOR SEQ ID NO:42:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:42:
   Glu Leu Lys Gln Thr Arg Val Asn Leu
   1               5
(2) INFORMATION FOR SEQ ID NO:43:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:43:
   Asn Leu Glu Val Ile His Asp Ala Leu
   1               5
(2) INFORMATION FOR SEQ ID NO:44:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:44:
   Glu Leu Lys Lys Val Lys Ser Val Leu
   1               5
(2) INFORMATION FOR SEQ ID NO:45:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:45:
   Pro Leu Ala Tyr Glu Arg Asp Lys Leu
   1 5
(2) INFORMATION FOR SEQ ID NO:46:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:46:
   Phe Leu Leu Ser Leu Gly Ile His Leu
   1 5
(2) INFORMATION FOR SEQ ID NO:47:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:47:
   Gly Leu Tyr Ser Ser Thr Val Pro Val
   1 5
(2) INFORMATION FOR SEQ ID NO:48:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:48:
   His Leu Tyr Ser His Pro Ile Ile Leu
   1 5
(2) INFORMATION FOR SEQ ID NO:49:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:49:
   Trp Ile Leu Arg Gly Thr Ser Phe Val
   1               5
(2) INFORMATION FOR SEQ ID NO:50:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:50:
   Asn Leu Ser Trp Leu Ser Leu Asp Val
   1               5
(2) INFORMATION FOR SEQ ID NO:51:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:51:
   Leu Leu Ser Ser Asn Leu Ser Trp Leu
   1               5
(2) INFORMATION FOR SEQ ID NO:52:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:52:
   Asn Leu Gln Ser Leu Thr Asn Leu Leu
   1               5
(2) INFORMATION FOR SEQ ID NO:53:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:53:
   His Leu Leu Val Gly Ser Ser Gly Leu
   1 5
(2) INFORMATION FOR SEQ ID NO:54:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:54:
   Leu Leu Asp Asp Glu Ala Gly Pro Leu
   1 5
(2) INFORMATION FOR SEQ ID NO:55:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:55:
   Pro Leu Glu Glu Glu Leu Pro Arg Leu
   1 5
(2) INFORMATION FOR SEQ ID NO:56:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:56:
   Asp Leu Asn Leu Gly Asn Leu Asn Val
   1 5
(2) INFORMATION FOR SEQ ID NO:57:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:57:
   Asn Leu Tyr Val Ser Leu Leu Leu Leu
   1 5
(2) INFORMATION FOR SEQ ID NO:58:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:58:
   Pro Leu Pro Ile His Thr Ala Glu Leu
   1 5

(2) INFORMATION FOR SEQ ID NO:59:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:59:
   Ile Leu Ser Pro Phe Leu Pro Leu Leu
   1 5
(2) INFORMATION FOR SEQ ID NO:60:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:60:
   Thr Leu Gln Asp Ile Val Leu His Leu
   1 5
(2) INFORMATION FOR SEQ ID NO:61:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:61:
   Trp Leu Ser Leu Leu Val Pro Phe Val
   1 5
(2) INFORMATION FOR SEQ ID NO:62:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:62:
   Phe Leu Leu Ser Leu Gly Ile His Leu
   1 5
(2) INFORMATION FOR SEQ ID NO:63:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:63:
   Gln Leu Phe Glu Asp Asn Tyr Ala Leu
   1 5
(2) INFORMATION FOR SEQ ID NO:64:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:64:
   Leu Met Val Thr Val Tyr Tyr Gly Val
   1 5
(2) INFORMATION FOR SEQ ID NO:65:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:65:
   Met Met Trp Phe Trp Gly Pro Ser Leu
   1 5
(2) INFORMATION FOR SEQ ID NO:66:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:66:
   Met Met Trp Tyr Trp Gly Pro Ser Leu
   1 5
(2) INFORMATION FOR SEQ ID NO:67:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:67:
   Phe Leu Thr Lys Gln Tyr Leu Asn Leu
   1               5
(2) INFORMATION FOR SEQ ID NO:68:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:68:
   Ile Leu His Asn Gly Ala Tyr Ser Leu
   1               5
(2) INFORMATION FOR SEQ ID NO:69:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:69:
   Leu Leu Leu Cys Leu Ile Phe Leu Leu
   1               5
(2) INFORMATION FOR SEQ ID NO:70:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:70:
   Gly Leu Tyr Ser Ser Thr Val Pro Val
   1 5
(2) INFORMATION FOR SEQ ID NO:71:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:71:
   His Leu Tyr Ser His Pro Ile Ile Leu
   1 5
(2) INFORMATION FOR SEQ ID NO:72:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:72:
   Arg Met Pro Glu Ala Ala Pro Pro Val
   1 5
(2) INFORMATION FOR SEQ ID NO:73:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:73:
   Leu Leu Met Gly Thr Leu Gly Ile Val
   1 5
(2) INFORMATION FOR SEQ ID NO:74:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:74:
   Ile Leu Ser Pro Phe Met Pro Leu Leu
   1               5
(2) INFORMATION FOR SEQ ID NO:75:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:75:
   Tyr Leu Val Ala Tyr Gln Ala Thr Val
   1               5
(2) INFORMATION FOR SEQ ID NO:76:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:76:
   Val Leu Leu Asp Tyr Gln Gly Met Leu
   1               5
(2) INFORMATION FOR SEQ ID NO:77:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:77:
   Val Leu Ala Glu Ala Met Ser Gln Val
   1 5
(2) INFORMATION FOR SEQ ID NO:78:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:78:
   Arg Leu Leu Gln Glu Thr Glu Leu Val
   1 5
(2) INFORMATION FOR SEQ ID NO:79:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:79:
   Ser Leu Tyr Ala Val Ser Pro Ser Val
   1 5
(2) INFORMATION FOR SEQ ID NO:80:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:80:
   Asp Leu Met Gly Tyr Ile Pro Leu Val
   1 5
(2) INFORMATION FOR SEQ ID NO:81:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:81:
   Leu Leu Cys Leu Ile Phe Leu Leu Val
   1 5
(2) INFORMATION FOR SEQ ID NO:82:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:82:
   Phe Leu Cys Gln Gln Tyr Leu His Leu
   1 5
(2) INFORMATION FOR SEQ ID NO:83:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:83:
   Asn Leu Tyr Val Ser Leu Met Leu Leu
   1 5
(2) INFORMATION FOR SEQ ID NO:84:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:84:
   Gly Met Leu Pro Val Cys Pro Leu Leu
   1 5
(2) INFORMATION FOR SEQ ID NO:85:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:85:
   Ile Leu Pro Cys Ser Phe Thr Thr Leu
   1 5
(2) INFORMATION FOR SEQ ID NO:86:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:86:
   Ala Leu Ser Thr Gly Leu Ile His Leu
   1 5
(2) INFORMATION FOR SEQ ID NO:87:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:87:
   Ile Leu Leu Leu Cys Leu Ile Phe Leu
   1 5
(2) INFORMATION FOR SEQ ID NO:88:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:88:
   His Leu Tyr Gln Gly Cys Gln Val Val
   1 5
(2) INFORMATION FOR SEQ ID NO:89:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:89:
   Cys Leu Thr Ser Thr Val Gln Leu Val
   1 5
(2) INFORMATION FOR SEQ ID NO:90:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:90:
   Pro Leu Leu Pro Ile Phe Phe Cys Leu
   1 5
(2) INFORMATION FOR SEQ ID NO:91:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:91:
   Ala Leu Cys Arg Trp Gly Leu Leu Leu
   1 5
(2) INFORMATION FOR SEQ ID NO:92:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:92:
   Val Leu Ile Gln Arg Asn Pro Gln Leu
   1 5
(2) INFORMATION FOR SEQ ID NO:93:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:93:
   Val Leu Gln Ala Cys Phe Phe Leu Leu
   1 5
(2) INFORMATION FOR SEQ ID NO:94:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:94:
   Leu Leu Trp Phe His Ile Ser Cys Leu
   1 5
(2) INFORMATION FOR SEQ ID NO:95:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:95:
   Ile Leu Ala Gly Tyr Gly Ala Gly Val
   1 5
(2) INFORMATION FOR SEQ ID NO:96:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:96:
   Gln Leu Met Pro Tyr Gly Cys Leu Leu
   1 5
(2) INFORMATION FOR SEQ ID NO:97:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:97:
   Leu Leu Trp Lys Gly Glu Gly Ala Val
   1 5
(2) INFORMATION FOR SEQ ID NO:98:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:98:
   Phe Leu Gly Gly Thr Pro Val Cys Leu
   1 5
(2) INFORMATION FOR SEQ ID NO:99:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:99:
   Ala Leu Ile His His Asn Thr His Leu
   1 5
(2) INFORMATION FOR SEQ ID NO:100:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:100:
   Val Leu Val His Pro Gln Trp Val Leu
   1 5
(2) INFORMATION FOR SEQ ID NO:101:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:101:
   Lys Leu Gln Cys Val Asp Leu His Val
   1 5
(2) INFORMATION FOR SEQ ID NO:102:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:102:
   Val Leu Pro Cys Ser Phe Thr Thr Leu
   1 5
(2) INFORMATION FOR SEQ ID NO:103:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:103:
   His Leu His Gln Asn Ile Val Asp Val
   1 5
(2) INFORMATION FOR SEQ ID NO:104:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:104:
   Ser Met Val Gly Asn Trp Ala Lys Val
   1 5
(2) INFORMATION FOR SEQ ID NO:105:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:105:
   Leu Leu Gly Arg Asn Ser Phe Glu Val
   1 5
(2) INFORMATION FOR SEQ ID NO:106:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:106:
   Gly Leu Tyr Arg Pro Leu Leu Ser Leu
   1 5
(2) INFORMATION FOR SEQ ID NO:107:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:107:
   Gly Leu Tyr Arg Pro Leu Leu Arg Leu
   1 5
(2) INFORMATION FOR SEQ ID NO:108:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:108:
   Asn Leu Ser Trp Leu Ser Leu Asp Val
   1 5
(2) INFORMATION FOR SEQ ID NO:109:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:109:
   Phe Leu Leu Leu Ala Asp Ala Arg Val
   1 5
(2) INFORMATION FOR SEQ ID NO:110:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:110:
   Tyr Leu Val Thr Arg His Ala Asp Val
   1 5
(2) INFORMATION FOR SEQ ID NO:111:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:111:
   Cys Leu Thr His Ile Val Asn Leu Leu
   1 5
(2) INFORMATION FOR SEQ ID NO:112:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:112:
   Glu Leu Met Asn Leu Ala Thr Trp Val
   1 5
(2) INFORMATION FOR SEQ ID NO:113:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:113:
   Lys Leu Pro Asp Leu Cys Thr Glu Leu
   1 5
(2) INFORMATION FOR SEQ ID NO:114:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:114:
   Ala Leu Gln Asp Ser Gly Leu Glu Val
   1 5
(2) INFORMATION FOR SEQ ID NO:115:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID N0:115:
(2) INFORMATION FOR SEQ ID NO:116:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:116:
   Ala Leu Ala His Gly Val Arg Val Leu
   1 5
(2) INFORMATION FOR SEQ ID NO:117:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:117:
   Thr Leu His Gly Pro Thr Pro Leu Leu
   1               5
(2) INFORMATION FOR SEQ ID NO:118:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:118:
   Lys Leu Leu Arg Gly Thr Lys Ala Leu
   1               5

(2) INFORMATION FOR SEQ ID NO:119:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:119:
   Cys Met Ser Ala Asp Leu Glu Val Val
   1               5
(2) INFORMATION FOR SEQ ID NO:120:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:120:
   Gly Leu Leu Gly Pro Leu Leu Val Leu
   1 5
(2) INFORMATION FOR SEQ ID NO:121:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:121:
   Thr Leu His Glu Tyr Met Leu Asp Leu
   1 5
(2) INFORMATION FOR SEQ ID NO:122:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:122:
   Lys Leu Leu Pro Glu Asn Asn Val Leu
   1 5
(2) INFORMATION FOR SEQ ID NO:123:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:123:
   Ile Leu Lys Glu Pro Val His Gly Val
   1 5
(2) INFORMATION FOR SEQ ID NO:124:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:124:
   Arg Leu Gly Leu Tyr Arg Pro Leu Leu
   1               5
(2) INFORMATION FOR SEQ ID NO:125:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:125:
   Gly Leu Pro Arg Tyr Val Ala Arg Leu
   1               5
(2) INFORMATION FOR SEQ ID NO:126:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:126:
   Lys Leu Ile Gly Thr Asp Asn Ser Val
   1               5
(2) INFORMATION FOR SEQ ID NO:127:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:127:
   Phe Leu Leu Ala Leu Leu Ser Cys Leu
   1 5
(2) INFORMATION FOR SEQ ID NO:128:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:128:
   Leu Leu Ser Ser Asn Leu Ser Trp Leu
   1 5
(2) INFORMATION FOR SEQ ID NO:129:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:129:
   Gln Leu Leu Arg Ile Pro Gln Ala Val
   1 5
(2) INFORMATION FOR SEQ ID NO:130:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:130:
   Gly Leu Arg Asp Leu Ala Val Ala Val
   1 5
(2) INFORMATION FOR SEQ ID NO:131:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:131:
   Thr Leu Asn Ala Trp Val Lys Val Ile
   1 5
(2) INFORMATION FOR SEQ ID NO:132:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:132:
   Asn Leu Gln Ser Leu Thr Asn Leu Leu
   1 5
(2) INFORMATION FOR SEQ ID NO:133:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:133:
   Gln Leu Gly Arg Lys Pro Thr Pro Leu
   1 5
(2) INFORMATION FOR SEQ ID NO:134:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:134:
   Ser Leu Asp Ser Trp Trp Thr Ser Leu
   1 5
(2) INFORMATION FOR SEQ ID NO:135:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:135:
   Ile Leu Ser Lys Thr Gly Asp Pro Val
   1 5
(2) INFORMATION FOR SEQ ID NO:136:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:136:
   Ile Leu Leu Val Val Val Leu Gly Val
   1 5
(2) INFORMATION FOR SEQ ID NO:137:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:137:
   Leu Leu His Lys Arg Thr Leu Gly Leu
   1 5
(2) INFORMATION FOR SEQ ID NO:138:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:138:
   Cys Leu Phe Lys Asp Trp Glu Glu Leu
   1 5
(2) INFORMATION FOR SEQ ID NO:139:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:139:
   Gly Leu Gly Ile Ser Trp Leu Gly Leu
   1 5
(2) INFORMATION FOR SEQ ID NO:140:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:140:
   His Leu Leu Val Gly Ser Ser Cys Leu
   1 5
(2) INFORMATION FOR SEQ ID NO:141:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:141:
   Thr Leu Glu Glu Ile Thr Gly Tyr Leu
   1 5
(2) INFORMATION FOR SEQ ID NO:142:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:142:
   Pro Leu Thr Ser Ile Ile Ser Ala Val
   1 5
(2) INFORMATION FOR SEQ ID NO:143:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:143:
   Leu Leu Gly Cys Ile Ile Thr Ser Leu
   1 5
(2) INFORMATION FOR SEQ ID NO:144:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:144:
   Ala Leu Asn Lys Met Phe Cys Gln Leu
   1 5
(2) INFORMATION FOR SEQ ID NO:145:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:145:
   His Leu Glu Gly Lys Ile Ile Leu Val
   1 5
(2) INFORMATION FOR SEQ ID NO:146:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:146:
   Gln Leu Arg Ser Leu Thr Glu Ile Leu
   1 5
(2) INFORMATION FOR SEQ ID NO:147:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:147:
   Asp Leu Ala Val Ala Val Glu Pro Val
   1 5
(2) INFORMATION FOR SEQ ID NO:148:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:148:
   Val Leu His Lys Arg Thr Leu Gly Leu
   1 5
(2) INFORMATION FOR SEQ ID NO:149:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:149:
   Phe Leu Cys Ile Leu Leu Leu Cys Leu
   1 5
(2) INFORMATION FOR SEQ ID NO:150:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:150:
   Leu Leu Asp Pro Arg Val Arg Gly Leu
   1 5
(2) INFORMATION FOR SEQ ID NO:151:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:151:
   Lys Leu Pro Gln Leu Cys Thr Glu Leu
   1 5
(2) INFORMATION FOR SEQ ID NO:152:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:152:
   His Leu Glu Gly Lys Val Ile Leu Val
   1               5
(2) INFORMATION FOR SEQ ID NO:153:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:153:
   Glu Met Met Thr Ala Cys Gln Gly Val
   1               5
(2) INFORMATION FOR SEQ ID NO:154:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:154:
   His Leu Ala Leu Gln Asp Ser Gly Leu
   1               5
(2) INFORMATION FOR SEQ ID NO:155:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:155:
   Val Leu Gly Val Val Pro Gly Ile Leu
   1 5
(2) INFORMATION FOR SEQ ID NO:156:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:156:
   Thr Leu Asn Ala Trp Val Lys Val Val
   1 5
(2) INFORMATION FOR SEQ ID NO:157:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:157:
   His Leu Glu Ser Leu Tyr Ala Ala Val
   1 5
(2) INFORMATION FOR SEQ ID NO:158:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:158:
   Cys Leu Ile Arg Leu Lys Pro Thr Leu
   1 5
(2) INFORMATION FOR SEQ ID NO:159:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:159:
   Cys Leu Gly Leu Ser Tyr Asp Gly Leu
   1 5
(2) INFORMATION FOR SEQ ID NO:160:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:160:
   His Leu Ser Leu Arg Gly Leu Pro Val
   1 5
(2) INFORMATION FOR SEQ ID NO:161:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:161:
   Ile Leu Glu Ser Leu Phe Arg Ala Val
   1 5
(2) INFORMATION FOR SEQ ID NO:162:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:162:
   Thr Leu Thr Cys Gly Phe Ala Asp Leu
   1 5
(2) INFORMATION FOR SEQ ID NO:163:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:163:
   Thr Leu Pro Ala Leu Ser Thr Gly Leu
   1 5
(2) INFORMATION FOR SEQ ID NO:164:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:164:
   Ala Leu Ala Ile Pro Gln Cys Arg Leu
   1 5
(2) INFORMATION FOR SEQ ID NO:165:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:165:
   Asp Leu Cys Cys Ser Val Phe Leu Val
   1 5
(2) INFORMATION FOR SEQ ID NO:166:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:166:
   Arg Leu Cys Val Gln Ser Thr His Val
   1               5
(2) INFORMATION FOR SEQ ID NO:167:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:167:
   Leu Leu Asp Asp Glu Ala Gly Pro Leu
   1               5
(2) INFORMATION FOR SEQ ID NO:168:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:168:
   Cys Leu Arg Arg Phe Ile Ile Phe Leu
   1               5
(2) INFORMATION FOR SEQ ID NO:169:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:169:
   Gly Leu Pro Val Cys Gln Asp His Leu
   1 5
(2) INFORMATION FOR SEQ ID NO:170:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:170:
   Pro Leu Glu Glu Glu Leu Pro Arg Leu
   1 5
(2) INFORMATION FOR SEQ ID NO:171:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:171:
   Pro Leu Leu Tyr Arg Leu Gly Ala Val
   1 5
(2) INFORMATION FOR SEQ ID NO:172:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:172:
   Glu Leu Ala Glu Asn Arg Glu Ile Leu
   1 5
(2) INFORMATION FOR SEQ ID NO:173:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:173:
   Glu Leu Gln Ala Ile His Leu Ala Leu
   1 5
(2) INFORMATION FOR SEQ ID NO:174:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:174:
   Leu Ile Pro Arg Arg Gly Pro Arg Leu
   1 5
(2) INFORMATION FOR SEQ ID NO:175:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:175:
   Asn Leu Gly Lys Val Ile Asp Thr Leu
   1 5
(2) INFORMATION FOR SEQ ID NO:176:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:176:
   Pro Leu Gly Gly Ala Ala Arg Ala Leu
   1 5
(2) INFORMATION FOR SEQ ID NO:177:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:177:
   Asp Leu Leu Asp Thr Ala Ser Ala Leu
   1 5
(2) INFORMATION FOR SEQ ID NO:178:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:178:
   Gln Leu Lys Gln Ser Arg Leu Gly Leu
   1 5

(2) INFORMATION FOR SEQ ID NO:179:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:179:
   Gly Leu Gln Pro Gln Gln Gly Ser Leu
   1 5
(2) INFORMATION FOR SEQ ID NO:180:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:180:
   Pro Leu Asp Gly Glu Tyr Phe Thr Leu
   1               5
(2) INFORMATION FOR SEQ ID NO:181:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:181:
   Val Leu Lys Asp Ala Ile Lys Asp Leu
   1               5
(2) INFORMATION FOR SEQ ID NO:182:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:182:
   Gln Leu Cys Tyr Gln Asp Thr Ile Leu
   1               5
(2) INFORMATION FOR SEQ ID NO:183:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:183:
   Asp Leu Asn Thr Met Leu Asn Thr Val
   1 5
(2) INFORMATION FOR SEQ ID NO:184:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:184:
   Glu Leu His Pro Asp Lys Trp Thr Val
   1 5
(2) INFORMATION FOR SEQ ID NO:185:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:185:
   Glu Leu Lys Lys Ile Ile Gly Gln Val
   1 5
(2) INFORMATION FOR SEQ ID NO:186:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:186:
   Glu Leu Val Val Ser Tyr Val Asn Val
   1 5
(2) INFORMATION FOR SEQ ID NO:187:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:187:
   Cys Leu Thr Pro Gly Arg Glu Thr Val
   1 5
(2) INFORMATION FOR SEQ ID NO:188:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:188:
   Asp Leu Asn Leu Gly Asn Leu Asn Val
   1 5
(2) INFORMATION FOR SEQ ID NO:189:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:189:
   Leu Leu Val Val Val Leu Gly Val Val
   1 5
(2) INFORMATION FOR SEQ ID NO:190:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:190:
   Asn Met Gly Leu Lys Ile Arg Gln Leu
   1 5
(2) INFORMATION FOR SEQ ID NO:191:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:191:
   Ser Leu Arg Glu Leu Gly Ser Gly Leu
   1 5
(2) INFORMATION FOR SEQ ID NO:192:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:192:
   Asp Leu Leu Glu Lys Gly Glu Arg Leu
   1 5
(2) INFORMATION FOR SEQ ID NO:193:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:193:
   Asp Leu Val Asp Ala Glu Glu Tyr Leu
   1 5
(2) INFORMATION FOR SEQ ID NO:194:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:194:
   Pro Leu Glu Glu Glu Leu Pro His Leu
   1 5
(2) INFORMATION FOR SEQ ID NO:195:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:195:
   Asp Leu Gln His Gly Arg Leu Val Leu
   1 5
(2) INFORMATION FOR SEQ ID NO:196:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:196:
   Pro Leu Pro Gly Pro Leu Gly Ala Leu
   1 5
(2) INFORMATION FOR SEQ ID NO:197:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:197:
   Leu Leu Thr Gln Ile Gly Cys Thr Leu
   1 5
(2) INFORMATION FOR SEQ ID NO:198:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:198:
   Pro Leu Val Lys Leu Trp Tyr Gln Leu
   1 5
(2) INFORMATION FOR SEQ ID NO:199:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:199:
   Phe Leu Phe Ile Leu Leu Leu Cys Leu
   1 5
(2) INFORMATION FOR SEQ ID NO:200:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:200:
   Asn Leu Tyr Val Ser Leu Leu Leu Leu
   1 5
(2) INFORMATION FOR SEQ ID NO:201:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:201:
   Pro Leu Gln Pro Glu Gln Leu Gln Val
   1 5
(2) INFORMATION FOR SEQ ID NO:202:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:202:
   Asp Leu Ala Ala Arg Asn Val Leu Val
   1 5
(2) INFORMATION FOR SEQ ID NO:203:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:203:
   Val Leu Val Lys Ser Pro Asn His Val
   1 5
(2) INFORMATION FOR SEQ ID NO:204:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:204:
   Thr Leu Ser Pro Gly Lys Asn Gly Val
   1 5
(2) INFORMATION FOR SEQ ID NO:205:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:205:
   Asp Leu Ser Trp Leu Ser Leu Asp Val
   1 5
(2) INFORMATION FOR SEQ ID NO:206:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:206:
   Asn Met Glu Asn Ile Ala Ser Gly Leu
   1 5
(2) INFORMATION FOR SEQ ID NO:207:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:207:
   Thr Leu Pro Gln Glu His Ile Val Leu
   1 5
(2) INFORMATION FOR SEQ ID NO:208:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:208:
   Lys Leu Lys Gln Cys Phe Arg Lys Leu
   1 5
(2) INFORMATION FOR SEQ ID NO:209:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:209:
   Pro Leu Pro Ile His Thr Ala Glu Leu
   1 5
(2) INFORMATION FOR SEQ ID NO:210:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:210:
   Gln Leu Asp Pro Ala Arg Asp Val Leu
   1 5
(2) INFORMATION FOR SEQ ID NO:211:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:211:
   Val Leu Gly Gly Cys Arg His Lys Leu
   1 5
(2) INFORMATION FOR SEQ ID NO:212:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:212:
   Asp Leu Gln Pro Glu Thr Thr Asp Leu
   1 5
(2) INFORMATION FOR SEQ ID NO:213:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:213:
   Ala Leu Glu Ala Gln Gln Glu Ala Leu
   1 5
(2) INFORMATION FOR SEQ ID NO:214:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:214:
   Asp Leu Pro Thr Gln Glu Pro Ala Leu
   1 5
(2) INFORMATION FOR SEQ ID NO:215:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:215:
   His Leu Ile Arg Val Glu Gly Asn Leu
   1 5
(2) INFORMATION FOR SEQ ID NO:216:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:216:
   Thr Leu Glu Asp Ser Ser Gly Asn Leu
   1 5
(2) INFORMATION FOR SEQ ID NO:217:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:217:
   Glu Leu Arg His Tyr Ser Asp Ser Val
   1 5
(2) INFORMATION FOR SEQ ID NO:218:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:218:
   Asp Leu His Val Ile Ser Asn Asp Val
   1 5
(2) INFORMATION FOR SEQ ID NO:219:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:219:
   Cys Leu Ile Phe Leu Leu Val Leu Leu
   1 5
(2) INFORMATION FOR SEQ ID NO:220:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:220:
   Leu Leu Phe Asn Ile Leu Gly Gly Trp Val
   1 5 10
(2) INFORMATION FOR SEQ ID NO:221:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:221:
   Leu Leu Val Pro Phe Val Gln Trp Phe Val
   1 5 10
(2) INFORMATION FOR SEQ ID NO:222:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:222:
   Leu Leu Ala Leu Leu Ser Cys Leu Thr Val
   1 5 10
(2) INFORMATION FOR SEQ ID NO:223:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:223:
   Ile Leu Leu Leu Cys Leu Ile Phe Leu Leu
   1 5 10
(2) INFORMATION FOR SEQ ID NO:224:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:224:
   Gly Leu Ser Pro Thr Val Trp Leu Ser Val
   1 5 10
(2) INFORMATION FOR SEQ ID NO:225:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:225:
   Ser Leu Tyr Asn Ile Leu Ser Pro Phe Leu
   1 5 10
(2) INFORMATION FOR SEQ ID NO:226:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:226:
   Leu Leu Val Leu Gln Ala Gly Phe Phe Leu
   1 5 10
(2) INFORMATION FOR SEQ ID NO:227:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:227:
   Phe Leu Gln Thr His Ile Glu Ala Glu Val
   1 5 10
(2) INFORMATION FOR SEQ ID NO:228:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:228:
   Gln Leu Phe Leu Asn Thr Leu Ser Phe Val
   1 5 10
(2) INFORMATION FOR SEQ ID NO:229:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:229:
   Leu Leu Pro Ile Phe Phe Cys Leu Trp Val
   1 5 10
(2) INFORMATION FOR SEQ ID NO:230:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:230:
   Leu Leu Leu Cys Leu Ile Phe Leu Leu Val
   1 5 10
(2) INFORMATION FOR SEQ ID NO:231:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:231:
   Leu Leu Asp Tyr Gln Gly Met Leu Pro Val
   1 5 10
(2) INFORMATION FOR SEQ ID NO:232:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:232:
   Trp Met Met Trp Tyr Trp Gly Pro Ser Leu
   1 5 10
(2) INFORMATION FOR SEQ ID NO:233:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:233:
   Gly Leu Tyr Ser Ser Thr Val Pro Val Leu
   1 5 10
(2) INFORMATION FOR SEQ ID NO:234:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:234:
   Tyr Leu Leu Pro Arg Arg Gly Pro Arg Leu
   1 5 10
(2) INFORMATION FOR SEQ ID NO:235:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:235:
   Gly Leu Leu Gly Cys Ile Ile Thr Ser Leu
   1 5 10
(2) INFORMATION FOR SEQ ID NO:236:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:236:
   Ile Leu Cys Trp Gly Glu Leu Met Asn Leu
   1 5 10
(2) INFORMATION FOR SEQ ID NO:237:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:237:
   Leu Leu Gly Ile Cys Leu Thr Ser Thr Val
   1 5 10
(2) INFORMATION FOR SEQ ID NO:238:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:238:
   Leu Leu Trp Lys Gly Glu Gly Ala Val Val
   1 5 10

(2) INFORMATION FOR SEQ ID NO:239:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:239:
   Leu Leu Ala Leu Leu Ser Cys Leu Thr Ile
   1 5 10
(2) INFORMATION FOR SEQ ID NO:240:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:240:
   Lys Leu Thr Asn Thr Gly Leu Tyr Asn Leu
   1 5 10
(2) INFORMATION FOR SEQ ID NO:241:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:241:
   Arg Leu Ile Val Phe Pro Asp Leu Gly Val
   1 5 10
(2) INFORMATION FOR SEQ ID NO:242:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:242:
(2)³ INFORMATION FOR SEQ ID NO:243:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:243:
(2) INFORMATION FOR SEQ ID NO:244:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:244:
   Ser Leu Asn Phe Leu Gly Gly Thr Pro Val
   1 5 10
(2) INFORMATION FOR SEQ ID NO:245:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:245:
   Ser Leu Gln Asp Ile Glu Ile Thr Cys Val
   1 5 10
(2) INFORMATION FOR SEQ ID NO:246:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:246:
   Ile Leu Ser Thr Leu Pro Glu Thr Thr Val
   1 5 10
(2) INFORMATION FOR SEQ ID NO:247:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:247:
   Arg Leu His Gly Leu Ser Ala Phe Ser Leu
   1 5 10
(2) INFORMATION FOR SEQ ID NO:248:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:248:
   Ile Leu Gly Gly Trp Val Ala Ala Gln Leu
   1 5 10
(2) INFORMATION FOR SEQ ID NO:249:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:249:
   Val Met Ala Gly Val Gly Ser Pro Tyr Val
   1 5 10
(2) INFORMATION FOR SEQ ID NO:250:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:250:
   Gln Leu Met Val Thr Val Tyr Tyr Gly Val
   1 5 10
(2) INFORMATION FOR SEQ ID NO:251:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:251:
   Ile Leu Arg Gly Thr Ser Phe Val Tyr Val
   1 5 10
(2) INFORMATION FOR SEQ ID NO:252:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:252:
   Ser Leu Thr Glu Ile Leu Lys Gly Gly Val
   1 5 10
(2) INFORMATION FOR SEQ ID NO:253:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:253:
   Leu Leu Gly Cys Ala Ala Asn Trp Ile Leu
   1 5 10
(2) INFORMATION FOR SEQ ID NO:254:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:254:
   Ala Leu Pro Pro Ala Ser Pro Ser Ala Val
   1 5 10
(2) INFORMATION FOR SEQ ID NO:255:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:255:
   Gln Leu Leu Trp Phe His Ile Ser Cys Leu
   1 5 10
(2) INFORMATION FOR SEQ ID NO:256:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:256:
   Asn Leu Leu Gly Arg Asn Ser Phe Glu Val
   1 5 10
(2) INFORMATION FOR SEQ ID NO:257:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:257:
   Val Leu Ala Ala Leu Ala Ala Tyr Cys Leu
   1 5 10
(2) INFORMATION FOR SEQ ID NO:258:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:258:
   Leu Leu Leu Asp Asp Glu Ala Gly Pro Leu
   1 5 10
(2) INFORMATION FOR SEQ ID NO:259:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:259:
   Ile Met Ala Lys Asn Glu Val Phe Cys Val
   1 5 10
(2) INFORMATION FOR SEQ ID NO:260:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:260:
   Val Leu Val Gly Gly Val Leu Ala Ala Leu
   1 5 10
(2) INFORMATION FOR SEQ ID NO:261:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:261:
   Leu Leu Cys Leu Ile Phe Leu Leu Val Leu
   1 5 10
(2) INFORMATION FOR SEQ ID NO:262:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:262:
   Lys Leu Met Val Thr Val Tyr Tyr Gly Val
   1               5                   10
(2) INFORMATION FOR SEQ ID NO:263:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:263:
   Asn Leu Met Val Thr Val Tyr Tyr Gly Val
   1               5                   10
(2) INFORMATION FOR SEQ ID NO:264:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:264:
   Cys Leu Ser Pro Thr Val Trp Leu Ser Ala
   1               5                   10
(2) INFORMATION FOR SEQ ID NO:265:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:265:
   Ser Leu Pro Thr His Asp Pro Ser Pro Leu
   1 5 10
(2) INFORMATION FOR SEQ ID NO:266:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:266:
   Val Leu Pro Glu Lys Asp Ser Trp Thr Val
   1 5 10
(2) INFORMATION FOR SEQ ID NO:267:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:267:
   Phe Leu His Ser Gly Thr Ala Lys Ser Val
   1 5 10
(2) INFORMATION FOR SEQ ID NO:268:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:268:
   Gly Leu Ile His Leu His Gln Asn Ile Val
   1 5 10
(2) INFORMATION FOR SEQ ID NO:269:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:269:
   Ser Leu Tyr Ala Ala Val Thr Asn Phe Leu
   1 5 10
(2) INFORMATION FOR SEQ ID NO:270:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:270:
   Ile Met Pro Ala Arg Phe Tyr Pro Asn Val
   1 5 10
(2) INFORMATION FOR SEQ ID NO:271:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:271:
   Tyr Leu Thr Arg Asp Pro Thr Thr Pro Leu
   1 5 10
(2) INFORMATION FOR SEQ ID NO:272:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:272:
   Gly Leu Tyr Asn Leu Leu Ile Arg Cys Leu
   1 5 10
(2) INFORMATION FOR SEQ ID NO:273:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:273:
   Tyr Leu Glu Tyr Gly Arg Cys Arg Thr Val
   1 5 10
(2) INFORMATION FOR SEQ ID NO:274:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:274:
   Ser Leu Phe Thr Ser Ile Thr Asn Phe Leu
   1 5 10
(2) INFORMATION FOR SEQ ID NO:275:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:275:
   Asn Leu Leu Ser Ser Asp Leu Ser Trp Leu
   1 5 10
(2) INFORMATION FOR SEQ ID NO:276:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:276:
   Arg Met Ala Arg Asp Pro Gln Arg Phe Val
   1 5 10
(2) INFORMATION FOR SEQ ID NO:277:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:277:
   Arg Met Arg Gly Thr Phe Val Val Pro Leu
   1 5 10
(2) INFORMATION FOR SEQ ID NO:278:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:278:
   Ser Leu Gln Leu Val Phe Gly Ile Asp Val
   1 5 10
(2) INFORMATION FOR SEQ ID NO:279:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:279:
   Lys Leu Leu His Lys Arg Thr Leu Gly Leu
   1 5 10
(2) INFORMATION FOR SEQ ID NO:280:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:280:
   Gly Leu Gly Met Glu His Leu Arg Glu Val
   1 5 10
(2) INFORMATION FOR SEQ ID NO:281:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:281:
   Gly Met Ser Tyr Leu Glu Asp Val Arg Leu
   1 5 10
(2) INFORMATION FOR SEQ ID NO:282:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:282:
   Asn Leu Leu Ser Ser Asn Leu Ser Trp Leu
   1 5 10
(2) INFORMATION FOR SEQ ID NO:283:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:283:
   Gln Leu Thr Val Trp Gly Ile Lys Gln Leu
   1 5 10
(2) INFORMATION FOR SEQ ID NO:284:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:284:
   Val Leu Glu Tyr Leu Val Ser Phe Gly Val
   1 5 10
(2) INFORMATION FOR SEQ ID NO:285:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:285:
   Val Leu Asn Pro Ser Val Ala Ala Thr Leu
   1 5 10
(2) INFORMATION FOR SEQ ID NO:286:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:286:
   Ser Leu Thr Asn Leu Leu Ser Ser Asn Leu
   1 5 10
(2) INFORMATION FOR SEQ ID NO:287:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:287:
   Ala Leu Leu Asp Pro Arg Val Arg Gly Leu
   1               5                   10
(2) INFORMATION FOR SEQ ID NO:288:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:288:
   Thr Leu Glu Asp Ser Ser Gly Asn Leu Leu
   1               5                   10
(2) INFORMATION FOR SEQ ID NO:289:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:289:
   Thr Leu Gln Gly Leu Gly Ile Ser Trp Leu
   1               5                   10
(2) INFORMATION FOR SEQ ID NO:290:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:290:
   Asp Leu Arg Ala Phe Gln Gln Leu Phe Leu
   1               5                   10
(2) INFORMATION FOR SEQ ID NO:291:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:291:
   Val Leu Gln Gly Leu Pro Arg Glu Tyr Val
   1 5 10
(2) INFORMATION FOR SEQ ID NO:292:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:292:
   Asp Leu Pro Pro Trp Phe Pro Pro Met Val
   1 5 10
(2) INFORMATION FOR SEQ ID NO:293:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:293:
   Asp Leu Ser Asp Gly Ser Trp Ser Thr Val
   1 5 10
(2) INFORMATION FOR SEQ ID NO:294:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:294:
   Cys Leu Ala Phe Ser Tyr Met Asp Asp Val
   1 5 10
(2) INFORMATION FOR SEQ ID NO:295:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:295:
   Pro Leu Val Leu Gly Thr Leu Glu Glu Val
   1 5 10
(2) INFORMATION FOR SEQ ID NO:296:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:296:
   Asn Leu Ala Thr Trp Val Gly Ser Asn Leu
   1 5 10
(2) INFORMATION FOR SEQ ID NO:297:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:297:
   Asn Leu Leu Thr Gln Ile Gly Cys Thr Leu
   1 5 10
(2) INFORMATION FOR SEQ ID NO:298:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:298:
   Gly Leu Thr His Ile Asp Ala His Pro Leu
   1               5                   10

(2) INFORMATION FOR SEQ ID NO:299:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:299:
   Val Leu Gly Ser Gly Ala Phe Gly Thr Val
   1               5                   10
(2) INFORMATION FOR SEQ ID NO:300:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:300:
   Gln Leu Ile Lys Lys Glu Lys Val Tyr Leu
   1               5                   10
(2) INFORMATION FOR SEQ ID NO:301:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:301:
   Lys Leu Leu Trp Lys Gly Glu Gly Ala Val
   1               5                   10
(2) INFORMATION FOR SEQ ID NO:302:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:302:
   Ser Met Val Gly Asn Trp Ala Lys Val Leu
   1               5                   10
(2) INFORMATION FOR SEQ ID NO:303:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:303:
   Lys Leu Ile Gly Thr Asp Asn Ser Val Val
   1               5                   10
(2) INFORMATION FOR SEQ ID NO:304:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:304:
   Ile Leu Leu Val Val Val Leu Gly Val Val
   1               5                   10
(2) INFORMATION FOR SEQ ID NO:305:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID N0:305:
(2) INFORMATION FOR SEQ ID NO:306:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:306:
   His Leu Lys Thr Ala Val Gln Met Ala Val
   1 5 10
(2) INFORMATION FOR SEQ ID NO:307:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:307:
   Asp Leu Leu Met Gly Thr Leu Gly Ile Val
   1 5 10
(2) INFORMATION FOR SEQ ID NO:308:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:308:
   Leu Leu Lys Leu Ala Gly Arg Trp Pro Val
   1 5 10
(2) INFORMATION FOR SEQ ID NO:309:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:309:
   Asp Leu Met Val Thr Val Tyr Tyr Gly Val
   1 5 10
(2) INFORMATION FOR SEQ ID NO:310:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:310:
   Thr Leu Glu Lys Leu Thr Asn Thr Gly Leu
   1 5 10
(2) INFORMATION FOR SEQ ID NO:311:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:311:
   Ser Leu Thr Asn Leu Leu Ser Ser Asp Leu
   1 5 10
(2) INFORMATION FOR SEQ ID NO:312:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:312:
   Asn Leu Glu Asp Pro Ala Ser Arg Glu Leu
   1 5 10
(2) INFORMATION FOR SEQ ID N0:313:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:313:
(2) INFORMATION FOR SEQ ID NO:314:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:314:
   Val Leu Val Ala Ser Arg Gly Arg Ala Val
   1 5 10
(2) INFORMATION FOR SEQ ID NO:315:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:315:
   Asp Leu Ser Val Phe Gln Asn Leu Gln Val
   1 5 10
(2) INFORMATION FOR SEQ ID NO:316:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:316:
   Gln Leu Phe Arg Asn Pro His Gln Ala Leu
   1 5 10
(2) INFORMATION FOR SEQ ID NO:317:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:317:
   Pro Leu Thr Ser Ile Ile Ser Ala Val Val
   1 5 10
(2) INFORMATION FOR SEQ ID NO:318:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:318:
   Tyr Leu Lys Gly Ser Ser Gly Gly Pro Leu
   1 5 10
(2) INFORMATION FOR SEQ ID NO:319:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:319:
   Gln Leu Pro Cys Glu Pro Glu Pro Asp Val
   1 5 10
(2) INFORMATION FOR SEQ ID NO:320:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:320:
   Cys Leu Thr Phe Gly Arg Glu Thr Val Leu
   1               5                   10
(2) INFORMATION FOR SEQ ID NO:321:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:321:
   Thr Leu Glu Asp Leu Leu Met Gly Thr Leu
   1               5                   10
(2) INFORMATION FOR SEQ ID NO:322:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:322:
   Ser Leu His Cys Lys Pro Glu Glu Ala Leu
   1               5                   10
(2) INFORMATION FOR SEQ ID NO:323:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:323:
   Glu Met Phe Arg Glu Leu Asn Glu Ala Leu
   1               5                   10
(2) INFORMATION FOR SEQ ID NO:324:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:324:
   Val Leu Lys Asp Ala Ile Lys Asp Leu Val
   1 5 10
(2) INFORMATION FOR SEQ ID NO:325:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:325:
   Asp Leu Val Asp Ala Glu Glu Tyr Leu Val
   1 5 10
(2) INFORMATION FOR SEQ ID NO:326:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:326:
   Arg Met Arg Gly Thr Phe Val Ser Pro Leu
   1 5 10
(2) INFORMATION FOR SEQ ID NO:327:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:327:
   Gln Leu Ala Lys Thr Cys Pro Val Gln Leu
   1 5 10
(2) INFORMATION FOR SEQ ID NO:328:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:328:
   Asn Leu Gln Val Ile Arg Gly Arg Ile Leu
   1 5 10
(2) INFORMATION FOR SEQ ID NO:329:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:329:
   Trp Leu Gly Leu Arg Ser Leu Arg Glu Leu
   1 5 10
(2) INFORMATION FOR SEQ ID NO:330:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:330:
   Asn Met Gly Leu Lys Ile Arg Gln Leu Leu
   1 5 10
(2) INFORMATION FOR SEQ ID NO:331:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:331:
   Pro Leu Ser Tyr Gln His Phe Arg Lys Leu
   1 5 10
(2) INFORMATION FOR SEQ ID NO:332:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:332:
   Glu Leu Pro Arg Leu Ala Asp Glu Gly Leu
   1 5 10
(2) INFORMATION FOR SEQ ID N0:333:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:333:
(2) INFORMATION FOR SEQ ID NO:334:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:334:
   Pro Leu Thr Val Asn Glu Lys Arg Arg Leu
   1 5 10
(2) INFORMATION FOR SEQ ID NO:335:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:335:
   Ile Met Pro Ala Arg Phe Tyr Pro Asn Leu
   1 5 10
(2) INFORMATION FOR SEQ ID NO:336:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:336:
   Pro Leu His Pro Ala Ala Met Pro His Leu
   1 5 10
(2) INFORMATION FOR SEQ ID NO:337:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:337:
   Asp Leu His Asp Ser Cys Ser Arg Asn Leu
   1 5 10
(2) INFORMATION FOR SEQ ID NO:338:
   (i) SEQUENCE CHARACTERISTICS:
      (A) LENGTH: 10 amino acids
      (B)' TYPE: amino acid
      (D) TOPOLOGY: linear
   (ii) MOLECULE TYPE: peptide
   (xi) SEQUENCE DESCRIPTION: SEQ ID NO:338:
   Leu Leu Tyr Lys Thr Phe Gly Arg Lys Leu
   1 5 10
(2) INFORMATION FOR SEQ ID NO:339:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:339:
   Pro Met Gly Val Gly Leu Ser Pro Phe Leu
   1 5 10
(2) INFORMATION FOR SEQ ID NO:340:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:340:
   Val Leu Gly Ala Lys Ser Val Gln His Leu
   1 5 10
(2) INFORMATION FOR SEQ ID NO:341:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:341:
   Pro Leu Pro Ile His Thr Ala Glu Leu Leu
   1 5 10
(2) INFORMATION FOR SEQ ID NO:342:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:342:
   Pro Leu Pro Ser Leu Ala Pro Ser Ala Val
   1 5 10
(2) INFORMATION FOR SEQ ID NO:343:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:343:
   Asp Leu Glu Ala Tyr Phe Lys Asp Cys Leu
   1 5 10
(2) INFORMATION FOR SEQ ID NO:344:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:344:
   Glu Leu Gly Glu Glu Ile Arg Leu Lys Val
   1 5 10
(2) INFORMATION FOR SEQ ID NO:345:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:345:
   Val Leu Gly Gly Cys Arg His Lys Leu Val
   1 5 10
(2) INFORMATION FOR SEQ ID NO:346:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:346:
   Thr Leu Glu Gln Gln Tyr Asn Lys Pro Leu
   1 5 10
(2) INFORMATION FOR SEQ ID NO:347:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:347:
   Asp Leu Cys Thr Glu Leu Asn Thr Ser Leu
   1 5 10
(2) INFORMATION FOR SEQ ID NO:348:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:348:
   Arg Leu Gln Arg Arg Arg Glu Thr Gln Val
   1 5 10
(2) INFORMATION FOR SEQ ID NO:349:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:349:
   His Leu Glu Pro Gln Asn Glu Ile Pro Val
   1 5 10
(2) INFORMATION FOR SEQ ID NO:350:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:350:
   Leu Leu Lys Tyr Arg Ala Arg Glu Pro Val
   1 5 10
(2) INFORMATION FOR SEQ ID NO:351:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:351:
   Cys Leu Gly Leu Ser Tyr Asp Gly Leu Leu
   1 5 10
(2) INFORMATION FOR SEQ ID NO:352:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:352:
   Asp Met Ser Leu Leu Lys Asn Arg Phe Leu
   1 5 10
(2) INFORMATION FOR SEQ ID NO:353:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:353:
   Leu Leu Arg Leu Ser Glu Pro Ala Glu Leu
   1 5 10
(2) INFORMATION FOR SEQ ID NO:354:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:354:
   Pro Leu Ser Gln Glu Thr Phe Ser Asp Leu
   1 5 10
(2) INFORMATION FOR SEQ ID NO:355:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:355:
   Pro Leu Pro Ser Gln Ala Met Asp Asp Leu
   1 5 10
(2) INFORMATION FOR SEQ ID NO:356:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:356:
   Glu Leu Ala Ala Leu Cys Arg Trp Gly Leu
   1 5 10
(2) INFORMATION FOR SEQ,ID NO:357:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:357:
(2) INFORMATION FOR SEQ ID NO:358:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:358:
   Arg Leu Arg Ile Val Arg Gly Thr Gln Leu
   1 5 10

(2) INFORMATION FOR SEQ ID NO:359:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:359:
   Gly Leu Arg Glu Leu Gln Leu Arg Ser Leu
   1               5                   10
(2) INFORMATION FOR SEQ ID NO:360:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:360:
   Gln Met Arg Ile Leu Lys Glu Thr Glu Leu
   1               5                   10
(2) INFORMATION FOR SEQ ID NO:361:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:361:
   Ile Leu Lys Glu Thr Glu Leu Arg Lys Val
   1               5                   10
(2) INFORMATION FOR SEQ ID NO:362:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:362:
   Pro Leu Asp Ser Thr Phe Tyr Arg Ser Leu
   1 5 10
(2) INFORMATION FOR SEQ ID NO:363:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ .ID NO:363:
   Leu Leu Glu Asp Asp Asp Met Gly Asp Leu
   1 5 10
(2) INFORMATION FOR SEQ ID NO:364:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:364:
   Asp Leu Gly Met Gly Ala Ala Lys Gly Leu
   1 5 10
(2) INFORMATION FOR SEQ ID NO:365:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:365:
   Pro Leu Pro Ser Glu Thr Asp Gly Tyr Val
   1 5 10
(2) INFORMATION FOR SEQ ID NO:366:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:366:
   Thr Leu Ser Pro Gly Lys Asn Gly Val Val
   1               5                   10
(2) INFORMATION FOR SEQ ID NO:367:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:367:
   Thr Leu Gln Asp Pro Arg Val Arg Ala Leu
   1               5                   10
(2) INFORMATION FOR SEQ ID NO:368:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:368:
   Asn Met Glu Asn Ile Ala Ser Gly Leu Leu
   1               5                   10
(2) INFORMATION FOR SEQ ID NO:369:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:369:
   Glu Leu Pro His Leu Ala Asp Glu Gly Leu
   1 5 10
(2) INFORMATION FOR SEQ ID NO:370:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:370:
   Gly Leu Asn Arg Pro Val Ala Glu Asp Leu
   1 5 10
(2) INFORMATION FOR SEQ ID NO:371:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:371:
   Pro Leu Thr Val Asn Glu Asn Arg Arg Leu
   1 5 10
(2) INFORMATION FOR SEQ ID NO:372:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:372:
   Leu Leu Tyr Lys Thr Tyr Gly Arg Lys Leu
   1 5 10
(2) INFORMATION FOR SEQ ID NO:373:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:373:
   Gly Leu Ser Ala Met Ser Pro Thr Asp Leu
   1 5 10
(2) INFORMATION FOR SEQ ID NO:374:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:374:
   Asp Leu Glu Ala Tyr Phe Lys Asp Cys Val
   1 5 10
(2) INFORMATION FOR SEQ ID NO:375:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:375:
   Thr Leu Gln Asp Pro Arg Val Arg Gly Leu
   1 5 10
(2) INFORMATION FOR SEQ ID NO:376:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:376:
   Asn Met Glu Asn Ile Thr Ser Gly Phe Leu
   1 5 10
(2) INFORMATION FOR SEQ ID NO:377:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:377:
   Asp Leu Val Asn Leu Leu Pro Ala Ile Leu
   1 5 10
(2) INFORMATION FOR SEQ ID NO:378:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:378:
   Pro Leu Thr Glu Glu Lys Ile Lys Ala Leu
   1 5 10
(2) INFORMATION FOR SEQ ID NO:379:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:379:
   Gln Leu Gly Ile Pro His Pro Ala Gly Leu
   1 5 10
(2) INFORMATION FOR SEQ ID NO:380:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:380:
   Gly Leu Lys Lys Lys Lys Ser Val Thr Val
   1 5 10
(2) INFORMATION FOR SEQ ID NO:381:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:381:
   Pro Ile Trp Lys Gly Pro Ala Lys Leu Leu
   1 5 10
(2) INFORMATION FOR SEQ ID NO:382:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:382:
   Asp Leu Ala Val Ala Val Glu Pro Val Val
   1 5 10
(2) INFORMATION FOR SEQ ID NO:383:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:383:
   Ser Leu Thr Gly Arg Asp Lys Asn Gln Val
   1               5                   10
(2) INFORMATION FOR SEQ ID NO:384:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:384:
   Asp Leu Glu Val Val Thr Ser Thr Trp Val
   1               5                   10
(2) INFORMATION FOR SEQ ID NO:385:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:385:
   Gly Leu Gly Lys Val Leu Ile Asp Ile Leu
   1               5                   10
(2) INFORMATION FOR SEQ ID NO:386:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:386:
   Val Leu Thr Thr Ser Cys Gly Asn Thr Leu
   1               5                   10
(2) INFORMATION FOR SEQ ID NO:387:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:387:
   Glu Leu Ile Thr Ser Cys Ser Ser Asn Val
   1               5                   10
(2) INFORMATION FOR SEQ ID NO:388:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:388:
   Cys Leu Arg Lys Leu Gly Val Pro Pro Leu
   1               5                   10
(2) INFORMATION FOR SEQ ID NO:389:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:389:
   Pro Leu Gly Phe Phe Pro Asp His Gln Leu
   1               5                   10
(2) INFORMATION FOR SEQ ID NO:390:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:390:
   Asn Met Glu Asn Thr Thr Ser Gly Phe Leu
   1 5 10
(2) INFORMATION FOR SEQ ID NO:391:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:391:
   Cys Leu Val Gly Ala Pro Phe Ala
   1 5
(2) INFORMATION FOR SEQ ID NO:392:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:392:
   Gly Ala Pro Phe Ala Ser Leu Val
   1 5
(2) INFORMATION FOR SEQ ID NO:393:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:393:
   Gly Leu Cys Phe Phe Gly Val Ala
   1 5
(2) INFORMATION FOR SEQ ID NO:394:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:394:
   Ala Leu Thr Gly Thr Glu Lys Leu
   1 5
(2) INFORMATION FOR SEQ ID NO:395:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:395:
   Leu Thr Gly Thr Glu Lys Leu Ile
   1 5
(2) INFORMATION FOR SEQ ID NO:396:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:396:
   Tyr Leu Ile Asn Val Ile His Ala
   1 5
(2) INFORMATION FOR SEQ ID NO:397:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:397:
   Val Ile His Ala Phe Gln Cys Val
   1               5
(2) INFORMATION FOR SEQ ID NO:398:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:398:
   Val Ile His Ala Phe Gln Tyr Val
   1               5
(2) INFORMATION FOR SEQ ID NO:399:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:399:
   Gly Thr Ala Ser Phe Phe Phe Leu
   1               5
(2) INFORMATION FOR SEQ ID NO:400:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:400:
   Phe Leu Tyr Gly Ala Leu Leu Leu
   1 5
(2) INFORMATION FOR SEQ ID NO:401:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:401:
   Thr Thr Gly Ala Val Arg Gln Ile
   1 5
(2) INFORMATION FOR SEQ ID NO:402:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:402:
   Thr Thr Ile Cys Gly Lys Gly Leu
   1 5
(2) INFORMATION FOR SEQ ID NO:403:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:403:
   Gln Ala His Ser Leu Glu Arg Val
   1 5
(2) INFORMATION FOR SEQ ID NO:404:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:404:
   Phe Val Gly Ile Thr Tyr Ala Leu
   1               5
(2) INFORMATION FOR SEQ ID NO:405:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:405:
   Gly Ile Thr Tyr Ala Leu Thr Val
   1               5
(2) INFORMATION FOR SEQ ID NO:406:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:406:
   Ile Thr Tyr Ala Leu Thr Val Val
   1               5
(2) INFORMATION FOR SEQ ID NO:407:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:407:
   Tyr Ala Leu Thr Val Val Trp Leu
   1 5
(2) INFORMATION FOR SEQ ID NO:408:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:408:
   Ala Leu Thr Val Val Trp Leu Leu
   1 5
(2) INFORMATION FOR SEQ ID NO:409:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:409:
   Leu Thr Val Val Trp Leu Leu Val
   1 5
(2) INFORMATION FOR SEQ ID NO:410:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:410:
   Leu Leu Val Phe Ala Cys Ser Ala
   1 5
(2) INFORMATION FOR SEQ ID NO:411:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:411:
   Leu Val Phe Ala Cys Ser Ala Val
   1 5
(2) INFORMATION FOR SEQ ID NO:412:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:412:
   Phe Ala Cys Ser Ala Val Pro Val
   1 5
(2) INFORMATION FOR SEQ ID NO:413:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:413:
   Ser Leu Cys Ala Asp Ala Arg Met
   1 5
(2) INFORMATION FOR SEQ ID NO:414:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:414:
   Asp Ala Arg Met Tyr Gly Val Leu
   1               5
(2) INFORMATION FOR SEQ ID NO:415:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:415:
   Trp Ile Ala Phe Pro Gly Lys Val
   1               5

(2) INFORMATION FOR SEQ ID NO:416:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:416:
   Lys Val Cys Gly Ser Asn Leu Leu
   1               5
(2) INFORMATION FOR SEQ ID NO:417:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:417:
   Leu Leu Ser Ile Cys Lys Thr Ala
   1 5
(2) INFORMATION FOR SEQ ID NO:418:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:418:
   Gln Met Thr Phe His Leu Phe Ile
   1 5
(2) INFORMATION FOR SEQ ID NO:419:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:419:
   His Leu Phe Ile Ala Ala Phe Val
   1 5
(2) INFORMATION FOR SEQ ID NO:420:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:420:
   Phe Val Gly Ala Ala Ala Thr Leu
   1 5
(2) INFORMATION FOR SEQ ID NO:421:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:421:
   Ala Ala Ala Thr Leu Val Ser Leu
   1 5
(2) INFORMATION FOR SEQ ID NO:422:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:422:
   Ala Ala Thr Leu Val Ser Leu Leu
   1 5
(2) INFORMATION FOR SEQ ID NO:423:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:423:
   Ser Leu Leu Thr Phe Met Ile Ala
   1 5
(2) INFORMATION FOR SEQ ID NO:424:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:424:
   Leu Leu Thr Phe Met Ile Ala Ala
   1 5
(2) INFORMATION FOR SEQ ID NO:425:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:425:
   Ala Ala Thr Tyr Asn Phe Ala Val
   1 5
(2) INFORMATION FOR SEQ ID NO:426:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:426:
   Ala Thr Tyr Asn Phe Ala Val Leu
   1 5
(2) INFORMATION FOR SEQ ID NO:427:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:427:
   Tyr Leu Ala Thr Ala Ser Thr Met
   1 5
(2) INFORMATION FOR SEQ ID NO:428:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:428:
   Asp Thr Gly Ile Leu Asp Ser Ile
   1 5
(2) INFORMATION FOR SEQ ID NO:429:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:429:
   Arg Thr Ala His Tyr Gly Ser Leu
   1 5
(2) INFORMATION FOR SEQ ID NO:430:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:430:
   His Ala Arg Ser Arg Pro Gly Leu
   1 5
(2) INFORMATION FOR SEQ ID NO:431:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:431:
   Arg Thr Gln Asp Glu Asn Pro Val
   1               5
(2) INFORMATION FOR SEQ ID NO:432:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:432:
   Val Val His Phe Phe Lys Asn Ile
   1               5
(2) INFORMATION FOR SEQ ID NO:433:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:433:
   Arg Thr Thr His Tyr Gly Ser Leu
   1               5
(2) INFORMATION FOR SEQ ID NO:434:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:434:
   Gly Val Asp Ala Gln Gly Thr Leu
   1               5
(2) INFORMATION FOR SEQ ID NO:435:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:435:
   Thr Leu Ser Lys Ile Phe Lys Leu
   1               5
(2) INFORMATION FOR SEQ ID NO:436:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:436:
   Trp Leu Leu Val Phe Ala Cys Ser Ala
   1               5
(2) INFORMATION FOR SEQ ID NO:437:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:437:
   Arg Met Tyr Gly Val Leu Pro Trp Ile
   1 5
(2) INFORMATION FOR SEQ ID NO:438:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:438:
   Trp Leu Gly His Pro Asp Lys Phe Val
   1 5
(2) INFORMATION FOR SEQ ID NO:439:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:439:
   Ser Leu Leu Thr Phe Met Ile Ala Ala
   1 5
(2) INFORMATION FOR SEQ ID NO:440:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:440:
   Leu Val Ser Leu Leu Thr Phe Met Ile
   1 5
(2) INFORMATION FOR SEQ ID NO:441:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:441:
   Met Ile Ala Ala Thr Tyr Asn Phe Ala
   1 5
(2) INFORMATION FOR SEQ ID NO:442:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:442:
   Phe Leu Tyr Gly Ala Leu Leu Leu Ala
   1 5
(2) INFORMATION FOR SEQ ID NO:443:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:443:
   Arg Met Tyr Gly Val Leu Pro Trp Asn
   1 5
(2) INFORMATION FOR SEQ ID NO:444:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:444:
   Val Ile His Ala Phe Gln Tyr Val Ile
   1 5
(2) INFORMATION FOR SEQ ID NO:445:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:445:
   Gly Leu Cys Phe Phe Gly Val Ala Leu
   1 5
(2) INFORMATION FOR SEQ ID NO:446:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:446:
   Asn Thr Trp Thr Thr Cys Gln Ser Ile
   1 5
(2) INFORMATION FOR SEQ ID NO:447:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:447:
   Phe Gln Met Thr Phe His Leu Phe Ile
   1 5
(2) INFORMATION FOR SEQ ID NO:448:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:448:
   Gln Met Thr Phe His Leu Phe Ile Ala
   1 5
(2) INFORMATION FOR SEQ ID NO:449:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:449:
   Ile Ala Ala Thr Tyr Asn Phe Ala Val
   1 5
(2) INFORMATION FOR SEQ ID NO:450:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:450:
   Tyr Ala Leu Thr Val Val Trp Leu Leu
   1 5
(2) INFORMATION FOR SEQ ID NO:451:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:451:
   Ala Ser Phe Phe Phe Leu Tyr Gly Ala
   1 5
(2) INFORMATION FOR SEQ ID NO:452:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:452:
   Ala Leu Thr Val Val Trp Leu Leu Val
   1 5
(2) INFORMATION FOR SEQ ID NO:453:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:453:
   Val Ser Leu Leu Thr Phe Met Ile Ala
   1 5
(2) INFORMATION FOR SEQ ID NO:454:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:454:
   Phe His Leu Phe Ile Ala Ala Phe Val
   1               5
(2) INFORMATION FOR SEQ ID NO:455:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:455:
   Gly Val Leu Pro Trp Asn Ala Phe Pro
   1               5
(2) INFORMATION FOR SEQ ID NO:456:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:456:
   Leu Leu Val Phe Ala Cys Ser Ala Val
   1               5
(2) INFORMATION FOR SEQ ID NO:457:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:457:
   Phe Phe Phe Leu Tyr Gly Ala Leu Leu
   1 5
(2) INFORMATION FOR SEQ ID NO:458:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:458:
   Thr Leu Val Ser Leu Leu Thr Phe Met
   1 5
(2) INFORMATION FOR SEQ ID NO:459:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:459:
   Gly Val Leu Pro Trp Ile Ala Phe Pro
   1 5
(2) INFORMATION FOR SEQ ID NO:460:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:460:
   Ala Leu Thr Gly Thr Glu Lys Leu Ile
   1 5
(2) INFORMATION FOR SEQ ID NO:461:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:461:
   Phe Ile Ala Ala Phe Val Gly Ala Ala
   1 5
(2) INFORMATION FOR SEQ ID NO:462:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:462:
   Met Thr Phe His Leu Phe Ile Ala Ala
   1 5
(2) INFORMATION FOR SEQ ID NO:463:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:463:
   Phe Val Gly Ala Ala Ala Thr Leu Val
   1 5
(2) INFORMATION FOR SEQ ID NO:464:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:464:
   Val Ile His Ala Phe Gln Cys Val Ile
   1               5
(2) INFORMATION FOR SEQ ID NO:465:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:465:
   Val Gly Ala Pro Phe Ala Ser Leu Val
   1               5
(2) INFORMATION FOR SEQ ID NO:466:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:466:
   Lys Leu Ile Glu Thr Tyr Phe Ser Lys
   1               5
(2) INFORMATION FOR SEQ ID NO:467:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:467:
   Ala Leu Phe Cys Gly Cys Gly His Glu
   1               5
(2) INFORMATION FOR SEQ ID NO:468:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:468:
   Arg Cys Leu Val Gly Ala Pro Phe Ala
   1               5

(2) INFORMATION FOR SEQ ID NO:469:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:469:
   Phe Pro Ser Lys Thr Ser Ala Ser Ile
   1               5
(2) INFORMATION FOR SEQ ID NO:470:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:470:
   Val Ile Tyr Gly Thr Ala Ser Phe Phe
   1 5
(2) INFORMATION FOR SEQ ID NO:471:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:471:
   Tyr Gly Thr Ala Ser Phe Phe Phe Leu
   1 5
(2) INFORMATION FOR SEQ ID NO:472:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:472:
   Leu Val Gly Ala Pro Phe Ala Ser Leu
   1 5
(2) INFORMATION FOR SEQ ID NO:473:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:473:
   Leu Leu Ala Glu Gly Phe Tyr Thr Thr
   1 5
(2) INFORMATION FOR SEQ ID NO:474:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:474:
   Asn Val Ile His Ala Phe Gln Cys Val
   1               5
(2) INFORMATION FOR SEQ ID NO:475:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:475:
   Trp Ile Ala Phe Pro Gly Lys Val Cys
   1               5
(2) INFORMATION FOR SEQ ID NO:476:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:476:
   Asn Leu Leu Ser Ile Cys Lys Thr Ala
   1               5
(2) INFORMATION FOR SEQ ID NO:477:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:477:
   Ser Leu Cys Ala Asp Ala Arg Met Tyr
   1 5
(2) INFORMATION FOR SEQ ID NO:478:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:478:
   Asn Thr Trp Thr Thr Cys Asp Ser Ile
   1 5
(2) INFORMATION FOR SEQ ID NO:479:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:479:
   Cys Ala Asp Ala Arg Met Tyr Gly Val
   1 5
(2) INFORMATION FOR SEQ ID NO:480:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:480:
   Gly Leu Ser Ala Thr Val Thr Gly Gly
   1 5
(2) INFORMATION FOR SEQ ID NO:481:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:481:
   Val Val Trp Leu Leu Val Phe Ala Cys
   1 5
(2) INFORMATION FOR SEQ ID NO:482:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:482:
   Tyr Ile Tyr Phe Asn Thr Trp Thr Thr
   1 5
(2) INFORMATION FOR SEQ ID NO:483:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:483:
   Gln Asp Tyr Glu Tyr Leu Ile Asn Val
   1 5
(2) INFORMATION FOR SEQ ID NO:484:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:484:
   Ile Ala Ala Phe Val Gly Ala Ala Ala
   1 5
(2) INFORMATION FOR SEQ ID NO:485:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:485:
   Gly Ile Thr Tyr Ala Leu Thr Val Val
   1 5
(2) INFORMATION FOR SEQ ID NO:486:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:486:
   Phe Met Ile Ala Ala Thr Tyr Asn Phe
   1 5
(2) INFORMATION FOR SEQ ID NO:487:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:487:
   Ser Ile Gly Ser Leu Cys Ala Asp Ala
   1 5
(2) INFORMATION FOR SEQ ID NO:488:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:488:
   Ser Leu Val Ala Thr Gly Leu Cys Phe
   1 5
(2) INFORMATION FOR SEQ ID NO:489:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:489:
   Ala Thr Tyr Asn Phe Ala Val Leu Lys
   1 5
(2) INFORMATION FOR SEQ ID NO:490:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:490:
   Ala Val Pro Val Tyr Ile Tyr Phe Asn
   1 5
(2) INFORMATION FOR SEQ ID NO:491:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:491:
   Tyr Val Ile Tyr Gly Thr Ala Ser Phe
   1 5
(2) INFORMATION FOR SEQ ID NO:492:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:492:
   Thr Gly Leu Cys Phe Phe Gly Val Ala
   1 5
(2) INFORMATION FOR SEQ ID NO:493:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:493:
   Ser Leu Glu Arg Val Cys His Cys Leu
   1 5
(2) INFORMATION FOR SEQ ID NO:494:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:494:
   Phe Ala Ser Leu Val Ala Thr Gly Leu
   1               5
(2) INFORMATION FOR SEQ ID NO:495:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:495:
   Gly Thr Ala Ser Phe Phe Phe Leu Tyr
   1               5
(2) INFORMATION FOR SEQ ID NO:496:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:496:
   Phe Phe Leu Tyr Gly Ala Leu Leu Leu
   1               5
(2) INFORMATION FOR SEQ ID NO:497:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:497:
   Gly Ala Ala Ala Thr Leu Val Ser Leu
   1 5
(2) INFORMATION FOR SEQ ID NO:498:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:498:
   Trp Thr Thr Cys Asp Ser Ile Ala Phe
   1 5
(2) INFORMATION FOR SEQ ID NO:499:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:499:
   Gly Val Ala Leu Phe Cys Gly Cys Gly
   1 5
(2) INFORMATION FOR SEQ ID NO:500:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:500:
   Ala Ala Ala Thr Leu Val Ser Leu Leu
   1 5
(2) INFORMATION FOR SEQ ID NO:501:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:501:
   Val Cys Gly Ser Asn Leu Leu Ser Ile
   1 5
(2) INFORMATION FOR SEQ ID NO:502:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:502:
   Thr Val Val Trp Leu Leu Val Phe Ala
   1 5
(2) INFORMATION FOR SEQ ID NO:503:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:503:
   Asn Tyr Gln Asp Tyr Glu Tyr Leu Ile
   1 5
(2) INFORMATION FOR SEQ ID NO:504:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:504:
   Thr Ile Cys Gly Lys Gly Leu Ser Ala
   1 5
(2) INFORMATION FOR SEQ ID NO:505:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:505:
   Val Phe Ala Cys Ser Ala Val Pro Val
   1 5
(2) INFORMATION FOR SEQ ID NO:506:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:506:
   Gly Leu Leu Glu Cys Cys Ala Arg Cys
   1 5
(2) INFORMATION FOR SEQ ID NO:507:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:507:
   Phe Ala Cys Ser Ala Val Pro Val Tyr
   1 5
(2) INFORMATION FOR SEQ ID NO:508:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:508:
   Ala Ala Thr Tyr Asn Phe Ala Val Leu
   1 5
(2) INFORMATION FOR SEQ ID NO:509:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:509:
   Phe Val Gly Ile Thr Tyr Ala Leu Thr
   1 5
(2) INFORMATION FOR SEQ ID NO:510:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:510:
   Ile Ala Phe Pro Ser Lys Thr Ser Ala
   1 5
(2) INFORMATION FOR SEQ ID NO:511:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:511:
   Asn Val Ile His Ala Phe Gln Tyr Val
   1               5
(2) INFORMATION FOR SEQ ID NO:512:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:512:
   Tyr Leu Ile Asn Val Ile His Ala Phe
   1               5
(2) INFORMATION FOR SEQ ID NO:513:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:513:
   Leu Leu Leu Ala Glu Gly Phe Tyr Thr
   1               5
(2) INFORMATION FOR SEQ ID NO:514:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:514:
   Leu Pro Trp Asn Ala Phe Pro Gly Lys
   1 5
(2) INFORMATION FOR SEQ ID NO:515:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:515:
   Gly Ser Leu Cys Ala Asp Ala Arg Met
   1 5
(2) INFORMATION FOR SEQ ID NO:516:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:516:
   Val Ala Thr Gly Leu Cys Phe Phe Gly
   1 5
(2) INFORMATION FOR SEQ ID NO:517:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:517:
   Tyr Asn Phe Ala Val Leu Lys Leu Met
   1 5
(2) INFORMATION FOR SEQ ID NO:518:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:518:
   Val Leu Pro Trp Asn Ala Phe Pro Gly
   1 5
(2) INFORMATION FOR SEQ ID NO:519:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:519:
   Ala Leu Leu Leu Ala Glu Gly Phe Tyr
   1 5
(2) INFORMATION FOR SEQ ID NO:520:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:520:
   Met Tyr Gly Val Leu Pro Trp Ile Ala
   1 5
(2) INFORMATION FOR SEQ ID NO:521:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:521:
   Val Gly Ile Thr Tyr Ala Leu Thr Val
   1 5
(2) INFORMATION FOR SEQ ID NO:522:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:522:
   Lys Leu Met Gly Arg Gly Thr Lys Phe
   1 5

(2) INFORMATION FOR SEQ ID NO:523:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:523:
   Val Cys His Cys Leu Gly Lys Trp Leu
   1 5
(2) INFORMATION FOR SEQ ID NO:524:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:524:
   Leu Leu Glu Cys Cys Ala Arg Cys Leu
   1 5
(2) INFORMATION FOR SEQ ID NO:525:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:525:
   Tyr Thr Thr Gly Ala Val Arg Gln Ile
   1 5
(2) INFORMATION FOR SEQ ID NO:526:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:526:
   Ala Thr Gly Leu Cys Phe Phe Gly Val
   1 5
(2) INFORMATION FOR SEQ ID NO:527:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:527:
   Lys Thr Ser Ala Ser Ile Gly Ser Leu
   1 5
(2) INFORMATION FOR SEQ ID NO:528:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:528:
   Glu Ala Leu Thr Gly Thr Glu Lys Leu
   1 5
(2) INFORMATION FOR SEQ ID NO:529:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:529:
   Lys Thr Thr Ile Cys Gly Lys Gly Leu
   1 5
(2) INFORMATION FOR SEQ ID NO:530:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:530:
   Ser Leu Ser Arg Phe Ser Trp Gly Ala
   1 5
(2) INFORMATION FOR SEQ ID NO:531:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:531:
   Tyr Leu Ala Thr Ala Ser Thr Met Asp
   1 5
(2) INFORMATION FOR SEQ ID NO:532:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:532:
   Trp Leu Lys Gln Ser Arg Ser Pro Leu
   1 5
(2) INFORMATION FOR SEQ ID NO:533:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:533:
   Val Val His Phe Phe Lys Asn Ile Val
   1 5
(2) INFORMATION FOR SEQ ID NO:534:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:534:
   Arg Gly Ser Gly Lys Val Pro Trp Leu
   1 5
(2) INFORMATION FOR SEQ ID NO:535:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:535:
   Ala Thr Ala Ser Thr Met Asp His Ala
   1 5
(2) INFORMATION FOR SEQ ID NO:536:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:536:
   Ile Leu Asp Ser Ile Gly Arg Phe Phe
   1 5
(2) INFORMATION FOR SEQ ID NO:537:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:537:
   Gly Leu Ser Leu Ser Arg Phe Ser Trp
   1 5
(2) INFORMATION FOR SEQ ID NO:538:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:538:
   Ile Val Thr Pro Arg Thr Pro Pro Pro
   1 5
(2) INFORMATION FOR SEQ ID NO:539:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:539:
   Ser Arg Ser Pro Leu Pro Ser His Ala
   1 5
(2) INFORMATION FOR SEQ ID NO:540:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:540:
   Arg Thr Gln Asp Glu Asn Pro Val Val
   1 5
(2) INFORMATION FOR SEQ ID NO:541:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:541:
   Gly Arg Ala Ser Asp Tyr Lys Ser Ala
   1 5
(2) INFORMATION FOR SEQ ID NO:542:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:542:
   Met Asp His Ala Arg His Gly Phe Leu
   1 5
(2) INFORMATION FOR SEQ ID NO:543:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:543:
   Asp Ser Arg Ser Gly Ser Pro Met Ala
   1 5
(2) INFORMATION FOR SEQ ID NO:544:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:544:
   Pro Gly Leu Cys His Met Tyr Lys Asp
   1 5
(2) INFORMATION FOR SEQ ID NO:545:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:545:
   Ser Arg Phe Ser Trp Gly Ala Glu Gly
   1 5
(2) INFORMATION FOR SEQ ID NO:546:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:546:
   Arg Ser Gly Ser Pro Met Ala Arg Arg
   1 5
(2) INFORMATION FOR SEQ ID NO:547:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:547:
   Arg Asp Ser Arg Ser Gly Ser Pro Met
   1 5
(2) INFORMATION FOR SEQ ID NO:548:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:548:
   Pro Val Val His Phe Phe Lys Asn Ile
   1 5
(2) INFORMATION FOR SEQ ID NO:549:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:549:
   Ser Ala His Lys Gly Phe Lys Gly Val
   1 5
(2) INFORMATION FOR SEQ ID NO:550:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:550:
   Thr Leu Ser Lys Ile Phe Lys Leu Gly
   1 5
(2) INFORMATION FOR SEQ ID NO:551:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:551:
   Lys Gly Phe Lys Gly Ala Tyr Asp Ala
   1 5
(2) INFORMATION FOR SEQ ID NO:552:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:552:
   Ala Arg Thr Ala His Tyr Gly Ser Leu
   1 5
(2) INFORMATION FOR SEQ ID NO:553:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:553:
   Gly Ala Tyr Asp Ala Gln Gly Thr Leu
   1 5
(2) INFORMATION FOR SEQ ID NO:554:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:554:
   Gly Thr Leu Ser Lys Ile Phe Lys Leu
   1 5
(2) INFORMATION FOR SEQ ID NO:555:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:555:
   Asp Ala Gln Gly Thr Leu Ser Lys Ile
   1 5
(2) INFORMATION FOR SEQ ID NO:556:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:556:
   Phe Asn Thr Trp Thr Thr Cys Gln Ser Ile
   1 5 10
(2) INFORMATION FOR SEQ ID NO:557:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:557:
   Phe Asn Thr Trp Thr Thr Cys Asp Ser Ile
   1 5 10
(2) INFORMATION FOR SEQ ID NO:558:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:558:
   Ala Arg Met Tyr Gly Val Leu Pro Trp Ile
   1 5 10
(2) INFORMATION FOR SEQ ID NO:559:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:559:
   Trp Leu Leu Val Phe Ala Cys Ser Ala Val
   1 5 10
(2) INFORMATION FOR SEQ ID NO:560:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:560:
   Lys Val Cys Gly Ser Asn Leu Leu Ser Ile
   1 5 10
(2) INFORMATION FOR SEQ ID NO:561:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:561:
   Thr Leu Val Ser Leu Leu Thr Phe Met Ile
   1 5 10
(2) INFORMATION FOR SEQ ID NO:562:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:562:
   Leu Val Ala Thr Gly Leu Cys Phe Phe Gly
   1 5 10
(2) INFORMATION FOR SEQ ID NO:563:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:563:
   Phe Phe Phe Leu Tyr Gly Ala Leu Leu Leu
   1 5 10
(2) INFORMATION FOR SEQ ID NO:564:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:564:
   Tyr Ala Leu Thr Val Val Trp Leu Leu Val
   1 5 10
(2) INFORMATION FOR SEQ ID NO:565:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:565:
   Ala Leu Leu Leu Ala Glu Gly Phe Tyr Thr
   1 5 10
(2) INFORMATION FOR SEQ ID NO:566:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:566:
   Phe Gln Met Thr Phe His Leu Phe Ile Ala
   1 5 10
(2) INFORMATION FOR SEQ ID NO:567:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:567:
   Phe Leu Tyr Gly Ala Leu Leu Leu Ala Glu
   1 5 10
(2) INFORMATION FOR SEQ ID NO:568:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:568:
   Phe Ala Cys Ser Ala Val Pro Val Tyr Ile
   1               5                   10
(2) INFORMATION FOR SEQ ID NO:569:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:569:
   Leu Val Phe Ala Cys Ser Ala Val Pro Val
   1               5                   10
(2) INFORMATION FOR SEQ ID NO:570:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:570:
   Leu Glu Cys Cys Ala Arg Cys Leu Val Gly
   1               5                   10
(2) INFORMATION FOR SEQ ID NO:571:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:571:
   Ser Leu Leu Thr Phe Met Ile Ala Ala Thr
   1               5                   10
(2) INFORMATION FOR SEQ ID NO:572:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:572:
   Leu Glu Arg Val Cys His Cys Leu Gly Lys
   1               5                   10
(2) INFORMATION FOR SEQ ID NO:573:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:573:
   Ile Tyr Phe Asn Thr Trp Thr Thr Cys Asp
   1               5                   10
(2) INFORMATION FOR SEQ ID NO:574:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:574:
   Leu Cys Phe Phe Gly Val Ala Leu Phe Cys
   1 5 10
(2) INFORMATION FOR SEQ ID NO:575:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:575:
   Leu Gly His Pro Asp Lys Phe Val Gly Ile
   1 5 10

(2) INFORMATION FOR SEQ ID NO:576:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:576:
   Phe His Leu Phe Ile Ala Ala Phe Val Gly
   1 5 10
(2) INFORMATION FOR SEQ ID NO:577:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:577:
   Gln Asp Tyr Glu Tyr Leu Ile Asn Val Ile
   1 5 10
(2) INFORMATION FOR SEQ ID NO:578:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:578:
   Ala Arg Met Tyr Gly Val Leu Pro Trp Asn
   1 5 10
(2) INFORMATION FOR SEQ ID NO:579:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:579:
   Ala Leu Thr Val Val Trp Leu Leu Val Phe
   1 5 10
(2) INFORMATION FOR SEQ ID NO:580:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:580:
   Arg Val Cys His Cys Leu Gly Lys Trp Leu
   1 5 10
(2) INFORMATION FOR SEQ ID NO:581:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:581:
   Ile Tyr Gly Thr Ala Ser Phe Phe Phe Leu
   1 5 10
(2) INFORMATION FOR SEQ ID NO:582:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:582:
   Asn Val Ile His Ala Phe Gln Tyr Val Ile
   1 5 10
(2) INFORMATION FOR SEQ ID NO:583:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:583:
   Gly Val Leu Pro Trp Ile Ala Phe Pro Gly
   1 5 10
(2) INFORMATION FOR SEQ ID NO:584:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:584:
   Phe Gly Val Ala Leu Phe Cys Gly Cys Gly
   1 5 10
(2) INFORMATION FOR SEQ ID NO:585:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:585:
   Leu Leu Leu Ala Glu Gly Phe Tyr Thr Thr
   1 5 10
(2) INFORMATION FOR SEQ ID NO:586:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:586:
   Ile Asn Val Ile His Ala Phe Gln Cys Val
   1 5 10
(2) INFORMATION FOR SEQ ID NO:587:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:587:
   Ser Asn Leu Leu Ser Ile Cys Lys Thr Ala
   1 5 10
(2) INFORMATION FOR SEQ ID NO:588:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:588:
   Ala Ser Phe Phe Phe Leu Tyr Gly Ala Leu
   1               5                   10
(2) INFORMATION FOR SEQ ID NO:589:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:589:
   Gly Val Leu Pro Trp Asn Ala Phe Pro Gly
   1               5                   10
(2) INFORMATION FOR SEQ ID NO:590:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:590:
   Tyr Gly Val Leu Pro Trp Ile Ala Phe Pro
   1               5                   10
(2) INFORMATION FOR SEQ ID NO:591:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:591:
   Phe Phe Leu Tyr Gly Ala Leu Leu Leu Ala
   1 5 10
(2) INFORMATION FOR SEQ ID NO:592:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:592:
   Thr Phe His Leu Phe Ile Ala Ala Phe Val
   1 5 10
(2) INFORMATION FOR SEQ ID NO:593:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:593:
   Phe Ile Ala Ala Phe Val Gly Ala Ala Ala
   1 5 10
(2) INFORMATION FOR SEQ ID NO:594:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:594:
   Trp Thr Thr Cys Asp Ser Ile Ala Phe Pro
   1 5 10
(2) INFORMATION FOR SEQ ID NO:595:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ.ID NO:595:
   Leu Leu Ser Ile Cys Lys Thr Ala Glu Phe
   1 5 10
(2) INFORMATION FOR SEQ ID NO:596:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:596:
   Cys Leu Val Gly Ala Pro Phe Ala Ser Leu
   1 5 10
(2) INFORMATION FOR SEQ ID NO:597:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:597:
   Phe Val Gly Ile Thr Tyr Ala Leu Thr Val
   1 5 10
(2) INFORMATION FOR SEQ ID NO:598:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:598:
   Ile Asn Val Ile His Ala Phe Gln Tyr Val
   1 5 10
(2) INFORMATION FOR SEQ ID NO:599:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:599:
   Ala Phe Pro Gly Lys Val Cys Gly Ser Asn
   1 5 10
(2) INFORMATION FOR SEQ ID NO:600:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:600:
   Ala Phe Pro Ser Lys Thr Ser Ala Ser Ile
   1 5 10
(2) INFORMATION FOR SEQ ID NO:601:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:601:
   Gln Ile Phe Gly Asp Tyr Lys Thr Thr Ile
   1 5 10
(2) INFORMATION FOR SEQ ID NO:602:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:602:
   Ser Leu Val Ala Thr Gly Leu Cys Phe Phe
   1 5 10
(2) INFORMATION FOR SEQ ID NO:603:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:603:
   Leu Leu Glu Cys Cys Ala Arg Cys Leu Val
   1 5 10
(2) INFORMATION FOR SEQ ID NO:604:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:604:
   Ala Ser Leu Val Ala Thr Gly Leu Cys Phe
   1 5 10
(2) INFORMATION FOR SEQ ID NO:605:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:605:
   Lys Trp Leu Gly His Pro Asp Lys Phe Val
   1 5 10
(2) INFORMATION FOR SEQ ID NO:606:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:606:
   Trp Thr Thr Cys Gln Ser Ile Ala Phe Pro
   1 5 10
(2) INFORMATION FOR SEQ ID NO:607:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:607:
   Leu Thr Val Val Trp Leu Leu Val Phe Ala
   1 5 10
(2) INFORMATION FOR SEQ ID NO:608:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:608:
   Val Tyr Ile Tyr Phe Asn Thr Trp Thr Thr
   1               5                   10
(2) INFORMATION FOR SEQ ID NO:609:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:609:
   Ala Ala Thr Leu Val Ser Leu Leu Thr Phe
   1               5                   10
(2) INFORMATION FOR SEQ ID NO:610:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:610:
   Gly Leu Cys Phe Phe Gly Val Ala Leu Phe
   1               5                   10
(2) INFORMATION FOR SEQ ID NO:611:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:611:
   Val Leu Pro Trp Ile Ala Phe Pro Gly Lys
   1 5 10
(2) INFORMATION FOR SEQ ID NO:612:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:612:
   Val Ala Leu Phe Cys Gly Cys Gly His Glu
   1 5 10
(2) INFORMATION FOR SEQ ID NO:613:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:613:
   Ala Thr Tyr Asn Phe Ala Val Leu Lys Leu
   1 5 10
(2) INFORMATION FOR SEQ ID NO:614:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:614:
   Asn Val Ile His Ala Phe Gln Cys Val Ile
   1 5 10
(2) INFORMATION FOR SEQ ID NO:615:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:615:
   Gly Thr Ala Ser Phe Phe Phe Leu Tyr Gly
   1 5 10
(2) INFORMATION FOR SEQ ID NO:616:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:616:
   Ile Ala Ala Thr Tyr Asn Phe Ala Val Leu
   1 5 10
(2) INFORMATION FOR SEQ ID NO:617:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:617:
   Ala Ala Phe Val Gly Ala Ala Ala Thr Leu
   1 5 10
(2) INFORMATION FOR SEQ ID NO:618:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:618:
   Gly Leu Leu Glu Cys Cys Ala Arg Cys Leu
   1               5                   10
(2) INFORMATION FOR SEQ ID NO:619:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:619:
   Phe Met Ile Ala Ala Thr Tyr Asn Phe Ala
   1               5                   10
(2) INFORMATION FOR SEQ ID NO:620:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:620:
   Val Ala Thr Gly Leu Cys Phe Phe Gly Val
   1               5                   10
(2) INFORMATION FOR SEQ ID NO:621:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:621:
   Arg Met Tyr Gly Val Leu Pro Trp Asn Ala
   1 5 10
(2) INFORMATION FOR SEQ ID NO:622:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:622:
   Ile Thr Tyr Ala Leu Thr Val Val Trp Leu
   1 5 10
(2) INFORMATION FOR SEQ ID NO:623:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:623:
   Ala Leu Phe Cys Gly Cys Gly His Glu Ala
   1 5 10
(2) INFORMATION FOR SEQ ID NO:624:
   (i) SEQUENCE CHARACTERISTICS:
      (A) LENGTH: 10 amino acids
      (B) TYPE: amino/acid
      (C) STRANDEDNESS: single
      (D) TOPOLOGY: unknown
   (ii) MOLECULE TYPE: peptide
   (xi) SEQUENCE DESCRIPTION: SEQ ID NO:624:
   Arg Met Tyr Gly Val Leu Pro Trp Ile Ala
   1 5 10
(2) INFORMATION FOR SEQ ID NO:625:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:625:
   Met Ile Ala Ala Thr Tyr Asn Phe Ala Val
   1 5 10
(2) INFORMATION FOR SEQ ID NO:626:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:626:
   Gln Met Thr Phe His Leu Phe Ile Ala Ala
   1 5 10
(2) INFORMATION FOR SEQ ID NO:627:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:627:
   His Leu Phe Ile Ala Ala Phe Val Gly Ala
   1 5 10
(2) INFORMATION FOR SEQ ID NO:628:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:628:
   Gly Ala Ala Ala Thr Leu Val Ser Leu Leu
   1 5 10

(2) INFORMATION FOR SEQ ID NO:629:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:629:
   Glu Ala Leu Thr Gly Thr Glu Lys Leu Ile
   1 5 10
(2) INFORMATION FOR SEQ ID NO:630:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:630:
   Thr Ala Glu Phe Gln Met Thr Phe His Leu
   1 5 10
(2) INFORMATION FOR SEQ ID NO:631:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:631:
   Leu Val Gly Ala Pro Phe Ala Ser Leu Val
   1               5                   10
(2) INFORMATION FOR SEQ ID NO:632:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:632:
   Cys Ala Asp Ala Arg Met Tyr Gly Val Leu
   1               5                   10
(2) INFORMATION FOR SEQ ID NO:633:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:633:
   Gly Leu Leu Glu Cys Cys Ala Arg Cys Leu Val
   1               5                   10
(2) INFORMATION FOR SEQ ID NO:634:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:634:
   Cys Leu Val Gly Ala Pro Phe Ala Ser Leu Val
   1 5 10
(2) INFORMATION FOR SEQ ID NO:635:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:635:
   Leu Val Ala Thr Gly Leu Cys Phe Phe Gly Val
   1 5 10
(2) INFORMATION FOR SEQ ID NO:636:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:636:
   Ala Thr Gly Leu Cys Phe Phe Gly Val Ala Leu
   1 5 10
(2) INFORMATION FOR SEQ ID NO:637:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:637:
   Ala Leu Phe Cys Gly Cys Gly His Glu Ala Leu
   1 5 10
(2) INFORMATION FOR SEQ ID NO:638:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:638:
   Leu Ile Asn Val Ile His Ala Phe Gln Tyr Val
   1               5                   10
(2) INFORMATION FOR SEQ ID NO:639:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:639:
   Leu Ile Asn Val Ile His Ala Phe Gln Cys Val
   1               5                   10
(2) INFORMATION FOR SEQ ID NO:640:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:640:
   Val Ile Tyr Gly Thr Ala Ser Phe Phe Phe Leu
   1               5                   10
(2) INFORMATION FOR SEQ ID NO:641:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:641:
   Thr Ala Ser Phe Phe Phe Leu Tyr Gly Ala Leu
   1 5 10
(2) INFORMATION FOR SEQ ID NO:642:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:642:
   Leu Leu Ala Glu Gly Phe Tyr Thr Thr Gly Ala
   1 5 10
(2) INFORMATION FOR SEQ ID NO:643:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:643:
   Leu Ala Glu Gly Phe Tyr Thr Thr Gly Ala Val
   1 5 10
(2) INFORMATION FOR SEQ ID NO:644:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:644:
   Thr Ile Cys Gly Lys Gly Leu Ser Ala Thr Val
   1 5 10
(2) INFORMATION FOR SEQ ID NO:645:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:645:
   Trp Leu Gly His Pro Asp Lys Phe Val Gly Ile
   1 5 10
(2) INFORMATION FOR SEQ ID NO:646:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:646:
   Phe Val Gly Ile Thr Tyr Ala Leu Thr Val Val
   1 5 10
(2) INFORMATION FOR SEQ ID NO:647:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:647:
   Gly Ile Thr Tyr Ala Leu Thr Val Val Trp Leu
   1 5 10
(2) INFORMATION FOR SEQ ID NO:648:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:648:
   Ile Thr Tyr Ala Leu Thr Val Val Trp Leu Leu
   1 5 10
(2) INFORMATION FOR SEQ ID NO:649:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:649:
   Ala Leu Thr Val Val Trp Leu Leu Val Phe Ala
   1 5 10
(2) INFORMATION FOR SEQ ID NO:650:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:650:
   Leu Leu Val Phe Ala Cys Ser Ala Val Pro Val
   1 5 10
(2) INFORMATION FOR SEQ ID NO:651:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:651:
   Ile Ala Phe Pro Ser Lys Thr Ser Ala Ser Ile
   1 5 10
(2) INFORMATION FOR SEQ ID NO:652:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:652:
   Ser Leu Cys Ala Asp Ala Arg Met Tyr Gly Val
   1 5 10
(2) INFORMATION FOR SEQ ID NO:653:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:653:
   Asp Ala Arg Met Tyr Gly Val Leu Pro Trp Ile
   1 5 10
(2) INFORMATION FOR SEQ ID NO:654:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:654:
   Val Leu Pro Trp Ile Ala Phe Pro Gly Lys Val
   1 5 10
(2) INFORMATION FOR SEQ ID NO:655:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:655:
   Val Leu Pro Trp Asn Ala Phe Pro Gly Lys Val
   1 5 10
(2) INFORMATION FOR SEQ ID NO:656:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:656:
   Lys Thr Ala Glu Phe Gln Met Thr Phe His Leu
   1 5 10
(2) INFORMATION FOR SEQ ID NO:657:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:657:
   Met Thr Phe His Leu Phe Ile Ala Ala Phe Val
   1 5 10
(2) INFORMATION FOR SEQ ID NO:658:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:658:
   His Leu Phe Ile Ala Ala Phe Val Gly Ala Ala
   1               5                   10
(2) INFORMATION FOR SEQ ID NO:659:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:659:
   Ile Ala Ala Phe Val Gly Ala Ala Ala Thr Leu
   1               5                   10
(2) INFORMATION FOR SEQ ID NO:660:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:660:
   Ala Ala Phe Val Gly Ala Ala Ala Thr Leu Val
   1               5                   10
(2) INFORMATION FOR SEQ ID NO:661:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:661:
   Phe Val Gly Ala Ala Ala Thr Leu Val Ser Leu
   1 5 10
(2) INFORMATION FOR SEQ ID NO:662:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:662:
   Ala Thr Leu Val Ser Leu Leu Thr Phe Met Ile
   1 5 10
(2) INFORMATION FOR SEQ ID NO:663:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:663:
   Thr Leu Val Ser Leu Leu Thr Phe Met Ile Ala
   1 5 10
(2) INFORMATION FOR SEQ ID NO:664:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:664:
   Phe Met Ile Ala Ala Thr Tyr Asn Phe Ala Val
   1 5 10
(2) INFORMATION FOR SEQ ID NO:665:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:665:
   Met Ile Ala Ala Thr Tyr Asn Phe Ala Val Leu
   1 5 10
(2) INFORMATION FOR SEQ ID NO:666:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:666:
   Ala Ala Thr Tyr Asn Phe Ala Val Leu Lys Leu
   1 5 10
(2) INFORMATION FOR SEQ ID NO:667:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:667:
   Ile Leu Asp Ser Ile Gly Arg Phe Phe Gly
   1 5 10
(2) INFORMATION FOR SEQ ID NO:668:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:668:
   Phe Leu Pro Arg His Arg Asp Thr Gly Ile
   1               5                   10
(2) INFORMATION FOR SEQ ID NO:669:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:669:
   Ala Tyr Asp Ala Gln Gly Thr Leu Ser Lys
   1               5                   10
(2) INFORMATION FOR SEQ ID NO:670:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:670:
   Phe Phe Lys Asn Ile Val Thr Pro Arg Thr
   1               5                   10
(2) INFORMATION FOR SEQ ID NO:671:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:671:
   Tyr Leu Ala Thr Ala Ser Thr Met Asp His
   1 5 10
(2) INFORMATION FOR SEQ ID NO:672:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:672:
   Asn Pro Val Val His Phe Phe Lys Asn Ile
   1 5 10
(2) INFORMATION FOR SEQ ID NO:673:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:673:
   His Arg Asp Thr Gly Ile Leu Asp Ser Ile
   1 5 10
(2) INFORMATION FOR SEQ ID NO:674:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:674:
   Ser Leu Ser Arg Phe Ser Trp Gly Ala Glu
   1 5 10
(2) INFORMATION FOR SEQ ID NO:675:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:675:
   Pro Val Val His Phe Phe Lys Asn Ile Val
   1               5                   10
(2) INFORMATION FOR SEQ ID NO:676:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:676:
   His Thr Arg Thr Thr His Tyr Gly Ser Leu
   1               5                   10
(2) INFORMATION FOR SEQ ID NO:677:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:677:
   Thr Met Asp His Ala Arg His Gly Phe Leu
   1               5                   10
(2) INFORMATION FOR SEQ ID NO:678:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:678:
   Pro Ala Arg Thr Ala His Tyr Gly Ser Leu
   1               5                   10
(2) INFORMATION FOR SEQ ID NO:679:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:679:
   Gly Ala Pro Lys Arg Gly Ser Gly Lys Val
   1               5                   10
(2) INFORMATION FOR SEQ ID NO:680:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:680:
   Tyr Leu Ala Thr Ala Ser Thr Met Asp His Ala
   1               5                   10
(2) INFORMATION FOR SEQ ID NO:681:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:681:
   Ser Thr Met Asp His Ala Arg His Gly Phe Leu
   1 5 10
(2) INFORMATION FOR SEQ ID NO:682:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:682:
   Phe Leu Pro Arg His Arg Asp Thr Gly Ile Leu
   1 5 10

(2) INFORMATION FOR SEQ ID NO:683:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:683:
   Gly Leu Ser Leu Ser Arg Phe Ser Trp Gly Ala
   1 5 10
(2) INFORMATION FOR SEQ ID NO:684:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:684:
   Gly Val Asp Ala Gln Gly Thr Leu Ser Lys Ile
   1 5 10
(2) INFORMATION FOR SEQ ID NO:685:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:685:
   Ala Leu Ala Lys Ala Ala Ala Ala Val
   1 5
(2) INFORMATION FOR SEQ ID NO:686:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:686:
   Ala Leu Xaa Gly Gly Xaa Val Asn Val
   1 5
(2) INFORMATION FOR SEQ ID NO:687:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:687:
   Leu Leu Asp Val Pro Thr Ala Ala Val
   1 5
(2) INFORMATION FOR SEQ ID NO:688:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:688:
   Gly Xaa Val Pro Phe Xaa Val Ser Val
   1 5
(2) INFORMATION FOR SEQ ID NO:689:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:689:
   Ser Leu Leu Pro Ala Ile Val Glu Leu
   1 5
(2) INFORMATION FOR SEQ ID NO:690:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:690:
   Ser Xaa Xaa Val Arg Ala Xaa Glu Val
   1 5
(2) INFORMATION FOR SEQ ID NO:691:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:691:
   Tyr Met Asn Gly Thr Met Ser Gln Val
   1 5
(2) INFORMATION FOR SEQ ID NO:692:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:692:
   Lys Xaa Asn Glu Pro Val Xaa Xaa Xaa
   1               5
(2) INFORMATION FOR SEQ ID NO:693:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:693:
   Tyr Leu Leu Pro Ala Ile Val His Ile
   1               5
(2) INFORMATION FOR SEQ ID NO:694:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:694:
   Ala Xaa Trp Gly Phe Phe Pro Val Xaa
   1               5
(2) INFORMATION FOR SEQ ID NO:695:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:695:
   Thr Leu Trp Val Asp Pro Tyr Glu Val
   1 5
(2) INFORMATION FOR SEQ ID NO:696:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:696:
   Gly Xaa Val Pro Phe Xaa Val Ser Val
   1 5
(2) INFORMATION FOR SEQ ID NO:697:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:697:
   Lys Ile Phe Gly Ser Leu Ala Phe Leu
   1 5
(2) INFORMATION FOR SEQ ID NO:698:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:698:
   Arg Ile Leu His Asn Gly Ala Tyr Ser Leu
   1 5 10
(2) INFORMATION FOR SEQ ID NO:699:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:699:
   Ile Ile Ser Ala Val Val Gly Ile Leu Leu
   1               5                   10
(2) INFORMATION FOR SEQ ID NO:700:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:700:
   Met Met Trp Phe Val Val Leu Thr Val
   1               5
(2) INFORMATION FOR SEQ ID NO:701:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:701:
   Tyr Leu Leu Leu Tyr Phe Ser Pro Val
   1               5
(2) INFORMATION FOR SEQ ID NO:702:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:702:
   Tyr Leu Tyr Arg Leu Asn Phe Cys Leu
   1 5
(2) INFORMATION FOR SEQ ID NO:703:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:703:
   Phe Met Trp Thr Tyr Leu Val Thr Leu
   1 5
(2) INFORMATION FOR SEQ ID NO:704:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:704:
   Leu Leu Trp Trp Ile Thr Ile Leu Leu
   1 5
(2) INFORMATION FOR SEQ ID NO:705:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:705:
   Gly Leu Trp Cys Val Leu Phe Phe Val
   1 5
(2) INFORMATION FOR SEQ ID NO:706:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:706:
   Leu Met Ile Arg Gly Val Leu Glu Val
   1 5
(2) INFORMATION FOR SEQ ID NO:707:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:707:
   Leu Leu Leu Cys Arg Leu Pro Phe Leu
   1 5
(2) INFORMATION FOR SEQ ID NO:708:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:708:
   Ala Met Ser Arg Asn Leu Phe Arg Val
   1 5
(2) INFORMATION FOR SEQ ID NO:709:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:709:
   Ala Met Leu Thr Ala Cys Val Glu Val
   1               5
(2) INFORMATION FOR SEQ ID NO:710:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:710:
   Arg Leu Gln Pro Asn Val Pro Leu Val
   1               5
(2) INFORMATION FOR SEQ ID NO:711:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:711:
   Val Leu Ala Arg Thr Phe Thr Pro Val
   1               5
(2) INFORMATION FOR SEQ ID NO:712:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:712:
   Arg Leu Leu Arg Gly Leu Ile Arg Leu
   1 5
(2) INFORMATION FOR SEQ ID NO:713:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:713:
   Trp Met Trp Phe Pro Ser Val Leu Leu
   1 5
(2) INFORMATION FOR SEQ ID NO:714:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:714:
   Tyr Leu Cys Cys Gly Ile Thr Leu Leu
   1 5
(2) INFORMATION FOR SEQ ID NO:715:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:715:
   Ser Leu Leu Thr Glu Val Glu Thr Tyr Val
   1 5 10
(2) INFORMATION FOR SEQ ID NO:716:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:716:
   Leu Leu Thr Glu Val Glu Thr Tyr Val
   1 5
(2) INFORMATION FOR SEQ ID NO:717:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:717:
   Leu Leu Thr Glu Val Glu Thr Tyr Val Leu
   1 5 10
(2) INFORMATION FOR SEQ ID NO:718:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:718:
   Gly Ile Leu Gly Phe Val Phe Thr Leu
   1 5
(2) INFORMATION FOR SEQ ID NO:719:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:719:
   Gly Ile Leu Gly Phe Val Phe Thr Leu Thr
   1 5 10
(2) INFORMATION FOR SEQ ID NO:720:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:720:
   Ile Leu Gly Phe Val Phe Thr Leu Thr
   1 5
(2) INFORMATION FOR SEQ ID NO:721:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:721:
   Ile Leu Gly Phe Val Phe Thr Leu Thr Val
   1 5 10
(2) INFORMATION FOR SEQ ID NO:722:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:722:
   Ala Leu Ala Ser Cys Met Gly Leu Ile
   1 5
(2) INFORMATION FOR SEQ ID NO:723:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:723:
   Arg Met Gly Ala Val Thr Thr Glu Val
   1 5
(2) INFORMATION FOR SEQ ID NO:724:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:724:
   Val Thr Thr Glu Val Ala Phe Gly Leu
   1 5
(2) INFORMATION FOR SEQ ID NO:725:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:725:
   Met Val Thr Thr Thr Asn Pro Leu Ile
   1 5
(2) INFORMATION FOR SEQ ID NO:726:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:726:
   Phe Thr Phe Ser Pro Thr Tyr Lys Ala
   1 5
(2) INFORMATION FOR SEQ ID NO:727:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:727:
   Tyr Leu His Thr Leu Trp Lys Ala Gly Ile
   1 5 10
(2) INFORMATION FOR SEQ ID NO:728:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:728:
   Leu Met Leu Gln Ala Gly Phe Phe Leu Val
   1 5 10
(2) INFORMATION FOR SEQ ID NO:729:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:729:
   Arg Met Leu Thr Ile Pro Gln Ser Val
   1 5
(2) INFORMATION FOR SEQ ID NO:730:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:730:
   Ser Leu Asp Ser Trp Trp Thr Ser Val
   1 5
(2) INFORMATION FOR SEQ ID NO:731:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:731:
   Phe Met Leu Leu Leu Cys Leu Ile Phe Leu
   1 5 10
(2) INFORMATION FOR SEQ ID NO:732:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:732:
   Leu Leu Pro Phe Val Gln Trp Phe Val
   1 5
(2) INFORMATION FOR SEQ ID NO:733:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:733:
   Leu Met Pro Phe Val Gln Trp Phe Val
   1 5
(2) INFORMATION FOR SEQ ID NO:734:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:734:
   Phe Leu Gly Leu Ser Pro Thr Val Trp Val
   1 5 10
(2) INFORMATION FOR SEQ ID NO:735:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:735:
   Ser Met Leu Ser Pro Phe Leu Pro Leu Val
   1 5 10
(2) INFORMATION FOR SEQ ID NO:736:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:736:
   Gly Leu Trp Ile Arg Thr Pro Pro Val
   1               5

(2) INFORMATION FOR SEQ ID NO:737:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:737:
   Asn Leu Gly Asn Leu Asn Val Ser Val
   1               5
(2) INFORMATION FOR SEQ ID NO:738:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:738:
   Tyr Leu His Thr Leu Trp Lys Ala Gly Val
   1               5                   10
(2) INFORMATION FOR SEQ ID NO:739:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:739:
   Arg Leu Thr Gly Gly Val Phe Leu Val
   1 5
(2) INFORMATION FOR SEQ ID NO:740:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:740:
   Arg Met Thr Gly Gly Val Phe Leu Val
   1 5
(2) INFORMATION FOR SEQ ID NO:741:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:741:
   Arg Leu Thr Gly Gly Val Phe Leu Val
   1 5
(2) INFORMATION FOR SEQ ID NO:742:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:742:
   Ile Leu Gly Leu Leu Gly Phe Ala Val
   1 5
(2) INFORMATION FOR SEQ ID NO:743:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:743:
   Gly Leu Cys Gln Val Phe Ala Asp Val
   1 5
(2) INFORMATION FOR SEQ ID NO:744:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:744:
   Trp Leu Leu Arg Gly Thr Ser Phe Val
   1 5
(2) INFORMATION FOR SEQ ID NO:745:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:745:
   Tyr Leu Pro Ser Ala Leu Asn Pro Val
   1 5
(2) INFORMATION FOR SEQ ID NO:746:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:746:
   Leu Leu Val Pro Phe Val Gln Trp Phe Ala
   1 5 10
(2) INFORMATION FOR SEQ ID NO:747:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:747:
   Phe Leu Pro Ser Asp Phe Phe Pro Ser Ile
   1 5 10
(2) INFORMATION FOR SEQ ID NO:748:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:748:
   Val Val Ser Tyr Val Asn Val Asn Met
   1 5
(2) INFORMATION FOR SEQ ID NO:749:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:749:
   His Leu Pro Asp Arg Val His Phe Ala
   1 5
(2) INFORMATION FOR SEQ ID NO:750:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:750:
   Ser Leu Ala Phe Ser Ala Val Pro Ala
   1 5
(2) INFORMATION FOR SEQ ID NO:751:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:751:
   Phe Leu Leu Thr Lys Ile Leu Thr Ile
   1 5
(2) INFORMATION FOR SEQ ID NO:752:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:752:
   Ser Leu Tyr Asn Ile Leu Ser Pro Phe Met
   1 5 10
(2) INFORMATION FOR SEQ ID NO:753:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:753:
   Cys Leu Phe His Ile Val Asn Leu Ile
   1               5
(2) INFORMATION FOR SEQ ID NO:754:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:754:
   Arg Leu Pro Asp Arg Val His Phe Ala
   1               5
(2) INFORMATION FOR SEQ ID NO:755:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:755:
   Ala Leu Pro Pro Ala Ser Pro Ser Ala
   1               5
(2) INFORMATION FOR SEQ ID NO:756:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:756:
   Gly Leu Leu Gly Trp Ser Pro Gln Ala
   1 5
(2) INFORMATION FOR SEQ ID NO:757:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:757:
   Phe Leu Gly Pro Leu Leu Val Leu Gln Ala
   1 5 10
(2) INFORMATION FOR SEQ ID NO:758:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:758:
   Phe Leu Leu Thr Arg Ile Leu Thr Ile
   1 5
(2) INFORMATION FOR SEQ ID NO:759:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:759:
   Gly Met Leu Pro Val Cys Pro Leu Ile
   1 5
(2) INFORMATION FOR SEQ ID NO:760:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:760:
   Gln Leu Phe His Leu Cys Leu Ile Ile
   1 5
(2) INFORMATION FOR SEQ ID NO:761:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:761:
   Lys Leu Cys Leu Gly Trp Leu Trp Gly Met
   1 5 10
(2) INFORMATION FOR SEQ ID NO:762:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:762:
   Leu Leu Trp Phe His Ile Ser Cys Leu Ile
   1 5 10
(2) INFORMATION FOR SEQ ID NO:763:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:763:
   Tyr Leu Val Ser Phe Gly Val Trp Ile
   1 5
(2) INFORMATION FOR SEQ ID NO:764:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:764:
   Leu Leu Glu Asp Trp Gly Pro Cys Ala
   1 5
(2) INFORMATION FOR SEQ ID NO:765:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:765:
   Lys Leu His Leu Tyr Ser His Pro Ile
   1 5
(2) INFORMATION FOR SEQ ID NO:766:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:766:
   Phe Leu Leu Ala Gln Phe Thr Ser Ala
   1               5
(2) INFORMATION FOR SEQ ID NO:767:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:767:
   Leu Leu Ala Gln Phe Thr Ser Ala Ile
   1               5
(2) INFORMATION FOR SEQ ID NO:768:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:768:
   Tyr Met Asp Asp Val Val Leu Gly Ala
   1               5
(2) INFORMATION FOR SEQ ID NO:769:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:769:
   Ala Leu Met Pro Leu Tyr Ala Cys Ile
   1 5
(2) INFORMATION FOR SEQ ID NO:770:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:770:
   Gly Leu Cys Gln Val Phe Ala Asp Ala
   1 5
(2) INFORMATION FOR SEQ ID NO:771:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:771:
   His Leu Pro Asp Leu Val His Phe Ala
   1 5
(2) INFORMATION FOR SEQ ID NO:772:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:772:
   Arg Leu Cys Cys Gln Leu Asp Pro Ala
   1 5
(2) INFORMATION FOR SEQ ID NO:773:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:773:
   Ala Leu Met Pro Leu Tyr Ala Cys Ile
   1 5
(2) INFORMATION FOR SEQ ID NO:774:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:774:
   Phe Leu Cys Lys Gln Tyr Leu Asn Leu
   1 5
(2) INFORMATION FOR SEQ ID NO:775:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:775:
   Ser Leu Tyr Ala Asp Ser Pro Ser Val
   1 5
(2) INFORMATION FOR SEQ ID NO:776:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:776:
   Ala Leu Met Pro Leu Tyr Ala Ser Ile
   1 5
(2) INFORMATION FOR SEQ ID NO:777:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:777:
   Asn Leu Asn Asn Leu Asn Val Ser Ile
   1 5
(2) INFORMATION FOR SEQ ID NO:778:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:778:
   Ala Leu Ser Leu Ile Val Asn Leu Leu
   1 5
(2) INFORMATION FOR SEQ ID NO:779:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:779:
   Lys Leu His Leu Tyr Ser His Pro Ile
   1 5
(2) INFORMATION FOR SEQ ID NO:780:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:780:
   Trp Ile Leu Arg Gly Thr Ser Phe Val
   1 5
(2) INFORMATION FOR SEQ ID NO:781:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:781:
   Leu Val Leu Gln Ala Gly Phe Phe Leu Leu
   1 5 10
(2) INFORMATION FOR SEQ ID NO:782:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:782:
   Phe Ile Leu Leu Leu Cys Leu Ile Phe Leu
   1 5 10
(2) INFORMATION FOR SEQ ID NO:783:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:783:
   Trp Ile Leu Arg Gly Thr Ser Phe Val
   1               5
(2) INFORMATION FOR SEQ ID NO:784:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:784:
   Ile Ile Ser Cys Thr Cys Pro Thr Val
   1               5
(2) INFORMATION FOR SEQ ID NO:785:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:785:
   Leu Val Pro Phe Val Gln Trp Phe Val
   1               5
(2) INFORMATION FOR SEQ ID NO:786:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:786:
   Leu Ile Ile Ser Cys Ser Cys Pro Thr Val
   1 5 10
(2) INFORMATION FOR SEQ ID NO:787:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:787:
   Phe Leu Pro Ser Asp Phe Phe Pro Ser Ile
   1 5 10
(2) INFORMATION FOR SEQ ID NO:788:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:788:
   Leu Leu Cys Leu Gly Trp Leu Trp Gly Met
   1 5 10
(2) INFORMATION FOR SEQ ID NO:789:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:789:
   Gln Leu Phe His Leu Cys Leu Ile Ile
   1 5
(2) INFORMATION FOR SEQ ID NO:790:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:790:
   Cys Leu Gly Trp Leu Thr Gly Met Asp Ile
   1 5 10

(2) INFORMATION FOR SEQ ID NO:791:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:791:
   Phe Leu Gly Gly Thr Thr Val Cys Leu
   1 5
(2) INFORMATION FOR SEQ ID NO:792:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:792:
   Ser Leu Tyr Ser Ile Leu Ser Pro Phe Leu
   1 5 10
(2) INFORMATION FOR SEQ ID NO:793:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:793:
   Phe Leu Pro Ser Asp Phe Phe Pro Ser Val
   1               5                   10
(2) INFORMATION FOR SEQ ID NO:794:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:794:
   Ile Leu Cys Trp Gly Glu Leu Met Thr Leu
   1               5                   10
(2) INFORMATION FOR SEQ ID NO:795:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:795:
   Leu Met Thr Leu Ala Thr Trp Val Gly Val
   1               5                   10
(2) INFORMATION FOR SEQ ID NO:796:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:796:
   Thr Leu Ala Thr Trp Val Gly Val Asn Leu
   1 5 10
(2) INFORMATION FOR SEQ ID NO:797:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:797:
   Gly Leu Ser Arg Tyr Val Ala Arg Leu
   1 5
(2) INFORMATION FOR SEQ ID NO:798:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:798:
   Phe Leu Cys Lys Gln Tyr Leu Asn Leu
   1 5
(2) INFORMATION FOR SEQ ID NO:799:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:799:
   Arg Met Arg Gly Thr Phe Ser Ala Pro Leu
   1 5 10
(2) INFORMATION FOR SEQ ID NO:800:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:800:
   Ser Leu Tyr Ala Asp Ser Pro Ser Val
   1 5
(2) INFORMATION FOR SEQ ID NO:801:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:801:
   Tyr Leu Tyr Gly Val Gly Ser Ala Val
   1 5
(2) INFORMATION FOR SEQ ID NO:802:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:802:
   Leu Leu Ser Thr Thr Glu Trp Gln Val
   1 5
(2) INFORMATION FOR SEQ ID NO:803:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:803:
   Ile Ile Gly Ala Glu Thr Phe Tyr Val
   1               5
(2) INFORMATION FOR SEQ ID NO:804:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:804:
   Gln Leu Trp Val Thr Val Tyr Tyr Gly Val
   1               5                   10
(2) INFORMATION FOR SEQ ID NO:805:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:805:
   Asn Leu Trp Val Thr Val Tyr Tyr Gly Val
   1               5                   10
(2) INFORMATION FOR SEQ ID NO:806
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:806:
   Lys Leu Trp Val Thr Val Tyr Tyr Gly Val
   1 5 10
(2) INFORMATION FOR SEQ ID NO:807:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:807:
   Lys Leu Trp Val Thr Val Tyr Tyr Gly Val
   1 5 10
(2) INFORMATION FOR SEQ ID NO:808:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:808:
   Tyr Met Leu Asp Leu Gln Pro Glu Thr
   1 5
(2) INFORMATION FOR SEQ ID NO:809:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:809:
   Thr Leu Gly Ile Val Cys Pro Ile
   1 5
(2) INFORMATION FOR SEQ ID NO:810:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:810:
   Tyr Leu Leu Asp Leu Gln Glu Pro Val
   1 5
(2) INFORMATION FOR SEQ ID NO:811:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:811:
   Tyr Met Leu Asp Leu Gln Pro Glu Val
   1 5
(2) INFORMATION FOR SEQ ID NO:812:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:812:
   Met Leu Asp Leu Gln Pro Glu Thr Thr
   1 5
(2) INFORMATION FOR SEQ ID NO:813:
   (i) SEQUENCE CHARACTERISTICS:
      (A) LENGTH: 15 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:813:
   Ser Leu Gln Asp Ile Glu Ile Thr Cys Val Tyr Cys Lys Thr Val
   1 5 10 15
(2) INFORMATION FOR SEQ ID NO:814:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:814:
   Arg Leu Leu Thr Ser Leu Phe Phe Leu
   1 5
(2) INFORMATION FOR SEQ ID NO:815:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:815:
   Arg Leu Leu Thr Ser Leu Phe Phe Leu
   1 5
(2) INFORMATION FOR SEQ ID NO:816:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:816:
   Leu Leu Leu Tyr Tyr Asp Tyr Ser Leu
   1               5
(2) INFORMATION FOR SEQ ID NO:817:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:817:
   Asp Met Leu Gly Arg Val Phe Phe Val
   1               5
(2) INFORMATION FOR SEQ ID NO:818:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:818:
   Thr Met Phe Glu Ala Leu Pro His Ile
   1               5
(2) INFORMATION FOR SEQ ID NO:819:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:819:
   Ala Leu Ile Ser Phe Leu Leu Leu Ala
   1 5
(2) INFORMATION FOR SEQ ID NO:820:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:820:
   Thr Leu Met Ser Ile Val Ser Ser Leu
   1 5
(2) INFORMATION FOR SEQ ID NO:821:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:821:
   Asn Ile Ser Gly Tyr Asn Phe Ser Leu
   1 5
(2) INFORMATION FOR SEQ ID NO:822:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:822:
   Ala Leu Leu Asp Gly Gly Asn Met Leu
   1 5
(2) INFORMATION FOR SEQ ID NO:823:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:823:
   Ala Leu His Leu Phe Lys Thr Thr Val
   1               5
(2) INFORMATION FOR SEQ ID NO:824:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:824:
   Ser Leu Ile Ser Asp Gln Leu Leu Met
   1               5
(2) INFORMATION FOR SEQ ID NO:825:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:825:
   Trp Leu Val Thr Asn Gly Ser Tyr Leu
   1               5
(2) INFORMATION FOR SEQ ID NO:826:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:826:
   Ala Leu Met Asp Leu Leu Met Phe Ser
   1 5
(2) INFORMATION FOR SEQ ID NO:827:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:827:
   Leu Met Asp Leu Leu Met Phe Ser Thr
   1 5
(2) INFORMATION FOR SEQ ID NO:828:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:828:
   Leu Met Phe Ser Thr Ser Ala Tyr Leu
   1 5
(2) INFORMATION FOR SEQ ID NO:829:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:829:
   Tyr Leu Val Ser Ile Phe Leu His Leu
   1 5
(2) INFORMATION FOR SEQ ID NO:830:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:830:
   Ser Leu His Cys Lys Pro Glu Glu Ala
   1 5
(2) INFORMATION FOR SEQ ID NO:831:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:831:
   Ala Leu Gly Leu Val Cys Val Gln Ala
   1 5
(2) INFORMATION FOR SEQ ID NO:832:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:832:
   Leu Val Leu Gly Thr Leu Glu Glu Val
   1 5
(2) INFORMATION FOR SEQ ID NO:833:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:833:
   Gly Thr Leu Glu Glu Val Pro Thr Ala
   1               5
(2) INFORMATION FOR SEQ ID NO:834:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:834:
   Cys Ile Leu Glu Ser Leu Phe Arg Ala
   1               5
(2) INFORMATION FOR SEQ ID NO:835:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:835:
   Lys Val Ala Asp Leu Val Gly Phe Leu Leu
   1               5                   10
(2) INFORMATION FOR SEQ ID NO:836:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:836:
   Lys Val Ala Asp Leu Val Gly Phe Leu Leu Leu
   1 5 10
(2) INFORMATION FOR SEQ ID NO:837:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:837:
   Val Met Ile Ala Met Glu Gly Gly His Ala
   1 5 10
(2) INFORMATION FOR SEQ ID NO:838:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:838:
   Ser Met His Cys Lys Pro Glu Glu Val
   1 5
(2) INFORMATION FOR SEQ ID NO:839:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:839:
   Ala Met Gly Leu Val Cys Val Gln Val
   1 5
(2) INFORMATION FOR SEQ ID NO:840:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:840:
   Leu Met Leu Gly Thr Leu Glu Glu Val
   1 5
(2) INFORMATION FOR SEQ ID NO:841:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:841:
   Lys Met Ala Asp Leu Val Gly Phe Leu Val
   1 5 10
(2) INFORMATION FOR SEQ ID NO:842:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:842:
   Val Met Val Thr Cys Leu Gly Leu Ser Val
   1 5 10
(2) INFORMATION FOR SEQ ID NO:843:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:843:
   Leu Leu Gly Asp Asn Gln Ile Met Val
   1               5

(2) INFORMATION FOR SEQ ID NO:844:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:844:
   Gln Met Met Pro Lys Thr Gly Phe Leu Val
   1               5                   10
(2) INFORMATION FOR SEQ ID NO:845:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:845:
   Val Met Ile Ala Met Glu Gly Gly His Val
   1               5                   10
(2) INFORMATION FOR SEQ ID NO:846:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:846:
   Trp Met Glu Leu Ser Val Met Glu Val
   1               5
(2) INFORMATION FOR SEQ ID NO:847:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:847:
   Phe Leu Trp Gly Pro Arg Ala Leu Ala
   1 5
(2) INFORMATION FOR SEQ ID NO:848:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:848:
   Arg Ala Leu Ala Glu Thr Ser Tyr Val
   1 5
(2) INFORMATION FOR SEQ ID NO:849:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:849:
   Ala Leu Ala Glu Thr Ser Tyr Val Lys Val Leu
   1 5 10
(2) INFORMATION FOR SEQ ID NO:850:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:850:
   Ala Leu Ala Glu Thr Ser Tyr Val Lys Val
   1 5 10
(2) INFORMATION FOR SEQ ID NO:851:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:851:
   Lys Val Leu Glu Tyr Val Ile Lys Val
   1 5
(2) INFORMATION FOR SEQ ID NO:852: -
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:852:
   Tyr Val Ile Lys Val Ser Ala Arg Val
   1 5
(2) INFORMATION FOR SEQ ID NO:853:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:853:
   Ala Leu Arg Glu Glu Glu Glu Gly Val
   1 5
(2) INFORMATION FOR SEQ ID NO:854:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:854:
   Tyr Met Phe Leu Trp Gly Pro Arg Val
   1 5
(2) INFORMATION FOR SEQ ID NO:855:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:855:
   Lys Met Val Glu Leu Val His Phe Leu Leu Leu
   1 5 10
(2) INFORMATION FOR SEQ ID NO:856:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:856:
   Lys Met Val Glu Leu Val His Phe Leu
   1 5
(2) INFORMATION FOR SEQ ID NO:857:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:857:
   Lys Met Val Glu Leu Val His Phe Leu Leu
   1 5 10
(2) INFORMATION FOR SEQ ID NO:858:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:858:
   Lys Ala Ser Glu Tyr Leu Gln Leu Val
   1 5
(2) INFORMATION FOR SEQ ID NO:859:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:859:
   Tyr Leu Gln Leu Val Phe Gly Ile Glu Val
   1 5 10
(2) INFORMATION FOR SEQ ID NO:860:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:860:
   Leu Val Phe Gly Ile Glu Val Val Glu Val
   1 5 10
(2) INFORMATION FOR SEQ ID NO:861:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:861:
   Gln Leu Val Phe Gly Ile Glu Leu Met Glu Val
   1 5 10
(2) INFORMATION FOR SEQ ID NO:862:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:862:
   Lys Val Ala Glu Leu Val His Phe Leu
   1 5
(2) INFORMATION FOR SEQ ID NO:863:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:863:
   Lys Val Ala Glu Leu Val His Phe Leu Leu
   1 5 10
(2) INFORMATION FOR SEQ ID NO:864:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:864:
   Glu Leu Met Glu Val Asp Pro Ile Gly His Leu
   1 5 10
(2) INFORMATION FOR SEQ ID NO:865:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:865:
   His Leu Tyr Ile Phe Ala Thr Cys Leu Gly Leu
   1 5 10
(2) INFORMATION FOR SEQ ID NO:866:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:866:
   Ile Met Pro Lys Ala Gly Leu Leu Ile Ile Val
   1 5 10
(2) INFORMATION FOR SEQ ID NO:867:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:867:
   Leu Val Phe Gly Ile Glu Leu Met Glu Val
   1 5 10
(2) INFORMATION FOR SEQ ID NO:868:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:868:
   Ala Leu Gly Arg Asn Ser Phe Glu Val
   1 5
(2) INFORMATION FOR SEQ ID.N0:869:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:869:
(2) INFORMATION FOR SEQ ID NO:870:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:870:
   Leu Leu Gly Arg Ala Ser Phe Glu Val
   1 5
(2) INFORMATION FOR SEQ ID NO:871:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:871:
   Leu Leu Gly Arg Asn Ala Phe Glu Val
   1 5
(2) INFORMATION FOR SEQ ID NO:872:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:872:
   Leu Leu Gly Arg Asn Ser Phe Ala Val
   1 5
(2) INFORMATION FOR SEQ ID NO:873:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:873:
   Arg Leu Gly Arg Asn Ser Phe Glu Val
   1 5
(2) INFORMATION FOR SEQ ID NO:874:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:874:
   Leu Leu Gly Arg Arg Ser Phe Glu Val
   1 5
(2) INFORMATION FOR SEQ ID NO:875:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:875:
   Leu Leu Gly Arg Asn Ser Phe Arg Val
   1 5
(2) INFORMATION FOR SEQ ID NO:876:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:876:
   Leu Leu Phe Phe Trp Leu Asp Arg Ser Val
   1 5 10
(2) INFORMATION FOR SEQ ID NO:877:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:877:
   Val Leu Ala Lys Glu Leu Lys Phe Val
   1 5
(2) INFORMATION FOR SEQ ID NO:878:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:878:
   Ile Leu Leu Trp Gln Pro Ile Pro Val
   1 5
(2) INFORMATION FOR SEQ ID NO:879:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:879:
   Ile Met Tyr Ser Ala His Asp Thr Thr Val
   1 5 10
(2) INFORMATION FOR SEQ ID NO:880:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:880:
   Phe Leu Thr Leu Ser Val Thr Trp Ile
   1 5
(2) INFORMATION FOR SEQ ID NO:881:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:881:
   Phe Leu Thr Leu Ser Val Thr Trp Ile Gly Ala
   1 5 10
(2) INFORMATION FOR SEQ ID NO:882:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:882:
   Phe Leu Thr Leu Ser Val Thr Trp Ile
   1 5
(2) INFORMATION FOR SEQ ID NO:883:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:883:
   Val Leu Val His Pro Gln Trp Val Leu Thr Ala
   1 5 10
(2) INFORMATION FOR SEQ ID NO:884:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:884:
   Ser Leu Phe His Pro Glu Asp Thr Gly Gln Val
   1               5                   10
(2) INFORMATION FOR SEQ ID NO:885:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:885:
   Met Leu Leu Arg Leu Ser Glu Pro Ala Glu Leu
   1               5                   10
(2) INFORMATION FOR SEQ ID NO:886:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:886:
   Ala Leu Gly Thr Thr Cys Tyr Ala
   1               5
(2) INFORMATION FOR SEQ ID NO:887:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:887:
   Lys Leu Gln Cys Val Asp Leu His Val Ile
   1               5                   10
(2) INFORMATION FOR SEQ ID NO:888:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:888:
   Phe Leu Pro Ser Asp Tyr Phe Pro Ser Val
   1               5                   10
(2) INFORMATION FOR SEQ ID NO:889:
   (i) SEQUENCE CHARACTERISTICS:
      (A) LENGTH: 12 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:889:
   Tyr Ser Phe Leu Pro Ser Asp Phe Phe Pro Ser Val
   1               5                   10
(2) INFORMATION FOR SEQ ID NO:890:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:890:
   Leu Leu Glu Cys Cys Ala Arg Cys Leu
   1 5
(2) INFORMATION FOR SEQ ID NO:891:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:891:
   Gly Leu Cys Phe Phe Gly Val Ala Leu
   1 5
(2) INFORMATION FOR SEQ ID NO:892:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:892:
   Ala Leu Thr Gly Thr Glu Lys Leu Ile
   1 5
(2) INFORMATION FOR SEQ ID NO:893:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:893:
   Ser Leu Glu Arg Val Cys His Cys Leu
   1 5
(2) INFORMATION FOR SEQ ID NO:894:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:894:
   Trp Leu Gly His Pro Asp Lys Phe Val
   1               5
(2) INFORMATION FOR SEQ ID NO:895:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:895:
   Ala Leu Thr Val Val Trp Leu Leu Val
   1               5
(2) INFORMATION FOR SEQ ID NO:896:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:896:
   Leu Leu Val Phe Ala Cys Ser Ala Val
   1               5

(2) INFORMATION FOR SEQ ID NO:897:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:897:
   Arg Met Tyr Gly Val Leu Pro Trp Ile
   1               5
(2) INFORMATION FOR SEQ ID NO:898:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:898:
   Gly Leu Leu Glu Cys Cys Ala Arg Cys Leu
   1               5                   10
(2) INFORMATION FOR SEQ ID NO:899:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:899:
   Leu Leu Glu Cys Cys Ala Arg Cys Leu Val
   1               5                   10
(2) INFORMATION FOR SEQ ID NO:900:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:900:
   Cys Leu Val Gly Ala Pro Phe Ala Ser Leu
   1 5 10
(2) INFORMATION FOR SEQ ID NO:901:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:901:
   Trp Leu Leu Val Phe Ala Cys Ser Ala Val
   1 5 10
(2) INFORMATION FOR SEQ ID NO:902:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:902:
   Thr Leu Val Ser Leu Leu Thr Phe Met Ile
   1 5 10
(2) INFORMATION FOR SEQ ID NO:903:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:903:
   Val Ile His Ala Phe Gln Tyr Val Ile
   1 5
(2) INFORMATION FOR SEQ ID NO:904:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:904:
   Phe Leu Tyr Gly Ala Leu Leu Leu Ala
   1               5
(2) INFORMATION FOR SEQ ID NO:905:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:905:
   Tyr Ala Leu Thr Val Val Trp Leu Leu
   1               5
(2) INFORMATION FOR SEQ ID NO:906:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:906:
   Trp Leu Leu Val Phe Ala Cys Ser Ala
   1               5
(2) INFORMATION FOR SEQ ID NO:907:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:907:
   Gln Met Thr Phe His Leu Phe Ile Ala
   1 5
(2) INFORMATION FOR SEQ ID NO:908:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide

   (xi) SEQUENCE DESCRIPTION: SEQ ID NO:908:
   Leu Val Ser Leu Leu Thr Phe Met Ile
   1 5
(2) INFORMATION FOR SEQ ID NO:909:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:909:
   Ser Leu Leu Thr Phe Met Ile Ala Ala
   1 5
(2) INFORMATION FOR SEQ ID NO:910:
   (i) SEQUENCE CHARACTERISTICS:
      (A) LENGTH: 9 amino acids '
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:910:
   Ile Ala Ala Thr Tyr Asn Phe Ala Val
   1 5
(2) INFORMATION FOR SEQ ID NO:911:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:911:
   Ala Leu Leu Leu Ala Glu Gly Phe Tyr Thr
   1 5 10
(2) INFORMATION FOR SEQ ID NO:912:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:912:
   Tyr Ala Leu Thr Val Val Trp Leu Leu Val
   1 5 10
(2) INFORMATION FOR SEQ ID NO:913:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:913:
   Leu Val Phe Ala Cys Ser Ala Val Pro Val
   1 5 10
(2) INFORMATION FOR SEQ ID NO:914:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:914:
   Lys Val Cys Gly Ser Asn Leu Leu Ser Ile
   1               5                   10
(2) INFORMATION FOR SEQ ID NO:915:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:915:
   Ser Leu Leu Thr Phe Met Ile Ala Ala Thr
   1               5                   10
(2) INFORMATION FOR SEQ ID NO:916:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:916:
   Ala Leu Met Gly Pro Leu Gly Leu Leu
   1               5
(2) INFORMATION FOR SEQ ID NO:917:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:917:
   Gly Leu Leu Gly Gln Ile Gly Pro Leu
   1 5
(2) INFORMATION FOR SEQ ID NO:918:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:918:
   Gly Met Leu Gly Gln Lys Gly Glu Ile
   1 5
(2) INFORMATION FOR SEQ ID NO:919:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:919:
   Pro Leu Gly Gln Asp Gly Leu Pro Val
   1 5
(2) INFORMATION FOR SEQ ID NO:920:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:920:
   Thr Leu Ala Leu Met Gly Pro Leu Gly Leu
   1 5 10
(2) INFORMATION FOR SEQ ID NO:921:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:921:
   Met Leu Gly Gln Lys Gly Glu Ile Gly Leu
   1 5 10
(2) INFORMATION FOR SEQ ID NO:922:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:922:
   Pro Leu Gly Lys Asp Gly Pro Pro Gly Val
   1 5 10
(2) INFORMATION FOR SEQ ID NO:923:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:923:
   Pro Leu Gly Leu Pro Gly Ala Ser Gly Leu
   1 5 10
(2) INFORMATION FOR SEQ ID NO:924:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:924:
   Gly Leu Ala Gly Gln Arg Gly Ile Val
   1 5
(2) INFORMATION FOR SEQ ID NO:925:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:925:
   Val Met Gln Gly Pro Met Gly Pro Met
   1 5
(2) INFORMATION FOR SEQ ID NO:926:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:926:
   Ser Leu Glu Glu Lys Ser Arg Leu Val
   1 5
(2) INFORMATION FOR SEQ ID NO:927:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:927:
   Phe Leu Leu Glu Val Val Asp Ile Leu
   1 5
(2) INFORMATION FOR SEQ ID NO:928:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:928:
   Leu Leu Glu Val Val Asp Ile Leu Leu
   1 5
(2) INFORMATION FOR SEQ ID NO:929:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:929:
   Gly Met Glu Gly Phe Asn Leu Glu Leu
   1 5
(2) INFORMATION FOR SEQ ID NO:930:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:930:
   Glu Leu Ser Asp His Pro Glu Ser Leu
   1 5
(2) INFORMATION FOR SEQ ID NO:931:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:931:
   Ile Leu Val Asp Cys Arg Asp Thr Leu
   1 5
(2) INFORMATION FOR SEQ ID NO:932:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:932:
   Gln Leu Ser Thr Gly Leu Asp Ile Ile
   1 5
(2) INFORMATION FOR SEQ ID NO:933:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:933:
   Thr Leu Lys Lys Met Arg Glu Ile Val
   1 5
(2) INFORMATION FOR SEQ ID NO:934:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:934:
   Gly Met Ala Ala Val Pro Lys Leu Val
   1 5
(2) INFORMATION FOR SEQ ID NO:935:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:935:
   Ala Leu Gly Phe Gly Thr Asp Asn Val
   1 5
(2) INFORMATION FOR SEQ ID NO:936:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:936:
   Val Leu Leu Gln Cys Ser Ala Ile Leu
   1 5
(2) INFORMATION FOR SEQ ID NO:937:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:937:
   Leu Leu Gln Cys Ser Ala Ile Leu Val
   1 5
(2) INFORMATION FOR SEQ ID NO:938:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:938:
   Ile Leu Val Lys Glu Lys Gly Ile Leu
   1 5
(2) INFORMATION FOR SEQ ID NO:939:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:939:
   Leu Met Trp Lys Ala Lys Gly Thr Val
   1 5
(2) INFORMATION FOR SEQ ID NO:940:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:940:
   Lys Leu His Lys Val Ala Pro Lys Ile
   1 5
(2) INFORMATION FOR SEQ ID NO:941:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:941:
   Met Met Glu Ser Gly Thr Thr Met Val
   1               5
(2) INFORMATION FOR SEQ ID NO:942:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:942:
   Phe Leu Ile Glu Glu Ile Glu Arg Leu
   1               5
(2) INFORMATION FOR SEQ ID NO:943:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:943:
   Lys Leu Gly Leu Lys Ile Cys Gly Phe Leu
   1               5                   10
(2) INFORMATION FOR SEQ ID NO:944:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:944:
   Phe Leu Leu Glu Val Val Asp Ile Leu Leu
   1 5 10
(2) INFORMATION FOR SEQ ID NO:945:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:945:
   Val Leu Asp Phe His His Pro His Gln Leu
   1 5 10
(2) INFORMATION FOR SEQ ID NO:946:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:946:
   Leu Leu Glu Gly Met Glu Gly Phe Asn Leu
   1 5 10
(2) INFORMATION FOR SEQ ID NO:947:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:947:
   Asn Met Phe Thr Tyr Glu Ile Ala Pro Val
   1 5 10
(2) INFORMATION FOR SEQ ID NO:948:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:948:
   Gly Met Ala Ala Val Pro Lys Leu Val Leu
   1 5 10
(2) INFORMATION FOR SEQ ID NO:949:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:949:
   Ala Leu Gly Phe Gly Thr Asp Asn Val Ile
   1 5 10
(2) INFORMATION FOR SEQ ID NO:950:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:950:
   Ile Leu Glu Ala Lys Gln Lys Gly Tyr Val
   1 5 10

(2) INFORMATION FOR SEQ ID NO:951:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:951:
   Leu Met Ser Arg Lys His Arg His Lys Leu
   1               5                   10
(2) INFORMATION FOR SEQ ID NO:952:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:952:
   Val Leu Leu Gln Cys Ser Ala Ile Leu Val
   1               5                   10
(2) INFORMATION FOR SEQ ID NO:953:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:953:
   Leu Leu Gln Pro Asp Lys Gln Tyr Asp Val
   1               5                   10
(2) INFORMATION FOR SEQ ID NO:954:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:954:
   Trp Leu Met Trp Lys Ala Lys Gly Thr Val
   1 5 10
(2) INFORMATION FOR SEQ ID NO:955:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:955:
   Glu Leu Ala Glu Tyr Leu Tyr Ala Lys Ile
   1 5 10
(2) INFORMATION FOR SEQ ID NO:956:
   (i) SEQUENCE CHARACTERISTICS:
      (A) LENGTH: 10 amino acids
      (B) TYPE: amino acid
      (C) STRANDEDNESS: single.
      (D) TOPOLOGY: unknown
   (ii) MOLECULE TYPE: peptide
   (xi) SEQUENCE DESCRIPTION: SEQ ID NO:956:
   Leu Met Met Glu Ser Gly Thr Thr Met Val
   1 5 10
(2) INFORMATION FOR SEQ ID NO:957:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:957:
   Gly Ala Ile Ser Asn Met Tyr Ser Ile
   1 5
(2) INFORMATION FOR SEQ ID NO:958:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:958:
   Asn Leu Trp Leu His Val Asp Ala Ala
   1               5
(2) INFORMATION FOR SEQ ID NO:959:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:959:
   Arg Met Val Ile Ser Asn Pro Ala Ala
   1               5
(2) INFORMATION FOR SEQ ID NO:960:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:960:
   Ala Ala Leu Gly Phe Gly Thr Asp Asn Val
   1               5                   10
(2) INFORMATION FOR SEQ ID NO:961:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:961:
   Met Met Gly Val Leu Leu Gln Cys Ser Ala
   1               5                   10
(2) INFORMATION FOR SEQ ID NO:962:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:962:
   Ile Leu Gln Gly Cys Asn Gln Met Cys Ala
   1               5                   10
(2) INFORMATION FOR SEQ ID NO:963:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:963:
   Ala Leu Phe Leu Gly Phe Leu Gly Ala Ala
   1               5                   10
(2) INFORMATION FOR SEQ ID NO:964:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:964:
   Met Leu Gln Leu Thr Val Trp Gly Ile
   1 5
(2) INFORMATION FOR SEQ ID NO:965:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:965:
   Arg Val Ile Glu Val Leu Gln Arg Ala
   1 5
(2) INFORMATION FOR SEQ ID NO:966:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:966:
   Lys Leu Thr Pro Leu Cys Val Thr Leu
   1 5
(2) INFORMATION FOR SEQ ID NO:967:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:967:
   Leu Leu Ile Ala Ala Arg Ile Val Glu Leu
   1 5 10
(2) INFORMATION FOR SEQ ID NO:968:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:968:
   Ser Leu Leu Asn Ala Thr Asp Ile Ala Val
   1               5                   10
(2) INFORMATION FOR SEQ ID NO:969:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:969:
   Ala Leu Phe Leu Gly Phe Leu Gly Ala
   1               5
(2) INFORMATION FOR SEQ ID NO:970:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:970:
   His Met Leu Gln Leu Thr Val Trp Gly Ile
   1               5                   10
(2) INFORMATION FOR SEQ ID NO:971:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:971:
   Leu Leu Asn Ala Thr Asp Ile Ala Val
   1 5
(2) INFORMATION FOR SEQ ID NO:972:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:972:
   Ala Leu Leu Tyr Lys Leu Asp Ile Val
   1 5
(2) INFORMATION FOR SEQ ID NO:973:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:973:
   Trp Leu Trp Tyr Ile Lys Ile Phe Ile
   1 5
(2) INFORMATION FOR SEQ ID NO:974:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:974:
   Thr Ile Ile Val His Leu Asn Glu Ser Val
   1 5 10
(2) INFORMATION FOR SEQ ID NO:975:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:975:
   Leu Leu Gln Tyr Trp Ser Gln Glu Leu
   1 5
(2) INFORMATION FOR SEQ ID NO:976:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:976:
   Ile Met Ile Val Gly Gly Leu Val Gly Leu
   1 5 10
(2) INFORMATION FOR SEQ ID NO:977:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:977:
   Leu Leu Tyr Lys Leu Asp Ile Val Ser Ile
   1 5 10
(2) INFORMATION FOR SEQ ID NO:978:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:978:
   Phe Leu Ala Ile Ile Trp Val Asp Leu
   1               5
(2) INFORMATION FOR SEQ ID NO:979:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:979:
   Thr Leu Gln Cys Lys Ile Lys Gln Ile Ile
   1               5                   10
(2) INFORMATION FOR SEQ ID NO:980:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:980:
   Gly Leu Val Gly Leu Arg Ile Val Phe Ala
   1               5                   10
(2) INFORMATION FOR SEQ ID NO:981:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:981:
   Phe Leu Gly Ala Ala Gly Ser Thr Met
   1 5
(2) INFORMATION FOR SEQ ID NO:982:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:982:
   Ile Ile Ser Leu Trp Asp Gln Ser Leu
   1 5
(2) INFORMATION FOR SEQ ID NO:983:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:983:
   Thr Val Trp Gly Ile Lys Gln Leu Gln Ala
   1 5 10
(2) INFORMATION FOR SEQ ID NO:984:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:984:
   Leu Leu Gly Arg Arg Gly Trp Glu Val
   1 5
(2) INFORMATION FOR SEQ ID NO:985:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:985:
   Ala Val Leu Ser Ile Val Asn Arg Val
   1 5
(2) INFORMATION FOR SEQ ID NO:986:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:986:
   Phe Ile Met Ile Val Gly Gly Leu Val
   1 5
(2) INFORMATION FOR SEQ ID NO:987:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:987:
   Leu Leu Asn Ala Thr Asp Ile Ala Val Ala
   1 5 10
(2) INFORMATION FOR SEQ ID NO:988:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:988:
   Phe Leu Tyr Gly Ala Leu Leu Leu Ala
   1               5
(2) INFORMATION FOR SEQ ID NO:989:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:989:
   Ser Leu Leu Thr Phe Met Ile Ala Ala
   1               5
(2) INFORMATION FOR SEQ ID NO:990:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:990:
   Phe Met Ile Ala Ala Thr Tyr Asn Phe Ala Val
   1               5                   10
(2) INFORMATION FOR SEQ ID NO:991:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:991:
   Arg Met Tyr Gly Val Leu Pro Trp Ile
   1               5
(2) INFORMATION FOR SEQ ID NO:992:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:992:
   Ile Ala Ala Thr Tyr Asn Phe Ala Val
   1               5
(2) INFORMATION FOR SEQ ID NO:993:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:993:
   Gly Leu Leu Glu Cys Cys Ala Arg Cys Leu Val
   1               5                   10
(2) INFORMATION FOR SEQ ID NO:994:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:994:
   Tyr Ala Leu Thr Val Val Trp Leu Leu
   1               5
(2) INFORMATION FOR SEQ ID NO:995:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:995:
   Ala Leu Thr Val Val Trp Leu Leu Val
   1               5
(2) INFORMATION FOR SEQ ID NO:996:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:996:
   Phe Leu Tyr Gly Ala Leu Leu Leu
   1               5
(2) INFORMATION FOR SEQ ID NO:997:
   (i) SEQUENCE CHARACTERISTICS:
      (A) LENGTH: 11 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:997:
   Ser Leu Cys Ala Asp Ala Arg Met Tyr Gly Val
   1               5                   10
(2) INFORMATION FOR SEQ ID NO:998:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:998:
   Leu Leu Val Phe Ala Cys Ser Ala Val
   1 5
(2) INFORMATION FOR SEQ ID NO:999:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:999:
   Lys Met Val Glu Leu Val His Phe Leu Leu
   1 5 10
(2) INFORMATION FOR SEQ ID NO:1000:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1000:
   Lys Val Ala Glu Leu Val His Phe Leu
   1 5
(2) INFORMATION FOR SEQ ID NO:1001:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1001:
   Arg Ala Leu Ala Glu Thr Ser Tyr Val
   1 5
(2) INFORMATION FOR SEQ ID NO:1002:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1002:
   Leu Val Phe Gly Ile Glu Leu Met Glu Val
   1 5 10
(2) INFORMATION FOR SEQ ID NO:1003:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1003:
   Phe Leu Trp Gly Pro Arg Ala Leu Ala
   1 5

(2) INFORMATION FOR SEQ ID NO:1004:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1004:
   Ala Leu Ala Glu Thr Ser Tyr Val Lys Val
   1 5 10
(2) INFORMATION FOR SEQ ID NO:1005:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1005:
   Leu Val Leu Gly Thr Leu Glu Glu Val
   1 5
(2) INFORMATION FOR SEQ ID NO:1006:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1006:
   Leu Leu Trp Lys Gly Glu Gly Ala Val Val
   1 5 10
(2) INFORMATION FOR SEQ ID NO:1007:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1007:
   Ile Ile Gly Ala Glu Thr Phe Tyr Val
   1 5
(2) INFORMATION FOR SEQ ID NO:1008:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1008:
   Leu Met Val Thr Val Tyr Tyr Gly Val
   1 5
(2) INFORMATION FOR SEQ ID NO:1009:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1009:
   Leu Leu Phe Asn Ile Leu Gly Gly Trp Val
   1               5                   10
(2) INFORMATION FOR SEQ ID NO:1010:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1010:
   Leu Leu Ala Leu Leu Ser Cys Leu Thr Val
   1               5                   10
(2) INFORMATION FOR SEQ ID NO:1011:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1011:
   Tyr Leu Val Ala Tyr Gln Ala Thr Val
   1               5
(2) INFORMATION FOR SEQ ID NO:1012:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1012:
   Phe Leu Leu Leu Ala Asp Ala Arg Val
   1 5
(2) INFORMATION FOR SEQ ID NO:1013:
   (i) SEQUENCE CHARACTERISTICS:
      (A) LENGTH: 9 amino acids
      (B)TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1013:
   Ile Leu Ala Gly Tyr Gly Ala Gly Val
   1 5
(2) INFORMATION FOR SEQ ID NO:1014:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1014:
   Tyr Leu Leu Pro Arg Arg Gly Pro Arg Leu
   1 5 10
(2) INFORMATION FOR SEQ ID NO:1015:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1015:
   Gly Leu Leu Gly Cys Ile Ile Thr Ser Leu
   1 5 10
(2) INFORMATION FOR SEQ ID NO:1016:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1016:
   Asp Leu Met Gly Tyr Ile Pro Leu Val
   1 5
(2) INFORMATION FOR SEQ ID NO:1017:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1017:
   Leu Leu Ala Leu Leu Ser Cys Leu Thr Ile
   1 5 10
(2) INFORMATION FOR SEQ ID NO:1018:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1018:
   Val Leu Ala Ala Leu Ala Ala Tyr Cys Leu
   1 5 10
(2) INFORMATION FOR SEQ ID NO:1019:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1019:
   Leu Leu Val Pro Phe Val Gln Trp Phe Val
   1 5 10
(2) INFORMATION FOR SEQ ID NO:1020:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1020:
   Phe Leu Leu Ala Gln Phe Thr Ser Ala
   1 5
(2) INFORMATION FOR SEQ ID NO:1021:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1021:
   Phe Leu Leu Ser Leu Gly Ile His Leu
   1 5
(2) INFORMATION FOR SEQ ID NO:1022:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1022:
   Ala Leu Met Pro Leu Tyr Ala Cys Ile
   1               5
(2) INFORMATION FOR SEQ ID NO:1023:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1023:
   Ile Leu Leu Leu Cys Leu Ile Phe Leu Leu
   1               5                   10
(2) INFORMATION FOR SEQ ID NO:1024:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1024:
   Leu Leu Pro Ile Phe Phe Cys Leu Trp Val
   1               5                   10
(2) INFORMATION FOR SEQ ID NO:1025:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1025:
   Tyr Leu His Thr Leu Trp Lys Ala Gly Ile
   1               5                   10
(2) INFORMATION FOR SEQ ID NO:1026:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1026:
   Tyr Leu His Thr Leu Trp Lys Ala Gly Val
   1 5 10
(2) INFORMATION FOR SEQ ID NO:1027:
   (i) SEQUENCE CHARACTERISTICS:
      (A) LENGTH: 8 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1027:
   Thr Leu Gly Ile Val Cys Pro Ile
   1 5
(2) INFORMATION FOR SEQ ID NO:1028:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1028:
   Thr Leu Gly Ile Val Cys Pro Ile Cys
   1 5
(2) INFORMATION FOR SEQ ID NO:1029:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1029:
   Gly Thr Leu Gly Ile Val Cys Pro Ile
   1 5
(2) INFORMATION FOR SEQ ID NO:1030:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1030:
   Tyr Met Leu Asp Leu Gln Pro Glu Thr Thr
   1 5 10
(2) INFORMATION FOR SEQ ID NO:1031:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1031:
   Tyr Met Leu Asp Leu Gln Pro Glu Thr
   1 5
(2) INFORMATION FOR SEQ ID NO:1032:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1032:
   Met Leu Asp Leu Gln Pro Glu Thr Thr
   1 5
(2) INFORMATION FOR SEQ ID NO:1033:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1033:
   Phe Ala Phe Arg Asp Leu Cys Ile Val
   1 5
(2) INFORMATION FOR SEQ ID NO:1034:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1034:
   Leu Leu Met Gly Thr Leu Gly Ile Val
   1 5
(2) INFORMATION FOR SEQ ID NO:1035:
   (i) SEQUENCE CHARACTERISTICS:
      (A) LENGTH: 10 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1035:
   Thr Ile His Asp Ile Ile Leu Glu Cys Val
   1 5 10
(2) INFORMATION FOR SEQ ID NO:1036:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1036:
   Thr Leu His Glu Tyr Met Leu Asp Leu
   1 5
(2) INFORMATION FOR SEQ ID NO:1037:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1037:
   Lys Leu Pro Gln Leu Cys Thr Glu Leu
   1 5
(2) INFORMATION FOR SEQ ID NO:1038:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1038:
   Ala Met Phe Gln Asp Pro Gln Glu Arg
   1 5
(2) INFORMATION FOR SEQ ID NO:1039:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1039:
   Leu Gln Thr Thr Ile His Asp Ile Ile
   1               5
(2) INFORMATION FOR SEQ ID NO:1040:
   (i) SEQUENCE CHARACTERISTICS:
      (A) LENGTH: 9 amino acids
      (B) TYPE: amino acid
      (C) STRANDEDNESS: single
      (D) TOPOLOGY: unknown
   (ii) MOLECULE TYPE: peptide
   (xi) SEQUENCE DESCRIPTION: SEQ ID NO:1040:
   His Val Asp Ile Arg Thr Leu Glu Asp
   1               5
(2) INFORMATION FOR SEQ ID NO: 1041:
   (i) SEQUENCE CHARACTERISTICS:
      (A) LENGTH: 14 amino acids
      (B) TYPE: amino acid
      (D) TOPOLOGY: linear
   (ii) MOLECULE TYPE: peptide
   (xi) SEQUENCE DESCRIPTION: SEQ ID NO: 1041:
   Gln Tyr Ile Lys Ala Asn Ser Lys Phe Ile Gly Ile Thr Glu
   1               5                   10
(2) INFORMATION FOR SEQ ID NO: 1042:
   (i) SEQUENCE CHARACTERISTICS:
      (A) LENGTH: 21 amino acids
      (B) TYPE: amino acid
      (D) TOPOLOGY: linear
   (ii) MOLECULE TYPE: peptide
   (xi) SEQUENCE DESCRIPTION: SEQ ID NO: 1042:
   Asp Ile Glu Lys Lys Ile Ala Lys Met Glu Lys Ala Ser Ser Val Phe
   1 5 10 15
   Asn Val Val Asn Ser
   20
(2) INFORMATION FOR SEQ ID NO: 1043:
   (i) SEQUENCE CHARACTERISTICS:
      (A) LENGTH: 17 amino acids
      (B) TYPE: amino acid
      (D) TOPOLOGY: linear
   (ii) MOLECULE TYPE: peptide
   (xi) SEQUENCE DESCRIPTION: SEQ ID NO: 1043:
   Tyr Gly Ala Val Asp Ser Ile Leu Gly Gly Val Ala Thr Tyr Gly Ala
   1 5 10 15
   Ala